(19) Europäisches Patentamt
European Patent Office
Office européen des brevets

(11) EP 4 737 442 A1

(12) EUROPEAN PATENT APPLICATION
published in accordance with Art. 153(4) EPC

(43) Date of publication:
06.05.2026 Bulletin 2026/19

(21) Application number: 24831928.7

(22) Date of filing: 25.06.2024

(51) International Patent Classification (IPC):
*C07D 213/38* *(2006.01)*

(52) Cooperative Patent Classification (CPC):
A01N 35/06; A01N 43/10; A01N 43/16; A01N 43/40; A01N 43/46; A01N 43/54; A01N 43/56; A01N 43/58; A01N 43/78; A01N 43/80; A01N 47/02; A01N 47/06; A01N 47/16; A01P 5/00; A01P 7/04; (Cont.)

(86) International application number:
PCT/JP2024/022931

(87) International publication number:
WO 2025/005068 (02.01.2025 Gazette 2025/01)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 26.06.2023 JP 2023104156

(71) Applicants:
- ADEKA CORPORATION
Arakawa-ku
Tokyo
116-8554 (JP)
- Nihon Nohyaku Co., Ltd.
Tokyo 104-8386 (JP)

(72) Inventors:
- HARA, Kenji
Tokyo 116-8554 (JP)
- SUGIHARA, Kousuke
Tokyo 116-8554 (JP)
- MASUDA, Tsuyoshi
Tokyo 116-8554 (JP)
- FUJII, Kazuki
Kawachinagano-shi, Osaka 586-0094 (JP)
- FUKATSU, Kosuke
Kawachinagano-shi, Osaka 586-0094 (JP)
- FUJITA, Naoya
Kawachinagano-shi, Osaka 586-0094 (JP)
- OIKAWA, Hinoki
Kawachinagano-shi, Osaka 586-0094 (JP)
- SAWAMOTO, Daisuke
Tokyo 116-8554 (JP)
- SUGIYAMA, Ryoji
Tokyo 116-8554 (JP)
- YOKOI, Taiki
Kawachinagano-shi, Osaka 586-0094 (JP)
- YONEMURA, Ikki
Kawachinagano-shi, Osaka 586-0094 (JP)
- YAMASHITA, Yudai
Kawachinagano-shi, Osaka 586-0094 (JP)
- OTSUKA, Yuga
Kawachinagano-shi, Osaka 586-0094 (JP)
- SAKITA, Ryo
Kawachinagano-shi, Osaka 586-0094 (JP)

(74) Representative: J A Kemp LLP
80 Turnmill Street
London EC1M 5QU (GB)

# (54) DIAMINOVINYLIDENE DERIVATIVE OR SALT THEREOF, PEST CONTROL AGENT CONTAINING SAID COMPOUND, AND METHODS FOR USING THESE

(57) The present invention aims to provide a novel ectoparasite control agent for animals and endoparasite control agent for animals.

The present invention provides a compound represented by the formula (1)

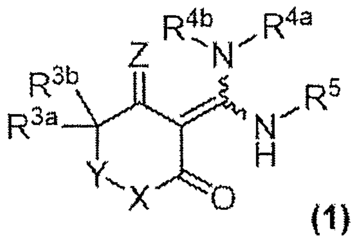

(1)

EP 4 737 442 A1

wherein each symbol is as defined in the present specification, or a salt thereof, and a pest control agent containing the compound as an active ingredient, and a method for using same.

(52) Cooperative Patent Classification (CPC): (Cont.)
**A61K 31/136; A61K 31/341; A61K 31/343; A61K 31/351; A61K 31/381; A61K 31/42; A61K 31/426; A61K 31/4402; A61K 31/4439; A61K 31/444; A61K 31/4965; A61K 31/497; A61K 31/505; A61K 31/506; A61P 33/00; C07C 225/18; C07D 213/38; C07D 213/50; C07D 213/69; C07D 213/74; C07D 223/10; C07D 231/12; C07D 231/38; C07D 237/20; C07D 239/26; C07D 239/42; C07D 239/47; C07D 241/12; C07D 241/20; C07D 261/08; C07D 261/14; C07D 263/32; C07D 277/28; C07D 307/14; C07D 307/52; C07D 307/81; C07D 309/04; C07D 311/74; C07D 333/20; C07D 333/22; C07D 401/04; C07D 401/12; C07D 401/14; C07D 403/04; C07D 405/14; C07D 409/12; C07D 409/14; C07D 413/12; C07D 413/14; C07D 417/12; C07D 417/14**

## Description

[Technical Field]

The present invention relates to a diaminovinylidene derivative or a salt thereof, a pest control agent containing the compound, and a method for use thereof.

[Background Art]

Patent Literatures 1 to 3 describe that certain tetrahydropyridine derivatives, tetrahydropyridazine derivatives, and cyclohexanedione derivatives have control activity against insect pests in the agricultural and horticultural field. In addition, Patent Literatures 4 to 6 report certain dihydropyran derivatives, piperidinone derivatives, and azepanedione derivatives as pharmaceutical compounds or intermediates therefor. However, these Literatures do not describe or suggest a compound having the diaminovinylidene skeleton of the present invention. Thus, the control activity of this compound against insect pests in the agricultural and horticultural field and animal parasites is not known at all.

[Citation List]

[Patent Literature]

[Patent Literature 1]
WO 2021/261562
[Patent Literature 2]
WO 2021/261563
[Patent Literature 3]
US Patent No. 3976785
[Patent Literature 4]
WO 2008/014311
[Patent Literature 5]
WO 2017/102649
[Patent Literature 6]
WO 2014/021281


[Summary of Invention]

[Technical Problem]

In the production of useful crops in agriculture, horticulture and the like, the damage caused by insect pests is enormous, and many effective agrohorticultural insect pest control agents are on the market. However, due to the demand for a drug that is highly effective against insect pests that are resistant to existing drugs, a drug with physical properties suitable for various labor-saving application methods, a drug superior in human and animal safety with reduced acute toxicity and the like, and a drug superior in environmental safety, such as appropriate persistence in soil and the like, the development of a novel agrohorticultural insect pest control agent is still one of the important problems in the field of agriculture and horticulture. In addition, parasite control is an important problem when raising animals such as farm animal, companion animal (pet) and the like. In addition to the parasite infection itself, the viruses, bacteria and the like transmitted by the parasites cause considerable suffering to animals, resulting in huge economic losses for farm animals. Therefore, the development of a novel ectoparasite control agent for animals and endoparasite control agent for animals has been desired.

[Solution to Problem]

The present inventors have conducted intensive studies in an attempt to develop a novel pest control agent, particularly an agrohorticultural insect pest control agent, an ectoparasite control agent for animals, and an endoparasite control agent for animals, and found that a diaminovinylidene compound represented by the formula (1) of the present invention or a salt thereof is useful as a pest control agent, which resulted in the completion of the present invention.
Accordingly, the present invention relates to the following.

[1] A compound represented by the formula (1)

[Chem. 1]

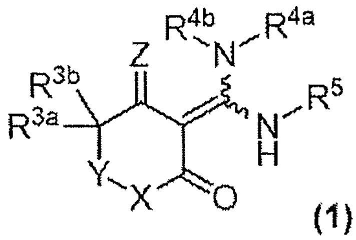

(1)

wherein

X is $CR^{1a}R^{1b}$, $NR^{1c}$, or an oxygen atom,
$R^{1a}$ and $R^{1b}$ are each

(a1) a hydrogen atom;
(a2) a halogen atom;
(a3) a $(C_1-C_6)$alkyl group;
(a4) a halo $(C_1-C_6)$alkyl group;
(a5) a phenyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a $(C_1-C_6)$alkyl group;
(b3) a $(C_2-C_6)$alkenyl group;
(b4) a $(C_2-C_6)$alkynyl group;
(b5) a $(C_3-C_6)$cycloalkyl group;
(b6) a halo $(C_1-C_6)$ alkyl group;
(b7) a halo$(C_2-C_6)$alkenyl group;
(b8) a halo$(C_2-C_6)$alkynyl group;
(b9) a halo$(C_3-C_6)$cycloalkyl group;
(b10) a $(C_1-C_6)$alkyl group having 1 to 3 substituents each independently selected from substituent group A;
(b11) a $(C_1-C_6)$alkylcarbonyl group;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b18) a pyridazinyl group;
(b19) a pyridazinyl group having 1 to 3 substituents each independently selected from substituent group B;
(b20) a pyrimidinyl group;
(b21) a pyrimidinyl group having 1 to 3 substituents each independently selected from substituent group B;
(b22) a pyrazinyl group;
(b23) a pyrazinyl group having 1 to 3 substituents each independently selected from substituent group B;
(b24) a phenyl$(C_1-C_6)$ alkyl group;
(b25) a phenyl$(C_1-C_6)$alkyl group having 1 to 5 substituents each independently selected from substituent group B;
(b26) a pyridyl$(C_1-C_6)$ alkyl group;
(b27) a pyridyl$(C_1-C_6)$alkyl group having 1 to 4 substituents each independently selected from substituent

group B;
(b28) a thiazolyl($C_1$-$C_6$)alkyl group; or
(b29) a thiazolyl($C_1$-$C_6$)alkyl group having 1 to 2 substituents each independently selected from substituent group B,

Y is $CR^{2a}R^{2b}$, $NR^{2c}$, or $CH_2CH_2$,
$R^{2a}$ is

(c1) a hydrogen atom;
(c2) a phenyl group;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c28) a triazinyl group;
(c29) a triazinyl group having 1 to 2 substituents each independently selected from substituent group B;
(c30) a thiadiazolyl group;
(c31) a thiadiazolyl group having one substituent selected from substituent group B;
(c32) a pyrrolyl group;
(c33) a pyrrolyl group having 1 to 4 substituents each independently selected from substituent group B;
(c34) an imidazolyl group;
(c35) an imidazolyl group having 1 to 3 substituents each independently selected from substituent group B;
(c36) a triazolyl group;
(c37) a triazolyl group having 1 to 2 substituents each independently selected from substituent group B;
(c38) a tetrazolyl group;
(c39) a tetrazolyl group having one substituent selected from substituent group B;
(c40) a naphthyl group;
(c41) a naphthyl group having 1 to 7 substituents each independently selected from substituent group B;
(c42) a quinolyl group;
(c43) a quinolyl group having 1 to 6 substituents each independently selected from substituent group B;
(c44) a carboxyl group; or
(c45) a ($C_1$-$C_6$)alkoxycarbonyl group,

$R^{2b}$ is

(d1) a hydrogen atom; or
(d2) a ($C_1$-$C_6$)alkyl group,

$R^{2c}$ is

(e1) a ($C_1$-$C_6$)alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e10) a pyrazinyl group;
(e11) a pyrazinyl group having 1 to 3 substituents each independently selected from substituent group B;
(e12) a furyl group;
(e13) a furyl group having 1 to 3 substituents each independently selected from substituent group B;
(e14) a thienyl group;
(e15) a thienyl group having 1 to 3 substituents each independently selected from substituent group B;
(e16) an oxazolyl group;
(e17) an oxazolyl group having 1 to 2 substituents each independently selected from substituent group B;
(e18) an isoxazolyl group;
(e19) an isoxazolyl group having 1 to 2 substituents each independently selected from substituent group B;
(e20) a thiazolyl group;
(e21) a thiazolyl group having 1 to 2 substituents each independently selected from substituent group B;
(e22) an isothiazolyl group;
(e23) an isothiazolyl group having 1 to 2 substituents each independently selected from substituent group B;
(e24) a triazinyl group;
(e25) a triazinyl group having 1 to 2 substituents each independently selected from substituent group B;
(e26) an oxadiazolyl group;
(e27) an oxadiazolyl group having one substituent selected from substituent group B;
(e28) a thiadiazolyl group;
(e29) a thiadiazolyl group having one substituent selected from substituent group B;
(e30) a pyrrolyl group;
(e31) a pyrrolyl group having 1 to 4 substituents each independently selected from substituent group B;
(e32) a pyrazolyl group;
(e33) a pyrazolyl group having 1 to 3 substituents each independently selected from substituent group B;
(e34) an imidazolyl group;
(e35) an imidazolyl group having 1 to 3 substituents each independently selected from substituent group B;
(e36) a triazolyl group;
(e37) a triazolyl group having 1 to 2 substituents each independently selected from substituent group B;
(e38) a tetrazolyl group;
(e39) a tetrazolyl group having one substituent selected from substituent group B;
(e40) a naphthyl group;
(e41) a naphthyl group having 1 to 7 substituents each independently selected from substituent group B;
(e42) a quinolyl group;
(e43) a quinolyl group having 1 to 6 substituents each independently selected from substituent group B;
(e44) a pyridylcarbonyl group;
(e45) a pyridylcarbonyl group having 1 to 4 substituents each independently selected from substituent group B; or
(e46) a hydrogen atom,

$R^{3a}$ is

(f1) a hydrogen atom;
(f2) a ($C_1$-$C_6$)alkyl group;
(f3) a halo ($C_1$-$C_6$)alkyl group;
(f4) a phenyl group;
(f5) a phenyl group having 1 to 5 substituents each independently selected from substituent group B;
(f6) a pyridyl group;

(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom;
(g2) a $(C_1-C_6)$alkyl group; or
(g3) a halo$(C_1-C_6)$alkyl group,

provided that $R^{1a}$, $R^{1b}$, $R^{2a}$, $R^{2ab}$, $R^{3a}$, and $R^{3b}$ are not hydrogen atoms at the same time,
$R^{4a}$ and $R^5$ are each

(h1) a hydrogen atom;
(h2) a hydroxyl group;
(h3) a cyano group;
(h4) a $(C_1-C_6)$alkyl group;
(h5) a $(C_2-C_6)$alkenyl group;
(h6) a $(C_2-C_6)$alkynyl group;
(h7) a $(C_3-C_6)$cycloalkyl group;
(h8) a $(C_1-C_6)$alkoxy group;
(h9) a halo$(C_1-C_6)$alkyl group;
(h10) a halo$(C_2-C_6)$alkenyl group;
(h11) a halo$(C_2-C_6)$alkynyl group;
(h12) a halo$(C_3-C_6)$cycloalkyl group;
(h13) a halo$(C_1-C_6)$ alkoxy group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C
(h29) a phenyl $(C_1-C_6)$alkyl group;
(h30) a phenyl $(C_1-C_6)$alkyl group having 1 to 5 substituents each independently selected from substituent group C;
(h31) a pyridyl$(C_1-C_6)$alkyl group;
(h32) a pyridyl$(C_1-C_6)$alkyl group having 1 to 4 substituents each independently selected from substituent group C;
(h33) a pyridazinyl $(C_1-C_6)$ alkyl group;
(h34) a pyridazinyl$(C_1-C_6)$alkyl group having 1 to 3 substituents each independently selected from substituent group C;
(h35) a pyrimidinyl$(C_1-C_6)$alkyl group;
(h36) a pyrimidinyl$(C_1-C_6)$alkyl group having 1 to 3 substituents each independently selected from substituent group C;
(h37) a pyrazinyl $(C_1-C_6)$alkyl group;
(h38) a pyrazinyl$(C_1-C_6)$alkyl group having 1 to 3 substituents each independently selected from substituent group C;

(h39) a thienyl($C_1$-$C_6$)alkyl group;
(h40) a thienyl($C_1$-$C_6$)alkyl group having 1 to 3 substituents each independently selected from substituent group C;
(h41) a furyl($C_1$-$C_6$)alkyl group;
(h42) a furyl($C_1$-$C_6$)alkyl group having 1 to 3 substituents each independently selected from substituent group C;
(h43) an oxazolyl($C_1$-$C_6$)alkyl group;
(h44) an oxazolyl($C_1$-$C_6$)alkyl group having 1 to 2 substituents each independently selected from substituent group C;
(h45) a thiazolyl($C_1$-$C_6$)alkyl group;
(h46) a thiazolyl($C_1$-$C_6$)alkyl group having 1 to 2 substituents each independently selected from substituent group C;
(h47) a benzofuranyl($C_1$-$C_6$)alkyl group;
(h48) a benzofuranyl($C_1$-$C_6$)alkyl group having 1 to 5 substituents each independently selected from substituent group C;
(h49) a tetrahydropyranyl($C_1$-$C_6$)alkyl group;
(h50) a tetrahydrofuryl ($C_1$-$C_6$)alkyl group; or
(h51) a dihydrobenzopyranyl group,

$R^{4a}$ and $R^5$ may be the same or different,
$R^{4b}$ is

(i1) a hydrogen atom;
(i2) a ($C_1$-$C_6$)alkyl group; or
(i3) a halo ($C_1$-$C_6$) alkyl group,

when $R^{4a}$ is (h4) a ($C_1$-$C_6$)alkyl group, or (h14) a ($C_1$-$C_6$)alkyl group having 1 to 3 substituents each independently selected from substituent group A, and $R^{4b}$ is (i2) a ($C_1$-$C_6$)alkyl group, then $R^{4a}$ and $R^{4b}$ may form a 3- to 6-membered ring together with the adjacent nitrogen atom, and
Z is an oxygen atom, a sulfur atom, or $NR^7$ wherein $R^7$ is a hydroxyl group, a ($C_1$-$C_6$)alkoxy group, a halo($C_1$-$C_6$) alkoxy group, a ($C_1$-$C_6$)alkyl group, a halo($C_1$-$C_6$)alkyl group, a ($C_3$-$C_6$)cycloalkyl group, an amino group, a ($C_1$-$C_6$)alkylamino group, or a di($C_1$-$C_6$)alkylamino group (the ($C_1$-$C_6$)alkyl groups may be the same or different),
substituent group A consists of

(j1) a cyano group;
(j2) a ($C_3$-$C_6$)cycloalkyl group;
(j3) a ($C_1$-$C_6$)alkoxy group;
(j4) a ($C_1$-$C_6$)alkylsulfanyl group;
(j5) a ($C_1$-$C_6$)alkylsulfinyl group;
(j6) a ($C_1$-$C_6$)alkylsulfonyl group;
(j7) a ($C_1$-$C_6$)alkylamino group;
(j8) a di($C_1$-$C_6$) alkylamino group (the ($C_1$-$C_6$)alkyl may be the same or different);
(j9) a halo ($C_3$-$C_6$)cycloalkyl group;
(j10) a halo ($C_1$-$C_6$)alkoxy group;
(j11) a halo($C_1$-$C_6$)alkylsulfanyl group;
(j12) a halo($C_1$-$C_6$)alkylsulfinyl group; and
(j13) a halo($C_1$-$C_6$)alkylsulfonyl group,

substituent group B consists of

(k1) a halogen atom;
(k2) a cyano group;
(k3) a nitro group;
(k4) an amino group;
(k5) a hydroxyl group;
(k6) a carboxyl group;
(k7) a ($C_1$-$C_6$)alkyl group;
(k8) a ($C_2$-$C_6$)alkenyl group;

(k9) a $(C_2-C_6)$alkynyl group;
(k10) a $(C_1-C_6)$alkoxy group;
(k11) a $(C_3-C_6)$cycloalkyl group;
(k12) a $(C_1-C_6)$alkylsulfanyl group;
(k13) a $(C_1-C_6)$alkylsulfinyl group;
(k14) a $(C_1-C_6)$alkylsulfonyl group;
(k15) a $(C_1-C_6)$alkylamino group;
(k16) a di$(C_1-C_6)$alkylamino group (the $(C_1-C_6)$alkyl may be the same or different);
(k17) a halo$(C_1-C_6)$ alkyl group;
(k18) a halo$(C_2-C_6)$alkenyl group;
(k19) a halo$(C_2-C_6)$alkynyl group;
(k20) a halo $(C_1-C_6)$ alkoxy group;
(k21) a halo $(C_3-C_6)$cycloalkyl group;
(k22) a halo$(C_1-C_6)$alkylsulfanyl group;
(k23) a halo$(C_1-C_6)$alkylsulfinyl group;
(k24) a halo$(C_1-C_6)$alkylsulfonyl group;
(k25) a $(C_1-C_6)$alkoxy$(C_1-C_6)$alkyl group;
(k26) a phenyl group;
(k27) a phenyl group having 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a halo$(C_1-C_6)$alkyl group, and a halo $(C_1-C_6)$alkoxy group;
(k28) a pyridyl group; and
(k29) a pyridyl group having 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a halo$(C_1-C_6)$alkyl group, and a halo $(C_1-C_6)$alkoxy group,

substituent group C consists of

(l1) a halogen atom;
(l2) a cyano group;
(l3) a nitro group;
(l4) an amino group;
(l5) a hydroxyl group;
(l6) a carboxyl group;
(l7) a $(C_1-C_6)$alkyl group;
(l8) a $(C_2-C_6)$alkenyl group;
(l9) a $(C_2-C_6)$alkynyl group;
(l10) a $(C_1-C_6)$alkoxy group;
(l11) a $(C_3-C_6)$cycloalkyl group;
(l12) a $(C_1-C_6)$alkylsulfanyl group;
(l13) a $(C_1-C_6)$alkylsulfinyl group;
(l14) a $(C_1-C_6)$alkylsulfonyl group;
(l15) a $(C_1-C_6)$alkylamino group;
(l16) a di$(C_1-C_6)$alkylamino group (the $(C_1-C_6)$alkyl may be the same or different);
(l17) a halo $(C_1-C_6)$ alkyl group;
(l18) a halo$(C_2-C_6)$alkenyl group;
(l19) a halo$(C_2-C_6)$alkynyl group;
(l20) a halo $(C_1-C_6)$ alkoxy group;
(l21) a halo $(C_3-C_6)$cycloalkyl group;
(l22) a halo$(C_1-C_6)$alkylsulfanyl group;
(l23) a halo$(C_1-C_6)$alkylsulfinyl group;
(l24) a halo$(C_1-C_6)$alkylsulfonyl group; and
(l25) a $(C_1-C_6)$alkoxy$(C_1-C_6)$alkyl group

(hereinafter at times referred to as compound (1)), or a salt thereof.

[2] The compound of the above-mentioned [1], wherein

X is $CR^{1a}R^{1b}$, $NR^{1c}$, or an oxygen atom,
$R^{1a}$ and $R^{1b}$ are each

(a1) a hydrogen atom;
(a2) a halogen atom;
(a3) a $(C_1-C_6)$alkyl group;
(a4) a halo$(C_1-C_6)$alkyl group;
(a5) a phenyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a $(C_1-C_6)$alkyl group;
(b5) a $(C_3-C_6)$cycloalkyl group;
(b6) a halo$(C_1-C_6)$alkyl group;
(b9) a halo $(C_3-C_6)$cycloalkyl group;
(b10) a $(C_1-C_6)$alkyl group having 1 to 3 substituents each independently selected from substituent group A;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b18) a pyridazinyl group;
(b19) a pyridazinyl group having 1 to 3 substituents each independently selected from substituent group B;
(b20) a pyrimidinyl group;
(b21) a pyrimidinyl group having 1 to 3 substituents each independently selected from substituent group B;
(b22) a pyrazinyl group;
(b23) a pyrazinyl group having 1 to 3 substituents each independently selected from substituent group B;
(b24) a phenyl $(C_1-C_6)$ alkyl group;
(b25) a phenyl $(C_1-C_6)$alkyl group having 1 to 5 substituents each independently selected from substituent group B;
(b26) a pyridyl$(C_1-C_6)$alkyl group;
(b27) a pyridyl$(C_1-C_6)$alkyl group having 1 to 4 substituents each independently selected from substituent group B;
(b28) a thiazolyl$(C_1-C_6)$alkyl group; or
(b29) a thiazolyl$(C_1-C_6)$alkyl group having 1 to 2 substituents each independently selected from substituent group B,

Y is $CR^{2a}R^{2b}$, $NR^{2c}$ or $CH_2CH_2$,
$R^{2a}$ is

(c1) a hydrogen atom;
(c2) a phenyl group;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;

(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c44) a carboxyl group; or
(c45) a $(C_1-C_6)$alkoxycarbonyl group,

$R^{2b}$ is

(d1) a hydrogen atom; or
(d2) a $(C_1-C_6)$alkyl group,

$R^{2c}$ is

(e1) a $(C_1-C_6)$alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e10) a pyrazinyl group;
(e11) a pyrazinyl group having 1 to 3 substituents each independently selected from substituent group B;
(e12) a furyl group;
(e13) a furyl group having 1 to 3 substituents each independently selected from substituent group B;
(e14) a thienyl group;
(e15) a thienyl group having 1 to 3 substituents each independently selected from substituent group B;
(e16) an oxazolyl group;
(e17) an oxazolyl group having 1 to 2 substituents each independently selected from substituent group B;
(e20) a thiazolyl group;
(e21) a thiazolyl group having 1 to 2 substituents each independently selected from substituent group B;
(e44) a pyridylcarbonyl group;
(e45) a pyridylcarbonyl group having 1 to 4 substituents each independently selected from substituent group B; or
(e46) a hydrogen atom,

$R^{3a}$ is

(f1) a hydrogen atom;
(f2) a $(C_1-C_6)$alkyl group;
(f4) a phenyl group;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;

(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom;
(g2) a $(C_1-C_6)$alkyl group; or
(g3) a halo $(C_1-C_6)$ alkyl group,

provided that $R^{1a}$, $R^{1b}$, $R^{2a}$, $R^{2ab}$, $R^{3a}$, and $R^{3b}$ are not hydrogen atoms at the same time,
$R^{4a}$ and $R^5$ are each

(h1) a hydrogen atom;
(h2) a hydroxyl group;
(h3) a cyano group;
(h4) a $(C_1-C_6)$alkyl group;
(h5) a $(C_2-C_6)$alkenyl group;
(h6) a $(C_2-C_6)$alkynyl group;
(h7) a $(C_3-C_6)$cycloalkyl group;
(h8) a $(C_1-C_6)$alkoxy group;
(h9) a halo $(C_1-C_6)$ alkyl group;
(h12) a halo$(C_3-C_6)$cycloalkyl group;
(h13) a halo $(C_1-C_6)$alkoxy group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C;
(h29) a phenyl $(C_1-C_6)$ alkyl group;
(h30) a phenyl$(C_1-C_6)$alkyl group having 1 to 5 substituents each independently selected from substituent group C;
(h31) a pyridyl$(C_1-C_6)$alkyl group;
(h32) a pyridyl$(C_1-C_6)$alkyl group having 1 to 4 substituents each independently selected from substituent group C;
(h37) a pyrazinyl$(C_1-C_6)$alkyl group;
(h38) a pyrazinyl$(C_1-C_6)$alkyl group having 1 to 3 substituents each independently selected from substituent group C;
(h39) a thienyl$(C_1-C_6)$alkyl group;
(h40) a thienyl$(C_1-C_6)$alkyl group having 1 to 3 substituents each independently selected from substituent group C;
(h41) a furyl$(C_1-C_6)$alkyl group;
(h42) a furyl$(C_1-C_6)$alkyl group having 1 to 3 substituents each independently selected from substituent group C;
(h43) an oxazolyl$(C_1-C_6)$alkyl group;
(h44) an oxazolyl$(C_1-C_6)$alkyl group having 1 to 2 substituents each independently selected from substituent group C;
(h45) a thiazolyl$(C_1-C_6)$alkyl group;

(h46) a thiazolyl($C_1$-$C_6$)alkyl group having 1 to 2 substituents each independently selected from substituent group C;
(h47) a benzofuranyl($C_1$-$C_6$)alkyl group;
(h48) a benzofuranyl($C_1$-$C_6$)alkyl group having 1 to 5 substituents each independently selected from substituent group C;
(h49) a tetrahydropyranyl($C_1$-$C_6$)alkyl group;
(h50) a tetrahydrofuryl($C_1$-$C_6$)alkyl group; or
(h51) a dihydrobenzopyranyl group,

$R^{4a}$ and $R^5$ may be the same or different,
$R^{4b}$ is

(i1) a hydrogen atom;
(i2) a ($C_1$-$C_6$)alkyl group; or
(i3) a halo ($C_1$-$C_6$) alkyl group,

when $R^{4a}$ is (h4) a ($C_1$-$C_6$)alkyl group, or (h14) a ($C_1$-$C_6$)alkyl group having 1 to 3 substituents each independently selected from substituent group A, and $R^{4b}$ is (i2) a ($C_1$-$C_6$)alkyl group, then $R^{4a}$ and $R^{4b}$ may form a 3- to 6-membered ring together with the adjacent nitrogen atom, and
Z is an oxygen atom, a sulfur atom, or $NR^7$ wherein $R^7$ is a hydroxyl group or a ($C_1$-$C_6$)alkoxy group,
or a salt thereof.

[2'] The compound of the above-mentioned [2], wherein

the substituent group A consists of

(j1) a cyano group;
(j2) a ($C_3$-$C_6$)cycloalkyl group;
(j3) a ($C_1$-$C_6$)alkoxy group;
(j4) a ($C_1$-$C_6$)alkylsulfanyl group;
(j5) a ($C_1$-$C_6$)alkylsulfinyl group;
(j6) a ($C_1$-$C_6$)alkylsulfonyl group;
(j7) a ($C_1$-$C_6$)alkylamino group;
(j8) a di($C_1$-$C_6$)alkylamino group (the ($C_1$-$C_6$)alkyl may be the same or different); and
(j10) a halo($C_1$-$C_6$)alkoxy group,

the substituent group B consists of

(k1) a halogen atom;
(k2) a cyano group;
(k7) a ($C_1$-$C_6$)alkyl group;
(k10) a ($C_1$-$C_6$)alkoxy group;
(k11) a ($C_3$-$C_6$)cycloalkyl group;
(k12) a ($C_1$-$C_6$)alkylsulfanyl group;
(k13) a ($C_1$-$C_6$)alkylsulfinyl group;
(k14) a ($C_1$-$C_6$)alkylsulfonyl group;
(k17) a halo ($C_1$-$C_6$)alkyl group;
(k20) a halo($C_1$-$C_6$)alkoxy group;
(k22) a halo($C_1$-$C_6$)alkylsulfanyl group;
(k23) a halo($C_1$-$C_6$)alkylsulfinyl group;
(k24) a halo($C_1$-$C_6$)alkylsulfonyl group;
(k26) a phenyl group;
(k27) a phenyl group having 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a halo($C_1$-$C_6$)alkyl group, and a halo($C_1$-$C_6$)alkoxy group;
(k28) a pyridyl group; and
(k29) a pyridyl group having 1 to 4 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a

halo($C_1$-$C_6$)alkyl group, and a halo ($C_1$-$C_6$) alkoxy group, and

the substituent group C consists of

(11) a halogen atom;
(12) a cyano group;
(17) a ($C_1$-$C_6$)alkyl group;
(110) a ($C_1$-$C_6$)alkoxy group;
(111) a ($C_3$-$C_6$)cycloalkyl group;
(112) a ($C_1$-$C_6$)alkylsulfanyl group;
(113) a ($C_1$-$C_6$)alkylsulfinyl group;
(114) a ($C_1$-$C_6$)alkylsulfonyl group;
(117) a halo($C_1$-$C_6$)alkyl group;
(120) a halo($C_1$-$C_6$)alkoxy group;
(122) a halo($C_1$-$C_6$)alkylsulfanyl group;
(123) a halo ($C_1$-$C_6$)alkylsulfinyl group; and
(124) a halo ($C_1$-$C_6$)alkylsulfonyl group,

or a salt thereof.

[3] The compound of the above-mentioned [1], wherein

X is $CR^{1a}R^{1b}$, $NR^{1c}$, or an oxygen atom,
$R^{1a}$ and $R^{1b}$ are each

(a1) a hydrogen atom;
(a3) a ($C_1$-$C_6$)alkyl group;
(a4) a halo ($C_1$-$C_6$) alkyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a ($C_1$-$C_6$)alkyl group;
(b10) a ($C_1$-$C_6$)alkyl group having 1 to 3 substituents each independently selected from substituent group A;
(b12) a ($C_1$-$C_6$)alkoxycarbonyl group;
(b13) a ($C_1$-$C_6$)alkoxy($C_1$-$C_6$)alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b24) a phenyl($C_1$-$C_6$) alkyl group;
(b25) a phenyl($C_1$-$C_6$) alkyl group having 1 to 5 substituents each independently selected from substituent group B;
(b28) a thiazolyl($C_1$-$C_6$)alkyl group; or
(b29) a thiazolyl($C_1$-$C_6$)alkyl group having 1 to 2 substituents each independently selected from substituent group B,

Y is $CR^{2a}R^{2b}$, $NR^{2c}$, or $CH_2CH_2$,
$R^{2a}$ is

(c1) a hydrogen atom;
(c2) a phenyl group;
(c3) a phenyl group having 1 to 5 substituents each independently selected from substituent group B;
(c4) a pyridyl group;

(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c44) a carboxyl group; or
(c45) a ($C_1$-$C_6$)alkoxycarbonyl group,

$R^{2b}$ is

(d1) a hydrogen atom; or
(d2) a ($C_1$-$C_6$)alkyl group,

$R^{2c}$ is

(e1) a ($C_1$-$C_6$)alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e44) a pyridylcarbonyl group;
(e45) a pyridylcarbonyl group having 1 to 4 substituents each independently selected from substituent group B; or
(e46) a hydrogen atom,

$R^{3a}$ is

(f1) a hydrogen atom;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom; or
(g2) a $(C_1-C_6)$alkyl group,

provided that $R^{1a}$, $R^{1b}$, $R^{2a}$, $R^{2b}$, $R^{3a}$, and $R^{3b}$ are not hydrogen atoms at the same time,
$R^{4a}$ is

(h1) a hydrogen atom;
(h2) a hydroxyl group;
(h3) a cyano group;
(h4) a $(C_1-C_6)$alkyl group;
(h7) a $(C_3-C_6)$cycloalkyl group;
(h8) a $(C_1-C_6)$ alkoxy group;
(h9) a halo $(C_1-C_6)$ alkyl group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C;
(h29) a phenyl $(C_1-C_6)$alkyl group;
(h30) a phenyl $(C_1-C_6)$alkyl group having 1 to 5 substituents each independently selected from substituent group C;
(h31) a pyridyl $(C_1-C_6)$ alkyl group;
(h32) a pyridyl $(C_1-C_6)$alkyl group having 1 to 4 substituents each independently selected from substituent group C;
(h37) a pyrazinyl $(C_1-C_6)$alkyl group;
(h38) a pyrazinyl $(C_1-C_6)$alkyl group having 1 to 3 substituents each independently selected from substituent group C;
(h39) a thienyl $(C_1-C_6)$ alkyl group;
(h40) a thienyl$(C_1-C_6)$alkyl group having 1 to 3 substituents each independently selected from substituent group C;
(h41) a furyl$(C_1-C_6)$alkyl group;
(h42) a furyl$(C_1-C_6)$alkyl group having 1 to 3 substituents each independently selected from substituent group C;
(h45) a thiazolyl$(C_1-C_6)$alkyl group;
(h46) a thiazolyl$(C_1-C_6)$alkyl group having 1 to 2 substituents each independently selected from substituent group C;
(h47) a benzofuranyl $(C_1-C_6)$ alkyl group;
(h48) a benzofuranyl$(C_1-C_6)$alkyl group having 1 to 5 substituents each independently selected from substituent group C;
(h49) a tetrahydropyranyl$(C_1-C_6)$alkyl group; or (h50) a tetrahydrofuryl $(C_1-C_6)$ alkyl group,

$R^{4b}$ is

(i1) a hydrogen atom; or
(i2) a $(C_1-C_6)$alkyl group,

when $R^{4a}$ is (h4) a $(C_1-C_6)$alkyl group and $R^{4b}$ is (i2) a $(C_1-C_6)$alkyl group, then $R^{4a}$ and $R^{4b}$ may form a 3- to 6-membered ring together with the adjacent nitrogen atom,

$R^5$ is

(h1) a hydrogen atom;
(h4) a $(C_1-C_6)$alkyl group;
(h5) a $(C_2-C_6)$alkenyl group;
(h6) a $(C_2-C_6)$alkynyl group;
(h7) a $(C_3-C_6)$cycloalkyl group;
(h9) a halo$(C_1-C_6)$alkyl group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h27) a pyrazolyl group;
(h28) a pyrazolyl group having 1 to 3 substituents each independently selected from substituent group C;
(h29) a phenyl$(C_1-C_6)$ alkyl group;
(h30) a phenyl$(C_1-C_6)$alkyl group having 1 to 5 substituents each independently selected from substituent group C;
(h37) a pyrazinyl$(C_1-C_6)$ alkyl group;
(h38) a pyrazinyl$(C_1-C_6)$ alkyl group having 1 to 3 substituents each independently selected from substituent group C; or
(h51) a dihydrobenzopyranyl group, and

Z is an oxygen atom,
or a salt thereof.

[3'] The compound of the above-mentioned [3], wherein

the substituent group A consists of

(j1) a cyano group;
(j2) a $(C_3-C_6)$cycloalkyl group;
(j3) a $(C_1-C_6)$alkoxy group; and
(j8) a di$(C_1-C_6)$alkylamino group (the $(C_1-C_6)$alkyl may be the same or different),

the substituent group B consists of

(k1) a halogen atom;
(k2) a cyano group;
(k7) a $(C_1-C_6)$alkyl group;
(k10) a $(C_1-C_6)$alkoxy group;
(k17) a halo$(C_1-C_6)$alkyl group;
(k20) a halo$(C_1-C_6)$alkoxy group;
(k26) a phenyl group; and
(k27) a phenyl group having 1 to 5 substituents each independently selected from the group consisting of a halogen atom, a cyano group, a $(C_1-C_6)$alkyl group, a $(C_1-C_6)$alkoxy group, a $(C_3-C_6)$cycloalkyl group, a halo$(C_1-C_6)$alkyl group, and a halo $(C_1-C_6)$alkoxy group;
(k28) a pyridyl group; and
(k29) a pyridyl group having 1 to 4 substituents each independently selected from the group consisting of a halogen atom, a cyano group, a $(C_1-C_6)$alkyl group, a $(C_1-C_6)$alkoxy group, a $(C_3-C_6)$cycloalkyl group, a halo$(C_1-C_6)$alkyl group, and a halo $(C_1-C_6)$ alkoxy group, and

the substituent group C consists of

(11) a halogen atom;
(17) a ($C_1$-$C_6$)alkyl group;
(110) a ($C_1$-$C_6$)alkoxy group; and
(120) a halo($C_1$-$C_6$)alkoxy group,

or a salt thereof.

[4] The compound of the above-mentioned [1], wherein Y is $CR^{2a}R^{2b}$ or $NR^{2c}$, or a salt thereof.
[5] The compound of the above-mentioned [2] or [2'], wherein Y is $CR^{2a}R^{2b}$ or $NR^{2c}$, or a salt thereof.
[6] The compound of the above-mentioned [3] or [3'], wherein Y is $CR^{2a}R^{2b}$ or $NR^{2c}$, or a salt thereof.
[7] A pest control agent comprising the compound of any of the above-mentioned [1] to [6], [2'], and [3'], or a salt thereof as an active ingredient.
[8] An agrohorticultural insect pest control agent comprising the compound of any of the above-mentioned [1] to [6], [2'], and [3'], or a salt thereof as an active ingredient.
[9] An ectoparasite control agent for animals, comprising the compound of any of the above-mentioned [1] to [6], [2'], and [3'], or a salt thereof as an active ingredient.
[10] An endoparasite control agent for animals, comprising the compound of any of the above-mentioned [1] to [6], [2'], and [3'], or a salt thereof as an active ingredient.
[11] A method for controlling an agrohorticultural insect pest, comprising treating a plant or soil with an effective amount of the agrohorticultural insect pest control agent of the above-mentioned [8].
[12] A method for controlling an ectoparasite in an animal, comprising orally or parenterally administering an effective amount of the ectoparasite control agent for animals of the above-mentioned [9] to a target animal.
[13] A method for controlling an endoparasite in an animal, comprising orally or parenterally administering an effective amount of the endoparasite control agent for animals of the above-mentioned [10] to a target animal.
[14] Use of the compound of any of the above-mentioned [1] to [6], [2'], and [3'], or a salt thereof as a pest control agent.
[15] A compound represented by the formula (3)

[Chem. 2]

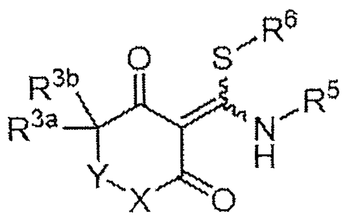

(3)

wherein

X is $CR^{1a}R^{1b}$, $NR^{1c}$, or an oxygen atom,
$R^{1a}$ and $R^{1b}$ are each

(a1) a hydrogen atom;
(a2) a halogen atom;
(a3) a ($C_1$-$C_6$)alkyl group;
(a4) a halo($C_1$-$C_6$)alkyl group;
(a5) a phenyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;

(b2) a $(C_1-C_6)$alkyl group;
(b5) a $(C_3-C_6)$cycloalkyl group;
(b6) a halo$(C_1-C_6)$alkyl group;
(b9) a halo $(C_3-C_6)$cycloalkyl group;
(b10) a $(C_1-C_6)$alkyl group having 1 to 3 substituents each independently selected from substituent group A;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b18) a pyridazinyl group;
(b19) a pyridazinyl group having 1 to 3 substituents each independently selected from substituent group B;
(b20) a pyrimidinyl group;
(b21) a pyrimidinyl group having 1 to 3 substituents each independently selected from substituent group B;
(b22) a pyrazinyl group;
(b23) a pyrazinyl group having 1 to 3 substituents each independently selected from substituent group B;
(b24) a phenyl$(C_1-C_6)$alkyl group;
(b25) a phenyl$(C_1-C_6)$alkyl group having 1 to 5 substituents each independently selected from substituent group B;
(b26) a pyridyl $(C_1-C_6)$ alkyl group;
(b27) a pyridyl$(C_1-C_6)$alkyl group having 1 to 4 substituents each independently selected from substituent group B;
(b28) a thiazolyl$(C_1-C_6)$alkyl group; or
(b29) a thiazolyl$(C_1-C_6)$alkyl group having 1 to 2 substituents each independently selected from substituent group B,

Y is $CR^{2a}R^{2b}$, $NR^{2c}$, or $CH_2CH_2$,
$R^{2a}$ is

(c1) a hydrogen atom;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c28) a carboxyl group;
(c29) a $(C_1-C_6)$alkoxycarbonyl group

(c44) a carboxyl group; or
(c45) a ($C_1$-$C_6$)alkoxycarbonyl group,

$R^{2b}$ is

(d1) a hydrogen atom; or
(d2) a ($C_1$-$C_6$)alkyl group,

$R^{2c}$ is

(e1) a ($C_1$-$C_6$)alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e10) a pyrazinyl group;
(e11) a pyrazinyl group having 1 to 3 substituents each independently selected from substituent group B;
(e12) a furyl group;
(e13) a furyl group having 1 to 3 substituents each independently selected from substituent group B;
(e14) a thienyl group;
(e15) a thienyl group having 1 to 3 substituents each independently selected from substituent group B;
(e16) an oxazolyl group;
(e17) an oxazolyl group having 1 to 2 substituents each independently selected from substituent group B;
(e20) a thiazolyl group;
(e21) a thiazolyl group having 1 to 2 substituents each independently selected from substituent group B;
(e44) a pyridylcarbonyl group; or
(e45) a pyridylcarbonyl group having 1 to 4 substituents each independently selected from substituent group B,

$R^{3a}$ is

(f1) a hydrogen atom;
(f2) a ($C_1$-$C_6$)alkyl group;
(f4) a phenyl group;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom;
(g2) a ($C_1$-$C_6$)alkyl group; or
(g3) a halo($C_1$-$C_6$)alkyl group,

provided that $R^{1a}$, $R^{1b}$, $R^{2a}$, $R^{2ab}$, $R^{3a}$, and $R^{3b}$ are not hydrogen atoms at the same time, and X is not $NR^{1c}$ when Y is$NR^{2c}$,
$R^5$ is

(h1) a hydrogen atom;
(h2) a hydroxyl group;

(h3) a cyano group;
(h4) a ($C_1$-$C_6$)alkyl group;
(h5) a ($C_2$-$C_6$)alkenyl group;
(h6) a ($C_2$-$C_6$)alkynyl group;
(h7) a ($C_3$-$C_6$)cycloalkyl group;
(h8) a ($C_1$-$C_6$)alkoxy group;
(h9) a halo($C_1$-$C_6$) alkyl group;
(h12) a halo($C_3$-$C_6$)cycloalkyl group;
(h13) a halo($C_1$-$C_6$)alkoxy group;
(h14) a ($C_1$-$C_6$)alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C;
(h29) a phenyl ($C_1$-$C_6$) alkyl group;
(h30) a phenyl($C_1$-$C_6$) alkyl group having 1 to 5 substituents each independently selected from substituent group C;
(h31) a pyridyl($C_1$-$C_6$)alkyl group;
(h32) a pyridyl($C_1$-$C_6$)alkyl group having 1 to 4 substituents each independently selected from substituent group C;
(h37) a pyrazinyl ($C_1$-$C_6$)alkyl group;
(h38) a pyrazinyl($C_1$-$C_6$)alkyl group having 1 to 3 substituents each independently selected from substituent group C;
(h39) a thienyl($C_1$-$C_6$)alkyl group;
(h40) a thienyl($C_1$-$C_6$)alkyl group having 1 to 3 substituents each independently selected from substituent group C;
(h41) a furyl($C_1$-$C_6$)alkyl group;
(h42) a furyl($C_1$-$C_6$)alkyl group having 1 to 3 substituents each independently selected from substituent group C;
(h43) an oxazolyl($C_1$-$C_6$)alkyl group;
(h44) an oxazolyl($C_1$-$C_6$)alkyl group having 1 to 2 substituents each independently selected from substituent group C;
(h45) a thiazolyl($C_1$-$C_6$)alkyl group;
(h46) a thiazolyl($C_1$-$C_6$)alkyl group having 1 to 2 substituents each independently selected from substituent group C;
(h47) a benzofuranyl($C_1$-$C_6$)alkyl group;
(h48) a benzofuranyl($C_1$-$C_6$)alkyl group having 1 to 5 substituents each independently selected from substituent group C;
(h49) a tetrahydropyranyl($C_1$-$C_6$)alkyl group;
(h50) a tetrahydrofuryl($C_1$-$C_6$)alkyl group; or
(h51) a dihydrobenzopyranyl group, and

$R^6$ is

(m1) a ($C_1$-$C_6$)alkyl group;
(m2) a ($C_2$-$C_6$)alkenyl group;
(m3) a ($C_2$-$C_6$)alkynyl group;
(m4) a halo($C_1$-$C_6$)alkyl group;

(m5) a phenyl($C_1$-$C_6$)alkyl group; or
(m6) a phenyl($C_1$-$C_6$)alkyl group having 1 to 5 substituents each independently selected from substituent group C,

substituent group A consists of

(j1) a cyano group;
(j2) a ($C_3$-$C_6$)cycloalkyl group;
(j3) a ($C_1$-$C_6$)alkoxy group;
(j4) a ($C_1$-$C_6$)alkylsulfanyl group;
(j5) a ($C_1$-$C_6$)alkylsulfinyl group;
(j6) a ($C_1$-$C_6$)alkylsulfonyl group;
(j7) a ($C_1$-$C_6$)alkylamino group;
(j8) a di ($C_1$-$C_6$) alkylamino group (the ($C_1$-$C_6$) alkyl may be the same or different);
(j9) a halo($C_3$-$C_6$)cycloalkyl group;
(j10) a halo($C_1$-$C_6$) alkoxy group;
(j11) a halo($C_1$-$C_6$)alkylsulfanyl group;
(j12) a halo($C_1$-$C_6$) alkylsulfinyl group; and
(j13) a halo($C_1$-$C_6$)alkylsulfonyl group,

substituent group B consists of

(k1) a halogen atom;
(k2) a cyano group;
(k3) a nitro group;
(k4) an amino group;
(k5) a hydroxyl group;
(k6) a carboxyl group;
(k7) a ($C_1$-$C_6$)alkyl group;
(k8) a ($C_2$-$C_6$)alkenyl group;
(k9) a ($C_2$-$C_6$)alkynyl group;
(k10) a ($C_1$-$C_6$)alkoxy group;
(k11) a ($C_3$-$C_6$)cycloalkyl group;
(k12) a ($C_1$-$C_6$)alkylsulfanyl group;
(k13) a ($C_1$-$C_6$)alkylsulfinyl group;
(k14) a ($C_1$-$C_6$)alkylsulfonyl group;
(k15) a ($C_1$-$C_6$)alkylamino group;
(k16) a di($C_1$-$C_6$)alkylamino group (the ($C_1$-$C_6$) alkyl may be the same or different);
(k17) a halo($C_1$-$C_6$)alkyl group;
(k18) a halo($C_2$-$C_6$)alkenyl group;
(k19) a halo($C_2$-$C_6$)alkynyl group;
(k20) a halo($C_1$-$C_6$)alkoxy group;
(k21) a halo($C_3$-$C_6$)cycloalkyl group;
(k22) a halo($C_1$-$C_6$)alkylsulfanyl group;
(k23) a halo($C_1$-$C_6$)alkylsulfinyl group;
(k24) a halo($C_1$-$C_6$)alkylsulfonyl group;
(k25) a ($C_1$-$C_6$)alkoxy ($C_1$-$C_6$) alkyl group;
(k26) a phenyl group;
(k27) a phenyl group having 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a halo($C_1$-$C_6$)alkyl group, and a halo ($C_1$-$C_6$)alkoxy group;
(k28) a pyridyl group; and
(k29) a pyridyl group having 1 to 4 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a halo($C_1$-$C_6$)alkyl group, and a halo ($C_1$-$C_6$)alkoxy group,

substituent group C consists of

(11) a halogen atom;
(12) a cyano group;
(13) a nitro group;
(14) an amino group;
(15) a hydroxyl group;
(16) a carboxyl group;
(17) a $(C_1-C_6)$alkyl group;
(18) a $(C_2-C_6)$alkenyl group;
(19) a $(C_2-C_6)$alkynyl group;
(110) a $(C_1-C_6)$alkoxy group;
(111) a $(C_3-C_6)$cycloalkyl group;
(112) a $(C_1-C_6)$alkylsulfanyl group;
(113) a $(C_1-C_6)$alkylsulfinyl group;
(114) a $(C_1-C_6)$alkylsulfonyl group;
(115) a $(C_1-C_6)$alkylamino group;
(116) a di$(C_1-C_6)$alkylamino group (the $(C_1-C_6)$alkyl may be the same or different);
(117) a halo$(C_1-C_6)$alkyl group;
(118) a halo$(C_2-C_6)$alkenyl group;
(119) a halo $(C_2-C_6)$alkynyl group;
(120) a halo $(C_1-C_6)$ alkoxy group;
(121) a halo $(C_3-C_6)$cycloalkyl group;
(122) a halo$(C_1-C_6)$alkylsulfanyl group;
(123) a halo $(C_1-C_6)$alkylsulfinyl group;
(124) a halo $(C_1-C_6)$alkylsulfonyl group; and
(125) a $(C_1-C_6)$alkoxy$(C_1-C_6)$alkyl group

(hereinafter at times referred to as compound (3)), or a salt thereof.

[15'] The compound of the above-mentioned [15], wherein

the substituent group A consists of

(j1) a cyano group;
(j2) a $(C_3-C_6)$cycloalkyl group;
(j3) a $(C_1-C_6)$alkoxy group;
(j4) a $(C_1-C_6)$alkylsulfanyl group;
(j5) a $(C_1-C_6)$alkylsulfinyl group;
(j6) a $(C_1-C_6)$alkylsulfonyl group;
(j7) a $(C_1-C_6)$alkylamino group;
(j8) a di$(C_1-C_6)$alkylamino group (the $(C_1-C_6)$alkyl may be the same or different); and
(j10) a halo $(C_1-C_6)$ alkoxy group,

the substituent group B consists of

(k1) a halogen atom;
(k2) a cyano group;
(k7) a $(C_1-C_6)$alkyl group;
(k10) a $(C_1-C_6)$alkoxy group;
(k11) a $(C_3-C_6)$cycloalkyl group;
(k12) a $(C_1-C_6)$alkylsulfanyl group;
(k13) a $(C_1-C_6)$alkylsulfinyl group;
(k14) a $(C_1-C_6)$alkylsulfonyl group;
(k17) a halo$(C_1-C_6)$alkyl group;
(k20) a halo$(C_1-C_6)$alkoxy group;
(k22) a halo$(C_1-C_6)$alkylsulfanyl group;
(k23) a halo$(C_1-C_6)$alkylsulfinyl group;
(k24) a halo$(C_1-C_6)$alkylsulfonyl group;
(k26) a phenyl group;

(k27) a substituted phenyl group having on the ring 1 to 5 substituents each independently selected from the group consisting of a halogen atom, a cyano group, a $(C_1-C_6)$alkyl group, a $(C_1-C_6)$alkoxy group, a $(C_3-C_6)$ cycloalkyl group, a halo $(C_1-C_6)$alkyl group, and a halo$(C_1-C_6)$alkoxy group;
(k28) a pyridyl group; and
(k29) a substituted pyridyl group having on the ring 1 to 4 substituents each independently selected from the group consisting of a halogen atom, a cyano group, a $(C_1-C_6)$alkyl group, a $(C_1-C_6)$alkoxy group, a $(C_3-C_6)$ cycloalkyl group, a halo $(C_1-C_6)$alkyl group, and a halo $(C_1-C_6)$alkoxy group, and

the substituent group C consists of

(l1) a halogen atom;
(l2) a cyano group;
(l7) a $(C_1-C_6)$alkyl group;
(l10) a $(C_1-C_6)$alkoxy group;
(l11) a $(C_3-C_6)$cycloalkyl group;
(l12) a $(C_1-C_6)$alkylsulfanyl group;
(l13) a $(C_1-C_6)$alkylsulfinyl group;
(l14) a $(C_1-C_6)$alkylsulfonyl group;
(l17) a halo $(C_1-C_6)$ alkyl group;
(l20) a halo $(C_1-C_6)$alkoxy group;
(l22) a halo$(C_1-C_6)$alkylsulfanyl group;
(l23) a halo$(C_1-C_6)$alkylsulfinyl group; and
(l24) a halo$(C_1-C_6)$alkylsulfonyl group, or a salt thereof.
[16] The compound of the above-mentioned [15] or [15'], wherein

X is $CR^{1a}R^{1b}$, $NR^{1c}$, or an oxygen atom,
$R^{1a}$ and $R^{1b}$ are each

(a1) a hydrogen atom;
(a3) a $(C_1-C_6)$alkyl group;
(a4) a halo$(C_1-C_6)$alkyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a $(C_1-C_6)$alkyl group;
(b12) a $(C_1-C_6)$alkoxycarbonyl group;
(b14) a phenyl group;
(b15) a phenyl group having 1 to 5 substituents each independently selected from substituent group B;
(b16) a pyridyl group; or
(b17) a pyridyl group having 1 to 4 substituents each independently selected from substituent group B,

Y is $CR^{2a}R^{2b}$, $NR^{2c}$, or $CH_2CH_2$,
$R^{2a}$ is

(c1) a hydrogen atom;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;

(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c44) a carboxyl group; or
(c45) a $(C_1-C_6)$alkoxycarbonyl group,

$R^{2b}$ is

(d1) a hydrogen atom; or
(d2) a $(C_1-C_6)$alkyl group,

$R^{2c}$ is

(e1) a $(C_1-C_6)$alkoxycarbonyl group;
(e4) a pyridyl group;
(e5) a pyridyl group having 1 to 4 substituents each independently selected from substituent group B;
(e44) a pyridylcarbonyl group; or
(e45) a pyridylcarbonyl group having 1 to 4 substituents each independently selected from substituent group B,

$R^{3a}$ is

(f1) a hydrogen atom;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom; or
(g2) a $(C_1-C_6)$alkyl group,

provided that $R^{1a}$, $R^{1b}$, $R^{2a}$, $R^{2ab}$, $R^{3a}$, and $R^{3b}$ are not hydrogen atoms at the same time, and X is not $NR^{1c}$ when Y is $NR^{2c}$,
$R^5$ is

(h1) a hydrogen atom;
(h4) a $(C_1-C_6)$alkyl group;
(h5) a $(C_2-C_6)$alkenyl group;
(h6) a $(C_2-C_6)$alkynyl group;

(h7) a $(C_3-C_6)$cycloalkyl group;
(h9) a halo$(C_1-C_6)$ alkyl group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h27) a pyrazolyl group;
(h28) a pyrazolyl group having 1 to 3 substituents each independently selected from substituent group C;
(h29) a phenyl$(C_1-C_6)$ alkyl group;
(h30) a phenyl$(C_1-C_6)$ alkyl group having 1 to 5 substituents each independently selected from substituent group C;
(h37) a pyrazinyl $(C_1-C_6)$alkyl group;
(h38) a pyrazinyl$(C_1-C_6)$alkyl group having 1 to 3 substituents each independently selected from substituent group C; or
(h51) a dihydrobenzopyranyl group, and

$R^6$ is

(m1) a $(C_1-C_6)$alkyl group;
(m5) a phenyl $(C_1-C_6)$alkyl group; or
(m6) a phenyl $(C_1-C_6)$ alkyl group having 1 to 5 substituents each independently selected from substituent group C,

or a salt thereof.

[17] A compound represented by the formula (1A)

[Chem. 3]

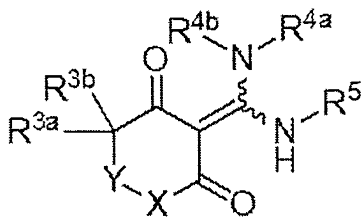

(1A)

wherein

X is $CR^{1a}R^{1b}$, $NR^{1c}$, or an oxygen atom,
$R^{1a}$ and $R^{1b}$ are each

(a1) a hydrogen atom;
(a2) a halogen atom;
(a3) a $(C_1-C_6)$alkyl group; or
(a4) a halo$(C_1-C_6)$alkyl group,

$R^{1a}$ and $R^{1b}$ may be the same or different,
$R^{1c}$ is

(b1) a hydrogen atom;

(b2) a $(C_1-C_6)$alkyl group;
(b3) a $(C_2-C_6)$alkenyl group;
(b4) a $(C_2-C_6)$alkynyl group;
(b5) a $(C_3-C_6)$cycloalkyl group;
(b6) a halo$(C_1-C_6)$ alkyl group;
(b7) a halo$(C_2-C_6)$alkenyl group;
(b8) a halo$(C_2-C_6)$alkynyl group;
(b9) a halo$(C_3-C_6)$cycloalkyl group;
(b10') a $(C_1-C_6)$alkyl group having 1 to 3 substituents each independently selected from substituent group $A_0$;
(b11) a $(C_1-C_6)$alkylcarbonyl group;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15') a phenyl group having 1 to 5 substituents each independently selected from substituent group $B_0$;
(b16) a pyridyl group;
(b17') a pyridyl group having 1 to 4 substituents each independently selected from substituent group $B_0$;
(b18) a pyridazinyl group;
(b19') a pyridazinyl group having 1 to 3 substituents each independently selected from substituent group $B_0$;
(b20) a pyrimidinyl group;
(b21') a pyrimidinyl group having 1 to 3 substituents each independently selected from substituent group $B_0$;
(b22) a pyrazinyl group;
(b23') a pyrazinyl group having 1 to 3 substituents each independently selected from substituent group $B_0$;
(b24) a phenyl$(C_1-C_6)$alkyl group;
(b25') a phenyl$(C_1-C_6)$alkyl group having 1 to 5 substituents each independently selected from substituent group $B_0$;
(b26) a pyridyl $(C_1-C_6)$alkyl group;
(b27') a pyridyl$(C_1-C_6)$alkyl group having 1 to 4 substituents each independently selected from substituent group $B_0$;
(b28) a thiazolyl$(C_1-C_6)$alkyl group; or
(b29') a thiazolyl$(C_1-C_6)$alkyl group having 1 to 2 substituents each independently selected from substituent group $B_0$,

Y is $CR^{2a}R^{2b}$, $NR^{2c}$ or $CH_2CH_2$,
$R^{2a}$ is

(c1) a hydrogen atom;
(c2) a phenyl group;
(c3') a phenyl group having 1 to 5 substituents each independently selected from substituent group $B_0$;
(c4) a pyridyl group;
(c5') a pyridyl group having 1 to 4 substituents each independently selected from substituent group $B_0$;
(c6) a pyridazinyl group;
(c7') a pyridazinyl group having 1 to 3 substituents each independently selected from substituent group $B_0$;
(c8) a pyrimidinyl group;
(c9') a pyrimidinyl group having 1 to 3 substituents each independently selected from substituent group $B_0$;
(c10) a pyrazinyl group;
(c11') a pyrazinyl group having 1 to 3 substituents each independently selected from substituent group $B_0$;
(c12) a furyl group;
(c13') a furyl group having 1 to 3 substituents each independently selected from substituent group $B_0$;
(c14) a thienyl group;
(c15') a thienyl group having 1 to 3 substituents each independently selected from substituent group $B_0$;
(c16) an oxazolyl group;
(c17') an oxazolyl group having 1 to 2 substituents each independently selected from substituent group $B_0$;
(c18) an isoxazolyl group;
(c19') a isoxazolyl group having 1 to 2 substituents each independently selected from substituent group $B_0$;
(c20) a thiazolyl group;
(c21') a thiazolyl group having 1 to 2 substituents each independently selected from substituent group $B_0$;
(c22) an isothiazolyl group; or
(c23') a isothiazolyl group having 1 to 2 substituents each independently selected from substituent group $B_0$,

$R^{2b}$ is

(d1) a hydrogen atom; or
(d2) a ($C_1$-$C_6$)alkyl group,

$R^{2c}$ is

(e1) a ($C_1$-$C_6$)alkoxycarbonyl group;
(e2) a phenyl group;
(e3') a phenyl group having 1 to 5 substituents each independently selected from substituent group $B_0$;
(e4) a pyridyl group;
(e5') a pyridyl group having 1 to 4 substituents each independently selected from substituent group $B_0$;
(e6) a pyridazinyl group;
(e7') a pyridazinyl group having 1 to 3 substituents each independently selected from substituent group $B_0$;
(e8) a pyrimidinyl group;
(e9') a pyrimidinyl group having 1 to 3 substituents each independently selected from substituent group $B_0$;
(e10) a pyrazinyl group;
(e11') a pyrazinyl group having 1 to 3 substituents each independently selected from substituent group $B_0$;
(e12) a furyl group;
(e13') a furyl group having 1 to 3 substituents each independently selected from substituent group $B_0$;
(e14) a thienyl group;
(e15') a thienyl group having 1 to 3 substituents each independently selected from substituent group $B_0$;
(e16) an oxazolyl group;
(e17') an oxazolyl group having 1 to 2 substituents each independently selected from substituent group $B_0$;
(e18) an isoxazolyl group;
(e19') a isoxazolyl group having 1 to 2 substituents each independently selected from substituent group $B_0$;
(e20) a thiazolyl group;
(e21') a thiazolyl group having 1 to 2 substituents each independently selected from substituent group $B_0$;
(e22) an isothiazolyl group; or
(e23') a isothiazolyl group having 1 to 2 substituents each independently selected from substituent group $B_0$,

$R^{3a}$ is

(f1) a hydrogen atom;
(f2) a ($C_1$-$C_6$)alkyl group;
(f3) a halo($C_1$-$C_6$)alkyl group;
(f4) a phenyl group;
(f5') a phenyl group having 1 to 5 substituents each independently selected from substituent group $B_0$;
(f6) a pyridyl group; or
(f7') a pyridyl group having 1 to 4 substituents each independently selected from substituent group $B_0$,

$R^{3b}$ is

(g1) a hydrogen atom;
(g2) a ($C_1$-$C_6$)alkyl group; or
(g3) a halo($C_1$-$C_6$)alkyl group,

$R^{4a}$ and $R^5$ are each

(h1) a hydrogen atom;
(h2) a hydroxyl group;
(h3) a cyano group;
(h4) a ($C_1$-$C_6$)alkyl group;
(h5) a ($C_2$-$C_6$)alkenyl group;
(h6) a ($C_2$-$C_6$)alkynyl group;
(h7) a ($C_3$-$C_6$)cycloalkyl group;
(h8) a ($C_1$-$C_6$) alkoxy group;
(h9) a halo($C_1$-$C_6$)alkyl group;

(h10) a halo($C_2$-$C_6$)alkenyl group;
(h11) a halo($C_2$-$C_6$)alkynyl group;
(h12) a halo($C_3$-$C_6$)cycloalkyl group;
(h13) a halo($C_1$-$C_6$)alkoxy group;
(h14') a ($C_1$-$C_6$)alkyl group having 1 to 3 substituents each independently selected from substituent group $A_0$;
(h15) a phenyl group;
(h16') a phenyl group having 1 to 5 substituents each independently selected from substituent group $C_0$;
(h17) a pyridyl group;
(h18') a pyridyl group having 1 to 4 substituents each independently selected from substituent group $C_0$;
(h19) a pyridazinyl group;
(h20') a pyridazinyl group having 1 to 3 substituents each independently selected from substituent group $C_0$;
(h21) a pyrimidinyl group;
(h22') a pyrimidinyl group having 1 to 3 substituents each independently selected from substituent group $C_0$;
(h23) a pyrazinyl group;
(h24') a pyrazinyl group having 1 to 3 substituents each independently selected from substituent group $C_0$;
(h25) an isoxazolyl group;
(h26') a isoxazolyl group having 1 to 2 substituents each independently selected from substituent group $C_0$;
(h27) a pyrazolyl group;
(h28') a pyrazolyl group having 1 to 3 substituents each independently selected from substituent group $C_0$;
(h29) a phenyl($C_1$-$C_6$)alkyl group;
(h30') a phenyl($C_1$-$C_6$)alkyl group having 1 to 5 substituents each independently selected from substituent group $C_0$;
(h31) a pyridyl($C_1$-$C_6$)alkyl group;
(h32') a pyridyl($C_1$-$C_6$)alkyl group having 1 to 4 substituents each independently selected from substituent group $C_0$;
(h33) a pyridazinyl($C_1$-$C_6$)alkyl group;
(h34') a pyridazinyl($C_1$-$C_6$)alkyl group having 1 to 3 substituents each independently selected from substituent group $C_0$;
(h35) a pyrimidinyl ($C_1$-$C_6$) alkyl group;
(h36') a pyrimidinyl($C_1$-$C_6$)alkyl group having 1 to 3 substituents each independently selected from substituent group $C_0$;
(h37) a pyrazinyl($C_1$-$C_6$)alkyl group;
(h38') a pyrazinyl($C_1$-$C_6$)alkyl group having 1 to 3 substituents each independently selected from substituent group $C_0$;
(h39) a thienyl($C_1$-$C_6$)alkyl group;
(h40') a thienyl($C_1$-$C_6$)alkyl group having 1 to 3 substituents each independently selected from substituent group $C_0$;
(h41) a furyl($C_1$-$C_6$)alkyl group;
(h42') a furyl($C_1$-$C_6$)alkyl group having 1 to 3 substituents each independently selected from substituent group $C_0$;
(h43) an oxazolyl($C_1$-$C_6$)alkyl group;
(h44') an oxazolyl($C_1$-$C_6$)alkyl group having 1 to 2 substituents each independently selected from substituent group $C_0$;
(h45) a thiazolyl($C_1$-$C_6$)alkyl group;
(h46') a thiazolyl($C_1$-$C_6$)alkyl group having 1 to 2 substituents each independently selected from substituent group $C_0$;
(h47) a benzofuranyl($C_1$-$C_6$)alkyl group;
(h48') a benzofuranyl($C_1$-$C_6$)alkyl group having 1 to 5 substituents each independently selected from substituent group $C_0$;
(h49) a tetrahydropyranyl($C_1$-$C_6$)alkyl group; or
(h50) a tetrahydrofuryl($C_1$-$C_6$)alkyl group,

$R^{4a}$ and $R^5$ may be the same or different,
$R^{4b}$ is

(i1) a hydrogen atom;
(i2) a ($C_1$-$C_6$)alkyl group; or
(i3) a halo ($C_1$-$C_6$) alkyl group,

when $R^{4a}$ is (h4) a ($C_1$-$C_6$)alkyl group or (h14') a ($C_1$-$C_6$)alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom,
substituent group $A_0$ consists of

(j1) a cyano group;
(j2) a ($C_3$-$C_6$)cycloalkyl group;
(j3) a ($C_1$-$C_6$)alkoxy group;
(j4) a ($C_1$-$C_6$)alkylsulfanyl group;
(j5) a ($C_1$-$C_6$)alkylsulfinyl group;
(j6) a ($C_1$-$C_6$)alkylsulfonyl group;
(j7) a ($C_1$-$C_6$)alkylamino group;
(j8) a di($C_1$-$C_6$)alkylamino group (the ($C_1$-$C_6$)alkyl may be the same or different);
(j9) a halo ($C_3$-$C_6$) cycloalkyl group;
(j10) a halo ($C_1$-$C_6$) alkoxy group;
(j11) a halo($C_1$-$C_6$)alkylsulfanyl group;
(j12) a halo($C_1$-$C_6$)alkylsulfinyl group; and
(j13) a halo($C_1$-$C_6$)alkylsulfonyl group,

substituent group $B_0$ consists of

(k1) a halogen atom;
(k2) a cyano group;
(k3) a nitro group;
(k4) an amino group;
(k5) a hydroxyl group;
(k6) a carboxyl group;
(k7) a ($C_1$-$C_6$)alkyl group;
(k8) a ($C_2$-$C_6$)alkenyl group;
(k9) a ($C_2$-$C_6$)alkynyl group;
(k10) a ($C_1$-$C_6$)alkoxy group;
(k11) a ($C_3$-$C_6$) cycloalkyl group;
(k12) a ($C_1$-$C_6$)alkylsulfanyl group;
(k13) a ($C_1$-$C_6$)alkylsulfinyl group;
(k14) a ($C_1$-$C_6$)alkylsulfonyl group;
(k15) a ($C_1$-$C_6$)alkylamino group;
(k16) a di($C_1$-$C_6$)alkylamino group (the ($C_1$-$C_6$)alkyl may be the same or different);
(k17) a halo($C_1$-$C_6$)alkyl group;
(k18) a halo($C_2$-$C_6$)alkenyl group;
(k19) a halo($C_2$-$C_6$)alkynyl group;
(k20) a halo ($C_1$-$C_6$)alkoxy group;
(k21) a halo ($C_3$-$C_6$) cycloalkyl group;
(k22) a halo($C_1$-$C_6$)alkylsulfanyl group;
(k23) a halo($C_1$-$C_6$)alkylsulfinyl group;
(k24) a halo($C_1$-$C_6$)alkylsulfonyl group; and
(k25) a ($C_1$-$C_6$)alkoxy($C_1$-$C_6$)alkyl group, and

substituent group $C_0$ consists of

(11) a halogen atom;
(12) a cyano group;
(13) a nitro group;
(14) an amino group;
(15) a hydroxyl group;
(16) a carboxyl group;
(l7) a ($C_1$-$C_6$)alkyl group;
(18) a ($C_2$-$C_6$)alkenyl group;
(19) a ($C_2$-$C_6$)alkynyl group;

(l10) a $(C_1-C_6)$alkoxy group;
(111) a $(C_3-C_6)$ cycloalkyl group;
(112) a $(C_1-C_6)$alkylsulfanyl group;
(113) a $(C_1-C_6)$alkylsulfinyl group;
(114) a $(C_1-C_6)$alkylsulfonyl group;
(l15) a $(C_1-C_6)$alkylamino group;
(116) a di$(C_1-C_6)$alkylamino group (the $(C_1-C_6)$alkyl may be the same or different);
(117) a halo $(C_1-C_6)$ alkyl group;
(118) a halo $(C_2-C_6)$alkenyl group;
(l19) a halo$(C_2-C_6)$alkynyl group;
(120) a halo $(C_1-C_6)$ alkoxy group;
(121) a halo $(C_3-C_6)$ cycloalkyl group;
(122) a halo$(C_1-C_6)$alkylsulfanyl group;
(123) a halo $(C_1-C_6)$alkylsulfinyl group;
(124) a halo$(C_1-C_6)$alkylsulfonyl group; and
(125) a $(C_1-C_6)$alkoxy$(C_1-C_6)$alkyl group,

provided that the (c3') substituted phenyl group, (c5') substituted pyridyl group, (c7') substituted pyridazinyl group,

(c9') substituted pyrimidinyl group, (c11') substituted pyrazinyl group, (c13') substituted furyl group, (c15') substituted thienyl group, (c17') substituted oxazolyl group,
(c19') substituted isoxazolyl group, (c21') substituted thiazolyl group, and (c23') substituted isothiazolyl group, for $R^{2a}$, and the (f5') substituted phenyl group and (f7') substituted pyridyl group, for $R^{3a}$ are not substituted with a $(C_1-C_6)$alkylsulfonyl group or a halo$(C_1-C_6)$alkylsulfonyl group on the atom adjacent to the atom bonded to the carbon atom, and the (b15') substituted phenyl group, (b17') substituted pyridyl group, (b19') substituted pyridazinyl group, (b21') substituted pyrimidinyl group, and (b23') substituted pyrazinyl group, for $R^{1c}$, and the (e3') substituted phenyl group, (e5') substituted pyridyl group, (e7') substituted pyridazinyl group, (e9') substituted pyrimidinyl group, (e11') substituted pyrazinyl group, (e13') substituted furyl group, (e15') substituted thienyl group, (e17') substituted oxazolyl group, (e19') substituted isoxazolyl group, (e21') substituted thiazolyl group, and (e23') substituted isothiazolyl group, for $R^{2c}$ are not substituted with a $(C_1-C_6)$alkylsulfonyl group or a halo$(C_1-C_6)$alkylsulfonyl group on the atom adjacent to the atom bonded to the nitrogen atom (hereinafter at times referred to as compound (1A)), or a salt thereof.

[18] The compound of the above-mentioned [17], wherein

X is $CR^{1a}R^{1b}$, $NR^{1c}$, or an oxygen atom,
$R^{1a}$ and $R^{1b}$ are each

(a1) a hydrogen atom;
(a2) a halogen atom; or
(a3) a $(C_1-C_6)$alkyl group,

$R^{1a}$ and $R^{1b}$ may be the same or different,
$R^{1c}$ is

(b1) a hydrogen atom;
(b2) a $(C_1-C_6)$alkyl group;
(b5) a $(C_3-C_6)$ cycloalkyl group;
(b6) a halo $(C_1-C_6)$ alkyl group;
(b9) a halo$(C_3-C_6)$cycloalkyl group;
(b10') a $(C_1-C_6)$alkyl group having 1 to 3 substituents each independently selected from substituent group $A_0$;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15') a phenyl group having 1 to 5 substituents each independently selected from substituent group $B_0$;
(b16) a pyridyl group;
(b17') a pyridyl group having 1 to 4 substituents each independently selected from substituent group $B_0$;

(b18) a pyridazinyl group;
(b19') a pyridazinyl group having 1 to 3 substituents each independently selected from substituent group $B_0$;
(b20) a pyrimidinyl group;
(b21') a pyrimidinyl group having 1 to 3 substituents each independently selected from substituent group $B_0$;
(b22) a pyrazinyl group;
(b23') a pyrazinyl group having 1 to 3 substituents each independently selected from substituent group $B_0$;
(b24) a phenyl ($C_1$-$C_6$) alkyl group;
(b25') a phenyl ($C_1$-$C_6$) alkyl group having 1 to 5 substituents each independently selected from substituent group $B_0$;
(b26) a pyridyl($C_1$-$C_6$)alkyl group;
(b27') a pyridyl($C_1$-$C_6$)alkyl group having 1 to 4 substituents each independently selected from substituent group $B_0$;
(b28) a thiazolyl($C_1$-$C_6$)alkyl group; or
(b29') a thiazolyl($C_1$-$C_6$)alkyl group having 1 to 2 substituents each independently selected from substituent group $B_0$,

Y is $CR^{2a}R^{2b}$, $NR^{2c}$, or $CH_2CH_2$,
$R^{2a}$ is

(c1) a hydrogen atom;
(c2) a phenyl group;
(c3') a phenyl group having 1 to 5 substituents each independently selected from substituent group $B_0$;
(c4) a pyridyl group;
(c5') a pyridyl group having 1 to 4 substituents each independently selected from substituent group $B_0$;
(c6) a pyridazinyl group;
(c7') a pyridazinyl group having 1 to 3 substituents each independently selected from substituent group $B_0$;
(c8) a pyrimidinyl group;
(c9') a pyrimidinyl group having 1 to 3 substituents each independently selected from substituent group $B_0$;
(c10) a pyrazinyl group;
(c11') a pyrazinyl group having 1 to 3 substituents each independently selected from substituent group $B_0$;
(c12) a furyl group;
(c13') a furyl group having 1 to 3 substituents each independently selected from substituent group $B_0$;
(c14) a thienyl group; or
(c15') a thienyl group having 1 to 3 substituents each independently selected from substituent group $B_0$,

$R^{2b}$ is

(d1) a hydrogen atom; or
(d2) a ($C_1$-$C_6$)alkyl group,

$R^{2c}$ is

(e1) a ($C_1$-$C_6$)alkoxycarbonyl group;
(e2) a phenyl group;
(e3') a phenyl group having 1 to 5 substituents each independently selected from substituent group $B_0$;
(e4) a pyridyl group;
(e5') a pyridyl group having 1 to 4 substituents each independently selected from substituent group $B_0$;
(e6) a pyridazinyl group;
(e7') a pyridazinyl group having 1 to 3 substituents each independently selected from substituent group $B_0$;
(e8) a pyrimidinyl group;
(e9') a pyrimidinyl group having 1 to 3 substituents each independently selected from substituent group $B_0$;
(e10) a pyrazinyl group;
(e11') a pyrazinyl group having 1 to 3 substituents each independently selected from substituent group $B_0$;
(e12) a furyl group;
(e13') a furyl group having 1 to 3 substituents each independently selected from substituent group $B_0$;
(e14) a thienyl group;
(e15') a thienyl group having 1 to 3 substituents each independently selected from substituent group $B_0$;
(e16) an oxazolyl group;

(e17') an oxazolyl group having 1 to 2 substituents each independently selected from substituent group $B_0$;
(e20) a thiazolyl group; or
(e21') a thiazolyl group having 1 to 2 substituents each independently selected from substituent group $B_0$,

$R^{3a}$ is

(f1) a hydrogen atom;
(f2) a $(C_1-C_6)$alkyl group;
(f4) a phenyl group;
(f5') a phenyl group having 1 to 5 substituents each independently selected from substituent group $B_0$;
(f6) a pyridyl group; or
(f7') a pyridyl group having 1 to 4 substituents each independently selected from substituent group $B_0$,

$R^{3b}$ is

(g1) a hydrogen atom;
(g2) a $(C_1-C_6)$alkyl group; or
(g3) a halo $(C_1-C_6)$alkyl group,

$R^{4a}$ and $R^5$ are each

(h1) a hydrogen atom;
(h2) a hydroxyl group;
(h3) a cyano group;
(h4) a $(C_1-C_6)$alkyl group;
(h7) a $(C_3-C_6)$ cycloalkyl group;
(h8) a $(C_1-C_6)$alkoxy group;
(h9) a halo$(C_1-C_6)$alkyl group;
(h12) a halo $(C_3-C_6)$ cycloalkyl group;
(h13) a halo $(C_1-C_6)$alkoxy group;
(h14') a $(C_1-C_6)$alkyl group having 1 to 3 substituents each independently selected from substituent group $A_0$;
(h15) a phenyl group;
(h16') a phenyl group having 1 to 5 substituents each independently selected from substituent group $C_0$;
(h17) a pyridyl group;
(h18') a pyridyl group having 1 to 4 substituents each independently selected from substituent group $C_0$;
(h19) a pyridazinyl group;
(h20') a pyridazinyl group having 1 to 3 substituents each independently selected from substituent group $C_0$;
(h21) a pyrimidinyl group;
(h22') a pyrimidinyl group having 1 to 3 substituents each independently selected from substituent group $C_0$;
(h23) a pyrazinyl group;
(h24') a pyrazinyl group having 1 to 3 substituents each independently selected from substituent group $C_0$;
(h25) an isoxazolyl group;
(h26') a isoxazolyl group having 1 to 2 substituents each independently selected from substituent group $C_0$;
(h27) a pyrazolyl group;
(h28') a pyrazolyl group having 1 to 3 substituents each independently selected from substituent group $C_0$;
(h29) a phenyl$(C_1-C_6)$alkyl group;
(h30') a phenyl $(C_1-C_6)$ alkyl group having 1 to 5 substituents each independently selected from substituent group $C_0$;
(h31) a pyridyl $(C_1-C_6)$alkyl group;
(h32') a pyridyl$(C_1-C_6)$alkyl group having 1 to 4 substituents each independently selected from substituent group $C_0$;
(h37) a pyrazinyl $(C_1-C_6)$alkyl group;
(h38') a pyrazinyl $(C_1-C_6)$alkyl group having 1 to 3 substituents each independently selected from substituent group $C_0$;
(h39) a thienyl $(C_1-C_6)$alkyl group;
(h40') a thienyl$(C_1-C_6)$alkyl group having 1 to 3 substituents each independently selected from substituent group $C_0$;
(h41) a furyl$(C_1-C_6)$alkyl group;

(h42') a furyl($C_1$-$C_6$)alkyl group having 1 to 3 substituents each independently selected from substituent group $C_0$;
(h43) an oxazolyl($C_1$-$C_6$)alkyl group;
(h44') an oxazolyl($C_1$-$C_6$)alkyl group having 1 to 2 substituents each independently selected from substituent group $C_0$;
(h45) a thiazolyl($C_1$-$C_6$)alkyl group;
(h46') a thiazolyl($C_1$-$C_6$)alkyl group having 1 to 2 substituents each independently selected from substituent group $C_0$;
(h47) a benzofuranyl($C_1$-$C_6$)alkyl group;
(h48') a benzofuranyl($C_1$-$C_6$)alkyl group having 1 to 5 substituents each independently selected from substituent group $C_0$;
(h49) a tetrahydropyranyl($C_1$-$C_6$)alkyl group; or
(h50) a tetrahydrofuryl($C_1$-$C_6$)alkyl group,

$R^{4a}$ and $R^5$ may be the same or different,
$R^{4b}$ is

(i1) a hydrogen atom;
(i2) a ($C_1$-$C_6$)alkyl group; or
(i3) a halo ($C_1$-$C_6$) alkyl group,

when $R^{4a}$ is (h4) a ($C_1$-$C_6$)alkyl group or (h14') a ($C_1$-$C_6$)alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom,
or a salt thereof.

[18'] The compound of the above-mentioned [18], wherein

the substituent group $A_0$ consists of

(j1) a cyano group;
(j2) a ($C_3$-$C_6$) cycloalkyl group;
(j3) a ($C_1$-$C_6$)alkoxy group;
(j4) a ($C_1$-$C_6$)alkylsulfanyl group;
(j5) a ($C_1$-$C_6$)alkylsulfinyl group;
(j6) a ($C_1$-$C_6$)alkylsulfonyl group;
(j7) a ($C_1$-$C_6$)alkylamino group;
(j8) a di($C_1$-$C_6$)alkylamino group (the ($C_1$-$C_6$)alkyl may be the same or different);
(j10) a halo ($C_1$-$C_6$)alkoxy group,

the substituent group $B_0$ consists of,

(k1) a halogen atom;
(k2) a cyano group;
(k7) a ($C_1$-$C_6$)alkyl group;
(k10) a ($C_1$-$C_6$)alkoxy group;
(k11) a ($C_3$-$C_6$)cycloalkyl group;
(k12) a ($C_1$-$C_6$)alkylsulfanyl group;
(k13) a ($C_1$-$C_6$)alkylsulfinyl group;
(k14) a ($C_1$-$C_6$)alkylsulfonyl group;
(k17) a halo($C_1$-$C_6$)alkyl group;
(k20) a halo ($C_1$-$C_6$) alkoxy group;
(k22) a halo($C_1$-$C_6$)alkylsulfanyl group;
(k23) a halo ($C_1$-$C_6$)alkylsulfinyl group; and
(k24) a halo ($C_1$-$C_6$)alkylsulfonyl group, and

the substituent group $C_0$ consists of

(11) a halogen atom;
(12) a cyano group;
(17) a $(C_1-C_6)$alkyl group;
(110) a $(C_1-C_6)$alkoxy group;
(111) a $(C_3-C_6)$ cycloalkyl group;
(112) a $(C_1-C_6)$alkylsulfanyl group;
(113) a $(C_1-C_6)$alkylsulfinyl group;
(114) a $(C_1-C_6)$alkylsulfonyl group;
(117) a halo$(C_1-C_6)$alkyl group;
(120) a halo $(C_1-C_6)$alkoxy group;
(122) a halo $(C_1-C_6)$alkylsulfanyl group;
(123) a halo $(C_1-C_6)$alkylsulfinyl group; and
(124) a halo $(C_1-C_6)$alkylsulfonyl group,

or a salt thereof.

[19] The compound of the above-mentioned [17], wherein

X is $CR^{1a}R^{1b}$, $NR^{1c}$, or an oxygen atom,
$R^{1a}$ and $R^{1b}$ are each

(a1) a hydrogen atom; or
(a3) a $(C_1-C_6)$alkyl group,

$R^{1a}$ and $R^{1b}$ may be the same or different,
$R^{1c}$ is

(b1) a hydrogen atom;
(b2) a $(C_1-C_6)$alkyl group;
(b10') a $(C_1-C_6)$alkyl group having 1 to 3 substituents each independently selected from substituent group $A_0$;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15') a phenyl group having 1 to 5 substituents each independently selected from substituent group $B_0$;
(b16) a pyridyl group;
(b17') a pyridyl group having 1 to 4 substituents each independently selected from substituent group $B_0$;
(b24) a phenyl$(C_1-C_6)$alkyl group;
(b25') a phenyl$(C_1-C_6)$alkyl group having 1 to 5 substituents each independently selected from substituent group $B_0$;
(b28) a thiazolyl$(C_1-C_6)$alkyl group; or
(b29') a thiazolyl$(C_1-C_6)$alkyl group having 1 to 2 substituents each independently selected from substituent group $B_0$,

Y is $CR^{2a}R^{2b}$, $NR^{2c}$, or $CH_2CH_2$,
$R^{2a}$ is

(c1) a hydrogen atom;
(c2) a phenyl group;
(c3') a phenyl group having 1 to 5 substituents each independently selected from substituent group $B_0$;
(c4) a pyridyl group;
(c5') a pyridyl group having 1 to 4 substituents each independently selected from substituent group $B_0$;
(c12) a furyl group;
(c13') a furyl group having 1 to 3 substituents each independently selected from substituent group $B_0$;
(c14) a thienyl group; or
(c15') a thienyl group having 1 to 3 substituents each independently selected from substituent group $B_0$,

$R^{2b}$ is
(d1) a hydrogen atom,

$R^{2c}$ is

(e1) a ($C_1$-$C_6$)alkoxycarbonyl group;
(e2) a phenyl group;
(e3') a phenyl group having 1 to 5 substituents each independently selected from substituent group $B_0$;
(e4) a pyridyl group;
(e5') a pyridyl group having 1 to 4 substituents each independently selected from substituent group $B_0$;
(e6) a pyridazinyl group;
(e7') a pyridazinyl group having 1 to 3 substituents each independently selected from substituent group $B_0$;
(e8) a pyrimidinyl group; or
(e9') a pyrimidinyl group having 1 to 3 substituents each independently selected from substituent group $B_0$,

$R^{3a}$ is

(f1) a hydrogen atom;
(f6) a pyridyl group; or
(f7') a pyridyl group having 1 to 4 substituents each independently selected from substituent group $B_0$,

$R^{3b}$ is

(g1) a hydrogen atom; or
(g2) a ($C_1$-$C_6$)alkyl group,

$R^{4a}$ is

(h1) a hydrogen atom;
(h2) a hydroxyl group;
(h3) a cyano group;
(h4) a ($C_1$-$C_6$)alkyl group;
(h7) a ($C_3$-$C_6$) cycloalkyl group;
(h8) a ($C_1$-$C_6$)alkoxy group;
(h9) a halo ($C_1$-$C_6$) alkyl group;
(h14') a ($C_1$-$C_6$)alkyl group having 1 to 3 substituents each independently selected from substituent group $A_0$;
(h15) a phenyl group;
(h16') a phenyl group having 1 to 5 substituents each independently selected from substituent group $C_0$;
(h17) a pyridyl group;
(h18') a pyridyl group having 1 to 4 substituents each independently selected from substituent group $C_0$;
(h21) a pyrimidinyl group;
(h22') a pyrimidinyl group having 1 to 3 substituents each independently selected from substituent group $C_0$;
(h25) an isoxazolyl group;
(h26') a isoxazolyl group having 1 to 2 substituents each independently selected from substituent group $C_0$;
(h27) a pyrazolyl group;
(h28') a pyrazolyl group having 1 to 3 substituents each independently selected from substituent group $C_0$;
(h29) a phenyl ($C_1$-$C_6$)alkyl group;
(h30') a phenyl ($C_1$-$C_6$) alkyl group having 1 to 5 substituents each independently selected from substituent group $C_0$;
(h31) a pyridyl ($C_1$-$C_6$)alkyl group;
(h32') a pyridyl($C_1$-$C_6$)alkyl group having 1 to 4 substituents each independently selected from substituent group $C_0$;
(h37) a pyrazinyl ($C_1$-$C_6$)alkyl group;
(h38') a pyrazinyl($C_1$-$C_6$)alkyl group having 1 to 3 substituents each independently selected from substituent group $C_0$;
(h39) a thienyl($C_1$-$C_6$)alkyl group;
(h40') a thienyl($C_1$-$C_6$)alkyl group having 1 to 3 substituents each independently selected from substituent group $C_0$;
(h41) a furyl($C_1$-$C_6$)alkyl group;
(h42') a furyl($C_1$-$C_6$)alkyl group having 1 to 3 substituents each independently selected from substituent group $C_0$;

(h45) a thiazolyl($C_1$-$C_6$)alkyl group;
(h46') a thiazolyl($C_1$-$C_6$)alkyl group having 1 to 2 substituents each independently selected from substituent group $C_0$;
(h47) a benzofuranyl($C_1$-$C_6$)alkyl group;
(h48') a benzofuranyl($C_1$-$C_6$)alkyl group having 1 to 5 substituents each independently selected from substituent group $C_0$;
(h49) a tetrahydropyranyl($C_1$-$C_6$)alkyl group; or
(h50) a tetrahydrofuryl($C_1$-$C_6$)alkyl group,

$R^{4b}$ is

(i1) a hydrogen atom; or
(i2) a ($C_1$-$C_6$)alkyl group,

when $R^{4a}$ is (h4) a ($C_1$-$C_6$)alkyl group and $R^{4b}$ is (i2) a ($C_1$-$C_6$)alkyl group, then $R^{4a}$ and $R^{4b}$ may form a 3- to 6-membered ring together with the adjacent nitrogen atom,
$R^5$ is

(h1) a hydrogen atom;
(h4) a ($C_1$-$C_6$)alkyl group;
(h7) a ($C_3$-$C_6$) cycloalkyl group;
(h9) a halo($C_1$-$C_6$)alkyl group;
(h14') a ($C_1$-$C_6$)alkyl group having 1 to 3 substituents each independently selected from substituent group $A_0$;
(h15) a phenyl group;
(h16') a phenyl group having 1 to 5 substituents each independently selected from substituent group $C_0$;
(h17) a pyridyl group;
(h18') a pyridyl group having 1 to 4 substituents each independently selected from substituent group $C_0$;
(h29) a phenyl($C_1$-$C_6$)alkyl group; or
(h30') a phenyl($C_1$-$C_6$)alkyl group having 1 to 5 substituents each independently selected from substituent group $C_0$,

or a salt thereof.

[19'] The compound of the above-mentioned [19], wherein

the substituent group $A_0$ consists of

(j1) a cyano group;
(j2) a ($C_3$-$C_6$)cycloalkyl group;
(j3) a ($C_1$-$C_6$)alkoxy group; and
(j8) a di($C_1$-$C_6$)alkylamino group (the ($C_1$-$C_6$)alkyl may be the same or different),

the substituent group $B_0$ consists of

(k1) a halogen atom;
(k2) a cyano group;
(k10) a ($C_1$-$C_6$)alkoxy group; and
(k17) a halo ($C_1$-$C_6$) alkyl group, and

the substituent group $C_0$ consists of

(l1) a halogen atom;
(l7) a ($C_1$-$C_6$)alkyl group;
(l10) a ($C_1$-$C_6$)alkoxy group; and
(l20) a halo ($C_1$-$C_6$)alkoxy group,

or a salt thereof.

[20] A pest control agent comprising the compound of any of [17] to [19], [18'], and [19'], or a salt thereof as an active ingredient.

[21] An agrohorticultural insect pest control agent comprising the compound of any of [17] to [19], [18'], and [19'], or a salt thereof as an active ingredient.

[22] An ectoparasite control agent for animals, comprising the compound of any of [17] to [19], [18'], and [19'], or a salt thereof as an active ingredient.

[23] An endoparasite control agent for animals, comprising the compound of any of [17] to [19], [18'], and [19'], or a salt thereof as an active ingredient.

[24] A method for controlling an agrohorticultural insect pest, comprising treating a plant or soil with an effective amount of the agrohorticultural insect pest control agent of [21].

[25] A method for controlling an ectoparasite in an animal, comprising orally or parenterally administering an effective amount of the ectoparasite control agent for animals of [22] to a target animal.

[26] A method for controlling an endoparasite in an animal, comprising orally or parenterally administering an effective amount of the endoparasite control agent for animals of [23] to a target animal.

[27] Use of the compound of any of [17] to [19], [18'], and [19'], or a salt thereof as a pest control agent.

[Advantageous Effects of Invention]

The compound of the present invention or a salt thereof provides a diaminovinylidene derivative represented by the formula (1) and having superior effects as a pest control agent.

[Description of Embodiments]

In the definitions of the formulas (1), (1A), and (3) of the compound of the present invention, the "halo" means a "halogen atom", and is a chlorine atom, a bromine atom, an iodine atom, or a fluorine atom.

The "($C_1$-$C_6$)alkyl group" is, for example, a straight chain or branched chain alkyl group having 1 to 6 carbon atoms such as methyl group, ethyl group, normal propyl group, isopropyl group, normal butyl group, isobutyl group, secondary butyl group, tertiary butyl group, normal pentyl group, isopentyl group, tertiary pentyl group, neopentyl group, 2,3-dimethylpropyl group, 1-ethylpropyl group, 1-methylbutyl group, 2-methylbutyl group, normal hexyl group, isohexyl group, 2-hexyl group, 3-hexyl group, 2-methylpentyl group, 3-methylpentyl group, 1,1,2-trimethylpropyl group, 3,3-dimethylbutyl group, and the like.

The "($C_2$-$C_6$)alkenyl group" is, for example, a straight chain or branched chain alkenyl group having 2 to 6 carbon atoms such as vinyl group, allyl group, isopropenyl group, 1-butenyl group, 2-butenyl group, 2-methyl-2-propenyl group, 1-methyl-2-propenyl group, 2-methyl-1-propenyl group, pentenyl group, 1-hexenyl group, 3,3-dimethyl-1-butenyl group, and the like, and the "($C_2$-$C_6$)alkynyl group" is, for example, a straight chain or branched chain alkynyl group having 2 to 6 carbon atoms such as ethynyl group, 1-propynyl group, 2-propynyl group, 1-butynyl group, 2-butynyl group, 3-butynyl group, 3-methyl-1-propynyl group, 2-methyl-3-propynyl group, pentynyl group, 1-hexynyl group, 3-methyl-1-butynyl group, 3,3-dimethyl-1-butynyl group, and the like.

The "($C_3$-$C_6$)cycloalkyl group" is, for example, a cyclic alkyl group having 3 to 6 carbon atoms such as cyclopropyl group, cyclobutyl group, cyclopentyl group, cyclohexyl group, and the like.

The "($C_1$-$C_6$)alkoxy group" is, for example, a straight chain or branched chain alkoxy group having 1 to 6 carbon atoms such as methoxy group, ethoxy group, normal propoxy group, isopropoxy group, normal butoxy group, secondary butoxy group, tertiary butoxy group, normal pentyloxy group, isopentyloxy group, tertiary pentyloxy group, neopentyloxy group, 2,3-dimethylpropyloxy group, 1-ethylpropyloxy group, 1-methylbutyloxy group, normal hexyloxy group, isohexyloxy group, 1,1,2-trimethylpropyloxy group, and the like.

The "($C_1$-$C_6$)alkylsulfanyl group" is, for example, a straight chain or branched chain alkylsulfanyl group having 1 to 6 carbon atoms such as methylsulfanyl group, ethylsulfanyl group, normal propylsulfanyl group, isopropylsulfanyl group, normal butylsulfanyl group, secondary butylsulfanyl group, tertiary butylsulfanyl group, normal pentylsulfanyl group, isopentylsulfanyl group, tertiary pentylsulfanyl group, neopentylsulfanyl group, 2,3-dimethylpropylsulfanyl group, 1-ethylpropylsulfanyl group, 1-methylbutylsulfanyl group, normal hexylsulfanyl group, isohexyl sulfanyl group, 1,1,2-trimethylpropylsulfanyl group, and the like.

The "($C_1$-$C_6$)alkylsulfinyl group" is, for example, a straight chain or branched chain alkylsulfinyl group having 1 to 6 carbon atoms such as methylsulfinyl group, ethylsulfinyl group, normal propylsulfinyl group, isopropylsulfinyl group, normal butylsulfinyl group, secondary butylsulfinyl group, tertiary butylsulfinyl group, normal pentylsulfinyl group, isopentylsulfinyl group, tertiary pentylsulfinyl group, neopentylsulfinyl group, 2,3-dimethylpropylsulfinyl group, 1-ethylpropylsulfinyl group, 1-methylbutylsulfinyl group, normal hexylsulfinyl group, isohexyl sulfinyl group, 1,1,2-trimethylpropylsulfinyl group, and the like.

The "($C_1$-$C_6$)alkylsulfonyl group" is, for example, a straight chain or branched chain alkylsulfonyl group having 1

to 6 carbon atoms such as methylsulfonyl group, ethylsulfonyl group, normal propylsulfonyl group, isopropylsulfonyl group, normal butylsulfonyl group, secondary butylsulfonyl group, tertiary butylsulfonyl group, normal pentylsulfonyl group, isopentylsulfonyl group, tertiary pentylsulfonyl group, neopentylsulfonyl group, 2,3-dimethylpropylsulfonyl group, 1-ethylpropylsulfonyl group, 1-methylbutylsulfonyl group, normal hexylsulfonyl group, isohexylsulfonyl group, 1,1,2-trimethylpropylsulfonyl group, and the like.

The "($C_1$-$C_6$)alkylamino group" is, for example, a straight chain or branched chain alkylamino group having 1 to 6 carbon atoms such as methylamino group, ethylamino group, normal propylamino group, isopropylamino group, normal butylamino group, isobutylamino group, secondary butylamino group, tertiary butylamino group, normal pentylamino group, isopentylamino group, tertiary pentylamino group, neopentylamino group, normal hexylamino group, isohexylamino group and the like.

The "di($C_1$-$C_6$)alkylamino group" is, for example, a straight chain or branched chain dialkylamino group having 2 to 12 carbon atoms and having two linear or branched alkyl groups each having 1 to 6 carbon atoms, such as N,N-dimethylamino group, N,N-diethylamino group, N,N-di-normal-propylamino group, N,N-diisopropylamino group, N,N-di-normal-butylamino group, N,N-di-secondary-butylamino group, N,N-di-tertiary-butylamino group, N-methyl-N-ethylamino group, N-methyl-N-normal-propylamino group, N-methyl-N-isopropylamino group, N-methyl-N-normal-butylamino group, N-methyl-N-secondary butylamino group, N-methyl-N-tertiary butylamino group, N-methyl-N-normal-pentylamino group, N-methyl-N-isopentylamino group, N-methyl-N-tertiary pentylamino group, N-methyl-N-neopentylamino group, N-methyl-N-(2,3-dimethylpropyl)amino group, N-methyl-N-(1-ethylpropyl)amino group, N-methyl-N-(1-methylbutyl)amino group, N-methyl-N-normal-hexylamino group, N-methyl-N-isohexylamino group, N-methyl-N-(1,1,2-trimethylpropyl)amino group, and the like.

The "($C_1$-$C_6$)alkylcarbonyl group" is, for example, an alkylcarbonyl group having 2 to 7 carbon atoms, for example, an alkylcarbonyl group having the aforementioned ($C_1$-$C_6$)alkyl group, such as acetyl group, propanoyl group, butanoyl group, 2-methylpropanoyl group, pentanoyl group, 2-methylbutanoyl group, 3-methylbutanoyl group, pivaloyl group, hexanoyl group, and the like.

The "($C_1$-$C_6$)alkoxycarbonyl group" is, for example, an alkoxycarbonyl group having 2 to 7 carbon atoms, for example, an alkoxycarbonyl group having the aforementioned ($C_1$-$C_6$)alkoxy group, such as methoxycarbonyl group, ethoxycarbonyl group, normal propoxycarbonyl group, isopropoxycarbonyl group, normal butoxycarbonyl group, isobutoxycarbonyl group, secondary butoxycarbonyl group, tertiary butoxycarbonyl group, pentyloxycarbonyl group, and the like.

The above-mentioned " ($C_1$-$C_6$) alkyl group", " ($C_2$-$C_6$) alkenyl group", " ($C_2$-$C_6$)alkynyl group", " ($C_1$-$C_6$) alkoxy group", "($C_1$-$C_6$)alkylsulfanyl group", "($C_1$-$C_6$)alkylsulfinyl group", "($C_1$-$C_6$)alkylsulfonyl group", "($C_3$-$C_6$)cycloalkyl group", "($C_1$-$C_6$) alkylcarbonyl group", " ($C_1$-$C_6$) alkoxycarbonyl group", "($C_1$-$C_6$)alkylamino group", "di ($C_1$-$C_6$) alkylamino group", and the like may be substituted by one or more halogen atoms at substitutable position(s) and, when substituted by two or more halogen atoms, the halogen atoms may be the same or different.

Each of the above-mentioned groups substituted with one or more halogen atoms is referred to as "halo($C_1$-$C_6$) alkyl group", "halo ($C_2$-$C_6$)alkenyl group", "halo ($C_2$-$C_6$)alkynyl group", "halo ($C_1$-$C_6$) alkoxy group", "halo ($C_1$-$C_6$) alkylsulfanyl group", "halo ($C_1$-$C_6$)alkylsulfinyl group", "halo ($C_1$-$C_6$)alkylsulfonyl group", "halo ($C_3$-$C_6$) cycloalkyl group", "halo($C_1$-$C_6$)alkylcarbonyl group", "halo ($C_1$-$C_6$) alkoxycarbonyl group", "halo ($C_1$-$C_6$) alkylamino group", "dihalo($C_1$-$C_6$) alkylamino group", or the like.

The expressions of "($C_1$-$C_6$)", "($C_2$-$C_6$)", "($C_3$-$C_6$)", and the like show ranges of the carbon atom numbers of various substituents. Furthermore, the above-mentioned definition applies to the groups to which the above-mentioned substituents are bonded. For example, "($C_1$-$C_6$)alkoxy($C_1$-$C_6$)alkyl group" means that a straight chain or branched chain alkoxy group having 1 to 6 carbon atoms is bonded to a straight chain or branched chain alkyl group having 1 to 6 carbon atoms.

When $R^{4a}$ is a ($C_1$-$C_6$)alkyl group or a ($C_1$-$C_6$)alkyl group having 1 to 3 substituents each independently selected from substituent group A (or substituent group $A_0$) (substituted ($C_1$-$C_6$)alkyl group) (substituent group A and substituent group $A_0$ are as defined above), and $R^{4b}$ is a ($C_1$-$C_6$)alkyl group, then $R^{4a}$ and $R^{4b}$ may form a 3- to 6-membered ring together with the adjacent nitrogen atom. Examples of the "3- to 6-membered ring" include aziridine ring, azetidine ring, pyrrolidine ring, piperidine ring, morpholine ring, and the like.

Examples of the salts of the compounds represented by the formulas (1), (1A) and (3) of the present invention include inorganic acid salts such as hydrochloride, sulfate, nitrate, phosphate, and the like, organic acid salts such as acetate, fumarate, maleate, oxalate, methanesulfonate, benzenesulfonate, paratoluenesulfonate, and the like; inorganic base salts such as sodium salt, potassium salt, magnesium salt, calcium salt, and the like, organic base salts such as trimethylammonium salt and the like.

The compounds represented by the formulas (1), (1A), and (3) of the present invention and salts thereof may contain one or plural number of asymmetric centers in the structural formula, and in some cases, two or more optical isomers and diastereomers may be present. The present invention encompasses any of such optical isomers and mixtures containing them at any ratio. In addition, the compounds represented by the formulas (1), (1A), and (3) of the

present invention and salts thereof may have two types of geometric isomers derived from a carbon-carbon double bond in the structural formula. The present invention encompasses all of such geometric isomers and the mixtures containing them at any ratio. Furthermore, the compounds represented by the formulas (1), (1A), and (3) of the present invention and salts thereof may have a plurality of tautomers. The present invention encompasses all of such tautomers and the mixtures containing them at any ratio.

Preferred embodiments of the compounds represented by the formulas (1), (1A), and (3) of the present invention are shown below. A compound represented by the formula (3) is useful as a synthetic intermediate for a compound represented by the formula (1) or (1A).

In the formulas (1), (1A), and (3), X is preferably $CR^{1a}R^{1b}$, $NR^{1c}$, or an oxygen atom.

In the formulas (1), (1A), and (3), Y is preferably $CR^{2a}R^{2b}$, $NR^{2c}$, or $CH_2CH_2$.

In the formula (1), Y is also preferably $CR^{2a}R^{2b}$ or $NR^{2c}$.

In the formulas (1), (1A), and (3), it is also preferable that X is $CR^{1a}R^{1b}$ and Y is $CR^{2a}R^{2b}$.

In the formulas (1), (1A), and (3), it is also preferable that X is an oxygen atom and Y is $CR^{2a}R^{2b}$.

In the formulas (1), (1A), and (3), it is also preferable that X is $NR^{1c}$ and Y is $CR^{2a}R^{2b}$.

In the formulas (1), (1A), and (3), it is also preferable that X is $CR^{1a}R^{1b}$ and Y is $NR^{2c}$.

In the formulas (1), (1A), and (3), it is also preferable that X is $NR^{1c}$ and Y is $CH_2CH_2$.

In the formulas (1), (1A), and (3), it is also preferable that X is $NR^{1c}$ and Y is $NR^{2c}$.

In the formula (1), $R^{1a}$ and $R^{1b}$ are the same or different and each is preferably the above-mentioned group (a1), (a2), (a3), (a4), (a5), (a6), (a7), or (a8), more preferably the above-mentioned group (a1), (a3), (a4), (a6), (a7), or (a8).

In the formula (1A), $R^{1a}$ and $R^{1b}$ are the same or different and each is preferably the above-mentioned group (a1), (a2), or (a3), more preferably the above-mentioned group (a1) or (a3).

In the formula (3), $R^{1a}$ and $R^{1b}$ are the same or different and each is preferably the above-mentioned group (a1), (a2), (a3), (a4), (a5), (a6), (a7), or (a8), more preferably the above-mentioned group (a1), (a3), (a4), (a6), (a7), or (a8).

In the formula (1), $R^{1c}$ is preferably the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29), more preferably the above-mentioned group (b1), (b2), (b10), (b12), (b13), (b14), (b15), (b16), (b17), (b24), (b25), (b28), or (b29).

In the formula (1A), $R^{1c}$ is preferably the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'), more preferably the above-mentioned group (b1), (b2), (b10'), (b12), (b13), (b14), (b15'), (b16), (b17'), (b24), (b25'), (b28), or (b29').

In the formula (3), $R^{1c}$ is preferably the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29), more preferably the above-mentioned group (b1), (b2), (b12), (b14), (b15), (b16), or (b17).

In the formula (1), $R^{2a}$ is preferably the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45), more preferably the above-mentioned group (c1), (c2), (c3), (c4), (c5), (c6), (c7), (c8), (c9), (c10), (c11), (c12), (c13), (c14), or (c15), further preferably the above-mentioned group (c1), (c2), (c3), (c4), (c5), (c12), (c13), (c14), or (c15).

In the formula (1A), $R^{2a}$ is preferably the above-mentioned group (c1), (c2), (c3'), (c4), (c5'), (c6), (c7'), (c8), (c9'), (c10), (c11'), (c12), (c13'), (c14), or (c15'), more preferably the above-mentioned group (c1), (c2), (c3'), (c4), (c5'), (c12), (c13'), (c14), or (c15').

In the formula (3), $R^{2a}$ is preferably the above-mentioned group (c1), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c28), (c29), (c44), or (c45), more preferably the above-mentioned group (c1), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c44), or (c45).

In the formulas (1), (1A), and (3), $R^{2b}$ is preferably the above-mentioned group (d1) or (d2), more preferably the above-mentioned group (d1).

In the formula (1), $R^{2c}$ is preferably the above-mentioned group (e1), (e2), (e3), (e4), (e5), (e6), (e7), (e8), (e9), (e10), (e11), (e12), (e13), (e14), (e15), (e16), (e17), (e20), (e21), (e44), (e45), or (e46), more preferably the above-mentioned group (e1), (e2), (e3), (e4), (e5), (e6), (e7), (e8), (e9), (e44), (e45), or (e46). In another embodiment, $R^{2c}$ is preferably the above-mentioned group (e1), (e2), (e3), (e4), (e5), (e6), (e7), (e8), (e9), (e10), (e11), (e12), (e13), (e14), (e15), (e16), (e17), (e20), or (e21), more preferably the above-mentioned group (e1), (e2), (e3), (e4), (e5), (e6), (e7), (e8), or (e9).

In the formula (1A), $R^{2c}$ is preferably the above-mentioned group (e1), (e2), (e3'), (e4), (e5'), (e6), (e7'), (e8), (e9'), (e10), (e11'), (e12), (e13'), (e14), (e15'), (e16), (e17'), (e20), or (e21'), more preferably the above-mentioned group (e1), (e2), (e3'), (e4), (e5'), (e6), (e7'), (e8), or (e9').

In the formula (3), $R^{2c}$ is preferably the above-mentioned group (e1), (e2), (e3), (e4), (e5), (e6), (e7), (e8), (e9), (e10), (e11), (e12), (e13), (e14), (e15), (e16), (e17), (e20), or (e21), more preferably the above-mentioned group (e1), (e4), (e5), (e44), or (e45).

In the formula (1), $R^{3a}$ is preferably the above-mentioned group (f1), (f2), (f4), (f5), (f6), (f7), (f8), (f9), (f10), or (f11), more preferably the above-mentioned group (f1), (f5), (f6), (f7), (f8), (f9), (f10), or (f11), further preferably the above-mentioned group (fl), (f6), or (f7).

In the formula (1A), $R^{3a}$ is preferably the above-mentioned group (f1), (f2), (f4), (f5'), (f6), or (f7'), more preferably the above-mentioned group (fl), (f6), or (f7').

In the formula (3), $R^{3a}$ is preferably the above-mentioned group (f1), (f2), (f4), (f5), (f6), (f7), (f8), (f9), (f10), or (f11), more preferably the above-mentioned group (f1), (f5), (f6), (f7), (f8), (f9), (f10), or (f11), further preferably the above-mentioned group (f1), (f6), or (f7).

In the formulas (1), (1A), and (3), $R^{3b}$ is preferably the above-mentioned group (g1), (g2), or (g3), more preferably the above-mentioned group (g1) or (g2).

In the formula (3), $R^{1a}$, $R^{1b}$, $R^{2a}$, $R^{2b}$, $R^{3a}$, and $R^{3b}$ are not hydrogen atoms at the same time, and X is not $NR^{1c}$ when Y is $NR^{2c}$,

In the formula (1), $R^{4a}$ is preferably the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51), more preferably the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50), further preferably the above-mentioned group (h1), (h2), (h3), (h4), (h7), (h8), (h9), (h14), (h15), (h16), (h17), (h18), (h21), (h22), (h23), (h24), (h25), (h26), (h27), (h28), (h29), (h30), (h31), (h32), (h37), (h38), (h39), (h40), (h41), (h42), (h45), (h46), (h47), (h48), (h49), or (h50).

In the formula (1A), $R^{4a}$ is preferably the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50), more preferably the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50).

In the formula (1), $R^{4b}$ is preferably the above-mentioned group (i1), (i2), or (i3), more preferably the above-mentioned group (i1) or (i2).

When $R^{4a}$ is (h4) a $(C_1-C_6)$alkyl group or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ also preferably form a 3- to 6-membered ring together with the adjacent nitrogen atom. When $R^{4a}$ is (h4) a $(C_1-C_6)$ alkyl group, and $R^{4b}$ is (i2) a $(C_1-C_6)$ alkyl group, then $R^{4a}$ and $R^{4b}$ also more preferably form a 3- to 6-membered ring together with the adjacent nitrogen atom.

In the formula (1A), $R^{4b}$ is preferably the above-mentioned group (i1), (i2), or (i3), more preferably the above-mentioned group (i1) or (i2).

When $R^{4a}$ is (h4) a $(C_1-C_6)$alkyl group or (h14') a $(C_1-C_6)$alkyl group having 1 to 3 substituents each independently selected from substituent group $A_0$, and $R^{4b}$ is (i2) a $(C_1-C_6)$alkyl group, then $R^{4a}$ and $R^{4b}$ also preferably form a 3- to 6-membered ring together with the adjacent nitrogen atom. When $R^{4a}$ is (h4) a $(C_1-C_6)$ alkyl group, and $R^{4b}$ is (i2) a $(C_1-C_6)$ alkyl group, then $R^{4a}$ and $R^{4b}$ also more preferably form a 3- to 6-membered ring together with the adjacent nitrogen atom.

In the formula (1), $R^5$ is preferably the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51), more preferably the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51). In another preferred embodiment, $R^5$ is preferably the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50), more preferably the above-mentioned group (h1), (h4), (h7), (h9), (h14), (h15), (h16), (h17), (h18), (h29), or (h30).

In the formula (1A), $R^5$ is preferably the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'), more preferably the above-mentioned group (h1), (h4), (h7), (h9), (h14'), (h15), (h16'), (h17), (h18'), (h29), or (h30').

In the formula (3), $R^5$ is preferably the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51), more preferably the above-mentioned group (h1), (h4), (h5), (h6), (h7), (h9), (h14), (h15), (h16), (h17), (h18), (h19), (h20), (h21), (h22), (h27), (h28), (h29), (h30), (h37), (h38), or (h51).

In the formula (1), Z is preferably an oxygen atom, a sulfur atom, or $NR^7$, more preferably an oxygen atom.

In the formula (1), $R^7$ is preferably a hydroxyl group, a $(C_1\text{-}C_6)$alkoxy group, a halo $(C_1\text{-}C_6)$alkoxy group, a $(C_1\text{-}C_6)$ alkyl group, a halo $(C_1\text{-}C_6)$ alkyl group, a $(C_3\text{-}C_6)$cycloalkyl group, an amino group, a $(C_1\text{-}C_6)$alkylamino group, or a di$(C_1\text{-}C_6)$alkylamino group (the two $(C_1\text{-}C_6)$alkyl groups may be the same or different), more preferably a hydroxyl group or a $(C_1\text{-}C_6)$alkoxy group.

In the formula (3), $R^6$ is preferably the above-mentioned group (m1), (m2), (m3), (m4), (m5), or (m6), more preferably the above-mentioned group (m1), (m5), or (m6).

In the formula (1), the substituent group A preferably consists of the above-mentioned groups (j1), (j2), (j3), (j4), (j5), (j6), (j7), (j8), and (j10), more preferably the above-mentioned groups (j1), (j2), (j3), and (j8).

In the formula (1A), the substituent group $A_0$ preferably consists of the above-mentioned groups (j1), (j2), (j3), (j4), (j5), (j6), (j7), (j8), and (j10), more preferably the above-mentioned groups (j1), (j2), (j3), and (j8).

In the formula (1), the substituent group B preferably consists of the above-mentioned groups (k1), (k2), (k7), (k10), (k11), (k12), (k13), (k14), (k17), (k20), (k22), (k23), (k24), (k26), (k27), (k28) and (k29), more preferably the above-mentioned groups (k1), (k2), (k7), (k10), (k17), (k20), (k26), (k27), (k28) and (k29).

In the formula (1A), the substituent group $B_0$ preferably consists of the above-mentioned groups (k1), (k2), (k7), (k10), (k11), (k12), (k13), (k14), (k17), (k20), (k22), (k23), and (k24), more preferably the above-mentioned groups (k1), (k2), (k10), and (k17).

In the formula (1), the substituent group C preferably consists of the above-mentioned groups (11), (12), (17), (110), (111), (112), (113), (114), (117), (120), (122), (123), and (124), more preferably the above-mentioned groups (11), (17), (110), and (120).

In the formula (1A), the substituent group $C_0$ preferably consists of the above-mentioned groups (11), (12), (17), (110), (111), (112), (113), (l14), (117), (120), (122), (123), and (124), more preferably the above-mentioned groups (11), (17), (l10), and (120).

In the formula (3), the substituent group A preferably consists of the above-mentioned groups (j1), (j2), (j3), (j4), (j5), (j6), (j7), (j8), and (j10), more preferably the above-mentioned groups (j1), (j2), (j3), and (j8).

In the formula (3), the substituent group B preferably consists of the above-mentioned groups (k1), (k2), (k7), (k10), (k11), (k12), (k13), (k14), (k17), (k20), (k22), (k23), (k24), (k26), (k27), (k28), and (k29), more preferably the above-mentioned groups (k1), (k2), (k7), (k10), (k17), (k26), and (k27) .

In the formula (3), the substituent group C preferably consists of the above-mentioned groups (11), (12), (17), (110), (111), (112), (113), (114), (117), (120), (122), (123), and (124), more preferably the above-mentioned groups (11), (17), (110), and (120).

As compound (1), the following compounds are preferred.

[Compound (1-0-i)]

Compound (1) wherein

X is $CR^{1a}R^{1b}$, $NR^{1c}$, or an oxygen atom,
$R^{1a}$ and $R^{1b}$ are the same or different and each is the above-mentioned group (a1), (a2), (a3), (a4), (a5), (a6), (a7), or (a8),
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CR^{2a}R^{2b}$, $NR^{2c}$, or $CH_2CH_2$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{2c}$ is the above-mentioned group (e1), (e2), (e3), (e4), (e5), (e6), (e7), (e8), (e9), (e10, (e11), (e12), (e13), (e14), (e15), (e16), (e17), (e20), (e21), (e44), (e45), or (e46),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
provided that $R^{1a}$, $R^{1b}$, $R^{2a}$, $R^{2b}$, $R^{3a}$, and $R^{3b}$ are not hydrogen atoms at the same time,
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1\text{-}C_6)$ alkyl group or (h14) a $(C_1\text{-}C_6)$ alkyl group having 1 to 3 substituents each independently selected from substituent group A, and $R^{4b}$ is (i2) a $(C_1\text{-}C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16),

(h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1-C_6)$alkoxy group,
or a salt thereof.

[Compound (1-0-ii)]

Compound (1) wherein

X is $CR^{1a}R^{1b}$, $NR^{1c}$, or an oxygen atom,
$R^{1a}$ and $R^{1b}$ are the same or different and each is the above-mentioned group (a1), (a3), (a4), (a6), (a7), or (a8),
$R^{1c}$ is the above-mentioned group (b1), (b2), (b10), (b12), (b13), (b14), (b15), (b16), (b17), (b24), (b25), (b28), or (b29),
Y is $CR^{2a}R^{2b}$, $NR^{2c}$, or $CH_2CH_2$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), (f7), (f8), (f9), (f10), or (f11),
$R^{3b}$ is the above-mentioned group (g1) or (g2),
provided that $R^{1a}$, $R^{1b}$, $R^{2a}$, $R^{2b}$, $R^{3a}$, and $R^{3b}$ are not hydrogen atoms at the same time,
$R^{4a}$ is the above-mentioned group (h1), (h2), (h3), (h4), (h7), (h8), (h9), (h14), (h15), (h16), (h17), (h18), (h21), (h22), (h23), (h24), (h25), (h26), (h27), (h28), (h29), (h30), (h31), (h32), (h37), (h38), (h39), (h40), (h41), (h42), (h45), (h46), (h47), (h48), (h49), or (h50),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$ alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38),
or (h51), and
Z is an oxygen atom,
or a salt thereof.

[Compound (1-0-iii)]

Compound (1) wherein

X is $CR^{1a}R^{1b}$, $NR^{1c}$, or an oxygen atom,
$R^{1a}$ and $R^{1b}$ are the same or different and each is the above-mentioned group (a1), (a2), (a3), (a4), (a5), (a6), (a7), or (a8),
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CR^{2a}R^{2b}$ or $NR^{2c}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{2c}$ is the above-mentioned group (e1), (e2), (e3), (e4), (e5), (e6), (e7), (e8), (e9), (e10), (e11), (e12), (e13), (e14), (e15), (e16), (e17), (e20), (e21), (e44), (e45), or (e46),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
provided that $R^{1a}$, $R^{1b}$, $R^{2a}$, $R^{2b}$, $R^{3a}$, and $R^{3b}$ are not hydrogen atoms at the same time,
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$alkyl group or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered

ring together with the adjacent nitrogen atom,
$R^S$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1-C_6)$alkoxy group,
or a salt thereof.

[Compound (1-0-iv)]

Compound (1) wherein

X is $CR^{1a}R^{1b}$, $NR^{1c}$, or an oxygen atom,
$R^{1a}$ and $R^{1b}$ are the same or different and each is the above-mentioned group (a1), (a3), (a4), (a6), (a7), or (a8),
$R^{1c}$ is the above-mentioned group (b1), (b2), (b10), (b12), (b13), (b14), (b15), (b16), (b17), (b24), (b25), (b28), or (b29),
Y is $CR^{2a}R^{2b}$ or $NR^{2c}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), (f7), (f8), (f9), (f10), or (f11),
$R^{3b}$ is the above-mentioned group (g1) or (g2),
provided that $R^{1a}$, $R^{1b}$, $R^{2a}$, $R^{2b}$, $R^{3a}$, and $R^{3b}$ are not hydrogen atoms at the same time,
$R^{4a}$ is the above-mentioned group (h1), (h2), (h3), (h4), (h7), (h8), (h9), (h14), (h15), (h16), (h17), (h18), (h21), (h22), (h23), (h24), (h25), (h26), (h27), (h28), (h29), (h30), (h31), (h32), (h37), (h38), (h39), (h40), (h41), (h42), (h45), (h46), (h47), (h48), (h49), or (h50),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h23), (h24), (h27), (h28), (h29), (h30), (h37), (h38), or (h51), and
Z is an oxygen atom,
or a salt thereof.

[Compound (1-i)]

Compound (1) wherein

X is $CR^{1a}R^{1b}$,
$R^{1a}$ and $R^{1b}$ are the same or different and each is the above-mentioned group (a1), (a2), (a3), (a4), (a6), (a7), or (a8),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$ alkyl group or (h14) a $(C_1-C_6)$ alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and

$R^7$ is a hydroxyl group, a $(C_1-C_6)$alkoxy group, a halo$(C_1-C_6)$alkoxy group, a $(C_1-C_6)$alkyl group, a halo$(C_1-C_6)$alkyl group, a $(C_3-C_6)$cycloalkyl group, an amino group, a $(C_1-C_6)$alkylamino group, or a di$(C_1-C_6)$alkylamino group (the two $(C_1-C_6)$alkyl groups may be the same or different),
or a salt thereof.

[Compound (1-ii)]

Compound (1) wherein

X is an oxygen atom,
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$alkyl group or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group, a $(C_1-C_6)$alkoxy group, a halo$(C_1-C_6)$alkoxy group, a $(C_1-C_6)$alkyl group, a halo $(C_1-C_6)$ alkyl group, a $(C_3-C_6)$cycloalkyl group, an amino group, a $(C_1-C_6)$alkylamino group, or a di$(C_1-C_6)$alkylamino group (the two $(C_1-C_6)$alkyl groups may be the same or different),
or a salt thereof.

[Compound (1-iii)]

Compound (1) wherein

X is $NR^{1c}$,
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), or (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$alkyl group or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group, a $(C_1-C_6)$alkoxy group, a halo$(C_1-C_6)$alkoxy group, a $(C_1-C_6)$alkyl group, a halo$(C_1-C_6)$alkyl group, a $(C_3-C_6)$cycloalkyl group, an amino group, a $(C_1-C_6)$alkylamino group,

or a di($C_1$-$C_6$)alkylamino group (the two ($C_1$-$C_6$)alkyl group may be the same or different),
or a salt thereof.

[Compound (1-iv)]

Compound (1) wherein

X is $CR^{1a}R^{1b}$,
$R^{1a}$ and $R^{1b}$ are the same or different and each is the above-mentioned group (a1), (a2), (a3), (a4), (a5), (a6), (a7), or (a8),
Y is $NR^{2c}$,
$R^{2c}$ is the above-mentioned group (e1), (e2), (e3), (e4), (e5), (e6), (e7), (e8), (e9), (e10), (e11), (e12), (e13), (e14), (e15), (e16), (e17), (e20), (e21), (e44), (e45), or (e46),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a ($C_1$-$C_6$) alkyl group or (h14) a ($C_1$-$C_6$) alkyl group having 1 to 3 substituents each independently selected from substituent group A, and $R^{4b}$ is (i2) a ($C_1$-$C_6$)alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group, a ($C_1$-$C_6$)alkoxy group, a halo($C_1$-$C_6$)alkoxy group, a ($C_1$-$C_6$)alkyl group, a halo($C_1$-$C_6$)alkyl group, a ($C_3$-$C_6$)cycloalkyl group, an amino group, a ($C_1$-$C_6$)alkylamino group,
or di($C_1$-$C_6$)alkylamino group (the two ($C_1$-$C_6$)alkyl group may be the same or different),
or a salt thereof.

[Compound (1-v)]

Compound (1) wherein

X is $NR^{1c}$,
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CH_2CH_2$,
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a ($C_1$-$C_6$)alkyl group or (h14) a ($C_1$-$C_6$)alkyl group having 1 to 3 substituents each independently selected from substituent group A, and $R^{4b}$ is (i2) a ($C_1$-$C_6$)alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group, a ($C_1$-$C_6$)alkoxy group, a halo($C_1$-$C_6$)alkoxy group, a ($C_1$-$C_6$)alkyl group, a halo($C_1$-$C_6$)alkyl group, a ($C_3$-$C_6$)cycloalkyl group, an amino group, a ($C_1$-$C_6$)alkylamino group,
or a di($C_1$-$C_6$)alkylamino group (the two ($C_1$-$C_6$)alkyl group may be the same or different),
or a salt thereof.

[Compound (1-vi)]

Compound (1) wherein

X is $NR^{1c}$,
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $NR^{2c}$,
$R^{2c}$ is the above-mentioned group (e1), (e2), (e3), (e4), (e5), (e6), (e7), (e8), (e9), (e10), (e11), (e12), (e13), (e14), (e15), (e16), (e17), (e20), (e21), (e44), (e45), or (e46),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$alkyl group or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group, a $(C_1-C_6)$alkoxy group, a halo$(C_1-C_6)$alkoxy group, a $(C_1-C_6)$alkyl group, a halo$(C_1-C_6)$alkyl group, a $(C_3-C_6)$cycloalkyl group, an amino group, a $(C_1-C_6)$alkylamino group,
or a di$(C_1-C_6)$alkylamino group (the two $(C_1-C_6)$alkyl group may be the same or different),
or a salt thereof.

[Compound (1-vii)]

Compound (1) wherein
X is $CR^{1a}R^{1b}$,

$R^{1a}$ and $R^{1b}$ are the same or different and each is the above-mentioned group (a1), (a3), (a4), (a6), (a7), or (a8),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ is the above-mentioned group (f1), (f5), (f6), or (f7), (f8), (f9), (f10), or (f11),
$R^{3b}$ is the above-mentioned group (g1) or (g2),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$ alkyl group or (h14) a $(C_1-C_6)$ alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1-C_6)$alkoxy group,
or a salt thereof.

[Compound (1-viii)]

Compound (1) wherein

X is an oxygen atom,
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ is the above-mentioned group (f1), (f5), (f6), or (f7), (f8), (f9), (f10), or (f11),
$R^{3b}$ is the above-mentioned group (g1) or (g2),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$alkyl group or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1-C_6)$alkoxy group,
or a salt thereof.

[Compound (1-ix)]

Compound (1) wherein

X is $NR^{1c}$,
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ is the above-mentioned group (f1), (f5), (f6), or (f7), (f8), (f9), (f10), or (f11),
$R^{3b}$ is the above-mentioned group (g1) or (g2),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1-C_6)$alkyl group or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1-C_6)$alkoxy group,
or a salt thereof.

[Compound (1-x)]

Compound (1) wherein

X is $CR^{1a}R^{1b}$,
$R^{1a}$ and $R^{1b}$ are the same or different and each is the above-mentioned group (a1), (a3), (a4), (a6), (a7), or (a8),
Y is $NR^{2c}$,
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), or (f7), (f8), (f9), (f10), or (f11),
$R^{3b}$ is the above-mentioned group (g1) or (g2),

$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1\text{-}C_6)$alkyl group or (h14) a $(C_1\text{-}C_6)$alkyl group having 1 to 3 substituents each independently selected from substituent group A, and $R^{4b}$ is (i2) a $(C_1\text{-}C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1\text{-}C_6)$alkoxy group,
or a salt thereof.

[Compound (1-xi)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $CH_2CH_2$,
$R^{3a}$ is the above-mentioned group (f1), (f5), (f6), or (f7), (f8), (f9), (f10), or (f11),
$R^{3b}$ is the above-mentioned group (g1) or (g2),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1\text{-}C_6)$ alkyl group or (h14) a $(C_1\text{-}C_6)$ alkyl group having 1 to 3 substituents each independently selected from substituent group A, and $R^{4b}$ is (i2) a $(C_1\text{-}C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1\text{-}C_6)$alkoxy group,
or a salt thereof.

[Compound (1-xii)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $NR^{2c}$,
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), or (f7), (f8), (f9), (f10), or (f11),
$R^{3b}$ is the above-mentioned group (g1) or (g2),
$R^{4a}$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51),
$R^{4b}$ is the above-mentioned group (i1), (i2), or (i3),
when $R^{4a}$ is (h4) a $(C_1\text{-}C_6)$ alkyl group or (h14) a $(C_1\text{-}C_6)$ alkyl group having 1 to 3 substituents each independently selected from substituent group A, and $R^{4b}$ is (i2) a $(C_1\text{-}C_6)$alkyl group, then $R^{4a}$ and $R^{4b}$ may form a 3- to 6-membered ring together with the adjacent nitrogen atom,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38),

(h39), (h40), (h41), (h42), (h43), (h44), (h45), (h46), (h47), (h48), (h49), (h50), or (h51),
Z is an oxygen atom, a sulfur atom, or $NR^7$, and
$R^7$ is a hydroxyl group or a $(C_1-C_6)$alkoxy group,
or a salt thereof.

[Compound (1-xiii)]

Compound (1) wherein

X is $CR^{1a}R^{1b}$,
$R^{1a}$ and $R^{1b}$ are the same or different and each is the above-mentioned group (a1), (a2), (a3), (a4), (a5), (a6), (a7), or (a8),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xiv)]

Compound (1) wherein

X is an oxygen atom,
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_8)$alkyl group, and $R^{4b}$ is (i2) a $(C_1-C_6)$ alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xv)]

Compound (1) wherein

X is $NR^{1c}$,

$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xvi)]

Compound (1) wherein
X is $CR^{1a}R^{1b}$,

$R^{1a}$ and $R^{1b}$ are the same or different and each is the above-mentioned group (a1), (a2), (a3), (a4), (a5), (a6), (a7), or (a8),
Y is $NR^{2c}$,
$R^{2c}$ is the above-mentioned group (e1), (e2), (e3), (e4), (e5), (e6), (e7), (e8), (e9), (e10), (e11), (e12), (e13), (e14), (e15), (e16), (e17), (e20), (e21), (e44), (e45), or (e46),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_6)$ alkyl group, and $R^{4b}$ is (i2) a $(C_1-C_6)$ alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xvii)]

Compound (1) wherein

X is $NR^{1c}$,
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CH_2CH_2$,
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),

when $R^{4a}$ is (h4) a ($C_1$-$C_6$)alkyl group, and $R^{4b}$ is (i2) a ($C_1$-$C_6$)alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a ($C_1$-$C_6$)alkoxy group,
or a salt thereof.

[Compound (1-xviii)]

Compound (1) wherein

X is $NR^{1c}$,
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $NR^{2c}$,
$R^{2c}$ is the above-mentioned group (e1), (e2), (e3), (e4), (e5), (e6), (e7), (e8), (e9), (e10), (e11), (e12), (e13), (e14), (e15), (e16), (e17), (e20), (e21), (e44), (e45), or (e46),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a ($C_1$-$C_6$)alkyl group, and $R^{4b}$ is (i2) a ($C_1$-$C_6$)alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a ($C_1$-$C_6$)alkoxy group,
or a salt thereof.

[Compound (1-xix)]

Compound (1) wherein

X is an oxygen atom,
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ i s the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a ($C_1$-$C_6$)alkyl group, and $R^{4b}$ is (i2) a ($C_1$- $C_6$)alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a ($C_1$-$C_6$)alkoxy group,
or a salt thereof.

[Compound (1-xx)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1\text{-}C_6)$alkyl group, and $R^{4b}$ is (i2) a $(C_1\text{-}C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1\text{-}C_6)$alkoxy group,
or a salt thereof.

[Compound (1-xxi)]

Compound (1) wherein

X is $CR^{1a}R^{1b}$,
$R^{1a}$ and $R^{1b}$ are the same or different and each is the above-mentioned group (a1), (a3), (a4), (a6), (a7), or (a8),
Y is $NR^{2c}$,
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1\text{-}C_6)$alkyl group, and $R^{4b}$ is (i2) a $(C_1\text{-}C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1\text{-}C_6)$alkoxy group,
or a salt thereof.

[Compound (1-xxii)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $CH_2CH_2$,
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1\text{-}C_6)$alkyl group, and $R^{4b}$ is (i2) a $(C_1$-

$C_6$) alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a ($C_1$-$C_6$)alkoxy group,
or a salt thereof.

[Compound (1-xxiii)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $NR^{2c}$,
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a ($C_1$-$C_6$)alkyl group, and $R^{4b}$ is (i2) a ($C_1$-$C_6$)alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a ($C_1$-$C_6$)alkoxy group,
or a salt thereof.

[Compound (1-xxiv)]

Compound (1) wherein

X is $CR^{1a}R^{1b}$,
$R^{1a}$ and $R^{1b}$ are the same or different and each is the above-mentioned group (a1), (a3), (a4), (a6), (a7), or (a8),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a ($C_1$-$C_6$)alkyl group, and $R^{4b}$ is (i2) a ($C_1$-
$C_6$) alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a ($C_1$-$C_6$)alkoxy group,
or a salt thereof.

[Compound (1-xxv)]

Compound (1) wherein

X is an oxygen atom,
Y is $CR^{2a}R^{2b}$,
$R^{23}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_6)$ alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xxvi)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $CR^{2a}R^{2b}$,
$R^{2a}$ is the above-mentioned group (c1), (c2), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_6)$ alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xxvii)]

Compound (1) wherein

X is $CR^{1a}R^{1b}$,
$R^{1a}$ and $R^{1b}$ are the same or different and each is the above-mentioned group (a1), (a3), (a4), (a6), (a7), or (a8),
Y is $NR^{2c}$,
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),

when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xxviii)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $CH_2CH_2$,
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_6)$ alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

[Compound (1-xxix)]

Compound (1) wherein

X is $NR^{1c}$,
$R^{1c}$ is the above-mentioned group (b1), (b2), (b10), (b12), (b13), (b14), (b15), (b16), (b17), (b24), (b25), (b28), or (b29),
Y is $NR^{2c}$,
$R^{2c}$ is the above-mentioned group (e1), (e2), (e3), (e4), (e5), (e6), (e7), (e8), (e9), (e44), (e45), or (e46),
$R^{3a}$ is the above-mentioned group (f1), (f5), (f6), (f7), (f8), (f9), (f10), or (f11),
$R^{3b}$ is the above-mentioned group (g1) or (g2),
$R^{4a}$ is the above-mentioned group (h1), (h2), (h3), (h4), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), or (h50),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom,
$R^5$ is the above-mentioned group (h1), (h4), (h5), (h6), (h7), (h9), (h14), (h15), (h16), (h17), (h18), (h19), (h20), (h21), (h22), (h23), (h24), (h27), (h28), (h29), (h30), (h37), (h38), or (h51),
Z is an oxygen atom, a sulfur atom, or $NR^7$, and
$R^7$ is a hydroxyl group or a $(C_1-C_6)$alkoxy group,
or a salt thereof.

As compound (1A), the following compounds are preferred.

[Compound (1A-0-i)]

Compound (1A) wherein

X is $CR^{1a}R^{1b}$, $NR^{1c}$, or an oxygen atom,
$R^{1a}$ and $R^{1b}$ are the same or different and each is the above-mentioned group (a1), (a2), or (a3),
Y is $CR^{2a}R^{2b}$, $NR^{2c}$, or $CH_2CH_2$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
$R^{2a}$ is the above-mentioned group (c1), (c2), (c3'), (c4), (c5'), (c6), (c7'), (c8), (c9'), (c10), (c11'), (c12), (c13'), (c14), or (c15'),
$R^{2b}$ is the above-mentioned group (d1) or (d2),
$R^{2c}$ is the above-mentioned group (e1), (e2), (e3'), (e4), (e5'), (e6), (e7'), (e8), (e9'), (e10), (e11'), (e12), (e13'), (e14), (e15'), (e16), (e17'), (e20), or (e21'),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a $(C_1\text{-}C_6)$alkyl group or (h14') a $(C_1\text{-}C_6)$ alkyl group having 1 to 3 substituents each independently selected from substituent group $A_0$, and $R^{4b}$ is (i2) a $(C_1\text{-}C_6)$alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-0-ii)]

Compound (1A) wherein

X is $CR^{1a}R^{1b}$, $NR^{1c}$, or an oxygen atom,
$R^{1a}$ and $R^{1b}$ are the same or different and each is the above-mentioned group (a1) or (a3),
$R^{1c}$ is the above-mentioned group (b1), (b2), (b10'), (b12), (b13), (b14), (b15'), (b16), (b17'), (b24), (b25'), (b28), or (b29'),
Y is $CR^{2a}R^{2b}$, $NR^{2c}$, or $CH_2CH_2$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{2c}$ is the above-mentioned group (e1), (e2), (e3'), (e4), (e5'), (e6), (e7'), (e8), or (e9'),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1\text{-}C_6)$alkyl group, and $R^{4b}$ is (i2) a $(C_1\text{-}C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-i)]

Compound (1A) wherein

X is $CR^{1a}R^{1b}$,
$R^{1a}$ and $R^{1b}$ are the same or different and each is the above-mentioned group (a1), (a2), or (a3),
Y is $CR^{2a}R^{2b}$,
$R^{2a}$ is the above-mentioned group (c1), (c2), (c3'), (c4), (c5'), (c6), (c7'), (c8), (c9'), (c10), (c11'), (c12), (c13'), (c14), or (c15'),
$R^{2b}$ is the above-mentioned group (d1) or (d2),
$R^{3a}$ is the above-mentioned group (f1), (f2), (f4), (f5'), (f6), or (f7'),

$R^{3b}$ is the above-mentioned group (g1), (g2), or (g3),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-ii)]

Compound (1A) wherein

X is an oxygen atom,
Y is $CR^{2a}R^{2b}$,
$R^{2a}$ is the above-mentioned group (c1), (c2), (c3'), (c4), (c5'), (c6), (c7'), (c8), (c9'), (c10), (c11'), (c12), (c13'), (c14), or (c15'),
$R^{2b}$ is the above-mentioned group (d1) or (d2),
$R^{3a}$ is the above-mentioned group (f1), (f2), (f4), (f5') (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3)
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-iii)]

Compound (1A) wherein

X is $NR^{1c}$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
Y is $CR^{2a}R^{2b}$,
$R^{2a}$ is the above-mentioned group (c1), (c2), (c3'), (c4), (c5'), (c6), (c7'), (c8), (c9'), (c10), (c11'), (c12), (c13'), (c14), or (c15'),
$R^{2b}$ is the above-mentioned group (d1) or (d2),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39),

(h40'), (h41), (h42'), (h43), (h44'), (h45), (h46'), (h47), (h48'), (h49), or (h50'), or a salt thereof.

[Compound (1A-iv)]

Compound (1A) wherein

X is $CR^{1a}R^{1b}$,
$R^{1a}$ and $R^{1b}$ are the same or different and each is the above-mentioned group (a1), (a2), or (a3),
Y is $NR^{2c}$,
$R^{2c}$ is the above-mentioned group (e1), (e2), (e3'), (e4), (e5'), (e6), (e7'), (e8), (e9'), (e10), (e11'), (e12), (e13'), (e14), (e15'), (e16), (e17'), (e20), or (e21'),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-v)]

Compound (1A) wherein

X is $NR^{1c}$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
Y is $CH_2CH_2$,
$R^{3a}$ is the above-mentioned group (f1) , (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-vi)]

Compound (1A) wherein

X is $NR^{1c}$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
Y is $NR^{2c}$,
$R^{2c}$ is the above-mentioned group (e1), (e2), (e3'), (e4), (e5'), (e6), (e7'), (e8), (e9'), (e10), (e11'), (e12), (e13'), (e14), (e15'), (e16), (e17'), (e20), or (e21'),

$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$)alkyl group or (h14') a ($C_1$-$C_6$)alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-vii)]

Compound (1A) wherein

X is $CR^{1a}R^{1b}$,
$R^{1a}$ and $R^{1b}$ are the same or different and each is the above-mentioned group (a1) or (a3),
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$)alkyl group or (h14') a ($C_1$-$C_6$)alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-viii)]

Compound (1A) wherein

X is an oxygen atom,
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$)alkyl group or (h14') a ($C_1$-$C_6$)alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-ix)]

Compound (1A) wherein

X is $NR^{1c}$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-x)]

Compound (1A) wherein

X is $CR^{1a}R^{1b}$,
$R^{1a}$ and $R^{1b}$ are the same or different and each is the above-mentioned group (a1) or (a3),
Y is $NR^{2c}$,
$R^{2c}$ is the above-mentioned group (e1), (e2), (e3'), (e4), (e5'), (e6), (e7'), (e8), or (e9'),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-xi)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $CH_2CH_2$,
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39),

(h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-xii)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $NR^{2c}$,
$R^{2c}$ is the above-mentioned group (e1), (e2), (e3'), (e4), (e5'), (e6), (e7'), (e8), or (e9'),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
$R^{4b}$ is the above-mentioned group (i1), (i2), or (i3),
when $R^{4a}$ is (h4) a ($C_1$-$C_6$) alkyl group or (h14') a ($C_1$-$C_6$) alkyl group having 1 to 3 substituents each independently selected from substituent group $A_0$, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h2), (h3), (h4), (h7), (h8), (h9), (h12), (h13), (h14'), (h15), (h16'), (h17), (h18'), (h19), (h20'), (h21), (h22'), (h23), (h24'), (h25), (h26'), (h27), (h28'), (h29), (h30'), (h31), (h32'), (h37), (h38'), (h39), (h40'), (h41), (h42'), (h43), (h44'), (h45), (h46'), (h47), (h48'), (h49), or (h50'),
or a salt thereof.

[Compound (1A-xiii)]

Compound (1A) wherein

X is $CR^{1a}R^{1b}$,
$R^{1a}$ and $R^{1b}$ are the same or different and each is the above-mentioned group (a1), (a2), or (a3),
Y is $CR^{2a}R^{2b}$,
$R^{2a}$ is the above-mentioned group (c1), (c2), (c3'), (c4), (c5'), (c6), (c7'), (c8), (c9'), (c10), (c11'), (c12), (c13'), (c14), or (c15'),
$R^{2b}$ is the above-mentioned group (d1) or (d2),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a ($C_1$-$C_6$)alkyl group, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xiv)]

Compound (1A) wherein

X is an oxygen atom,
Y is $CR^{2a}R^{2b}$,
$R^{2a}$ is the above-mentioned group (c1), (c2), (c3'), (c4), (c5'), (c6), (c7'), (c8), (c9'), (c10), (c11'), (c12), (c13'), (c14), or (c15'),
$R^{2b}$ is the above-mentioned group (d1) or (d2),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xv)]

Compound (1A) wherein

X is $NR^{1c}$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
Y is $CR^{2a}R^{2b}$,
$R^{2a}$ is the above-mentioned group (c1), (c2), (c3'), (c4), (c5'), (c6), (c7'), (c8), (c9'), (c10), (c11'), (c12), (c13'), (c14), or (c15'),
$R^{2b}$ is the above-mentioned group (d1) or (d2),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$ alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xvi)]

Compound (1A) wherein

X is $CR^{1a}R^{1b}$,
$R^{1a}$ and $R^{1b}$ are the same or different and each is the above-mentioned group (a1), (a2), or (a3),
Y is $NR^{2c}$,
$R^{2c}$ is the above-mentioned group (e1), (e2), (e3'), (e4), (e5'), (e6), (e7'), (e8), (e9'), (e10), (e11'), (e12), (e13'), (e14), (e15'), (e16), (e17'), (e20), or (e21'),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xvii)]

Compound (1A) wherein

X is $NR^{1c}$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
Y is $CH_2CH_2$,
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a ($C_1$-$C_6$) alkyl group, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xviii)]

Compound (1A) wherein

X is $NR^{1c}$,
$R^{1c}$ is the above-mentioned group (b1), (b2), (b5), (b6), (b9), (b10'), (b12), (b13), (b14), (b15'), (b16), (b17'), (b18), (b19'), (b20), (b21'), (b22), (b23'), (b24), (b25'), (b26), (b27'), (b28), or (b29'),
Y is $NR^{2c}$,
$R^{2c}$ is the above-mentioned group (e1), (e2), (e3'), (e4), (e5'), (e6), (e7'), (e8), (e9'), (e10), (e11'), (e12), (e13'), (e14), (e15'), (e16), (e17'), (e20), or (e21'),
$R^{3a}$ is the above-mentioned group (f1), (f2), (f4), (f5'), (f6), or (f7'),
$R^{3b}$ is the above-mentioned group (g1), (g2), or (g3),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a ($C_1$-$C_6$)alkyl group, and $R^{4b}$ is (i2) a ($C_1$-$C_6$)alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xix)]

Compound (1A) wherein

X is an oxygen atom,
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a ($C_1$-$C_6$)alkyl group, and $R^{4b}$ is (i2) a ($C_1$-$C_6$) alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xx)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxi)]

Compound (1A) wherein

X is $CR^{1a}R^{1b}$,
$R^{1a}$ and $R^{1b}$ are the same or different and each is the above-mentioned group (a1) or (a3),
Y is $NR^{2c}$,
$R^{2c}$ is the above-mentioned group (e1), (e2), (e3'), (e4), (e5'), (e6), (e7'), (e8), or (e9'),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxii)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $CH_2CH_2$,
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxiii)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $NR^{2c}$,
$R^{2c}$ is the above-mentioned group (e1), (e2), (e3'), (e4), (e5'), (e6), (e7'), (e8), or (e9'),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxiv)]

Compound (1A) wherein

X is $CR^{1a}R^{1b}$,
$R^{1a}$ and $R^{1b}$ are the same or different and each is the above-mentioned group (a1) or (a3),
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxv)]

Compound (1A) wherein

X is an oxygen atom,
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$ alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxvi)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $CR^{2a}R^{2b}$,
$R^{2a}$ is the above-mentioned group (c1), (c2), (c3'), (c4), (c5'), (c12), (c13'), (c14), or (c15'),
$R^{2b}$ is the above-mentioned group (d1),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxvii)]

Compound (1A) wherein

X is $CR^{1a}R^{1b}$,
$R^{1a}$ and $R^{1b}$ are the same or different and each is the above-mentioned group (a1) or (a3),
Y is $NR^{2c}$,
$R^{2c}$ is the above-mentioned group (e1), (e2), (e3'), (e4), (e5'), (e6), (e7'), (e8), or (e9'),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$ alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxviii)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $CH_2CH_2$,
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

[Compound (1A-xxix)]

Compound (1A) wherein

X is $NR^{1c}$,
$R^{1c}$ is the above-mentioned group (b1), (b2), (b10'), (b12), (b13), (b14), (b15'), (b16), (b17'), (b24), (b25'), (b28), or (b29'),
Y is $NR^{2c}$,
$R^{2c}$ is the above-mentioned group (e1), (e2), (e3'), (e4), (e5'), (e6), (e7'), (e8), or (e9'),
$R^{3a}$ is the above-mentioned group (f1), (f6), or (f7'),
$R^{3b}$ is the above-mentioned group (g1) or (g2),
$R^{4a}$ is the above-mentioned group (h1), (h2), (h3), (h4), (h7), (h8), (h9), (h14'), (h15), (h16'), (h17), (h18'), (h21), (h22'), (h25), (h26'), (h27), (h28'), (h29), (h30'), (h31), (h32'), (h37), (h38'), (h39), (h40'), (h41), (h42'), (h45), (h46'), (h47), (h48'), (h49), or (h50'),
$R^{4b}$ is the above-mentioned group (i1) or (i2),
when $R^{4a}$ is (h4) a $(C_1-C_6)$alkyl group, and $R^{4b}$ is (i2) a $(C_1-C_6)$ alkyl group, then $R^{4a}$ and $R^{4b}$ may form a 3- to 6-membered ring together with the adjacent nitrogen atom, and
$R^5$ is the above-mentioned group (h1), (h4), (h7), (h9), (h14'), (h15), (h16'), (h17), (h18'), (h29), or (h30'),
or a salt thereof.

As compound (3), the following compounds are preferred.

[Compound (3-0-i)]

Compound (3) wherein

X is $CR^{1a}R^{1b}$, $NR^{1c}$, or an oxygen atom,
$R^{1a}$ and $R^{1b}$ are the same or different and each is the above-mentioned group (a1), (a2), (a3), (a4), (a5), (a6), (a7), or (a8),
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CR^{2a}R^{2b}$, $NR^{2c}$, or $CH_2CH_2$,
$R^{2a}$ is the above-mentioned group (c1), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c28), (c29), (c44), or (c45),
$R^{2b}$ is the above-mentioned group (d1) or (d2),
$R^{2c}$ is the above-mentioned group (e1), (e2), (e3), (e4), (e5), (e6), (e7), (e8), (e9), (e10), (e11), (e12), (e13), (e14), (e15), (e16), (e17), (e20), (e21), (e44), or (e45),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
provided that $R^{1a}$, $R^{1b}$, $R^{2a}$, $R^{2b}$, $R^{3a}$, and $R^{3b}$ are not hydrogen atoms at the same time, and X is not $NR^{1c}$ when Y is $NR^{2c}$,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51), and
$R^6$ is the above-mentioned group (m1), (m2), (m3), (m4), (m5), or (m6),
or a salt thereof.

[Compound (3-0-ii)]

Compound (3) wherein

X is $CR^{1a}R^{1b}$, $NR^{1c}$, or an oxygen atom,
$R^{1a}$ and $R^{1b}$ are the same or different and each is the above-mentioned group (a1), (a3), (a4), (a6), (a7), or (a8),
$R^{1c}$ is the above-mentioned group (b1), (b2), (b12), (b14), (b15), (b16), or (b17),
Y is $CR^{2a}R^{2b}$, $NR^{2c}$, or $CH_2CH_2$,
$R^{2a}$ is the above-mentioned group (c1), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),

$R^{2c}$ is the above-mentioned group (e1), (e4), (e5), (e44), or (e45),
$R^{3a}$ is the above-mentioned group (f1), (f5), (f6), (f7), (f8), (f9), (f10), or (f11),
$R^{3b}$ is the above-mentioned group (g1) or (g2),
provided that $R^{1a}$, $R^{1b}$, $R^{2a}$, $R^{2b}$, $R^{3a}$, and $R^{3b}$ are not hydrogen atoms at the same time, and X is not $NR^{1c}$ when Y is $NR^{2c}$,
$R^5$ is the above-mentioned group (h1), (h4), (h5), (h6), (h7), (h9), (h14), (h15), (h16), (h17), (h18), (h19), (h20), (h21), (h22), (h27), (h28), (h29), (h30), (h37), (h38), or (h51), and
$R^6$ is the above-mentioned group (m1), (m5), or (m6),
or a salt thereof.

[Compound (3-i)]

Compound (3) wherein

X is $CR^{1a}R^{1b}$, $NR^{1c}$, or an oxygen atom,
$R^{1a}$ and $R^{1b}$ are the same or different and each is the above-mentioned group (a1), (a2), (a3), (a4), (a5), (a6), (a7), or (a8),
$R^{1c}$ is the above-mentioned group (b1), (b2), (b5), (b6), (b9), (b10), (b12), (b13), (b14), (b15), (b16), (b17), (b18), (b19), (b20), (b21), (b22), (b23), (b24), (b25), (b26), (b27), (b28), or (b29),
Y is $CR^{2a}R^{2b}$, $NR^{2c}$, or $CH_2CH_2$,
$R^{2a}$ is the above-mentioned group (c1), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c28), (c29), (c44), or (c45),
$R^{2b}$ is the above-mentioned group (d1) or (d2),
$R^{2c}$ is the above-mentioned group (e1), (e2), (e3), (e4), (e5), (e6), (e7), (e8), (e9), (e10), (e11), (e12), (e13), (e14), (e15), (e16), (e17), (e20), (e21), (e44), or (e45),
$R^{3a}$ is the above-mentioned group (f1), (f2), (f4), (f5), (f6), (f7), (f8), (f9), (f10), or (f11),
$R^{3b}$ is the above-mentioned group (g1), (g2), or (g3),
provided that $R^{1a}$, $R^{1b}$, $R^{2a}$, $R^{2b}$, $R^{3a}$, and $R^{3b}$ are not hydrogen atoms at the same time, and X is not $NR^{1c}$ when Y is $NR^{2c}$,
$R^5$ is the above-mentioned group (h1), (h4), (h5), (h6), (h7), (h9), (h14), (h15), (h16), (h17), (h18), (h19), (h20), (h21), (h22), (h27), (h28), (h29), (h30), (h37), (h38), or (h51), and
$R^6$ is the above-mentioned group (m1), (m5), or (m6),
or a salt thereof.

[Compound (3-ii)]

Compound (3) wherein

X is $CR^{1a}R^{1b}$, $NR^{1c}$, or an oxygen atom,
$R^{1a}$ and $R^{1b}$ are the same or different and each is the above-mentioned group (a1), (a3), (a4), or (a7),
$R^{1c}$ is the above-mentioned group (b1), (b2), (b12), (b14), (b15), (b16), or (b17),
Y is $CR^{2a}R^{2b}$, $NR^{2c}$, or $CH_2CH_2$,
$R^{2a}$ is the above-mentioned group (c1), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1) or (d2),
$R^{2c}$ is the above-mentioned group (e1), (e4), (e5), (e44), or (e45),
$R^{3a}$ is the above-mentioned group (f1), (f5), (f6), (f7), (f8), (f9), (f10), or (f11),
$R^{3b}$ is the above-mentioned group (g1) or (g2),
provided that $R^{1a}$, $R^{1b}$, $R^{2a}$, $R^{2b}$, $R^{3a}$, and $R^{3b}$ are not hydrogen atoms at the same time, and X is not $NR^{1c}$ when Y is $NR^{2c}$,
$R^5$ is the above-mentioned group (h1), (h2), (h3), (h4), (h5), (h6), (h7), (h8), (h9), (h12), (h13), (h14), (h15), (h16), (h17), (h18), (h19), (h20), (h21), (h22), (h23), (h24), (h25), (h26), (h27), (h28), (h29), (h30), (h31), (h32), (h37), (h38), (h39), (h40), (h41), (h42), (h43), (h44), (h45), (h46), (h47), (h48), (h49), (h50), or (h51), and
$R^6$ is the above-mentioned group (m1), (m2), (m3), (m4), (m5), or (m6),
or a salt thereof.

[Compound (3-iii)]

Compound (3) wherein

X is $CR^{1a}R^{1b}$, $NR^{1c}$, or an oxygen atom,
$R^{1a}$ and $R^{1b}$ are the same or different and each is the above-mentioned group (a1), (a3), (a4), or (a7),
$R^{1c}$ is the above-mentioned group (b1), (b2), (b12), (b14), (b15), (b16), or (b17),
Y is $CR^{2a}R^{2b}$ or $NR^{2c}$,
$R^{2a}$ is the above-mentioned group (c1), (c3), (c4), (c5), (c6), (c7), (c8), (c9), (c10), (c11), (c12), (c13), (c14), (c15), (c16), (c17), (c18), (c19), (c20), (c21), (c22), (c23), (c24), (c25), (c26), (c27), (c44), or (c45),
$R^{2b}$ is the above-mentioned group (d1),
$R^{2c}$ is the above-mentioned group (e1), (e4), (e5), (e44), or (e45),
$R^{3a}$ is the above-mentioned group (f1), (f5), (f6), (f7), (f8), (f9), (f10), or (f11),
$R^{3b}$ is the above-mentioned group (g1) or (g2),
provided that $R^{1a}$, $R^{1b}$, $R^{2a}$, $R^{2b}$, $R^{3a}$, and $R^{3b}$ are not hydrogen atoms at the same time, and X is not $NR^{1c}$ when Y is $NR^{2c}$,
$R^5$ is the above-mentioned group (h1), (h4), (h5), (h6), (h7), (h9), (h14), (h15), (h16), (h17), (h18), (h19), (h20), (h21), (h22), (h27), (h28), (h29), (h30), (h37), (h38), or (h51), and
$R^6$ is the above-mentioned group (m1), (m5), or (m6),
or a salt thereof.

Specific examples of the preferred compound (1) (or compound (1A)) are compounds of compound Nos. 1-1 to 1-119, compounds of compound Nos. 2-1 to 2-7, compounds of compound Nos. 3-1 to 3-61, compounds of compound Nos. 4-1 to 4-16, compounds of compound Nos. 5-1 to 5-3, compounds of compound Nos. 6-1 to 6-48, compounds of compound Nos. 7-1 to 7-8, and salts thereof, described below.

Specific examples of preferred compound (3) are the compounds of the below-mentioned compound Nos. 9-1 to 9-57, or salts thereof.

Various compounds of the present invention can be produced by, for example, the following production methods, and the present invention is not limited thereto.

Production method 1

A compound represented by the formula (1A) of the present invention can be produced from a compound represented by the formula (2) by the following step [I-a]:

[Chem. 4]

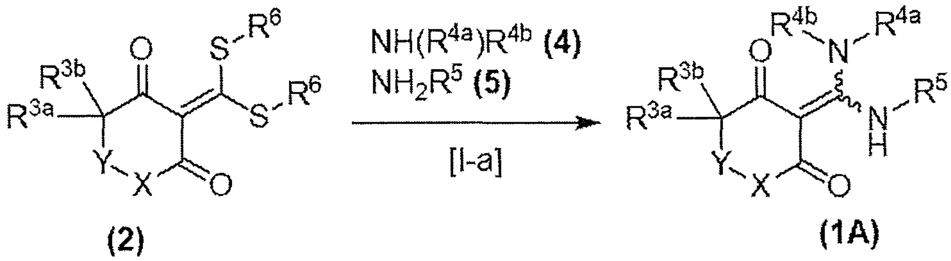


wherein X, Y, $R^{3a}$, $R^{3b}$, $R^{4a}$, $R^{4b}$, $R^5$, and $R^6$ are as defined above.

Production method of step [I-a]

A compound represented by the formula (1A) of the present invention can be produced by reacting a compound represented by the formula (2) with compounds represented by the formula (4) and the formula (5) in the presence of an inert solvent and in the presence or absence of a base.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium

carbonate, cesium carbonate, and the like; acetates such as lithium acetate, sodium acetate, potassium acetate, and the like; organic bases such as pyridine, picoline, lutidine, triethylamine, tributylamine, N,N-diisopropylethylamine, 1,8-diazabicyclo[5.4.0]-7-undecene (DBU), and the like, and the like. The amount thereof to be used is generally 1-fold mol to 10-fold mol with respect to a compound represented by the formula (2).

An inert solvent that can be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 L to 100 L per 1 mol of a compound represented by the formula (2).

Since this reaction is an equimolar reaction, each compound may be used in an equimolar amount, but it is also possible to use either compound in excess. The reaction temperature in this reaction may be generally from about 0°C to the boiling point of the solvent to be used. While the reaction time varies depending on the reaction scale, reaction temperature, and the like and is not constant, it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary.

Production method 2

A compound represented by the formula (1A) of the present invention can be produced from a compound represented by the formula (3) by the following step [I-b]:

[Chem. 5]

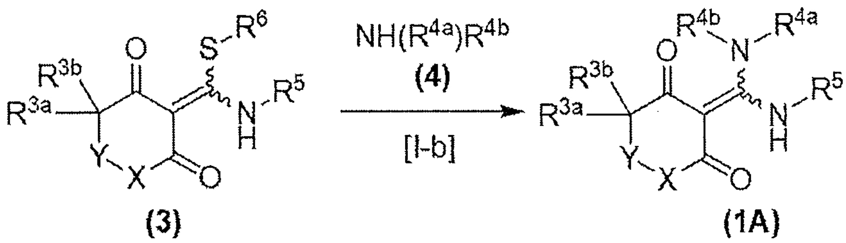


wherein X, Y, $R^{3a}$, $R^{3b}$, $R^{4a}$, $R^{4b}$, $R^5$, and $R^6$ are as defined above.

Production method of step [I-b]

A compound represented by the formula (1A) can be produced by reacting a compound represented by the formula (3) with a compound represented by the formula (4) in the presence of an inert solvent and in the presence or absence of a base.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like; acetates such as lithium acetate, sodium acetate, potassium acetate, and the like; organic bases such as pyridine, picoline, lutidine, triethylamine, tributylamine, N,N-diisopropylethylamine, DBU, and the like, and the like. The amount thereof to be used is generally 1-fold mol to 10-fold mol with respect to a compound represented by the formula (3).

An inert solvent that can be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents

such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like, and the like. These inert solvents can be used alone or a mixture of two or more kinds thereof may be used. The amount thereof to be used can be appropriately selected from the range of generally 0.1 L to 100 L per 1 mol of a compound represented by the formula (3).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. This reaction can also be performed under microwave irradiation. After completion of the reaction, the target product may be isolated from the reaction system containing the target product by a conventional method and the target product can be produced by purification using recrystallization, column chromatography, and the like as necessary.

Production method 3

A compound represented by the formula (1B) of the present invention can be produced from a compound represented by the formula (1A) of the present invention by the following step [I-c]:

[Chem. 6]

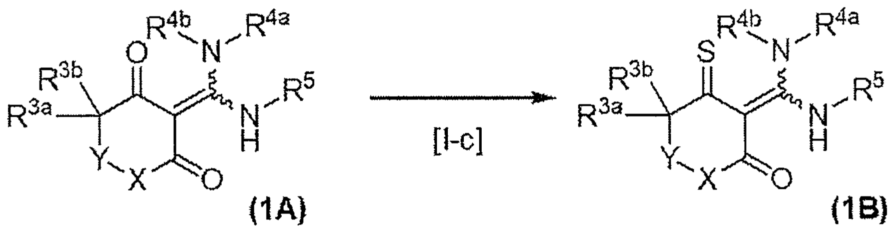


(1A) (1B)

wherein X, Y, $R^{3a}$, $R^{3b}$, $R^{4a}$, $R^{4b}$, and $R^5$ are as defined above.

Production method of step [I-c]

A compound represented by the formula (1B) can be produced by reacting a compound represented by the formula (1A) of the present invention with a sulfurizing agent in the presence of an inert solvent.

Examples of the sulfurizing agent that can be used in this reaction include Lawesson's Reagent, diphosphorus pentasulfide, and the like. The amount thereof to be used is generally 1.0-fold mol to 10-fold mol with respect to a compound represented by the formula (1A).

While a solvent may or may not be used in this reaction, examples of the inert solvent that can be used in this reaction include inert solvents, which may be any as long as they do not markedly inhibit the progress of this reaction, such as chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (1A).

The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. This reaction can also be performed under microwave irradiation. After completion of the reaction, the target product may be isolated from the reaction system containing the target product by a conventional method and the target product can be produced by purification using recrystallization, column chromatography, and the like as necessary.

Production method 4

A compound represented by the formula (1C) of the present invention can be produced from a compound represented by the formula (1A) of the present invention by the following step [I-d]:

[Chem. 7]

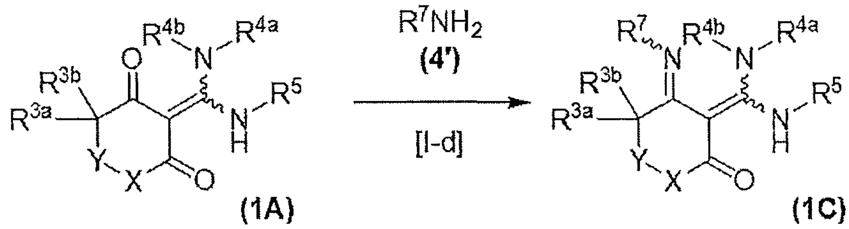

(1A) (1C)

wherein X, Y, $R^{3a}$, $R^{3b}$, $R^{4a}$, $R^{4b}$, $R^5$, and $R^7$ are as defined above.

Production method of step [I-d]

A compound represented by the formula (1C) can be produced by reacting a compound represented by the formula (1A) of the present invention with a compound represented by the formula (4') according to the method described in ORGANIC FUNCTIONAL GROUP PREPARATIONS III 2nd edition ACADEMIC PRESS, INC. or Greene's PROTECTIVE GROUPS in ORGANIC SYNTHESIS FOURTH EDITION (WILEY) or a method analogous thereto. After completion of the reaction, the target product may be isolated from the reaction system containing the target product by a conventional method and the target product can be produced by purification using recrystallization, column chromatography, and the like as necessary.

Production method of starting material - 1

A compound represented by the formula (2), which is the starting material for the compound represented by the formula (1A) of the present invention, can be produced from a compound represented by the formula (6) by the following step [II-a]:

[Chem. 8]

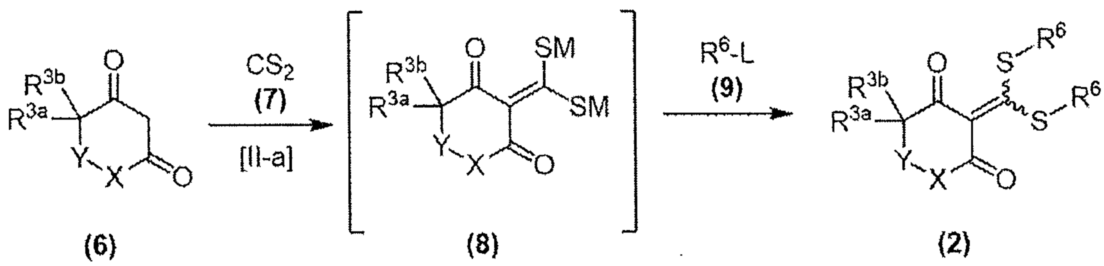

(6) (8) (2)

wherein X, Y, $R^{3a}$, $R^{3b}$, and $R^6$ are as defined above, L is, for example, a leaving group such as chlorine, bromine, iodine, $(C_1-C_6)$alkylcarbonyloxy group, a $(C_1-C_6)$alkoxycarbonyloxy group, and the like, and M is an alkali metal atom such as a lithium atom, a sodium atom, a potassium atom, and the like.

Production method of step [II-a]

Similar to the production method described in JP-61-57565 A, a compound represented by the formula (6) is reacted with carbon disulfide (7) in the presence of a base and an inert solvent to produce the corresponding dithiolate salt (8). This compound, with or without isolation, is then reacted with a compound represented by the formula (9) to produce a compound represented by the formula (2).

Examples of the base that can be used for this reaction include sodium carbonate, potassium carbonate, sodium hydroxide, potassium hydroxide and the like. These can be used as solids or used after dissolving in an inert solvent. The amount of the base to be used can be appropriately selected from the range of 2-fold mol to 8-fold mol per 1 mol of a compound represented by the formula (6), and is preferably 4-fold mol to 6-fold mol.

The inert solvent that can be used for this reaction may be any as long as it does not markedly inhibit the progress of the reaction. Examples thereof include alcohols such as methanol, ethanol, isopropyl alcohol, and the like; polar solvents such as dimethyl sulfoxide, N,N-dimethylformamide, 1,3-dimethyl-2-imidazolidinone, acetonitrile, and the like; water; or a mixed solvent thereof. The amount of the inert solvent to be used poses no problem as long as the reaction proceeds, and can be appropriately selected from the range of 0.5 L to 50 L per 1 mol of a compound represented by the

formula (6) or a compound represented by the formula (8).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of starting material - 2

A compound represented by the formula (3) of the present invention, which is the starting material for the compound represented by the formula (1A) of the present invention, can be produced from a compound represented by the formula (6) by the following steps [III-a] and [III-b]:

[Chem. 9]

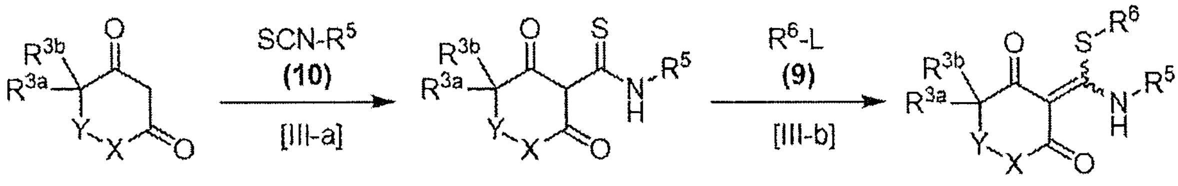


(6) (11) (3)

wherein X, Y, $R^{3a}$, $R^{3b}$, $R^5$, and $R^6$ are as defined above, and L is, for example, a leaving group such as chlorine, bromine, iodine, $(C_1-C_6)$alkylcarbonyloxy group, a $(C_1-C_6)$alkoxycarbonyloxy group, and the like.

Production method of step [III-a]

A compound represented by the formula (11) can be produced by reacting a compound represented by the formula (6) with a compound represented by the formula (10) in the presence of a base and an inert solvent.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like; acetates such as lithium acetate, sodium acetate, potassium acetate, and the like; organic bases such as pyridine, picoline, lutidine, triethylamine, tributylamine, N,N-diisopropylethylamine, DBU, and the like, and the like. The amount thereof to be used is generally 0.5-fold mol to 10-fold mol with respect to a compound represented by the formula (6).

The inert solvent that can be used for this reaction may be any as long as it does not markedly inhibit the progress of the reaction. Examples thereof include chain or cyclic saturated hydrocarbons such as pentane, hexane, cyclohexane, and the like; aromatic hydrocarbons such as benzene, toluene, xylene, and the like; halogenated hydrocarbons such as methylene chloride, chloroform, carbon tetrachloride, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (6).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [III-b]

A compound represented by the formula (3) can be produced by reacting a compound represented by the formula (11) with a compound represented by the formula (9) in the presence of a base and an inert solvent.

Examples of the base that can be used in this reaction include inorganic bases such as hydroxides of alkali metal atom such as sodium hydroxide, potassium hydroxide, and the like; hydrides of alkali metal such as sodium hydride, potassium hydride, and the like; alkoxides such as sodium ethoxide, potassium t-butoxide, and the like; carbonates such as sodium carbonate, potassium carbonate, cesium carbonate, sodium hydrogen carbonate, and the like, organic bases such as triethylamine, pyridine, N,N-dimethyl-4-aminopyridine, DBU, and the like, and the like. The amount thereof to be used is generally 1-fold mol to 10-fold mol with respect to a compound represented by the formula (11).

The inert solvent that can be used for this reaction may be any as long as it does not markedly inhibit the progress of the reaction. Examples thereof include chain or cyclic saturated hydrocarbons such as pentane, hexane, cyclohexane, and the like; aromatic hydrocarbons such as benzene, toluene, xylene, and the like; halogenated hydrocarbons such as methylene chloride, chloroform, carbon tetrachloride, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; esters such as methyl acetate and the like; ketones such as acetone, methyl ethyl ketone, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (11).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of starting material - 3

A compound represented by the formula (6), wherein X is $CR^{1a}R^{1b}$ wherein $R^{1a}$ and $R^{1b}$ are as defined above, and Y is $CR^{2a}R^{2b}$ wherein $R^{2a}$ and $R^{2b}$ are as defined above, can be produced by the method described in Journal of the Chemical Society, 539-540 (1935), or Bioorganic & Medicinal Chemistry (2012), 20, 1029-1045, or according to the method analogous thereto.

Production method of starting material - 4

A compound represented by the formula (6), wherein X is an oxygen atom, and Y is $CR^{2a}R^{2b}$ wherein $R^{2a}$ and $R^{2b}$ are as defined above, can be produced by the method described in WO 2008/014311, or according to the method analogous thereto.

Production method of starting material - 5

A compound represented by the formula (6A) which is a compound represented by the formula (6), wherein X is $NR^{1c}$ wherein $R^{1c}$ is as defined above, Y is $CR^{2a}R^{2b}$ wherein $R^{2a}$ and $R^{2b}$ are as defined above, and $R^{3a}$ and $R^{3b}$ are each a hydrogen atom, can be produced from a compound represented by the formula (12) by the following step [V-a]. A compound represented by the formula (12) can be produced by the method described in WO 2021/261562, or according to the method analogous thereto.

[Chem. 10]

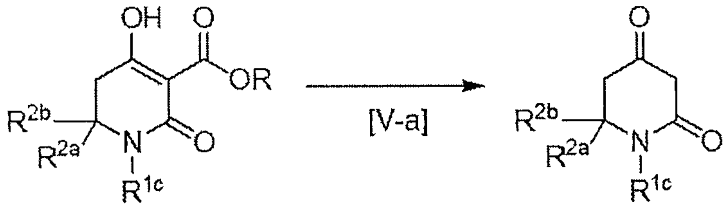
(12)
(6A)

wherein $R^{1c}$, $R^{2a}$, and $R^{2b}$ are as defined above. R is, for example, a $(C_1-C_6)$alkyl group such as methyl group, an ethyl group, and the like.

Production method of step [V-a]

A compound represented by the formula (6A) can be produced by reacting a compound represented by the formula (12) under heating conditions in the presence of an inert solvent and in the presence or absence of a base.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like; acetates such as lithium acetate, sodium acetate, potassium acetate, and the like; organic bases such as pyridine, picoline, lutidine, triethylamine, tributylamine, N,N-diisopropylethylamine, DBU, and the like; and the like. The amount thereof to be used is generally 1-fold mol to 10-fold mol with respect to a compound represented by the formula (12).

An inert solvent that can be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; water, and the like. These inert solvents can be used alone or a mixture of two or more kinds thereof may be used. The amount thereof to be used can be appropriately selected from the range of generally 0.1 L to 100 L per 1 mol of a compound represented by the formula (12).

The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of starting material - 6

A compound represented by the formula (6B) can be produced from a compound represented by the formula (13) by the following steps [VI-a], [VI-b], [VI-c], [VI-d], [VI-e], [VI-f], and [VI-g]. The reaction conditions of step [VI-g] are the same as those in the aforementioned step [V-a].

[Chem. 11]

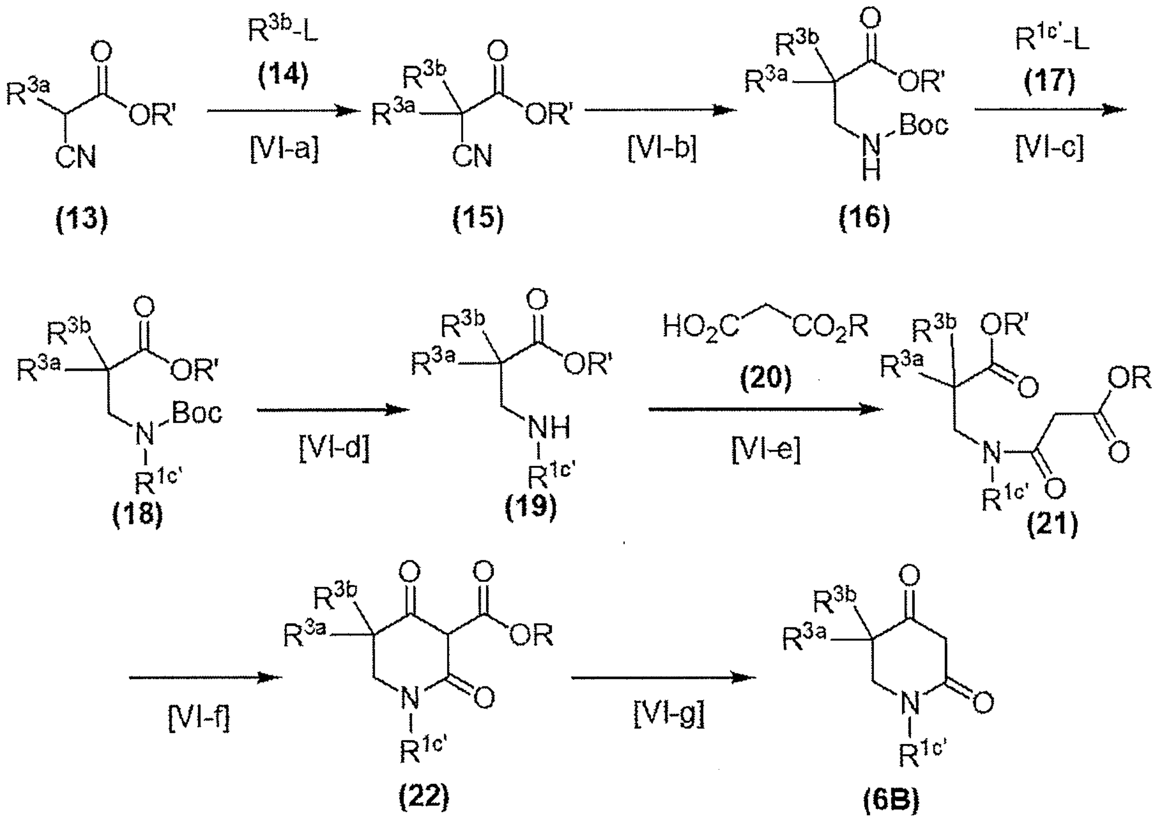


wherein $R^{3a}$ and $R^{3b}$ are as defined above, $R^{1c'}$ is a ($C_1$-$C_6$) alkyl group, a ($C_2$-$C_6$)alkenyl group, a ($C_2$-$C_6$)alkynyl group, a ($C_3$-$C_6$) cycloalkyl group, a halo($C_1$-$C_6$)alkyl group, a halo($C_2$-$C_6$)alkenyl group, a halo ($C_2$-$C_6$)alkynyl group, a halo($C_3$-$C_6$)cycloalkyl group, a ($C_1$-$C_6$)alkyl group having 1 to 3 substituents each independently selected from substituent group A or substituent group $A_0$ (substituent group A and substituent group $A_0$ are as defined above), a ($C_1$-$C_6$) alkylcarbonyl group, a ($C_1$-$C_6$)alkoxycarbonyl group, a ($C_1$-$C_6$)alkoxy($C_1$-$C_6$)alkoxycarbonyl group, a phenyl group, a phenyl group having 1 to 5 substituents each independently selected from substituent group B or substituent group $B_0$ (substituent group B and substituent group $B_0$ are as defined above), a pyridyl group, a pyridyl group having 1 to 4 substituents each independently selected from substituent group B or substituent group $B_0$ (substituent group B and substituent group $B_0$ are as defined above), a pyridazinyl group, a pyridazinyl group having 1 to 3 substituents each independently selected from substituent group B or substituent group $B_0$ (substituent group B and substituent group $B_0$ are as defined above), a pyrimidinyl group, a pyrimidinyl group having 1 to 3 substituents each independently selected from substituent group B or substituent group $B_0$ (substituent group B and substituent group $B_0$ are as defined above), a pyrazinyl group, a pyrazinyl group having 1 to 3 substituents each independently selected from substituent group B or substituent group $B_0$ (substituent group B and substituent group $B_0$ are as defined above), a phenyl ($C_1$-$C_6$) alkyl group, a phenyl ($C_1$-$C_6$) alkyl group having 1 to 5 substituents each independently selected from substituent group B or substituent group $B_0$ (substituent group B and substituent group $B_0$ are as defined above), a pyridyl($C_1$-$C_6$)alkyl group, a pyridyl ($C_1$-$C_6$)alkyl group having 1 to 4 substituents each independently selected from substituent group B or substituent group $B_0$ (substituent group B and substituent group $B_0$ are as defined above), a thiazolyl($C_1$-$C_6$)alkyl group, or a thiazolyl($C_1$-$C_6$) alkyl group having 1 or 2 substituents each independently selected from substituent group B or substituent group $B_0$ (substituent group B and substituent group $B_0$ are as defined above), R and R' are each, for example, a ($C_1$-$C_6$)alkyl group such as methyl group, ethyl group, and the like, L is, for example, a leaving group such as chlorine, bromine, iodine, ($C_1$-$C_6$) alkylcarbonyloxy group, a ($C_1$-$C_6$)alkoxycarbonyloxy group, and the like, and Boc is a tertiary butoxycarbonyl group.

Production method of step [VI-a]

A compound represented by the formula (15) can be produced by reacting a compound represented by the formula (13) with a compound represented by the formula (14) in the presence of a base and an inert solvent.

Examples of the base that can be used in this reaction include hydroxides such as sodium hydroxide, potassium hydroxide, and the like; alkali metal hydrides such as sodium hydride, potassium hydride, and the like; alkoxides such as

sodium ethoxide, potassium t-butoxide, and the like; carbonates such as sodium carbonate, potassium carbonate, cesium carbonate, sodium hydrogen carbonate, and the like; organic bases such as triethylamine, N,N-diisopropylethylamine, pyridine, DBU, and the like, and the like. The amount thereof to be used is generally 1-fold mol to 10-fold mol with respect to a compound represented by the formula (13).

An inert solvent that can be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated hydrocarbons such as methylene chloride, chloroform, carbon tetrachloride, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; esters such as methyl acetate and the like; ketones such as acetone, methyl ethyl ketone, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (13).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [VI-b]

A compound represented by the formula (16) can be produced by reacting a compound represented by the formula (15) with di-tertiary-butyl dicarbonate in the presence of metal halide, a reducing agent and an inert solvent.

Examples of the metal halide that can be used in this reaction include lithium chloride, lithium bromide, lithium iodide, sodium chloride, sodium bromide, sodium iodide, potassium chloride, potassium bromide, potassium iodide, cesium chloride, cesium bromide, cesium iodide, cobalt chloride, and the like. The amount thereof to be used is generally 1-fold mol to 10-fold mol with respect to a compound represented by the formula (15).

Examples of the reducing agent that can be used in this reaction include sodium borohydride, sodium cyanoborohydride, sodium bis(2-methoxyethoxy)aluminum hydride, hydrogen/palladium carbon, hydrogen/Raney nickel, and the like. The amount thereof to be used is generally 0.5-fold mol to 10-fold mol with respect to a compound represented by the formula (15).

An inert solvent that can be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated hydrocarbons such as methylene chloride, chloroform, carbon tetrachloride, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; esters such as methyl acetate and the like; ketones such as acetone, methyl ethyl ketone, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (15).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 96 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [VI-c]

A compound represented by the formula (18) can be produced by reacting a compound represented by the formula (16) with a compound represented by the formula (17) in the presence of a base and an inert solvent. The reaction

conditions of step [VI-c] are the same as those in the aforementioned step [VI-a].

Production method of step [VI-d]

A compound represented by the formula (19) or a salt thereof can be produced by hydrolyzing a compound represented by the formula (18) in the presence of an acid and in the presence or absence of an inert solvent.
Examples of the acid that can be used in this reaction include inorganic acids such as hydrochloric acid, sulfuric acid, nitric acid, and the like; organic acids such as formic acid, acetic acid, propionic acid, trifluoroacetic acid, benzoic acid, and the like; sulfonic acids such as methanesulfonic acid, trifluoromethanesulfonic acid, paratoluenesulfonic acid, and the like; phosphoric acid, and the like. The amount thereof to be used can be appropriately selected from the range of 0.01-fold mol to 10-fold mol with respect to a compound represented by the formula (18). The acid can also be used as a solvent.
An inert solvent that can be used in this reaction may be any as long as it does not markedly inhibit the progress of this reaction, and inert solvents such as aromatic hydrocarbons such as benzene, toluene, xylene, and the like; halogenated hydrocarbons such as methylene chloride, chloroform, carbon tetrachloride, and the like; halogenated aromatic hydrocarbons such as chlorobenzene, dichlorobenzene, and the like; chain or cyclic ethers such as diethyl ether, methyl tertiary butylether, dioxane, tetrahydrofuran, and the like; esters such as ethyl acetate and the like; amides such as N,N-dimethylformamide, N,N-dimethylacetamide, and the like; ketones such as acetone, methyl ethyl ketone, and the like; polar solvents such as dimethyl sulfoxide, 1,3-dimethyl-2-imidazolidinone, and the like can be mentioned. These inert solvents can be used alone or a mixture of two or more kinds thereof may be used. The amount of the inert solvent to be used is not particularly limited as long as it is an amount that dissolves the reaction reagent, and can be appropriately selected from the range of 0.5 L to 100 L per 1 mol of a compound represented by the formula (18). When the aforementioned acid is used as a solvent, a solvent may not be used.
The reaction temperature in this reaction may be generally from room temperature to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [VI-e]

A compound represented by the formula (21) can be produced by reacting a compound represented by the formula (19) or a salt thereof with a compound represented by the formula (20) in the presence of a base and a condensing agent and in the presence or absence of an inert solvent.
Examples of the condensing agent that can be used in this reaction include acid activation reagents such as phosgene, phosphorus trichloride, phosphorus oxychloride, oxalyl chloride, thionyl chloride, and the like; carbodiimides such as N,N'-dicyclohexylcarbodiimide (DCC), 1-(3-dimethylaminopropyl)-3-ethylcarbodiimide (EDCI), and the like; phosphorus pentoxide, polyphosphoric acid, N,N'-carbonyldiimidazole, 2-chloropyridine 1-methiodide (Mukaiyama reagent), 2-ethoxy-N-ethoxycarbonyl-1,2-dihydroquinoline (EEDQ), triphenylphosphine/carbon tetrachloride, bromotripyrrolidinophosphonium hexafluorophosphate (BROP), O-(1H-benzotriazol-1-yloxy)tris(dimethylamino)phosphonium hexafluorophosphate (BOP), N,N,N',N'-bis(tetramethylene)chlorouronium tetrafluoroborate, O-(1H-benzotriazol-1-yl)-N,N,N',N'-tetramethyluronium hexafluorophosphate (HBTU), O-(1H-benzotriazol-1-yl)-N,N,N',N'-bis(tetramethylene)uronium hexafluorophosphate, O-(1H-benzotriazol-1-yl)-N,N,N' ,N'-tetramethyluronium tetrafluoroborate (TBTU), O-(1H-benzotriazol-1-yl)-N,N,N',N'-bis(tetramethylene)uronium tetrafluoroborate, O-(7-azabenzotriazol-1-yl)-N,N,N',N'-tetramethyluronium hexafluorophosphate (HATU), 1-hydroxybenzotriazole (HOBt), propylphosphonic anhydride ($T_3P$), 4-(4,6-dimethoxy-1,3,5-triazin-2-yl)-4-methylmorpholinium salt (DMT-MM), and the like. These reagents can be used alone or a mixture of two or more kinds thereof may be used. The amount of the condensing agent to be used can be appropriately selected from the range of 0.5-fold mol to 5-fold mol with respect to a compound represented by the formula (19).
Examples of the base that can be used in this reaction include carbonates such as lithium carbonate, lithium hydrogen carbonate, sodium carbonate, sodium hydrogen carbonate, potassium carbonate, potassium hydrogen carbonate, cesium carbonate, calcium carbonate, magnesium carbonate, and the like; acetates such as lithium acetate, sodium acetate, potassium acetate, and the like; organic bases such as pyridine, picoline, lutidine, triethylamine, tributylamine, N,N-diisopropylethylamine, N,N-dimethyl-4-aminopyridine, DBU, and the like; and the like. The amount of the base to be used can be appropriately selected from the range of 0.5-fold mol to 5-fold mol with respect to a compound represented by the formula (19). The base can also be used as a solvent.
An inert solvent that can be used in this reaction may be any as long as it does not markedly inhibit the progress of this reaction. Examples thereof include chain or cyclic saturated hydrocarbons such as pentane, hexane, cyclohexane,

and the like; chain or cyclic ethers such as diethyl ether, tetrahydrofuran (THF), dioxane, and the like; aromatic hydrocarbons such as benzene, toluene, xylene, and the like; halogenated hydrocarbons such as methylene chloride, chloroform, carbon tetrachloride, and the like; nitriles such as acetonitrile, isopropylnitrile, and the like; polar solvents such as N,N-dimethylformamide, N,N-dimethylacetamide, dimethyl sulfoxide, 1,3-dimethyl-2-imidazolidinone; and the like. These inert solvents can be used alone or a mixture of two or more kinds thereof may be used. The amount of the inert solvent to be used is not particularly limited as long as it is an amount that dissolves the reaction reagent, and can be appropriately selected from the range of 0.5 L to 100 L per 1 mol of a compound represented by the formula (19). When the aforementioned base is used as a solvent, a solvent may not be used.

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [VI-f]

A compound represented by the formula (22) can be produced by subjecting a compound represented by the formula (21) to a cyclization reaction in the presence of a base and an inert solvent.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; carbonates such as lithium carbonate, lithium hydrogen carbonate, sodium carbonate, sodium hydrogen carbonate, potassium carbonate, potassium hydrogen carbonate, cesium carbonate, calcium carbonate, magnesium carbonate, and the like; acetates such as lithium acetate, sodium acetate, potassium acetate, and the like; organic bases such as pyridine, picoline, lutidine, triethylamine, tributylamine, N,N-diisopropylethylamine, DBU, and the like; and the like. The amount thereof to be used is generally 1-fold mol to 10-fold mol with respect to a compound represented by the formula (21).

An inert solvent that can be used in this reaction may be any as long as it does not markedly inhibit the progress of this reaction. Examples thereof include inert solvents such as chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (21).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of starting material - 7

A compound represented by the formula (6C) which is a compound represented by the formula (6), wherein X is $CH_2$, Y is $NR^{2c}$ wherein $R^{2c}$ is as defined above, and $R^{3a}$ and $R^{3b}$ are each a hydrogen atom, can be produced from a compound represented by the formula (23) by the following steps [VII-a], [VII-b], [VII-c], [VII-d], and [VII-e]. The reaction conditions of step [VII-c] are the same as those in the aforementioned step [VI-d]. A compound represented by the formula (6C) can be produced by the method described in WO 2006/003096 or WO 2007/051062, or in accordance with the method analogous thereto.

[Chem. 12]

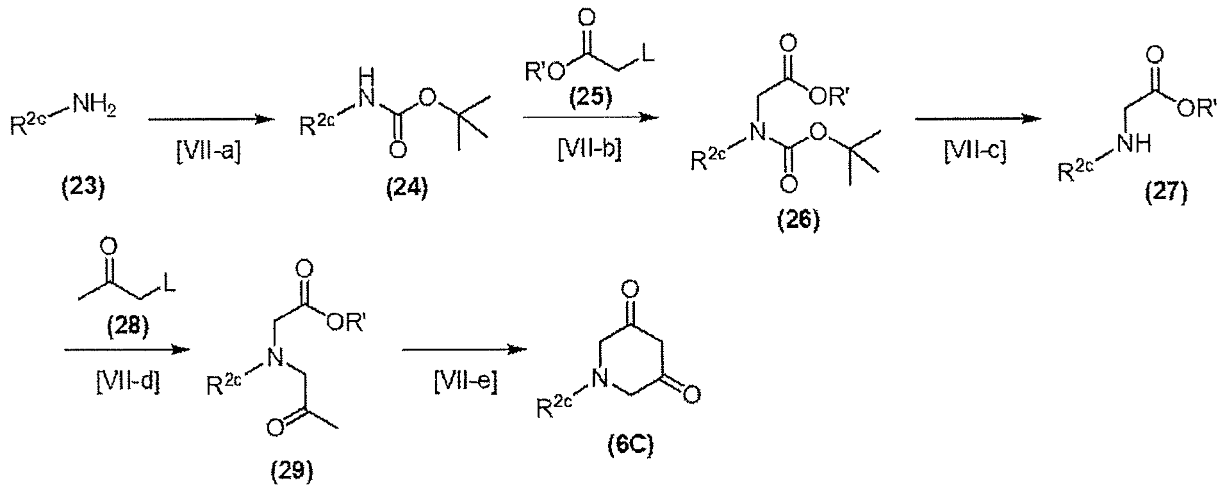


wherein $R^{2c}$ is as defined above, R' is, for example, a ($C_1$-$C_6$)alkyl group such as a methyl group, an ethyl group, and the like, and L is, for example, a leaving group such as chlorine, bromine, iodine, ($C_1$-$C_6$)alkylcarbonyloxy group, a ($C_1$-$C_6$) alkoxycarbonyloxy group, and the like.

Production method of step [VII-a]

A compound represented by the formula (24) can be produced by reacting a compound represented by the formula (23) with di-tertiary-butyl dicarbonate in the presence of a base and in the presence or absence of an inert solvent.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like; acetates such as lithium acetate, sodium acetate, potassium acetate, and the like; organic bases such as pyridine, picoline, lutidine, triethylamine, tributylamine, N,N-diisopropylethylamine, DBU, and the like; and the like. The amount thereof to be used can be appropriately selected from the range of generally 1-fold mol to 10-fold mol with respect to a compound represented by the formula (23). The base can also be used as a solvent.

An inert solvent that can be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated hydrocarbons such as methylene chloride, chloroform, carbon tetrachloride, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; esters such as methyl acetate and the like; ketones such as acetone, methyl ethyl ketone, and the like; aprotic polar solvents such as N,N-dimethylformamide, N,N-dimethylacetamide, dimethyl sulfoxide, 1,3-dimethyl-2-imidazolidinone, and the like; alcohols such as methanol, ethanol, propanol, butanol, 2-propanol, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (23). When the aforementioned base is used as a solvent, a solvent may not be used.

Since this reaction is an equimolar reaction, each compound may be used in an equimolar amount, but it is also possible to use either compound in excess. The reaction temperature in this reaction may be generally from about -100°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [VII-b]

A compound represented by the formula (26) can be produced by reacting a compound represented by the formula (24) with a compound represented by the formula (25) in the presence of a base and an inert solvent.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like; acetates such as lithium acetate, sodium acetate, potassium acetate; and the like, organic bases such as pyridine, picoline, lutidine, triethylamine, tributylamine, N,N-diisopropylethylamine, DBU, and the like; and the like. The amount thereof to be used can be appropriately selected from the range of generally 1-fold mol to 10-fold mol with respect to a compound represented by the formula (24).

An inert solvent to be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (24).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of step [VII-d]

A compound represented by the formula (29) can be produced by reacting a compound represented by the formula (27) with a compound represented by the formula (28) in the presence of a base and an inert solvent.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like; acetates such as lithium acetate, sodium acetate, potassium acetate, and the like; organic bases such as pyridine, picoline, lutidine, triethylamine, tributylamine, N,N-diisopropylethylamine, DBU, and the like; and the like. The amount thereof to be used can be appropriately selected from the range of generally 1-fold mol to 10-fold mol with respect to a compound represented by the formula (27).

An inert solvent to be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (27).

Since this reaction is an equimolar reaction, each compound may be used in an equimolar amount, but it is also possible to use either compound in excess. The reaction temperature in this reaction may be generally from about 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column

chromatography, and the like as necessary. The product can also be subjected to the next step without isolation.

Production method of step [VII-e]

A compound represented by the formula (6C) can be produced by subjecting a compound represented by the formula (29) to a cyclization reaction in the presence of a base and an inert solvent.

Examples of the base that can be used in this reaction include hydroxides such as lithium hydroxide, sodium hydroxide, potassium hydroxide, calcium hydroxide, and the like; alkoxides such as sodium methoxide, sodium ethoxide, sodium tertiary-butoxide, potassium tertiary-butoxide, and the like; alkali metal hydrides such as sodium hydride, potassium hydride, and the like; carbonates such as lithium carbonate, lithium hydrogen carbonate, sodium carbonate, sodium hydrogen carbonate, potassium carbonate, potassium hydrogen carbonate, calcium carbonate, magnesium carbonate, cesium carbonate, and the like; acetates such as lithium acetate, sodium acetate, potassium acetate, and the like; organic bases such as pyridine, picoline, lutidine, triethylamine, tributylamine, N,N-diisopropylethylamine, DBU, and the like; and the like. The amount thereof to be used can be appropriately selected from the range of generally 1-fold mol to 10-fold mol with respect to a compound represented by the formula (29).

An inert solvent to be used in this reaction may be any as long as it does not markedly inhibit the progress of this reaction. Examples thereof include chain or cyclic saturated hydrocarbons such as pentane, hexane, cyclohexane, and the like; aromatic hydrocarbons such as benzene, toluene, xylene, and the like; halogenated aromatic hydrocarbons such as chlorobenzene, dichlorobenzene, and the like; chain or cyclic ethers such as diethyl ether, methyl tertiary-butyl ether, dioxane, tetrahydrofuran, and the like; nitriles such as acetonitrile, propionitrile, and the like; aprotic polar solvents such as N,N-dimethylformamide, N,N-dimethylacetamide, dimethyl sulfoxide, 1,3-dimethyl-2-imidazolidinone, and the like; and the like. These inert solvents can be used alone or a mixture of two or more kinds thereof may be used. The amount thereof to be used can be appropriately selected from the range of generally 0.1 L to 100 L per 1 mol of a compound represented by the formula (29).

The reaction temperature in this reaction may be generally from 0°C to the boiling point of the solvent to be used. The reaction time varies depending on the reaction scale, reaction temperature, and the like, and is not constant, but it can be appropriately selected from the range of generally several minutes to 48 hours. After completion of the reaction, the target product may be isolated from the reaction system containing the target product by a conventional method and the target product can be produced by purification using recrystallization, column chromatography, and the like as necessary. The product can also be subjected to the next step without isolation.

Production method of starting material - 8

A compound represented by the formula (6), wherein X is $NR^{1c}$ wherein $R^{1c}$ is as defined above, and Y is $CH_2CH_2$, can be produced by the method described in Tetrahedron Letters, 1995, 36, 3659, or Journal of Heterocyclic Chemistry, 2017, 54, 1318, or in accordance with the method analogous thereto.

Production method of starting material - 9

A compound represented by the formula (6E) can be produced from a compound represented by the formula (6D) by the following step [IX-a]. The reaction conditions of step [IX-a] are the same as those in the aforementioned step [VI-a]. A compound represented by the formula (6D) can be produced by the method described in WO 2021/261563, or in accordance with the method analogous thereto.

[Chem. 13]

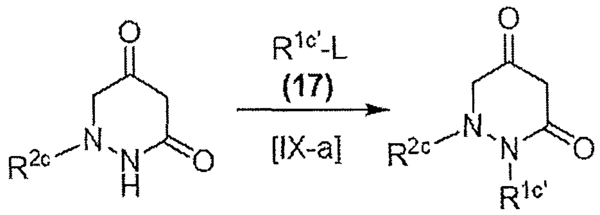


(6D) (6E)

wherein $R^{1c'}$ and $R^{2c}$ are as defined above, and L is, for example, a leaving group such as chlorine, bromine, iodine, $(C_1-C_6)$alkylcarbonyloxy group, a $(C_1-C_6)$alkoxycarbonyloxy group, and the like.

Representative examples of the compound (compound (1)) represented by the formula (1) of the present

invention are shown in the following Tables 1 to 7, but the present invention is not limited to them. Representative examples of compound (2) are shown in Table 8, and representative examples of compound (3) are shown in Table 9. In the following Tables, "Me" is a methyl group, "Et" is an ethyl group, "n-Pr" is a normal propyl group, "c-Pr" is a cyclopropyl group, "c-Bu" is a cyclobutyl group, "i-Bu" is an isobutyl group, "t-Bu" is a tertiary butyl group, "Ph" is a phenyl group, and "Bn" is a benzyl group. The property value is melting point (°C), refractive index $n_d$ (measurement temperature; °C), or $H^1$-NMR. $H^1$-NMR data is shown in Tenth Table.

[Chem. 14]

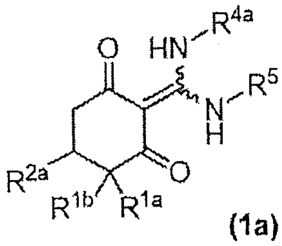

(1a)

[Table 1]

| First Table | | | | | | |
|---|---|---|---|---|---|---|
| compound No. | $R^{1a}$ | $R^{1b}$ | $R^{2a}$ | $R^{4a}$ | $R^5$ | property value |
| 1-1 | H | H | 4-$CF_3$-Ph | 5-F-pyridin-2-yl | 5-F-pyridin-2-yl | NMR |
| 1-2 | H | H | 5-Cl-thiophen-2-yl | 5-F-pyridin-2-yl | 5-F-pyridin-2-yl | 155-159 |
| 1-3 | H | H | 4-$CF_3$-Ph | 6-F-pyridin-3-yl | 6-F-pyridin-3-yl | 250-252 |
| 1-4 | H | H | 4-$CF_3$-Ph | 2-F-pyridin-4-yl | 2-F-pyridin-4-yl | 188-189 |
| 1-5 | H | H | 5-Br-pyridin-2-yl | 5-F-pyridin-2-yl | 5-F-pyridin-2-yl | 239-241 |
| 1-6 | H | H | 5-Br-pyridin-2-yl | 6-F-pyridin-3-yl | 6-F-pyridin-3-yl | 298-300 |
| 1-7 | H | H | 5-Br-pyridin-2-yl | 2-F-pyridin-4-yl | 2-F-pyridin-4-yl | 258-260 |
| 1-8 | H | H | 6-$CF_3$-pyridin-3-yl | 6-F-pyridin-3-yl | 6-F-pyridin-3-yl | 300 |
| 1-9 | H | H | 4-$CF_3$-Ph | 4-F-Ph | 4-F-Ph | 215-218 |
| 1-10 | H | H | 5-Br-pyridin-2-yl | Me | 4-F-Ph | NMR |
| 1-11 | H | H | furan-2-yl | Me | Me | 178-179 |
| 1-12 | H | H | furan-2-yl | Bn | Bn | 145-146 |
| 1-13 | H | H | 4-$CF_3$-Ph | (tetrahydropyran-4-yl)$CH_2$ | 4-F-Ph | 165-166 |
| 1-14 | H | H | 4-$CF_3$-Ph | (4-$CF_3O$-Ph)$CH_2$ | 4-F-Ph | 130-131 |
| 1-15 | H | H | 4-$CF_3$-Ph | 5-Me-isoxazol-3-yl | 4-F-Ph | 163-164 |
| 1-16 | H | H | 4-$CF_3$-Ph | (thiazol-2-yl)$CH_2$ | 4-F-Ph | 186-187 |
| 1-17 | H | H | 4-$CF_3$-Ph | pyrimidin-5-yl | 4-F-Ph | 231-232 |
| 1-18 | H | H | 4-$CF_3$-Ph | 1-Me-pyrazol-3-yl | 4-F-Ph | 212-213 |
| 1-19 | H | H | 4-$CF_3$-Ph | (pyridin-2-yl)$CH_2$ | 4-F-Ph | 176-177 |
| 1-20 | H | H | 4-$CF_3$-Ph | (furan-2-yl)$CH_2$ | 4-F-Ph | 171-172 |
| 1-21 | H | H | 4-$CF_3$-Ph | (1-benzofuran-6-yl)$CH_2$ | 4-F-Ph | 144-145 |
| 1-22 | H | H | 4-$CF_3$-Ph | (thiophen-2-yl)$CH_2$ | 4-F-Ph | 181-182 |
| 1-23 | H | H | 4-$CF_3$-Ph | $CF_3CH_2$ | 4-F-Ph | 121-122 |
| 1-24 | H | H | 4-$CF_3$-Ph | $BrCH_2CH_2$ | $BrCH_2CH_2$ | 130-131 |
| 1-25 | H | H | 4-$CF_3$-Ph | $MeOCH_2CH_2$ | 4-F-Ph | 134-135 |

(continued)

| First Table | | | | | | |
|---|---|---|---|---|---|---|
| compound No. | $R^{1a}$ | $R^{1b}$ | $R^{2a}$ | $R^{4a}$ | $R^5$ | property value |
| 1-26 | H | H | 4-$CF_3$-Ph | (c-Pr)$CH_2$ | 4-F-Ph | 141-142 |
| 1-27 | H | H | 4-$CF_3$-Ph | $BrCH_2CH_2$ | 4-F-Ph | 105-106 |
| 1-28 | H | H | 4-$CF_3$-Ph | $CF_3CH_2$ | $CF_3CH_2$ | NMR |
| 1-29 | H | H | 4-$CF_3$-Ph | $MeOCH_2CH_2$ | $MeOCH_2CH_2$ | NMR |
| 1-30 | H | H | 4-$CF_3$-Ph | (c-Pr)$CH_2$ | (c-Pr)$CH_2$ | 154-155 |

[Table 2]

| continued from First Table | | | | | | |
|---|---|---|---|---|---|---|
| compound No. | $R^{1a}$ | $R^{1b}$ | $R^{2a}$ | $R^{4a}$ | $R^5$ | property value |
| 1-31 | H | H | 4-$CF_3$-Ph | $CF_3CH_2CH_2$ | 4-F-Ph | 105-106 |
| 1-32 | H | H | 4-$CF_3$-Ph | (4-F-Ph)$CH_2CH_2$ | 4-F-Ph | 167-168 |
| 1-33 | H | H | 4-$CF_3$-Ph | t-BuO | 4-F-Ph | 177-178 |
| 1-34 | H | H | 4-$CF_3$-Ph | (4-F-Ph)$CH_2$ | 4-F-Ph | 156-157 |
| 1-35 | H | H | 4-$CF_3$-Ph | $CHF_2CH_2$ | 4-F-Ph | 176-177 |
| 1-36 | H | H | 4-$CF_3$-Ph | c-Pr | 4-F-Ph | 153-154 |
| 1-37 | H | H | 4-$CF_3$-Ph | c-Bu | 4-F-Ph | 185-186 |
| 1-38 | H | H | 4-$CF_3$-Ph | t-BuO | 4-F-Ph | 162-163 |
| 1-39 | H | H | 4-$CF_3$-Ph | (tetrahydrofuran-2-yl)$CH_2$ | 4-F-Ph | 207-208 |
| 1-40 | H | H | 4-$CF_3$-Ph | $CF_3CH_2CH_2$ | $CF_3CH_2CH_2$ | 135-136 |
| 1-41 | H | H | 4-$CF_3$-Ph | (4-F-Ph)$CH_2CH_2$ | (4-F-Ph)$CH_2CH_2$ | 163-164 |
| 1-42 | H | H | 4-$CF_3$-Ph | $Me_2NCH_2CH_2$ | $Me_2NCH_2CH_2$ | 251-252 |
| 1-43 | H | H | 4-$CF_3$-Ph | H | 4-F-Ph | 187-188 |
| 1-44 | Me | Me | 5-Br-pyridin-2-yl | 4-F-Ph | 4-F-Ph | 125-126 |
| 1-45 | Me | Me | 5-Br-pyridin-2-yl | 5-F-pyridin-2-yl | 5-F-pyridin-2-yl | 183-184 |
| 1-46 | Me | Me | 5-Br-pyridin-2-yl | 6-F-pyridin-3-yl | 6-F-pyridin-3-yl | 197-198 |
| 1-47 | Me | Me | 5-Br-pyridin-2-yl | 2-F-pyridin-4-yl | 2-F-pyridin-4-yl | 210-211 |

[Table 3]

| continued from First Table | | | | | | |
|---|---|---|---|---|---|---|
| compound No. | $R^{1a}$ | $R^{1b}$ | $R^{2a}$ | $R^{4a}$ | $R^5$ | property value |
| 1-48 | H | H | 6-$CF_3$-pyridin-3-yl | 5-F-pyridin-2-yl | 5-F-pyridin-2-yl | 228-230 |
| 1-49 | H | H | 5-Cl-thiophen-2-yl | 6-F-pyridin-3-yl | 6-F-pyridin-3-yl | 284-285 |
| 1-50 | H | H | 5-Cl-thiophen-2-yl | 4-F-Ph | 4-F-Ph | 228-229 |
| 1-51 | H | H | 5-Cl-thiophen-2-yl | 6-F-pyridin-3-yl | 4-F-Ph | 228-229 |
| 1-52 | H | $CF_3CH_2$ | H | 6-F-pyridin-3-yl | 6-F-pyridin-3-yl | 70-71 |

(continued)

| continued from First Table | | | | | | |
|---|---|---|---|---|---|---|
| compound No. | $R^{1a}$ | $R^{1b}$ | $R^{2a}$ | $R^{4a}$ | $R^5$ | property value |
| 1-53 | H | $CF_3CH_2$ | H | Me | 6-F-pyridin-3-yl | 88-89 |
| 1-54 | H | $CF_3CH_2$ | H | 4-F-Ph | 6-F-pyridin-3-yl | 101-102 |
| 1-55 | H | H | thiazol-5-yl | 6-F-pyridin-3-yl | 6-F-pyridin-3-yl | 283-284 |
| 1-56 | H | H | 4-$CF_3$-Ph | 6-F-pyridin-3-yl | Me | 199-200 |
| 1-57 | H | H | 4-$CF_3$-Ph | 4-F-Ph | Me | 192-194 |
| 1-58 | H | H | pyrimidin-5-yl | 6-F-pyridin-3-yl | 4-F-Ph | >300 |
| 1-59 | H | H | pyrimidin-5-yl | 4-F-Ph | 4-F-Ph | 280-281 |
| 1-60 | H | H | isothiazol-5-yl | 6-F-pyridin-3-yl | 6-F-pyridin-3-yl | 286-287 |
| 1-61 | H | H | 4-$CF_3$-Ph | 6-F-pyridin-3-yl | 5-F-pyridin-2-yl | 218-219 |
| 1-62 | H | H | 4-$CF_3$-Ph | 2-OMe-pyrimidin-5-yl | 2-OMe-pyrimidin-5-yl | 300-301 |
| 1-63 | H | H | 4-$CF_3$-Ph | 5-OMe-pyrazin-2-yl | 5-OMe-pyrazin-2-yl | 251-252 |
| 1-64 | H | H | 4-$CF_3$-Ph | 4-F-Ph | pyrazin-2-yl | 177-179 |
| 1-65 | H | H | 4-$CF_3$-Ph | 6-F-pyridin-3-yl | (pyrazin-2-yl)$CH_2$ | 212-219 |
| 1-66 | H | H | 3-(2-Cl-Ph)-isoxazol-5-yl | 6-F-pyridin-3-yl | 4-F-Ph | 135-136 |
| 1-67 | H | H | 3-(2-Cl-Ph)-isoxazol-5-yl | Me | 4-F-Ph | 198-199 |
| 1-68 | H | H | 4-$CF_3$-Ph | 4-F-Ph | propargyl | 193-194 |
| 1-69 | H | H | 4-$CF_3$-Ph | 4-F-Ph | 3,4-dihydro-2H-1-benzopyran-4-yl | 111-112 |
| 1-70 | H | pyridin-2-yl | 4-$CF_3$-Ph | 6-F-pyridin-3-vl | Me | 185-186 |
| 1-71 | H | pyridin-2-yl | 4-$CF_3$-Ph | 6-F-pyridin-3-yl | 6-F-pyridin-3-yl | 258-260 |
| 1-72 | H | pyridin-4-yl | 4-$CF_3$-Ph | 6-F-pyridin-3-yl | 6-F-pyridin-3-yl | 245-247 |
| 1-73 | H | pyridin-2-yl | 4-$CF_3$-Ph | 6-F-pyridin-3-yl | 4-F-Ph | 214-216 |
| 1-74 | H | H | 2-Me-oxazol-5-yl | 4-F-Ph | 6-F-pyridin-3-yl | 221-223 |
| 1-75 | H | H | 2-Me-oxazol-5-yl | 6-F-pyridin-3-yl | 6-F-pyridin-3-yl | 241-243 |
| 1-76 | H | H | isothiazol-5-yl | 6-F-pyridin-3-yl | 4-F-Ph | 256-258 |
| 1-77 | H | H | pyrazin-2-yl | 6-F-pyridin-3-yl | 4-F-Ph | 271-272 |

(continued)

| continued from First Table | | | | | | |
|---|---|---|---|---|---|---|
| compound No. | $R^{1a}$ | $R^{1b}$ | $R^{2a}$ | $R^{4a}$ | $R^5$ | property value |
| 1-78 | H | H | pyrazin-2-yl | 4-F-Ph | 4-F-Ph | 206-207 |
| 1-79 | H | H | 5-Cl-1-Me-3-Ph-pyrazol-4-yl | 6-F-pyridin-3-yl | 4-F-Ph | 199-200 |
| 1-80 | H | H | 5-Cl-1-Me-3-Ph-pyrazol-4-yl | 4-F-Ph | 4-F-Ph | 98-99 |
| 1-81 | H | H | pyrazin-2-yl | Me | 4-F-Ph | 184-185 |
| 1-82 | H | H | 5-Cl-1-Me-3-Ph-pyrazol-4-yl | Me | 4-F-Ph | 86-87 |
| 1-83 | H | pyridin-4-yl | 4-$CF_3$-Ph | 6-F-pyridin-3-yl | Me | NMR |
| 1-84 | H | pyridin-4-yl | 4-$CF_3$-Ph | 6-F-pyridin-3-yl | 4-F-Ph | 222-224 |
| 1-85 | H | pyridin-3-yl | 4-$CF_3$-Ph | 6-F-pyridin-3-yl | 6-F-pyridin-3-yl | 234-236 |
| 1-86 | H | pyridin-3-yl | 4-$CF_3$-Ph | 6-F-pyridin-3-yl | Me | 184-186 |
| 1-87 | H | pyridin-3-yl | 4-$CF_3$-Ph | 6-F-pyridin-3-yl | 4-F-Ph | 129-131 |
| 1-88 | H | 4-Cl-Ph | 4-$CF_3$-Ph | 6-F-pyridin-3-yl | 6-F-pyridin-3-yl | 251-253 |

[Table 4]

| continued from First Table | | | | | | |
|---|---|---|---|---|---|---|
| compound No. | $R^{1a}$ | $R^{1b}$ | $R^{2a}$ | $R^{4a}$ | $R^5$ | property value |
| 1-89 | H | 4-Cl-Ph | 4-$CF_3$-Ph | 6-F-pyridin-3-yl | Me | 195-197 |
| 1-90 | H | 4-$CF_3$-Ph | 4-$CF_3$-Ph | 6-F-pyridin-3-yl | 6-F-pyridin-3-yl | 250-252 |
| 1-91 | H | 4-$CF_3$-Ph | 4-$CF_3$-Ph | 6-F-pyridin-3-yl | Me | |
| 1-92 | H | 4-MeO-Ph | 4-$CF_3$-Ph | 6-F-pyridin-3-yl | 6-F-pyridin-3-yl | |
| 1-93 | H | 4-MeO-Ph | 4-$CF_3$-Ph | 6-F-pyridin-3-yl | Me | |
| 1-94 | H | 3-Cl-pyridin-2-yl | 4-$CF_3$-Ph | 6-F-pyridin-3-yl | 6-F-pyridin-3-yl | 218-220 |
| 1-95 | H | 3-Cl-pyridin-2-yl | 4-$CF_3$-Ph | 6-F-pyridin-3-yl | Me | 205-207 |
| 1-96 | H | 2-$CF_3$-pyridin-6-yl | 4-$CF_3$-Ph | 6-F-pyridin-3-yl | 6-F-pyridin-3-yl | 239-241 |
| 1-97 | H | 2-$CF_3$-pyridin-6-yl | 4-$CF_3$-Ph | 6-F-pyridin-3-yl | Me | |

(continued)

| continued from First Table | | | | | | |
|---|---|---|---|---|---|---|
| compound No. | $R^{1a}$ | $R^{1b}$ | $R^{2a}$ | $R^{4a}$ | $R^5$ | property value |
| 1-98 | H | 4-Me-pyridin-2-yl | 4-$CF_3$-Ph | 6-F-pyridin-3-yl | 6-F-pyridin-3-yl | |
| 1-99 | H | 4-Me-pyridin-2-yl | 4-$CF_3$-Ph | 6-F-pyridin-3-yl | Me | |
| 1-100 | H | 2-Cl-pyridin-5-yl | 4-$CF_3$-Ph | 6-F-pyridin-3-yl | 6-F-pyridin-3-yl | |
| 1-101 | H | 2-Cl-pyridin-5-yl | 4-$CF_3$-Ph | 6-F-pyridin-3-yl | Me | |
| 1-102 | H | 4-Me-Ph | 4-$CF_3$-Ph | 6-F-pyridin-3-yl | 6-F-pyridin-3-yl | 239-241 |
| 1-103 | H | 4-Me-Ph | 4-$CF_3$-Ph | 6-F-pyridin-3-yl | Me | |
| 1-104 | H | 2-Cl-pyridin-4-yi | 4-$CF_3$-Ph | 6-F-pyridin-3-yl | 6-F-pyridin-3-yl | 219-221 |
| 1-105 | H | 2-Cl-pyridin-4-yl | 4-$CF_3$-Ph | 6-F-pyridin-3-yl | Me | |
| 1-106 | H | H | 6-Cl-pyridazin-3-yl | 4-F-Ph | 4-F-Ph | |
| 1-107 | H | H | 6-Cl-pyridazin-3-yl | 4-F-Ph | 6-F-pyridin-3-yl | |
| 1-108 | H | H | 6-Cl-pyridazin-3-yl | 4-F-Ph | Me | 196-197 |
| 1-109 | H | H | 6-OEt-pyridazin-3-yl | 4-F-Ph | 4-F-Ph | |
| 1-110 | H | H | 6-OEt-pyridazin-3-yl | 4-F-Ph | 6-F-pyridin-3-yl | |
| 1-111 | H | H | 6-OEt-pyridazin-3-yl | 4-F-Ph | Me | 191-192 |
| 1-112 | H | H | 4-$CF_3$-Ph | Me | 5-F-pyridin-2-yl | 165-167 |
| 1-113 | H | H | 4-$CF_3$-Ph | H | 5-F-pyridin-2-yl | 173-175 |
| 1-114 | H | H | 4-$CF_3$-Ph | 6-F-pyridin-3-yl | 5-F-pyrimidin-2-yl | |
| 1-115 | H | H | 2-Me-oxazol-5-yl | 4-F-Ph | propen-3-yl | 89-90 |
| 1-116 | H | H | 4-$CF_3$-Ph | 6-F-pyridin-3-yl | pyridazin-3-yl | 244-245 |
| 1-117 | H | H | 4-$CF_3$-Ph | Me | 1-Me-pyrazol-3-yl | 155-157 |
| 1-118 | H | H | 4-$CF_3$-Ph | 2-OMe-pyridin-3-yl | 2-OMe-pyridin-3-yl | 107-108 |
| 1-119 | H | H | 4-$CF_3$-Ph | H | 6-F-pyridin-3-yl | 171-172 |

[Chem. 15]

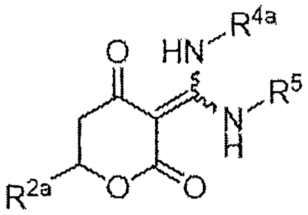

(1b)

[Table 5]

| Second Table | | | | |
|---|---|---|---|---|
| compound No. | $R^{2a}$ | $R^{4a}$ | $R^5$ | property value |
| 2-1 | 6-$CF_3$-pyridin-3-yl | 2-F-pyridin-4-yl | 2-F-pyridin-4-yl | 194-195 |
| 2-2 | 6-$CF_3$-pyridin-3-yl | 5-F-pyridin-2-yl | 5-F-pyridin-2-yl | 224 |
| 2-3 | 6-$CF_3$-pyridin-3-yl | 6-F-pyridin-3-yl | 6-F-pyridin-3-yl | 290 |
| 2-4 | 5-Br-pyridin-2-yl | 5-F-pyridin-2-yl | 5-F-pyridin-2-yl | 215-216 |
| 2-5 | 5-Br-pyridin-2-yl | 6-F-pyridin-3-yl | 6-F-pyridin-3-yl | 280-281 |
| 2-6 | 5-Br-pyridin-2-yl | 2-F-pyridin-4-yl | 2-F-pyridin-4-yl | 164-165 |
| 2-7 | 4-Br-Ph | Me | 4-F-Ph | 190-191 |

[Chem. 16]

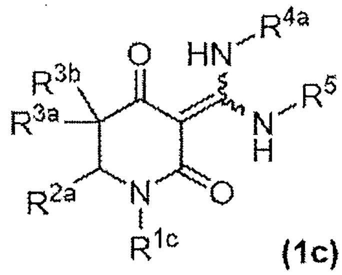

(1c)

[Table 6]

| Third Table | | | | | | | |
|---|---|---|---|---|---|---|---|
| compound No. | $R^{1c}$ | $R^{2a}$ | $R^{3a}$ | $R^{3b}$ | $R^{4a}$ | $R^5$ | property value |
| 3-1 | Me | 4-$CF_3$-Ph | H | H | Me | 4-F-Ph | NMR |
| 3-2 | Me | H | 6-$CF_3$-pyridin-3-yl | Et | Me | 4-F-Ph | NMR |
| 3-3 | Me | H | 6-$CF_3$-pyridin-3-yl | Et | H | 4-F-Ph | 182-183 |
| 3-4 | Me | 4-$CF_3$-Ph | H | H | H | 4-F-Ph | NMR |

[Table 7]

| continued from Third Table | | | | | | | |
|---|---|---|---|---|---|---|---|
| compound No. | $R^{1c}$ | $R^{2a}$ | $R^{3a}$ | $R^{3b}$ | $R^{4a}$ | $R^5$ | property value |
| 3-5 | H | 6-Br-pyridin-3-yl | H | H | 6-F-pyridin-3-yl | 6-F-pyridin-3-yl | >300 |
| 3-6 | H | 6-Br-pyridin-3-yl | H | H | Me | 6-F-pyridin-3-yl | 251-253 |
| 3-7 | H | 6-Br-pyridin-3-yl | H | H | 4-F-Ph | 6-F-pyridin-3-yl | 279-281 |
| 3-8 | Me | H | 4-$CF_3$-Ph | Et | Me | 6-F-pyridin-3-yl | 74-77 |
| 3-9 | Me | H | 4-$CF_3$-Ph | Et | 4-F-Ph | 6-F-pyridin-3-yl | 173-175 |
| 3-10 | Me | H | 4-$CF_3$-Ph | Et | 6-F-pyridin-3-yl | 6-F-pyridin-3-yl | 91-93 |
| 3-11 | H | 4-$CF_3$-Ph | H | H | Me | 6-F-pyridin-3-yl | 187-189 |
| 3-12 | H | 4-$CF_3$-Ph | H | H | 4-F-Ph | 6-F-pyridin-3-yl | 290-292 |
| 3-13 | H | 4-$CF_3$-Ph | H | H | 6-F-pyridin-3-yl | 6-F-pyridin-3-yl | 295-297 |
| 3-14 | H | 2-$CF_3$-thiazol-5-yl | H | H | Me | 6-F-pyridin-3-yl | 223-225 |
| 3-15 | H | 2-$CF_3$-thiazol-5-yl | H | H | 4-F-Ph | 6-F-pyridin-3-yl | 249-251 |
| 3-16 | H | 2-$CF_3$-thiazol-5-yl | H | H | 6-F-pyridin-3-yl | 6-F-pyridin-3-yl | 278-280 |
| 3-17 | $CO_2$t-Bu | $CO_2$t-Bu | H | H | Me | 4-F-Ph | 98-99 |
| 3-18 | H | $CO_2$t-Bu | H | H | Me | 4-F-Ph | 78-79 |
| 3-19 | H | $CO_2H$ | H | H | 4-F-Ph | 6-F-pyridin-3-yl | 202-203 |
| 3-20 | H | 1-Me-5-$OCHF_2$-pyra-zol-3-yl | H | H | Me | 6-F-pyridin-3-yl | 175-176 |
| 3-21 | H | 1-Me-5-$OCHF_2$-pyra-zol-3-yl | H | H | 4-F-Ph | 4-F-Ph | 60-61 |
| 3-22 | H | 1-Me-5-$OCHF_2$-pyra-zol-3-yl | H | H | 4-F-Ph | 6-F-pyridin-3-yl | 170-171 |
| 3-23 | H | 4-(4-F-Ph)-thiazol-2-yl | H | H | Me | 6-F-pyridin-3-yl | 198-199 |
| 3-24 | H | 4-(4-F-Ph)-thiazol-2-yl | H | H | 4-F-Ph | 6-F-pyridin-3-yl | 229-230 |
| 3-25 | H | 4-(4-F-Ph)-thiazol-2-yl | H | H | 6-F-pyridin-3-yl | 6-F-pyridin-3-yl | 267-268 |
| 3-26 | H | 5-Br-thiophen-2-yl | H | H | Me | 6-F-pyridin-3-yl | 205-206 |

(continued)

| continued from Third Table | | | | | | | |
|---|---|---|---|---|---|---|---|
| compound No. | $R^{1c}$ | $R^{2a}$ | $R^{3a}$ | $R^{3b}$ | $R^{4a}$ | $R^5$ | property value |
| 3-27 | H | 5-Br-thiophen-2-yl | H | H | 4-F-Ph | 6-F-pyridin-3-yl | 276-277 |

[Table 8]

| continued from Third Table | | | | | | | |
|---|---|---|---|---|---|---|---|
| compound No. | $R^{1c}$ | $R^{2a}$ | $R^{3a}$ | $R^{3b}$ | $R^{4a}$ | $R^5$ | property value |
| 3-28 | H | 5-Br-thiophen-2-yl | H | H | 6-F-pyridin-3-yl | 6-F-pyridin-3-yl | 300-301 |
| 3-29 | H | 5-CN-thiophen-2-yl | H | H | Me | 6-F-pyridin-3-yl | 244-245 |
| 3-30 | H | 5-CN-thiophen-2-yl | H | H | 4-F-Ph | 6-F-pyridin-3-yl | 260-261 |
| 3-31 | H | 5-CN-thiophen-2-yl | H | H | 6-F-pyridin-3-yl | 6-F-pyridin-3-yl | 299-300 |
| 3-32 | 4-$CF_3$-Ph | H | H | H | Me | 6-F-pyridin-3-yl | 172-173 |
| 3-33 | 4-$CF_3$-Ph | H | H | H | 4-F-Ph | 6-F-pyridin-3-yl | 220-221 |
| 3-34 | 4-$CF_3$-Ph | H | H | H | 6-F-pyridin-3-yl | 6-F-pyridin-3-yl | 274-275 |
| 3-35 | 6-Cl-pyridin-3-yl | H | H | H | Me | 6-F-pyridin-3-yl | 157-158 |
| 3-36 | 6-Cl-pyridin-3-yl | H | H | H | 4-F-Ph | 6-F-pyridin-3-yl | 178-179 |
| 3-37 | 6-Cl-pyridin-3-yl | H | H | H | 6-F-pyridin-3-yl | 6-F-pyridin-3-yl | 239-240 |
| 3-38 | H | 3-(2-F-pyridin-4-yl)-1,2,4-oxadiazol-5-yl | H | H | Me | 4-F-Ph | 167-168 |
| 3-39 | H | $CO_2$t-Bu | H | H | 4-F-Ph | 6-F-pyridin-3-yl | 86-87 |
| 3-40 | H | 3-(2-F-pyridin-4-yl)-1,2,4-oxadiazol-5-yl | H | H | 4-F-Ph | 6-F-pyridin-3-yl | 249-250 |
| 3-41 | H | 6-Br-pyridin-3-yl | H | H | 4-F-Ph | 4-F-Ph | 285-286 |
| 3-42 | H | 5-Br-6-Cl-pyridin-3-yl | H | H | H | 4-F-Ph | 138-139 |
| 3-43 | H | 5-Br-6-Cl-pyridin-3-yl | H | H | 6-F-pyridin-3-yl | 4-F-Ph | 285-286 |
| 3-44 | H | 5-(6-F-pyridin-3-yl)-6-Cl-pyridin-3-yl | H | H | H | 4-F-Ph | 119-121 |

(continued)

| continued from Third Table | | | | | | | |
|---|---|---|---|---|---|---|---|
| compound No. | $R^{1c}$ | $R^{2a}$ | $R^{3a}$ | $R^{3b}$ | $R^{4a}$ | $R^5$ | property value |
| 3-45 | H | 5-(6-F-pyridin-3-yl)-6-Cl-pyridin-3-yl | H | H | 6-F-pyridin-3-yl | 4-F-Ph | 198-200 |
| 3-46 | H | 5-Br-6-Cl-pyridin-3-yl | H | H | H | 6-F-pyridin-3-yl | |
| 3-47 | H | 5-Br-6-Cl-pyridin-3-yl | H | H | Me | 6-F-pyridin-3-yl | 125-126 |
| 3-48 | H | 5-(6-F-pyridin-3-yl)-6-Cl-pyridin-3-yl | H | H | H | 6-F-pyridin-3-yl | 124-127 |

[Table 9]

| continued from Third Table | | | | | | | |
|---|---|---|---|---|---|---|---|
| compound No. | $R^{1c}$ | $R^{2a}$ | $R^{3a}$ | $R^{3b}$ | $R^{4a}$ | $R^5$ | property value |
| 3-49 | H | 5-(6-F-pyridin-3-yl)-6-Cl-pyridin-3-yl | H | H | 6-F-pyridin-3-yl | 6-F-pyridin-3-yl | 162-165 |
| 3-50 | H | 5-(6-F-pyridin-3-yl)-6-Cl-pyridin-3-yl | H | H | 4-F-Ph | 4-F-Ph | 224-225 |
| 3-51 | H | 5-(6-F-pyridin-3-yl)-6-Cl-pyridin-3-yl | H | H | Me | 4-F-Ph | 92-95 |
| 3-52 | H | 5-(4-F-Ph)-6-Cl-pyridin-3-yl | H | H | Me | 6-F-pyridin-3-yl | |
| 3-53 | Me | H | 4-Cl-thiazol-2-yl | Et | 4-F-Ph | 6-F-pyridin-3-yl | 151-154 |
| 3-54 | Me | H | 4-Cl-thiazol-2-yl | Et | Me | 6-F-pyridin-3-yl | 73-77 |
| 3-55 | Me | H | quinolin-2-yl | Et | 4-F-Ph | 6-F-pyridin-3-yl | 184-185 |
| 3-56 | Me | H | quinolin-2-yl | Et | Me | 6-F-pyridin-3-yl | 70-74 |
| 3-57 | Me | H | 6-$CF_3$-pyridin-3-yl | Et | Me | 6-F-pyridin-3-yl | 141-144 |
| 3-58 | Me | H | 6-$CF_3$-pyridin-3-yl | Et | H | 6-F-pyridin-3-yl | 70-72 |
| 3-59 | H | 6-$CF_3$-pyridin-3-yl | H | H | 6-F-pyridin-3-yl | 6-F-pyridin-3-yl | 284-285 |
| 3-60 | H | 6-$CF_3$-pyridin-3-yl | H | H | Me | 6-F-pyridin-3-yl | 292-293 |
| 3-61 | H | 6-$CF_3$-pyridin-3-yl | H | H | 4-F-Ph | 6-F-pyridin-3-yl | 299-300 |

[Chem. 17]

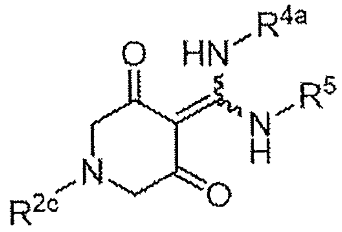

(1d)

[Table 10]

| Fourth Table | | | | |
|---|---|---|---|---|
| compound No. | $R^{2c}$ | $R^{4a}$ | $R^5$ | property value |
| 4-1 | 5-CN-pyridin-2-yl | Me | 4-F-Ph | 261-262 |
| 4-2 | 5-CN-pyridin-2-yl | (4-F-Ph)$CH_2$ | 4-F-Ph | 231-232 |
| 4-3 | 5-CN-pyridin-2-yl | n-Pr | 4-F-Ph | 206-207 |
| 4-4 | 5-CN-pyridin-2-yl | i-Bu | 4-F-Ph | 172-173 |
| 4-5 | 5-CN-pyridin-2-yl | (c-Pr)$CH_2$ | 4-F-Ph | 214-215 |
| 4-6 | 5-CN-pyridin-2-yl | $CHF_2CH_2$ | 4-F-Ph | 246-247 |
| 4-7 | 5-CN-pyridin-2-yl | (furan-2-yl)$CH_2$ | 4-F-Ph | 199-200 |
| 4-8 | 5-CN-pyridin-2-yl | Bn | 4-F-Ph | 185-186 |
| 4-9 | 5-CN-pyridin-2-yl | (pyrazin-2-yl)$CH_2$ | 4-F-Ph | 204-205 |
| 4-10 | $CO_2$t-Bu | 5-F-pyridin-2-yl | 5-F-pyridin-2-yl | 196-198 |
| 4-11 | CO(5-F-pyridin-2-yl) | 6-F-pyridin-3-yl | 6-F-pyridin-3-yl | 194-195 |
| 4-12 | CO(5-CF3-pyridin-2-yl) | 6-F-pyridin-3-yl | 6-F-pyridin-3-yl | 192-193 |
| 4-13 | $CO_2$t-Bu | Me | 5-F-pyridin-2-yl | |
| 4-14* | H | Me | 5-F-pyridin-2-yl | 209-211 |
| 4-15 | $CO_2$t-Bu | 6-F-pyridin-3-yl | 6-F-pyridin-3-yl | 226-227 |
| 4-16* | H | 6-F-pyridin-3-yl | 6-F-pyridin-3-yl | 236-237 |
| (In the Table, * indicates hydrochloride.) | | | | |

[Chem. 18]

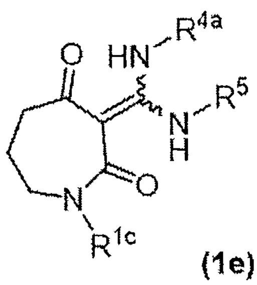

(1e)

[Table 11]

| Fifth Table | | | | |
|---|---|---|---|---|
| compound No. | $R^{1c}$ | $R^{4a}$ | $R^5$ | property value |
| 5-1 | 4-$CF_3$-Ph | H | 4-F-Ph | 220-221 |
| 5-2 | 4-$CF_3$-Ph | Me | 4-F-Ph | 155-156 |
| 5-3 | 4-$CF_3$-Ph | OH | 4-F-Ph | 145-146 |

[Chem. 19]

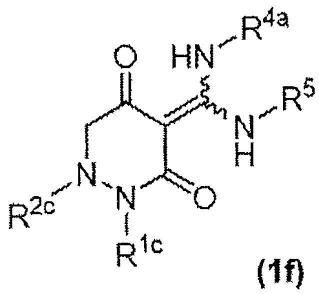
(1f)

[Table 12]

| Sixth Table | | | | | |
|---|---|---|---|---|---|
| compound No. | $R^{1c}$ | $R^{2c}$ | $R^{4a}$ | $R^5$ | property value |
| 6-1 | $CO_2Me$ | 6-CN-pyridin-3-yl | OMe | 4-F-Ph | 121-125 |
| 6-2 | $CO_2Me$ | 6-CN-pyridin-3-yl | OH | 4-F-Ph | 191-194 |
| 6-3 | $CO_2CH_2CH_2OMe$ | 6-CN-pyridin-3-yl | 3,4-$F_2$-Ph | 3,4-$F_2$-Ph | 128-130 |
| 6-4 | $CO_2Me$ | 6-CN-pyridin-3-yl | 4-F-Ph | 4-F-Ph | 149-151 |
| 6-5 | $CO_2Me$ | 6-CN-pyridin-3-yl | CN | 4-F-Ph | 239-241 |
| 6-6 | $CO_2CH_2CH_2OMe$ | 6-CN-pyridin-3-yl | 3-F-Ph | 3-F-Ph | 118-121 |
| 6-7 | $CO_2CH_2CH_2OMe$ | 6-CN-pyridin-3-yl | 4-OMe-Ph | 4-OMe-Ph | 179-182 |
| 6-8 | $CO_2CH_2CH_2OMe$ | 6-CN-pyridin-3-yl | 3-Me-Ph | 3-Me-Ph | 93-95 |
| 6-9 | H | 6-CN-pyridin-3-yl | 4-F-Ph | n-Pr | 152-155 |
| 6-10 | $CO_2Me$ | 6-CN-pyridin-3-yl | 4-F-Ph | n-Pr | 106-109 |
| 6-11 | $CO_2Me$ | 6-Br-pyridin-3-yl | 4-F-Ph | n-Pr | 112-113 |
| 6-12 | $CO_2Me$ | 4-CN-Ph | 6-Cl-pyridin-3-yl | 6-Cl-pyridin-3-yl | 270-273 |
| 6-13 | $CO_2Me$ | 6-CN-pyridin-3-yl | 4-F-Ph | Me | 203-206 |
| 6-14 | $CO_2Me$ | 6-CN-pyridin-3-yl | 4-F-Ph | (4-F-Ph)$CH_2$ | 135-138 |
| 6-15 | $CO_2Me$ | 6-CN-pyridin-3-yl | $CHF_2CH_2$ | 4-F-Ph | 115-120 |
| 6-16 | H | 5-Cl-6-CN-pyridin-3-yl | Me | 4-F-Ph | 195-199 |
| 6-17 | 6-CN-pyridin-3-yl | 6-CN-pyridin-3-yl | 4-F-Ph | Me | 180-181 |
| 6-18 | $CO_2Me$ | 5-Cl-6-CN-pyridin-3-yl | 3,4-$F_2$-Ph | Me | 241-243 |
| 6-19 | $CO_2Me$ | 5-Cl-6-CN-pyridin-3-yl | 4-F-Ph | Me | 215-217 |
| 6-20 | $CO_2Me$ | 5-Br-6-CN-pyridin-3-yl | 4-F-Ph | Me | 232-234 |

(continued)

| Sixth Table | | | | | |
|---|---|---|---|---|---|
| compound No. | $R^{1c}$ | $R^{2c}$ | $R^{4a}$ | $R^5$ | property value |
| 6-21 | $CO_2Me$ | 5-OMe-6-CN-pyridin-3-yl | 4-F-Ph | Me | 245-247 |
| 6-22 | $CO_2Me$ | 6-Br-pyridin-3-yl | 3,4-$F_2$-Ph | Me | 178-179 |
| 6-23 | $CO_2Me$ | 5-Cl-6-CN-pyridin-3-yl | 4-F-Ph | c-Pr | 203-205 |
| 6-24 | H | 3,4-$(CN)_2$-Ph | 4-F-Ph | Me | 165-167 |
| 6-25 | Me | 6-CN-pyridin-3-yl | 4-F-Ph | Me | 200-201 |
| 6-26 | $MeOCH_2$ | 6-CN-pyridin-3-yl | 4-F-Ph | Me | 163-164 |
| 6-27 | Bn | 6-CN-pyridin-3-yl | 4-F-Ph | Me | 120-121 |
| 6-28 | $CO_2Et$ | 3-F-4-CN-Ph | Me | 4-F-Ph | 115-116 |
| 6-29 | $CO_2Et$ | 3-F-4-CN-Ph | Me | 3,4-$F_2$-Ph | 98-99 |
| 6-30 | $CO_2Et$ | 3-Cl-4-CN-Ph | Me | 3-F-Ph | 215-216 |

[Table 13]

| continued from Sixth Table | | | | | |
|---|---|---|---|---|---|
| compound No. | $R^{1c}$ | $R^{2c}$ | $R^{4a}$ | $R^5$ | property value |
| 6-31 | $CO_2Et$ | 3-Cl-4-CN-Ph | (3-F-Ph)$CH_2$ | 3-F-Ph | 209-211 |
| 6-32 | $CO_2Me$ | 6-CN-pyridin-3-yl | 4-F-Ph | (3-F-Ph) $CH_2$ | 111-113 |
| 6-33 | $CO_2Me$ | 6-CN-pyridin-3-yl | 4-F-Ph | H | 201-203 |
| 6-34 | Et | 6-CN-pyridin-3-yl | 4-F-Ph | Me | 129-130 |
| 6-35 | Ph | Ph | 4-F-Ph | Me | 161-162 |
| 6-36 | (NC)$CH_2$ | 6-CN-pyridin-3-yl | 4-F-Ph | Me | 155-156 |
| 6-37 | (c-Pr)$CH_2$ | 6-CN-pyridin-3-yl | 4-F-Ph | Me | 162-163 |
| 6-38 | (2-Cl-thiazol-5-yl) $CH_2$ | 6-CN-pyridin-3-yl | 4-F-Ph | Me | 235-236 |
| 6-39 | H | 5-CN-pyrimidin-2-yl | Me | 4-F-Ph | 275-278 |
| 6-40 | $CO_2Me$ | 6-CN-pyridin-3-yl | $CHF_2CH_2$ | $CHF_2CH_2$ | 87-88 |
| 6-41 | $CO_2Me$ | 6-Cl-pyridazin-3-yl | Me | 4-F-Ph | 267-269 |
| 6-42 | $CO_2Me$ | 6-CN-pyridin-3-yl | $MeOCH_2CH_2CH_2$ | 4-F-Ph | 1.4436 (21.9) |
| 6-43 | $CO_2Me$ | 6-CN-pyridin-3-yl | $Me_2NCH_2CH_2$ | 4-F-Ph | 178-180 |
| 6-44 | Me | 5-Cl-6-CN-pyridin-3-yl | 4-F-Ph | Me | 129-131 |
| 6-45 | $CO_2Me$ | 6-CN-pyridin-3-yl | c-Pr | c-Pr | 97-100 |
| 6-46 | Me | 6-CN-pyridin-3-yl | c-Pr | c-Pr | 199-203 |
| 6-47 | Me | 5-Br-6-CN-pyridin-3-yl | 4-F-Ph | Me | 232-234 |
| 6-48 | H | 6-CN-pyridin-3-yl | c-Pr | c-Pr | 248-251 |

[Table 14]

| Seventh Table | | |
|---|---|---|
| compound No. | structural formula | property value |
| 7-1 | 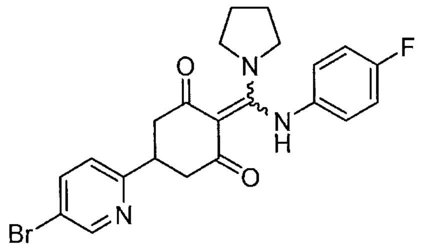 | 111-112 |
| 7-2 | 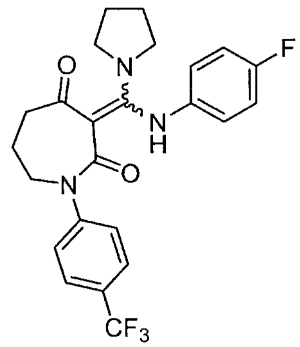 | 167-168 |
| 7-3 | 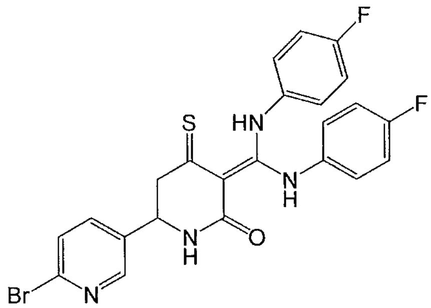 | |
| 7-4 | 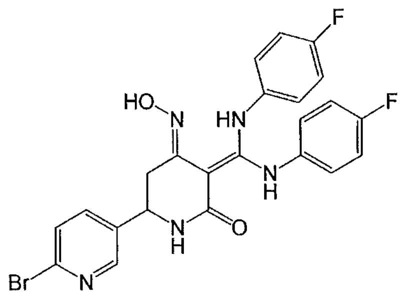 | |
| 7-5 | 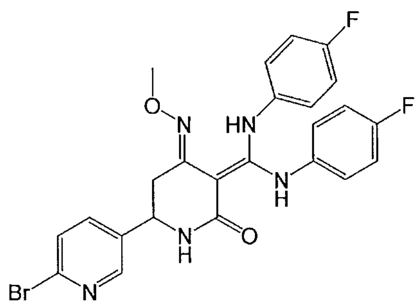 | |

[Table 15]

| continued from Seventh Table | | |
|---|---|---|
| compound No. | structural formula | property value |
| 7-6 | 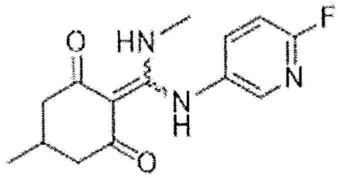 | 215-216 |
| 7-7 | 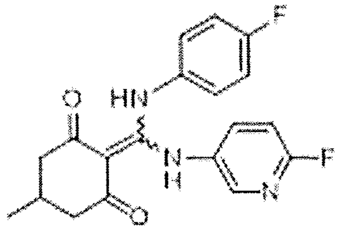 | 223-224 |
| 7-8 | 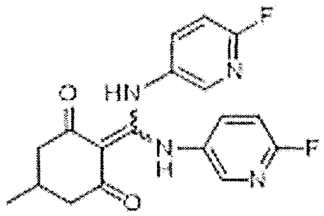 | 244-245 |

[Chem. 20]

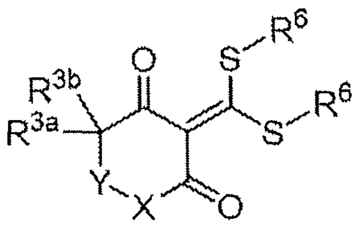

(2)

[Table 16]

| Eighth Table (in Eighth Table, $R^{3a}$ and $R^{3b}$ are each a hydrogen atom) | | | | |
|---|---|---|---|---|
| compound No. | X | Y | $R^6$ | property value |
| 8-1 | $CH_2$ | (5-$CF_3$-pyridin-2-yl)CH | Me | 88-89 |
| 8-2 | $CH_2$ | (4-SMe-Ph)CH | Me | 101-102 |
| 8-3 | $CH_2$ | (5-Br-pyridin-2-yl)CH | Me | 131-132 |
| 8-4 | $CMe_2$ | (5-Br-pyridin-2-yl)CH | Me | 61-62 |
| 8-5 | $CH_2$ | (3-Ph-isoxazol-5-yl)CH | Me | 128-131 |
| 8-6 | $CH_2$ | (thiazol-5-yl)CH | Me | 118-121 |
| 8-7 | $CH_2$ | (5-Cl-3-Me-1-Ph-pyrazol-4-yl)CH | Me | 149-153 |

(continued)

| Eighth Table (in Eighth Table, $R^{3a}$ and $R^{3b}$ are each a hydrogen atom) | | | | |
|---|---|---|---|---|
| compound No. | X | Y | $R^6$ | property value |
| 8-8 | $CH_2$ | (6-$CF_3$-pyridin-3-yl)CH | Me | 159-163 |
| 8-9 | $CH_2$ | (4-Me-Ph)CH | Me | 174-176 |
| 8-10 | $CH_2$ | (5-Cl-thiophen-2-yl)CH | Me | 141-145 |
| 8-11 | $CH_2$ | (5-Cl-thiophen-2-yl)CH | Bn | NMR |
| 8-12 | $CH_2$ | (furan-2-yl)CH | Me | 88-89 |
| 8-13 | $CH_2$ | (4-$CF_3$-Ph)CH | Me | 110-114 |
| 8-14 | $CH_2$ | (4-$CF_3$-Ph)CH | (4-t-Bu-Ph)$CH_2$ | NMR |
| 8-15 | $CH_2$ | (4-$CF_3$-Ph)CH | Bn | NMR |
| 8-16 | $CH_2$ | (5-Cl-3-Me-1-Ph-pyrazol-4-yl)CH | Me | 135-139 |
| 8-17 | O | (4-SMe-Ph)CH | Me | NMR |
| 8-18 | O | (3-Ph-isoxazol-5-yl)CH | Me | 83-86 |
| 8-19 | O | (6-$CF_3$-pyridin-3-yl)CH | Me | 151-155 |
| 8-20 | O | (4-$CF_3$-Ph)CH | Me | 131-133 |
| 8-21 | O | (4-$CF_3$-Ph)CH | Bn | NMR |
| 8-22 | $CH_2$ | t-BuOCON | Me | 95-97 |
| 8-23 | (4-$CF_3$O-Ph)$CH_2$N | $CH_2CH_2$ | Me | NMR |
| 8-24 | BnN | $CH_2CH_2$ | Me | NMR |

[Chem. 21]

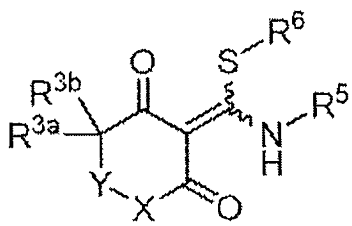

**(3)**

[Table 17]

| Ninth Table | | | | | | | |
|---|---|---|---|---|---|---|---|
| compound No. | X | Y | $R^{3a}$ | $R^{3b}$ | $R^6$ | $R^5$ | property value |
| 9-1 | $CH_2$ | (4-$CF_3$-Ph)CH | H | H | Me | 6-F-pyridin-3-yl | 132-135 |
| 9-2 | $CH_2$ | (5-Br-pyridin-2-yl) CH | H | H | Me | 4-F-Ph | 112-113 |
| 9-3 | $CH_2$ | (4-$CF_3$-Ph)CH | H | H | Me | (4-t-Bu-Ph) $CH_2$ | 125-126 |

(continued)

| Ninth Table | | | | | | | |
|---|---|---|---|---|---|---|---|
| compound No. | X | Y | $R^{3a}$ | $R^{3b}$ | $R^6$ | $R^5$ | property value |
| 9-4 | $CH_2$ | (4-$CF_3$-Ph)CH | H | H | (3-Br-Ph) $CH_2$ | 4-F-Ph | 122-123 |
| 9-5 | $CH_2$ | (furan-2-yl)CH | H | H | Me | Bn | NMR |
| 9-6 | O | (4-Br-Ph)CH | H | H | (4-t-Bu-Ph) $CH_2$ | 4-F-Ph | |
| 9-7 | NMe | (4-$CF_3$-Ph)CH | H | H | (4-t-Bu-Ph) $CH_2$ | 4-F-Ph | NMR |
| 9-8 | NMe | $CH_2$ | 6-$CF_3$-pyridin-3-yl | Et | (4-t-Bu-Ph) $CH_2$ | 4-F-Ph | 162-163 |
| 9-9 | $CH_2$ | (5-CN-pyridin-2-yl)N | H | H | Me | 3,4,5-$F_3$-Ph | 157-158 |
| 9-10 | $CH_2$ | (5-CN-pyridin-2-yl)N | H | H | Me | 4-F-Ph | 209-210 |
| 9-11 | $CH_2$ | (5-$CF_3$-pyrimidin-2-yl)N | H | H | Me | 4-F-Ph | NMR |
| 9-12 | $CH_2$ | t-BuOCON | H | H | Me | 4-F-Ph | NMR |
| 9-13 | (4-$CF_3$-Ph)N | $CH_2CH_2$ | H | H | (4-t-Bu-Ph) $CH_2$ | 4-F-Ph | 129-130 |

[Table 18]

| continued from Ninth Table | | | | | | | |
|---|---|---|---|---|---|---|---|
| compound No. | X | Y | $R^{3a}$ | $R^{3b}$ | $R^6$ | $R^5$ | property value |
| 9-14 | ($CO_2$t-Bu)N | ($CO_2$t-Bu)CH | H | H | Me | 4-F-Ph | |
| 9-15 | $CH_2$ | (3-(2-Cl-Ph)-isoxazol-5-yl)CH | H | H | Me | 4-F-Ph | |
| 9-16 | (pyridin-2-yl)CH | (4-$CF_3$-Ph)CH | H | H | Me | 6-F-pyridin-3-yl | NMR |
| 9-17 | $CH_2$ | (2-Me-oxazol-5-yl) CH | H | H | Me | 4-F-Ph | 43-44 |
| 9-18 | $CH_2$ | (pyrazin-2-yl)CH | H | H | (4-t-Bu-Ph) $CH_2$ | 4-F-Ph | NMR |
| 9-19 | $CH_2$ | (5-Cl-1-Me-3-Ph-pyrazol-4-yl)CH | H | H | (4-t-Bu-Ph) $CH_2$ | 4-F-Ph | NMR |
| 9-20 | $CH_2$ | (pyrimidin-2-yl)CH | H | H | (4-t-Bu-Ph) $CH_2$ | 4-F-Ph | 181-182 |
| 9-21 | NH | (2-$CF_3$-thiazol-5-yl)CH | H | H | Me | 6-F-pyridin-3-yl | 248-250 |
| 9-22 | $CH_2$ | (5-Cl-thiophen-2-yl)CH | H | H | Me | 6-F-pyridin-3-yl | 153-154 |
| 9-23 | NMe | $CH_2$ | 4-$CF_3$-Ph | Et | Me | 6-F-pyridin-3-yl | 78-80 |
| 9-24 | $CH_2$ | $CH_2$ | $CF_3CH_2$ | H | Me | 4-F-Ph | |

(continued)

| continued from Ninth Table | | | | | | | |
|---|---|---|---|---|---|---|---|
| compound No. | X | Y | $R^{3a}$ | $R^{3b}$ | $R^6$ | $R^5$ | property value |
| 9-25 | $CH_2$ | (isothiazol-5-yl)CH | H | H | (4-t-Bu-Ph) $CH_2$ | 6-F-pyr-idin-3-yl | 185-186 |
| 9-26 | $CH_2$ | (CO2t-Bu)N | H | H | Me | 5-F-pyridin-2-yl | |
| 9-27 | $CH_2$ | (Me)CH | H | H | Me | 6-F-pyridin-3-yl | |
| 9-28 | $CH_2$ | (4-$CF_3$-Ph)CH | H | H | (4-t-Bu-Ph) $CH_2$ | 6-F-pyr-idin-3-yl | 188-189 |
| 9-29 | NH | (4-$CF_3$-Ph)CH | H | H | Me | 6-F-pyridin-3-yl | 202-204 |
| 9-30 | NH | (6-Br-pyridin-3-yl) CH | H | H | Me | 6-F-pyridin-3-yl | 196-198 |
| 9-31 | NH | (5-Br-6-Cl-pyridin-3-yl)CH | H | H | Me | 4-F-Ph | 103-104 |
| 9-32 | NH | (5-Br-6-Cl-pyridin-3-yl)CH | H | H | (4-t-Bu-Ph) $CH_2$ | 6-F-pyr-idin-3-yl | 129-130 |
| 9-33 | NH | (5-Br-thiophen-2-yl)CH | H | H | Me | 6-F-pyridin-3-yl | 169-170 |
| 9-34 | (4-$CF_3$-Ph)N | $CH_2$ | H | H | Me | 6-F-pyridin-3-yl | 148-149 |
| 9-35 | NH | (1-Me-5-$OCHF_2$-pyrazol-3-yl)CH | H | H | Me | 6-F-pyridin-3-yl | 49-50 |
| 9-36 | NH | (4-(4-F-Ph)-thiazol-2-yl)CH | H | H | Me | 6-F-pyridin-3-yl | 51-52 |
| 9-37 | NMe | $CH_2$ | 6-$CF_3$-pyri-din-3-yl | Et | Me | 6-F-pyridin-3-yl | |
| 9-38 | NMe | $CH_2$ | quinolin-2-yl | Et | Me | 6-F-pyridin-3-yl | |
| 9-39 | NMe | $CH_2$ | 4-Cl-thiazol- | Et | Me | 6-F-pyridin-3-yl | |
| 9-40 | (4-$CF_3$-Ph)CH | (4-$CF_3$-Ph)CH | H | H | Me | 6-F-pyridin-3-yl | 198-200 |
| 9-41 | (pyridin-3-yl)CH | (4-$CF_3$-Ph)CH | H | H | Me | 6-F-pyridin-3-yl | NMR |

[Table 19]

| continued from Ninth Table | | | | | | | |
|---|---|---|---|---|---|---|---|
| compound No. | X | Y | $R^{3a}$ | $R^{3b}$ | $R^6$ | $R^5$ | property value |
| 9-42 | (pyridin-4-yl)CH | (4-$CF_3$-Ph)CH | H | H | Me | 6-F-pyridin-3-yl | NMR |
| 9-43 | $CH_2$ | (6-Cl-pyridazin-3-yl)CH | H | H | Me | 4-F-Ph | 57-58 |

(continued)

| continued from Ninth Table | | | | | | | |
|---|---|---|---|---|---|---|---|
| compound No. | X | Y | $R^{3a}$ | $R^{3b}$ | $R^6$ | $R^5$ | property value |
| 9-44 | (6-Cl-pyrdin-3-yl) N | $CH_2$ | H | H | Me | 6-F-pyridin-3-yl | 177-178 |
| 9-45 | $CH_2$ | (6-OEt-pyrida-zin-3-yl)CH | H | H | Me | 4-F-Ph | 235-236 |
| 9-46 | (4-Cl-Ph)CH | (4-$CF_3$-Ph)CH | H | H | Me | 6-F-pyridin-3-yl | 188-190 |
| 9-47 | (3-Cl-pyridin-2-yl) CH | (4-$CF_3$-Ph)CH | H | H | Me | 6-F-pyridin-3-yl | 171-173 |
| 9-48 | (2-$CF_3$-pyridin-6-yl)CH | (4-$CF_3$-Ph)CH | H | H | Me | 6-F-pyridin-3-yl | 150-152 |
| 9-49 | (4-Me-Ph)CH | (4-$CF_3$-Ph)CH | H | H | Me | 6-F-pyridin-3-yl | 147-149 |
| 9-50 | (2-Cl-pyridin-4-yl) CH | (4-$CF_3$-Ph)CH | H | H | Me | 6-F-pyridin-3-yl | 163-165 |
| 9-51 | NH | (5-(6-F-pyridin-3-yl)-6-Cl-pyridin-3-yl)CH | H | H | Me | 4-F-Ph | 109-111 |
| 9-52 | NH | (5-(6-F-pyridin-3-yl)-6-Cl-pyridin-3-yl)CH | H | H | Me | 6-F-pyridin-3-yl | 152-155 |
| 9-53 | NH | $CH_2$ | 3-(6-F-pyri-din-3-yl)-4-Cl-Ph | Me | Me | 4-F-Ph | |
| 9-54 | NH | $CH_2$ | 3-(6-F-pyri-din-3-yl)-4-Cl-Ph | Me | Me | 6-F-pyridin-3-yl | |
| 9-55 | NH | $CH_2$ | 5-(6-F-pyri-din-3-yl)-6-Cl-pyridin-3-yl | Me | Me | 6-F-pyridin-3-yl | |
| 9-56 | NH | (6-Br-pyridin-3-yl) CH | H | H | Me | 4-F-Ph | 62-63 |
| 9-57 | NH | (6-$CF_3$-pyridin-3-yl)CH | H | H | Me | 6-F-pyridin-3-yl | 222-224 |

[Table 20]

| Tenth Table | |
|---|---|
| compound No. | $^1$H-NMR data ($CDCl_3$/TMS, ppm) |
| 1-1 | δ 14.34(s, 2H), 7.76-7.70(m, 2H), 7.66-7.59(m, 2H), 7.42-7.36(m, 2H), 7.33-7.27(m, 2H), 7.06-7.00(m, 2H), 3.54-3.42(m, 1H), 2.99-2.78(m, 4H) |
| 1-10 | δ 13.21(s, 1H), 11.76(s, 1H), 8.64-8.61(m, 1H), 7.76(dd, 1H), 7.20-7.01(m, 5H), 3.50-3.37(m, 1H), 2.95(dd, 2H), 2.77(dd, 2H), 2.52(d, 3H) |
| 1-28 | δ 13.61(s, 1H), 12.58(s, 1H), 7.69-7.34(m, 2H), 7.22-7.09(m, 4H), 6.77(dd, 2H), 3.57-3.29(m, 3H), 2.96-2.69(m, 4H) |

(continued)

| Tenth Table | |
|---|---|
| compound No. | $^1$H-NMR data ($CDCl_3$/TMS, ppm) |
| 1-29 | δ 13.23(s, 1H), 12.00(s, 1H), 7.61(d, 2H), 7.37(d, 2H), 7.19-7.12(m, 2H), 7.07(t, 2H), 3.47-3.34(m, 5H), 2.94-2.72(m, 4H) |
| 3-1 | δ 12.9(d, 1H), 11.4(d, 1H), 7.61(d, 2H), 7.31(d, 2H), 7.10-7.13(m, 2H), 7.02-7.06(m, 2H), 4.60-4.62(m, 1H), 3.25-3.33(m, 1H), 3.00-3.02(m, 3H), 2.61-2.66(m, 1H), 2.51-2.54(m, 3H) |
| 3-2 | δ 12.5-13.4(m, 1H), 11.2-12.0(m, 1H), 8.68(s, 1H), 7.85(d, 1H), 7.63(d, 1H), 7.00-7.10(m, 4H), 3.71(d, 1H), 3.60(d, 1H), 3.09(s, 3H), 2.51(d, 3H), 2.14(quint., 1H), 1.95(quint., 1H), 0.78(t, 3H) |
| 3-4 | δ 12.7(d, 1H), 10.8(d, 1H), 7.55-7.70(m, 2H), 6.95-7.48(m, 6H), 5.20-5.30(m, 1H), 4.55-4.75(m, 1H), 3.45-4.60(m, 1H), 3.20-3.40(m, 1H), 2.95-3.05(m, 3H), |
| 8-11 | δ 7.35-7.27(m, 6H), 7.25-7.20(m, 4H), 6.72(d, 1H), 6.60(d, 1H), 4.13(s, 4H), 3.34-3.23(m, 1H), 2.95-2.87(m, 4H), 2.76-2.65(m, 4H) |
| 8-14 | δ 7.59(d, 2H), 7.38-7.25(m, 6H), 7.18(d, 4H), 3.31-3.14(m, 1H), 4.16(s, 4H), 2.90-2.63(m, 4H), 1.30(s, 18H) |
| 8-15 | δ 7.58-7.45(m, 4H), 7.38-7.25(m, 6H), 7.18(d, 4H), 3.30-3.14(m, 1H), 4.22(s, 4H), 2.92-2.62(m, 4H) |
| 8-17 | δ 7.33(d, 2H), 7.26(d, 2H), 5.49-5.44(m, 1H), 2.99-2.82(m, 2H), 2.56(s, 6H), 2.48(s, 3H) |
| 8-21 | δ 7.62-7.56(m, 2H), 7.41-7.33(m, 2H), 7.30-7.12(m, 6H), 5.21(s, 1H), 4.20-4.00(m, 4H), 2.04(s, 2H) |
| 8-23 | δ 7.43(d, 2H), 7.20(d, 2H), 4.74(s, 2H), 3.47(t, 2H), 2.54(s, 3H), 2.49(t, 2H), 2.45(s, 3H), 1.89-1.79(m, 2H) |
| 8-24 | δ 7.43-7.27(m, 5H), 4.75(s, 2H), 3.47(t, 2H), 2.57-2.42(m, 8H), 1.81-1.71(m, 2H) |
| 9-5 | δ 12.84(s, 1H), 7.42-7.28(m, 5H), 6.30(dd, 1H), 6.05(d, 1H), 4.78-4.71(m, 2H), 3.48-3.38(m, 1H), 2.86(dd, 2H), 2.72(dd, 2H), 2.42(s, 3H) |
| 9-7 | δ 14.1(s, 1H), 7.61(m, 2H), 7.21-7.35(m, 6H), 7.08(m, 2H), 6.95(m, 2H), 4.69(m, 1H), 3.55(m, 2H), 3.31(m, 1H), 3.11(m, 3H), 2.77(m, 1H), 1.27(m, 9H) |
| 9-11 | δ 14.4(br, 1H), 8.56(d, 2H), 7.31-7.27(m, 2H), 7.16-7.12(m, 2H), 4.69(s, 4H), 2.10(s, 3H) |
| 9-12 | δ 14.6(br, 0.75H), 9.76(br, 0.25), 7.42-7.40(m, 1H), 7.15-7.06(m, 3H), 4.22(s, 4H), 2.15(s, 3H), 1.48(s, 9H) |

[Table 21]

| continued from Tenth Table | |
|---|---|
| compound No. | H-NMR data ($CDCl_3$/TMS, ppm) |
| 1-83 | δ 13.37(s, 1H), 11.88(s, 1H), 8.45(d, 2H), 8.09(s, 1H), 7.65-7.60(m, 1H), 7.46(d, 2H), 7.19(d, 2H), 7.00-6.97(m, 3H), 3.97-3.94(m, 1H), 3.65-3.58(m, 1H), 3.02-2.83(m, 2H), 2.58(d, 3H) |
| 9-16 | δ 8.53-8.51(m, 1H), 8.20-8.18(m, 1H), 7.79-7.75(m, 1H), 7.62-7.43(m, 4H), 7.23-7.21(d, 2H), 7.10-7.07(m, 1H), 7.02-6.93(m, 1H), 4.12-3.92(m, 2H), 3.01-2.91(m, 2H), 2.07(s, 3H) |
| 9-18 | δ 14.93(br, 1H), 8.55-8.52(m, 2H), 8.48(d, 1H), 7.36-7.30(m, 2H), 7.29-7.24(m, 2H), 7.16-7.10(m, 2H), 7.04-7.00(m, 2H), 3.65(s, 2H), 3.60-3.50(m, 1H), 3.00(dd, 2H), 2.84(dd, 2H), 1.27(s, 9H) |
| 9-19 | δ 14.98(br, 1H), 7.44-7.36(m, 5H), 7.34-7.28(m, 2H), 7.25-7.21(m, 2H), 7.15-7.09(m, 2H), 7.01-6.97(m, 2H), 3.89(s, 3H), 3.56(s, 2H), 3.55-3.45(m, 1H), 3.11(dd, 2H), 2.65(dd, 2H), 1.26(s, 9H) |
| 9-41 | δ 8.42-8.40(m, 1H), 8.30-8.23(m, 2H), 7.83-7.78(m, 1H), 7.47(d, 2H), 7.36-7.33(m, 1H), 7.21-7.14(m, 3H), 7.06-7.03(m, 1H), 3.97(d, 1H), 3.71-3.64(m, 1H), 3.04-2.92(m, 2H), 2.06(s, 3H) |
| 9-42 | δ 8.46-8.44(m, 2H), 8.24-8.23(m, 1H), 7.83-7.78(m, 1H), 7.47(d, 2H), 7.19(d, 2H), 7.04-7.00(m, 1H), 6.97-6.96(m, 2H), 3.95(d, 1H), 3.71-3.64(m, 1H), 3.06-2.91(m, 2H), 2.05(s, 3H) |

Pest control agent containing the compound represented by the formula (1) of the present invention or a salt thereof as an active ingredient can be applied as an agrohorticultural insect pest control agent, an ectoparasite control agent for animal, and an endoparasite control agent for animal. Agrohorticultural insect pest control agents are applicable to controlling various insect pests such as agrohorticultural insect pests, stored grain insect pests, sanitary insect pests, nematodes, and the like, which are injurious to paddy rice, fruit trees, vegetables, other crops, flowers and ornamental plants.

In addition, agrohorticultural insecticides containing the compound represented by the formula (1) of the present invention or salts thereof as an active ingredient are superior in physical properties suitable for various labor-saving application methods, systemic transfer activity, and environmental safety such as appropriate soil persistence and the like.

Furthermore, agrohorticultural insecticides containing the compound represented by the formula (1) of the present invention or a salt thereof as an active ingredient have little impact on natural enemies; useful insects such as Apis mellifera, bumblebee, and the like; environmental organisms such as midge and the like.

Furthermore, ectoparasite control agents for animals and endoparasite control agents for animals, particularly, endoparasite control agents for animals, containing the compound represented by the formula (1) of the present invention or a salt thereof as an active ingredient are superior in safety and pharmacokinetic aspects.

Examples of the above-mentioned insect pest, Nematoda and the like include the following.

Examples of the insect pest of Lepidoptera include Parasa consocia, Anomis mesogona, Papilio xuthus, Matsumuraeses azukivora, Ostrinia scapulalis, Spodoptera exempta, Hyphantria cunea, Ostrinia furnacalis, Pseudaletia separata, Tinea translucens, Bactra furfuryla, Parnara guttata, Marasmia exigua, Sesamia inferens, Brachmia triannulella, Monema flavescens, Trichoplusia ni, Pleuroptya ruralis, Cystidia couaggaria, Lampides boeticus, Cephonodes hylas, Helicoverpa armigera, Phalerodonta manleyi, Eumeta japonica, Pieris brassicae, Malacosoma neustria testacea, Stathmopoda masinissa, Cuphodes diospyrosella, Archips xylosteanus, Agrotis segetum, Tetramoera schistaceana, Papilio machaon hippocrates, Endoclyta sinensis, Lyonetia prunifoliella, Phyllonorycter ringoneella, Cydia kurokoi, Eucoenogenes aestuosa, Lobesia botrana, Latoia sinica, Euzophera batangensis, Phalonidia mesotypa, Spilosoma imparilis, Glyphodes pyloalis, Olethreutes mori, Tineola bisselliella, Endoclyta excrescens, Nemapogon granellus, Synanthedon hector, Cydia pomonella, Plutella xylostella, Cnaphalocrocis medinalis, Sesamia calamistis, Scirpophaga incertulas, Pediasia teterrellus, Phthorimaea operculella, Stauropus fagi persimilis, Etiella zinckenella, Spodoptera exigua, Palpifer sexnotata, Spodoptera mauritia, Scirpophaga innotata, Xestia c-nigrum, Spodoptera depravata, Ephestia kuehniella, Angerona prunaria, Clostera anastomosis, Pseudoplusia includens, Matsumuraeses falcana, Helicoverpa assulta, Autographa nigrisigna, Agrotis ipsilon, Euproctis pseudoconspersa, Adoxophyes orana, Caloptilia theivora, Homona magnanima, Ephestia elutella, Eumeta minuscula, Clostera anachoreta, Heliothis maritima, Sparganothis pilleriana, Busseola fusca, Euproctis subflava, Biston robustum, Heliothis zea, Aedia leucomelas, Narosoideus flavidorsalis, Viminia rumicis, Bucculatrix pyrivorella, Grapholita molesta, Spulerina astaurota, Ectomyelois pyrivorella, Chilo suppressalis, Acrolepiopsis sapporensis, Plodia interpunctella, Hellula undalis, Sitotroga cerealella, Spodoptera litura, Eucosma aporema, Acleris comariana, Scopelodes contractus, Orgyia thyellina, Spodoptera frugiperda, Ostrinia zaguliaevi, Naranga aenescens, Andraca bipunctata, Paranthrene regalis, Acosmeryx castanea, Phyllocnistis toparcha, Endopiza viteana, Eupoecillia ambiguella, Anticarsia gemmatalis, Cnephasia cinereipalpana, Lymantria dispar, Dendrolimus spectabilis, Leguminivora glycinivorella, Maruca testulalis, Matsumuraeses phaseoli, Caloptilia soyella, Phyllocnistis citrella, Omiodes indicate, Archips fuscocupreanus, Acanthoplusia agnata, Bambalina sp., Carposina niponensis, Conogethes punctiferalis, Synanthedon sp., Lyonetia clerkella, Papilio helenus, Colias erate poliographus, Phalera flavescens, Pieridae such as Pieris rapae crucivora, Pieris rapae, and the like, Euproctis similis, Acrolepiopsis suzukiella, Ostrinia nubilalis, Mamestra brassicae, Ascotis selenaria, Phtheochroides clandestina, Hoshinoa adumbratana, Odonestis pruni japonensis, Triaena intermedia, Adoxophyes orana fasciata, Grapholita inopinata, Spilonota ocellana, Spilonota lechriaspis, Illiberis pruni, Argyresthia conjugella, Caloptilia zachrysa, Archips breviplicanus, Anomis flava, Pectinophora gossypiella, Notarcha derogata, Diaphania indica, Heliothis virescens, Earias cupreoviridis, and the like.

Examples of the insect pest of Hemiptera include Nezara antennata, Stenotus rubrovittatus, Graphosoma rubrolineatum, Trigonotylus coelestialium, etc., Aeschynteles maculatus, Creontiades pallidifer, Dysdercus cingulatus, Chrysomphalus ficus, Aonidiella aurantii, Graptopsaltria nigrofuscata, Blissusleucopterus, Icerya purchasi, Piezodorus hybneri, Lagynotomus elongatus, Thaia subrufa, Scotinophara lurida, Sitobion ibarae, Stariodes iwasakii, Aspidiotus destructor, Taylorilygus pallidulus, Myzusmumecola, Pseudaulacaspis prunicola, Acyrthosiphon pisum, Anacanthocoris striicornis, Ectometopterus micantulus, Eysarcoris lewisi, Molipteryx fuliginosa, Cicadella viridis, Rhopalosophum rufiabdominalis, Saissetia oleate, Trialeurodes vaporariorum, Aguriahana quercus, Lygus spp., Euceraphis punctipennis, Andaspis kashicola, Coccus pseudomagnoliarum, Cavelerius saccharivorus, Galeatus spinifrons, Macrosiphoniella sanborni, Aonidiella citrina, Halyomorpha mista, Stephanitis fasciicarina, Trioza camphorae, Leptocorisa chinensis, Trioza quercicola, Uhlerites latius, Erythroneura comes, Paromius exiguus, Duplaspidiotus claviger, Nephotettix nigropictus, Halticiellus insularis, Perkinsiella saccharicida, Psylla malivorella, Anomomeura mori, Pseudococcus longispinis, Pseudaulacaspis pentagona, Pulvinaria kuwacola, Apolygus lucorum, Togo hemipterus, Toxoptera aurantii, Saccharicoccus sacchari, Geoica lucifuga, Numata muiri, Comstockaspis perniciosa, Unaspis citri, Aulacorthum solani, Eysarcoris ventralis, Bemisia argentifolii, Cicadella spectra, Aspidiotus hederae, Liorhyssus hyalinus, Calophya nigridorsalis, Sogatella furcifera, Megoura crassicauda, Brevicoryne brassicae, Aphis glycines, Leptocorisa oratorius, Nephotettix virescens, Uroeucon formosanum, Cyrtopeltis tennuis, Bemisia tabaci, Lecanium persicae, Parlatoria theae, Pseudaonidia paeoniae, Empoasca onukii, Plautia stali, Dysaphis tulipae, Macrosiphum euphorbiae, Stephanitis pyrioides, Ceroplastes ceriferus, Parlatoria camelliae, Apolygus spinolai, Nephotettix cincticeps, Glaucias subpunctatus, Orthotylus flavosparsus, Rhopalosiphum maidis, Peregrinus maidis, Eysarcoris parvus, Cimex lectularius, Psylla abieti, Nilaparvata lugens, Psylla tobirae, Eurydema rugosum, Schizaphis piricola, Psylla pyricola, Parlatoreopsis pyri, Stephanitis nashi, Dysmicoccus wistariae, Lepholeucaspis japonica, Sappaphis piri, Lipaphis erysimi, Neotoxoptera formosana, Rhopalosophum nymphaeae, Edwardsianarosae, Pinnaspisaspidistrae, Psylla alni, Speusotettix subfusculus, Alnetoidia alneti, Sogatella panicicola, Adelphocoris lineolatus, Dysdercus poecilus, Parlatoria ziziphi, Uhlerites debile, Laodelphax striatellus, Eurydema pulchrum, Cletus trigonus, Clovia punctata, Empoasca sp., Coccus hesperidum, Pachybrachius luridus, Planococcus kraunhiae, Stenotus binotatus, Arboridia apicalis, Macrosteles fascifrons, Dolycoris baccarum, Adelphocoris triannulatus, Viteus vitifolii, Acanthocoris sordidus, Leptocorisa acuta, Macropes obnubilus, Cletus punctiger, Riptortus clavatus, Paratrioza cockerelli, Aphrophora costalis, Lygus disponsi, Lygus saundersi, Crisicoccus pini, Empoasca abietis, Crisicoccus matsumotoi, Aphis craccivora, Megacopta punctatissimum, Eysarcoris guttiger, Lepidosaphes beckii, Diaphorina citri, Toxoptera citricidus, Planococcus citri, Dialeurodes citri, Aleurocanthus spiniferus, Pseudococcus citriculus, Zyginella citri, Pulvinaria citricola, Coccus discrepans, Pseudaonidia duplex, Pulvinaria aurantii, Lecanium corni, Nezara viridula, Stenodema calcaratum, Rhopalosiphum padi, Sitobion akebiae, Schizaphis graminum, Sorhoanus tritici, Brachycaudus helichrysi, Carpocoris purpureipennis, Myzus persicae, Hyalopterus pruni, Aphis farinose yanagicola, Metasalis populi, Unaspis yanonensis, Mesohomotoma camphorae, Aphis spiraecola, Aphis pomi, Lepidosaphes ulmi, Psylla mali, Heterocordylus flavipes, Myzus malisuctus, Aphidonuguis mali, Orientus ishidai, Ovatus malicolens, Eriosoma lanigerum, Ceroplastes rubens, Aphis gossypii, and the like.

Examples of the insect pest of Coleoptera include Xystrocera globosa, Paederus fuscipes, Eucetonia roelofsi, Callosobruchus chinensis, Cylas formicarius, Hypera postica, Echinocnemus squameus, Oulema oryzae, Donacia provosti, Lissorhoptrus oryzophilus, Colasposoma dauricum, Euscepes postfasciatus, Epilachna varivestis, Acanthoscelides obtectus, Diabrotica virgifera virgifera, Involvulus cupreus, Aulacophora femoralis, Bruchus pisorum, Epilachna vigintioctomaculata, Carpophilus dimidiatus, Cassida nebulosa, Luperomorpha tunebrosa, Phyllotreta striolata, Psacothea hilaris, Aeolesthes chrysothrix, Curculio sikkimensis, Carpophilus hemipterus, Oxycetonia jucunda, Diabrotica spp., Mimela splendens, Sitophilus zeamais, Tribolium castaneum, Sitophilus oryzae, Palorus subdepressus, Melolontha japonica, Anoplophora malasiaca, Neatus picipes, Leptinotarsa decemlineata, Diabrotica undecimpunctata howardi, Sphenophorus venatus, Crioceris quatuordecimpunctata, Conotrachelus nenuphar, Ceuthorhynchidius albosuturalis, Phaedon brassicae, Lasioderma serricorne, Sitona japonicus, Adoretus tenuimaculatus, Tenebrio molitor, Basilepta balyi, Hypera nigrirostris, Chaetocnema concinna, Anomala cuprea, Heptophylla picea, Epilachna vigintioctopunctata, Diabrotica longicornis, Eucetonia pilifera, Agriotes spp., Attagenus unicolor japonicus, Pagria signata, Anomala rufocuprea, Palorus ratzeburgii, Alphitobius laevigatus, Anthrenus verbasci, Lyctus brunneus, Tribolium confusum, Medythia nigrobilineata, Xylotrechus pyrrhoderus, Epitrix cucumeris, Tomicus piniperda, Monochamus alternatus, Popillia japonica, Epicauta gorhami, Sitophilus zeamais, Rhynchites heros, Listroderes costirostris, Callosobruchus maculatus, Phyllobius armatus, Anthonomus pomorum, Linaeidea aenea, Anthonomus grandis, and the like.

Examples of the insect pest of Diptera include Culex pipiens pallens, Pegomya hyoscyami, Liriomyza huidobrensis, Musca domestica, Chlorops oryzae, Hydrellia sasakii, Agromyza oryzae, Hydrellia griseola, Ophiomyia phaseoli, Dacus cucurbitae, Drosophila suzukii, Rhacochlaena japonica, Muscina stabulans, Phoridae such as Megaselia spiracularis and the like, Clogmia albipunctata, Tipula aino, Phormia regina, Culex tritaeniorhynchus, Anopheles sinensis, Hylemya brassicae, Asphondylia sp., Delia platura, Delia antiqua, Rhagoletis cerasi, Culex pipiens molestus forskal, Ceratitis capitata, Bradysia agrestis, Pegomya cunicularia, Liriomyza sativae, Liriomyza bryoniae, Chromatomyia horticola, Liriomyza chinensis, Culex quinquefasciatus, Aedes aegypti, Aedes albopictus, Liriomyza trifolii, Liriomyza sativae, Dacus dorsalis, Dacus tsuneonis, Sitodiplosis mosellana, Meromuza nigriventris, Anastrepha ludens, Rhagoletis pomonella, and the like.

Examples of the insect pest of Hymenoptera include Pristomyrmex pungens, Bethylidae, Monomorium pharaohnis, Pheidole noda, Athalia rosae, Dryocosmus kuriphilus, Formica fusca japonica, Vespoidea, Athalia infumata infumata, Arge pagana, Athalia japonica, Acromyrmex spp., Solenopsis spp., Arge mali, Ochetellus glaber, and the like.

Examples of the insect pest of Orthoptera include Homorocoryphus lineosus, Gryllotalpa sp., Oxya hyla intricata, Oxya yezoensis, Locusta migratoria, Oxya japonica, Homorocoryphus jezoensis, Teleogryllus emma, and the like.

Examples of the insect pest of Thysanoptera include Selenothrips rubrocinctus, Stenchaetothrips biformis, Haplothrips aculeatus, Ponticulothrips diospyrosi, Thrips flavus, Anaphothrips obscurus, Liothrips floridensis, Thrips simplex, Thrips nigropilosus, Heliothrips haemorrhoidalis, Pseudodendrothrips mori, Microcephalothrips abdominalis, Leeuwenia pasanii, Litotetothrips pasaniae, Scirtothrips citri, Haplothrips chinensis, Mycterothrips glycines, Thrips setosus, Scirtothrips dorsalis, Dendrothrips minowai, Haplothrips niger, Thrips tabaci, Thrips alliorum, Thrips hawaiiensis, Haplothrips kurdjumovi, Chirothrips manicatus, Frankliniella intonsa, Thrips coloratus, Franklinella occidentalis, Thrips

palmi, Frankliniella lilivora, Liothrips vaneeckei, and the like.

Examples of the insect pest of Acari include Leptotrombidium akamushi, Tetranychus ludeni, Dermacentor variabilis, Tetranychus truncatus, Ornithonyssus bacoti, Demodex canis, Tetranychus viennensis, Tetranychus kanzawai, Ixodes such as Rhipicephalus sanguineus, and the like, Cheyletus malaccensis, Tyrophagus putrescentiae, Dermatophagoides farinae, Latrodectus hasseltii, Dermacentor taiwanicus, Acaphylla theavagrans, Polyphagotarsonemus latus, Aculops lycopersici, Ornithonyssus sylvairum, Tetranychus urticae, Eriophyes chibaensis, Sarcoptes scabiei, Haemaphysalis longicornis, Ixodes scapularis, Tyrophagus similis, Cheyletus eruditus, Panonychus citri, Cheyletus moorei, Brevipalpus phoenicis, Octodectes cynotis, Dermatophagoides ptrenyssnus, Haemaphysalis flava, Ixodes ovatus, Phyllocoptruta citri, Aculus schlechtendali, Panonychus ulmi, Amblyomma americanum, Dermanyssus gallinae, Rhyzoglyphus robini, Sancassania sp., and the like.

Examples of the insect pest of Isoptera include Reticulitermes miyatakei, Incisitermes minor, Coptotermes formosanus, Hodotermopsis japonica, Reticulitermes sp., Reticulitermes flaviceps amamianus, Glyptotermes kushimensis, Coptotermes guangzhoensis, Neotermes koshunensis, Glyptotermes kodamai, Glyptotermes satsumensis, Cryptotermes domesticus, Odontotermes formosanus, Glyptotermes nakajimai, Pericapritermes nitobei, Reticulitermes speratus, and the like.

Examples of the insect pest of Blattodea include Periplaneta fuliginosa, Blattella germanica, Blatta orientalis, Periplaneta brunnea, Blattella lituricollis, Periplaneta japonica, Periplaneta americana, and the like.

Examples of the Nematoda include Nothotylenchus acris, Aphelenchoides besseyi, Pratylenchus penetrans, Meloidogyne hapla, Meloidogyne incognita, Globodera rostochiensis, Meloidogyne javanica, Heterodera glycines, Pratylenchus coffeae, Pratylenchus neglectus, Tylenchus semipenetrans, and the like.

Examples of the Mollusca include Pomacea canaliculata, Achatina fulica, Meghimatium bilineatum, Lehmannina valentiana, Limax flavus, Acusta despecta sieboldiana, and the like.

The agrohorticultural insect pest control agent of the present invention also has a strong control effect on Tuta absoluta as other insect pest.

The agrohorticultural insect pest control agent containing the compound represented by the formula (1) of the present invention or salts thereof as an active ingredient has a marked control effect on the aforementioned insect pests, which are injurious to paddy field crops, upland crops, fruit trees, vegetables and other crops, flowers and ornamental plants, and the like. Therefore, the desired effect of the agrohorticultural insect pest control agent of the present invention can be exhibited by applying the agents to propagation facility, paddy field, field, fruit trees, vegetables, other crops, seeds of flowers and ornament plants, cultivation carriers such as paddy field water, stem and leaves, soil, and the like, and the like, at a season at which the insect pests are expected to appear, before their appearance or at the time when their appearance is confirmed. Particularly, preferred use form is application utilizing what is called systemic/penetration transferability by allowing absorption of the compound of the present invention from roots via or not via soil, by treating propagation soil for crops, flowers, ornamental plants, and the like, planting hole soil for transplantation, plant foot, irrigation water, culture water for hydroponic culture, and the like.

The useful plants, for which the agrohorticultural insect pest control agent of the present invention can be used, are not specifically limited, and examples thereof include plants such as cereals (e.g. rice, barley, wheat, rye, oat, corn, etc.), legume (soybean, adzuki bean, fava bean, bean, kidney bean, peanut, etc.), fruit trees and fruits (apple, citrus fruits, pear, grapes, peach, plum, cherry fruit, walnut, sweet chestnut, almond, banana, etc.), vegetables (cabbage, tomato, spinach, broccoli, lettuce, onion, green onion (chive, shallot), green pepper, eggplant, strawberry, pepper, okra, green chive, etc.), root vegetables (carrot, potato, sweet potato, tannia, radish, turnip, lotus root, burdock, garlic, Allium bakeri, etc.), crop for processing (cotton, hemp, beet, hop, sugar cane, sugar beet, olive, rubber, coffee, tobacco, tea, etc.), gourd (pumpkin, cucumber, watermelon, oriental melon, melon, etc.), feed crop (orchard grass, sorghum, timothy, clover, alfalfa, etc.), grass (Korean lawn grass, bent grass, etc.), ornamental plants such as spices (lavender, rosemary, thyme, parsley, pepper, ginger, etc.), flowers and ornamental plants (chrysanthemum, rose, carnation, orchid, tulip, lily, etc.), garden tree (gingko, Japanese cherry, aucuba, etc.), forest tree (Abies sachalinensis, Picea jezoensis, pines, Thujopsis dolabrata, Japanese cedar, Japanese cypress, eucalyptus, etc.), and the like.

The above-mentioned "plant" also includes plants imparted with resistance to, for example, HPPD inhibitors such as isoxaflutole and the like; ALS inhibitors such as imazethapyr, thifensulfuron methyl, and the like; EPSP synthetase inhibitors such as glyphosate and the like; glutamine synthetase inhibitors such as glufosinate and the like; acetyl CoA carboxylase inhibitors such as sethoxydim and the like; and herbicides such as broxynil, dicamba, 2,4-D, and the like, by a classical breeding method or gene recombination technique.

Examples of the "plant" imparted with resistance by a classical breeding method include colza, wheat, sunflower, and rice resistant to imidazolinone ALS inhibitory herbicides such as imazethapyr and the like, which are already commercially available as a trade name of "Clearfield" (registered trademark). Similarly, soybean resistant to sulfonylurea ALS inhibitory herbicides such as thifensulfuron methyl and the like by a classical breeding method is already commercially available as a trade name of "STS soybean". Similarly, examples of the plant imparted with resistance to acetyl CoA carboxylase inhibitors such as trione oxime-based and aryloxyphenoxypropionic acid-based herbicides and the like, by a

classical breeding method include SR corn and the like.

The plant imparted with resistance to acetyl CoA carboxylase inhibitors is described in Proc. Natl. Acad. Sci. USA, vol. 87, pages 7175 - 7179 (1990) and the like. In addition, mutated acetyl CoA carboxylase resistant to acetyl CoA carboxylase inhibitors is reported in Weed Science, vol. 53, pages 728 - 746 (2005) and the like, and a plant resistant to acetyl CoA carboxylase inhibitors can be created by introducing such mutated acetyl CoA carboxylase gene into a plant by a gene recombination technique or introducing a mutation involved in imparting resistance into acetyl CoA carboxylase of the plant. Furthermore, a plant resistant to acetyl CoA carboxylase inhibitor, ALS inhibitor, and the like can be created by introducing a mutation of site-specific amino acid substitution into the acetyl CoA carboxylase gene, ALS gene, and the like of the plant by introducing a base-substituted and mutation-introducing nucleic acid represented by a chimeraplasty technique (Gura T. 1999. Repairing the Genome's Spelling Mistakes. Science 285: 316-318.) into the plant cell. The agrohorticultural insect pest control agent of the present invention can also be used for these plants.

Furthermore, as the toxins produced in such genetically modified plants, insecticidal proteins derived from Bacillus cereus or Bacillus popilliae; insecticidal proteins such as δ-endotoxins (e.g. CrylAb, CrylAc, CrylF, CrylFa2, Cry2Ab, Cry3A, Cry3Bbl, Cry9C, etc.), VIP1, VIP2, VIP3, VIP3A, and the like, which are derived from Bacillus thuringiensis; insecticidal proteins derived from nematodes; toxins produced by animals, such as scorpion toxin, spider toxin, bee toxin, insect-specific neurotoxins, and the like; filamentous fungi toxins; plant lectins; agglutinin; protease inhibitors such as trypsin inhibitor, serine protease inhibitor, patatin, cystatin, papain inhibitor, and the like; ribosome-inactivating proteins (RIP) such as ricin, corn-RIP, abrin, rufin, sapolin, priodin, and the like; steroid metabolic enzymes such as 3-hydroxysteroid oxidase, ecdysteroid-UDP-glucosyltransferase, cholesterol oxidase, and the like; ecdysone inhibitors; HMG-CoA reductase; ion channel inhibitors such as a sodium channel inhibitor, calcium channel inhibitor, and the like; juvenile hormone esterase; diuretic hormone acceptor; stilbene synthetase; bibenzyl synthetase; chitinase; glucanase, and the like can be mentioned.

The toxins produced in such genetically modified plants also include hybrid toxins, partially deficient toxins, and modified toxins of δ-endotoxin proteins such as CrylAb, CrylAc, CrylF, CrylFa2, Cry2Ab, Cry3A, Cry3Bbl, Cry9C, Cry34Ab, and Cry35Ab; insecticidal proteins such as VIPl, VIP2, VIP3, VIP3A, and the like. The hybrid toxins are produced by a novel combination of the different domains of such a protein by adopting recombination technology. The known partially deficient toxin is CrylAb, in which a part of amino acid sequence is deficient. In the modified toxins, one or a plurality of amino acids of a natural toxin is/are replaced.

Examples of such toxins and genetically modified plants capable of synthesizing such toxins are described in EP-A-0 374 753, WO 93/07278, WO 95/34656, EP-A-0 427 529, EP-A-451 878, WO 03/052073, and the like.

The toxins contained in such genetically modified plants impart resistance to insect pests of Coleoptera, insect pests of Hemipteria, insect pests of Diptera, insect pests of Lepidoptera, and Nematoda to the plants. The agrohorticultural insect pest control agent of the present invention can also be used in combination with or by systematization with such technique.

For control of various insect pests, the agrohorticultural insect pest control agent of the present invention need only be applied to the plant expected to allow occurrence of insect pest and nematode, as it is or after being appropriately diluted with or suspended in water or the like, in an amount effective for control of the insect pest or nematode. For example, for insect pest and nematode that occur in fruit trees, cereals, vegetables, and the like, the agent is sprayed on the stem and leaf parts, or absorbed from the root by a seed treatment such as dipping the seeds in a drug, dressing of seeds, Calper treatment, and the like, a soil treatment such as soil whole layer blending, planting row application, bed soil blending, cell seedling treatment, planting hole treatment, plant foot/base treatment, top dress, nursery box treatment of rice, application on water surface, and the like, or the like. In addition, the agrohorticultural insect pest control agent can also be used by application to a culture fluid for culture fluid (hydroponic) culture, application by smoking, tree stem injection, or the like.

The agrohorticultural insect pest control agent of the present invention need only be applied to the place expected to allow development of insect pests, as it is or after being appropriately diluted with or suspended in water or the like, in an amount effective for control of the insect pests. For example, spraying on grain-storage insect pests, house insect pests, hygiene insect pests, forest insect pests, and the like, as well as application, smoking, bait to house and architectural materials and the like can also be employed for use.

As methods for the seed treatment, a method comprising penetration of a liquid agent obtained by diluting or not a liquid formulation or a solid formulation by dipping seeds in the agent, a method including adhering a solid formulation or liquid formulation to the surfaces of seeds by admixing and dressing the seeds with the formulation, a method comprising coating seeds by admixing the seeds with adhesive carriers such as resin, polymer, and the like, a method comprising applying near the seeds simultaneously with planting, and the like can be mentioned.

The "seed" to be subjected to the seed treatment means a plant body in an early stage of growth, which is used for propagation, and examples thereof include seeds, as well as bulb, tuber, seed potato, germ, propagule, scaly bulb, a plant body for vegetative propagation by cutting propagation, and the like.

The "soil" and "cultivation carrier" for plant when practicing the method of use of the present invention mean supports for cultivation of plants, particularly supports for growing roots, and the material is not particularly limited. The

material may be any as long as plants can grow therein and specific materials include, for example, soils, nursery mat, water, and the like. As a specific material, for example, sand pumice, vermiculite, diatomaceous earth, agar, gelled substance, polymer substance, rock wool, glass wool, wood chip, bark, and the like can also be used.

As a method of application to crop plant stem and leaves, grain storage insect pests, house insect pests, sanitary insect pests, forest insect pests, or the like, a method including appropriately diluting a liquid formulation such as emulsion, flowable formulation, and the like, or a solid formulation such as a wettable powder, water dispersible granule, and the like with water, and spraying same, a method of spraying a dust, smoking, and the like can be mentioned.

For application to the soil, for example, a method comprising application of a liquid formulation to the plant foot of plant body, nursery for raising seedling and the like with or without dilution in water; a method comprising application of granules to the plant foot of plant body or nursery for raising seedling; a method comprising application of dust, wettable powder, water dispersible granule, granule, and the like before sowing or transplanting to allow them to be incorporated into the entire soil; a method comprising application of dust, wettable powder, water dispersible granule, granule, and the like to planting hole, planting row, and the like before sowing or planting, and the like can be mentioned.

In the case of the paddy rice nursery box, the formulation may vary depending on the timing of, for example, application on sowing, application during greening, application during transplanting, and the like. The formulations of dust, water dispersible granule, granule, and the like can be employed. Application is also possible by incorporation into the grove soil, wherein the grove soil may be mixed with dust, water dispersible granule, granule, and the like and, for example, incorporation into bed soil, incorporation into cover soil, incorporation into the entire grove soil, and the like can be employed. Simply, a grove soil and various formulations may be applied in alternate layers.

As a method of application to a paddy field, a solid formulation such as jumbo, pack, granule, water dispersible granule, and the like, a liquid formulation such as flowable, emulsion, and the like are generally sprayed on a flooded paddy field. Alternatively, during rice planting, a suitable formulation can also be applied or injected to soil directly or after blending with a fertilizer. Moreover, by utilizing a liquid agent such as emulsion, flowable, and the like to a water inlet and the flow source of water into paddy fields such as an irrigation apparatus and the like, a saving application along with the supply of water can also be performed.

For upland crops, treatment of seeds, a cultivation carrier to be placed near the plant body, and the like during the period of from sowing to raising seedling is possible. For plants to be directly sown in the field, a direct treatment of seeds, a treatment of a plant foot of the plant under cultivation, and the like are preferred. An application of granules, a soil injection treatment with a liquid agent with or without dilution with water, and the like can be performed. It is also a preferred treatment to blend granules with a cultivation carrier before seeding, and seed the blend.

For a treatment on sowing or during raising seedling of a cultivated plant to be transplanted, a direct treatment of seeds, a soil injection treatment of nursery for raising seedling with a liquid agent, and a dispersal treatment thereof with granules are preferred. In addition, treatment of a planting hole with granules and mixing of a cultivation carrier to be placed near the transplantation site with the granules during fix planting are also preferred treatments.

The agrohorticultural insect pest control agent of the present invention is generally prepared into conveniently usable forms according to an ordinary manner for preparation of agrochemicals.

That is, the compound represented by the formula (1) or a salt thereof of the present invention are blended with a suitable inert carrier and optionally, an adjuvant in a proper proportion and prepared into a suitable preparation form such as a suspension, emulsion, liquid formulation, wettable powder, granular wettable powder, granules, dust, tablets, pack, or the like through dissolution, dispersion, suspension, mixing, impregnation, adsorption, or sticking.

The agrohorticultural insect pest control agent of the present invention can contain, besides active ingredients as necessary, additional components generally used for pesticide preparation. Examples of the additional component include carriers such as solid carrier, liquid carrier, and the like, surfactant, dispersant, wetting agent, binder, tackifier, thickener, colorant, spreader, sticker, antifreezing agent, anticaking agent, disintegrant, decomposition inhibitor, and the like. Where necessary, preservative, plant detritus, and the like may be used as other additional components. These addition components may be used alone or in a mixture of two or more kinds thereof.

Examples of the solid carrier include natural minerals such as quartz, clay, kaolinite, pyrophyllite, sericite, talc, bentonite, acid clay, attapulgite, zeolite, diatomaceous earth, and the like; inorganic salts such as calcium carbonate, ammonium sulfate, sodium sulfate, potassium chloride, and the like; organic solid carriers such as synthetic silicic acid, synthetic silicate, starch, cellulose, vegetable powder (e.g., sawdust, coconut shell, corn cob, tobacco stalk, etc.), and the like; plastic carriers such as polyethylene, polypropylene, poly(vinylidene chloride), and the like; urea; hollow inorganic bodies; hollow plastic bodies; fumed silica (white carbon), and the like. These may be used alone or in a mixture of two or more kinds thereof.

Examples of the liquid carrier include monohydric alcohols such as methanol, ethanol, propanol, isopropanol, butanol, and the like; polyhydric alcohols such as ethylene glycol, diethylene glycol, propylene glycol, hexylene glycol, polyethylene glycol, polypropylene glycol, glycerin, and the like; polyhydric alcohol ethers such as propylene glycol ether and the like; ketones such as acetone, methyl ethyl ketone, methyl isobutyl ketone, diisobutyl ketone, cyclohexanone, and the like; ethers such as ethyl ether, dioxane, ethylene glycol monoethyl ether, dipropyl ether, THF, and the like; aliphatic

hydrocarbons such as normal paraffin, naphthene, isoparaffin, kerosene, mineral oil, and the like; aromatic hydrocarbons such as benzene, toluene, xylene, solvent naphtha, alkylnaphthalene, and the like; halogenated hydrocarbons such as dichloromethane, chloroform, carbon tetrachloride, and the like; esters such as ethyl acetate, diisopropyl phthalate, dibutyl phthalate, dioctyl phthalate, dimethyl adipate, and the like; lactones such as γ-butyrolactone and the like; amides such as N,N-dimethylformamide, diethylformamide, N,N-dimethylacetamide, N-alkylpyrrolidinone, and the like; nitriles such as acetonitrile and the like; sulfur compounds such as dimethyl sulfoxide and the like; vegetable oils such as soybean oil, rapeseed oil, cottonseed oil, castor oil, and the like; water, and the like. These may be used alone or in a mixture of two or more kinds thereof.

Examples of the surfactant to be used as a dispersant or moistening agent include nonionic surfactants such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl aryl ether, polyoxyethylene alkyl phenyl ether, polyoxyethylene dialkylphenyl ether, polyoxyethylene alkyl phenyl ether-formalin condensate, polyoxyethylene-polyoxypropylene block copolymer, polyoxystyrene-polyoxyethylene block copolymer, alkyl polyoxyethylene-polypropylene block copolymer ether, polyoxyethylenealkylamine, polyoxyethylene fatty acid amide, polyoxyethylene fatty acid bisphenyl ether, polyalkylene benzyl phenyl ether, polyoxyalkylene styryl phenyl ether, acetylene diol, polyoxyalkylene-added acetylene diol, polyoxyethylene ether-type silicone-, ester-type silicone, and fluorine-surfactants, polyoxyethylene castor oil, hydrogenated polyoxyethylene castor oil, and the like; anionic surfactants such as alkyl sulfate salt, polyoxyethylene alkyl ether sulfate salt, polyoxyethylene alkyl phenyl ether sulfate salt, polyoxyethylene styryl phenyl ether sulfate salt, alkylbenzenesulfonate salt, alkylarylsulfonate salt, lignin sulfonate salt, alkylsulfosuccinate salt, naphthalenesulfonate salt, alkylnaphthalenesulfonate salt, salt of formalin condensate of naphthalenesulfonic acid, salt of formalin condensate of alkylnaphthalenesulfonic acid, fatty acid salt, polycarboxylate salt, polyacrylate salt, N-methyl-fatty acid sarcosinate, resin acid salt, polyoxyethylene alkyl ether phosphate salt, polyoxyethylene alkyl phenyl ether phosphate salt, and the like; cationic surfactants such as laurylamine hydrochloride, stearylamine hydrochloride, oleylamine hydrochloride, stearylamine acetate salt, stearylaminopropylamine acetate salt, alkyltrimethylammonium chloride, alkyldimethylbenzalkonium chloride, and the like; ampholytic surfactants such as amino acid- and betaine-surfactants and the like, and the like. These surfactants may be used alone or in a mixture of two or more kinds thereof.

Examples of the binder and tackifier include carboxymethylcellulose or salt thereof, dextrin, water-soluble starch, xanthan gum, guar gum, sucrose, polyvinylpyrrolidone, gum arabic, poly(vinyl alcohol), polyvinyl acetate, sodium polyacrylate, polyethylene glycol having an average molecular weight of 6,000 to 20,000, polyethylene oxide having an average molecular weight of 100,000 to 5,000,000, phospholipid (e.g. cephalin, lecithin, etc.), cellulose powder, dextrin, processed starch, polyaminocarboxylic acid chelate compound, crosslinked polyvinylpyrrolidone, maleic acid-styrene copolymer, (meth)acrylic acid copolymer, half ester of polyhydric alcohol polymer and dicarboxylic anhydride, water-soluble salt of polystyrenesulfonic acid, paraffin, terpene, polyamide resin, polyacrylic acid salt, polyoxyethylene, wax, polyvinylalkyl ether, alkylphenol formaldehyde condensate, synthetic resin emulsion, and the like.

Examples of the thickener include water-soluble polymers such as xanthan gum, guar gum, diutan gum, carboxymethylcellulose, polyvinylpyrrolidone, carboxyvinyl polymer, acrylic polymer, starch compound, polysaccharide, and the like; inorganic fine powders such as high-purity bentonite and fumed silica (white carbon), and the like.

Examples of the colorant include inorganic pigments such as iron oxide, titanium oxide, Prussian blue, and the like; organic dyes such as an alizarin dye, azo dye, and metal phthalocyanine dye, and the like.

Examples of the antifreezing agent include polyhydric alcohols such as ethylene glycol, diethylene glycol, propylene glycol, glycerin, and the like, and the like.

Examples of the aids for anticaking or disintegrating include starch, alginic acid, mannose, galactose, and the like, polyvinylpyrrolidone, fumed silica (white carbon), ester gum, petroleum resin, sodium tripolyphosphate, sodium hexametaphosphate, metal stearate, cellulose powder, dextrin, methacrylate copolymer, polyvinylpyrrolidone, polyaminocarboxylic acid chelate compound, sulfonated styrene-isobutylene-maleic anhydride copolymer, and starch-polyacrylonitrile graft copolymer, and the like.

Examples of the decomposition inhibitor include desiccants such as zeolite, calcined lime, magnesium oxide, and the like; antioxidants such as phenol compound, amine compound, sulfur compound, phosphoric acid compound, and the like; ultraviolet absorbers such as salicylic acid compound, benzophenone compound, and the like, and the like.

Examples of the preservative include potassium sorbate, 1,2-benzothiazolin-3-one, and the like.

Further, functional spreading agents; active enhancers for metabolic decomposition inhibitors such as piperonyl butoxide and the like; anti-freezing agents such as propylene glycol and the like; antioxidants such as BHT and the like; ultraviolet absorbers, and other aids may also be used as necessary.

The content of the compound as an active ingredient may be increased or decreased as necessary, and the compound may be used in a proportion appropriately chosen from the range of 0.01 part by weight to 90 parts by weight per 100 parts by weight of the agrohorticultural insect control agent of the present invention. For example, in the case of dusts, granules, emulsion, or wettable powders, the suitable content is from 0.01 part by weight to 50 parts by weight (0.01 wt% to

50 wt% of the total weight of the agrohorticultural insect control agent).

The amount of use of the agrohorticultural insect pest control agent of the present invention varies depending on various factors such as purpose, target insect pest, growth state of plant, tendency of insect pest outbreak, weather, environmental conditions, dosage form, application method, application site, application time, and the like. It may be appropriately chosen from the range of 0.001 g to 10 kg, preferably 0.01 g to 1 kg, in terms of the compound as an active ingredient, per 10 ares depending on the purpose.

The agrohorticultural insect pest control agent of the present invention may be used in admixture with other agrohorticultural insecticides, acaricides, nematicides, fungicides, biological control agents, or the like in order to expand control target insect pests and the period suitable for control, or to reduce the drug amount. Furthermore, it may be used in admixture with herbicides, plant growth regulators, fertilizers, and the like, depending on the usage situation.

Examples of other agrohorticultural insecticide, acaricide and nematicide to be used for such purpose include 3,5-xylyl methylcarbamate (XMC), fenobucarb (BPMC), Bt toxin insecticide compound, CPCBS (chlorfenson), DCIP (dichlorodiisopropyl ether), D-D (1,3-Dichloropropene), DDT, NAC, O-4-dimethylsulfamoylphenyl O,O-diethyl phosphorothioate (DSP), O-ethyl O-4-nitrophenyl phenylphosphonothioate (EPN), tripropylisocyanurate (TPIC), acrinathrin, azadirachtin, acynonapyr, azinphos-methyl, acequinocyl, acetamiprid, acetoprole, acephate, abamectin, afidopyropen, avermectin-B, amidoflumet, amitraz, alanycarb, aldicarb, aldoxycarb, aldrin, alpha-endosulfan, alpha-cypermethrin, albendazole, allethrin, isazofos, isamidofos, isoamidofos, isoxathion, isocycloseram, isofenphos, isofluallanam, isoprocarb (MIPC), epsilon-metofluthrin, epsilon-momfluorothrin, ivermectin, imicyafos, imidacloprid, imiprothrin, indazapyroxamet, indoxacarb, esfenvalerate, ethiofencarb, ethion, ethiprole, etoxazole, ethofenprox, ethoprophos, etrimfos, emamectin, emamectin-benzoate, endosulfan, empenthrin, oxazosulfyl, oxamyl, oxydemeton-methyl, oxydeprofos (ESP), oxibendazole, oxfendazole, potassium oleate, sodium oleate, cadusafos, kappa-bifenthrin, cartap, carbaryl, carbosulfan, carbofuran, gamma-cyhalothrin, xylylcarb, quinalphos, kinoprene, chinomethionat, cloethocarb, clothianidin, clofentezine, chromafenozide, chlorantraniliprole, chlorethoxyfos, chlordimeform, chlordane, chlorpyrifos, chlorpyrifos-methyl, chlorphenapyr, chlorfenson, chlorfenvinphos, chlorfluazuron, chlorobenzilate, chlorobenzoate, chloroprallethrin, Kelthane (dicofol), salithion, cyhalodiamide, cyanophos (CYAP), diafenthiuron, diamidafos, cyantraniliprole, theta-cypermethrin, dienochlor, cyetpyrafen, cyenopyrafen, dioxabenzofos, diofenolan, sigma-cypermethrin, cyclaniliprole, dichlofenthion (ECP), cycloprothrin, dichlorvos (DDVP), dicloromezotiaz, disulfoton, dinotefuran, cyhalodiamide, cyhalothrin, cyphenothrin, cyfluthrin, diflubenzuron, cyflumetofen, diflovidazin, cyproflanilide, cyhexatin, cypermethrin, cybenzoxasulfyl, dimethylvinphos, dimethoate, dimpropyridaz, dimefluthrin, silafluofen, cyromazine, spidoxamat, spinetoram, spinosad, spirodiclofen, spirotetramat, spiropidion, spirobudifen, spiromesifen, sulfiflumin, sulfluramid, sulprofos, sulfoxaflor, zeta-cypermethrin, diazinon, tau-fluvalinate, dazomet, thiacloprid, tiapyrachlor, thiamethoxam, tioxazafen, thiodicarb, thiocyclam, thiosultap, thiosultap-sodium, thionazin, thiometon, tiorantraniliprole, deet, dieldrin, tetrachlorantraniliprole, tyclopyrazoflor, tetrachlorvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, doramectin, tralopyril, tralomethrin, transfluthrin, triazamate, triazuron, trichlamide, trichlorphon (DEP), trifluenfuronate, triflumezopyrium, triflumuron, tolfenpyrad, naled (BRP), nicofluprole, nithiazine, nitenpyram, novaluron, noviflumuron, hydroprene, vaniliprole, vamidothion, parathion, parathion-methyl, halfenprox, halofenozide, pioxaniliprole, bistrifluron, bisultap, bisulflufen, hydramethylnon, hydroxy propyl starch, binapacryl, pyflubumide, bifenazate, bifenthrin, piperflanilide, pymetrozine, pyraclorfos, pyrafluprole, pyridafenthion, pyridaben, pyridalyl, pyrifluquinazon, pyriprole, pyriproxyfen, pirimicarb, pyrimidifen, pyriminostrobin, pirimiphos-methyl, pyrethrins, fipronil, fenazaquin, fenamiphos, bromopropylate, fenitrothion (MEP), fenoxycarb, fenothiocarb, phenothrin, fenobucarb, fensulfothion, fenthion (MPP), phenthoate (PAP), fenvalerate, fenpyroximate, fenpropathrin, fenbendazole, fenmezoditiaz, butathiofos, buprofezin, furathiocarb, prallethrin, fluacrypyrim, fluazaindolizine, fluazinam, fluazuron, fluensulfone, fluxametamide, fluchlordiniliprole, flucycloxuron, flucythrinate, fluvalinate, flupyradifurone, flufiprole, flupyradifurone, flupyrazofos, flupyrimin, flufenerim, flufenoxystrobin, flufenoxuron, flufenzine, flufenoprox, flupyroxystrobin, fluproxyfen, flubrocythrinate, fluhexafon, flubendiamide, flupentiofenox, flumethrin, flurimfen, prothiofos, protrifenbute, flonicamid, propaphos, propargite (BPPS), profenofos, broflanilide, profluthrin, propoxur (PHC), flometoquin, alpha-bromadiolone, bromopropylate, beta-cyfluthrin, hexaflumuron, hexythiazox, heptafluthrin, heptenophos, permethrin, benclothiaz, bendiocarb, benzpyrimoxan, bensultap, benzoximate, bentioflumin, benfuracarb, phoxim, phosalone, fosthiazate, fosthietan, phosphamidon, phosphocarb, phosmet (PMP), polynactin complex, formetanate, formothion, phorate, machine oil, malathion, milbemycin, milbemycin-A, milbemectin, mecarbam, mesulfenfos, methomyl, metaldehyde, metaflumizone, methamidophos, metam-ammonium, metam-sodium, methiocarb, methidathion (DMTP), methylisothiocyanate, methylneodecanamide, methylparathion, metoxadiazone, methoxychlor, methoxyfenozide, metofluthrin, methoprene, metolcarb, meperfluthrin, mevinphos, monocrotophos, monosultap, momfluorothrin, lambda-cyhalothrin, ryanodine, lufenuron, rescalure, resmethrin, lepimectin, rotenone, levamisol hydrochloride, fenbutatin oxide, morantel tartarate, methyl bromide, cyhexatin, calcium cyanamide, calcium polysulfide, sulfur, nicotine-sulfate, and the like.

Examples of the agrohorticultural fungicide to be used for such purposes include aureofungin, azaconazole, azithiram, acypetacs, acibenzolar, acibenzolar-S-methyl, azoxystrobin, anilazine, amisulbrom, ampropylfos, ametoctradin, allyl alcohol, aldimorph, amobam, isotianil, isovaledione, isopyrazam, isofetamid, isoflucypram, isoprothiolane, ipconazole, ipfentrifluconazole, ipflufenoquin, iprodione, iprovalicarb, iprobenfos, imazalil, iminoctadine, metam, iminoctadine-albesilate, iminoctadine-triacetate, imibenconazole, inpyrfluxam, uniconazole, uniconazole-P, echlomezole, edifenphos, etaconazole, ethaboxam, ethirimol, etem, ethoxyquin, etridiazole, enestroburin, enoxastrobin, epoxiconazole, oxadixyl, oxathiapiprolin, oxycarboxin, copper-8-quinolinolate, oxytetracycline, copper-oxinate, oxpoconazole, oxpoconazole-fumarate, oxolinic acid, octhilinone, ofurace, orysastrobin, carbam (metam-sodium), kasugamycin, galquin, carbamorph, carpropamid, carbendazim, carboxin, carvone, quinazamid, quinacetol, quinoxyfen, quinofumelin, chinomethionat, quinomethionate, captafol, captan, kiralaxyl, quinconazole, quintozene, guazatine, cufraneb, cuprobam, coumoxystrobin, glyodin, griseofulvin, climbazole, cresol, kresoxim-methyl, chlozolinate, clotrimazole, chlobenthiazone, chloraniformethan, chloranil, chlorquinox, chloropicrin, chlorfenazole, chloroinconazide, chlorodinitronaphthalene, chlorothalonil, chloroneb, salicylanilide, zarilamid, cyazofamid, diethyl pyrocarbonate, diethofencarb, cyclafuramid, diclocymet, dichlozoline, diclobutrazol, cyclobutrifluram, dichlofluanid, cycloheximide, dichlobentiazox, diclomezine, dicloran, dichlorophen, dichlone, disulfiram, ditalimfos, dithianon, diniconazole, diniconazole-M, zineb, dinocap, dinocton, dinosulfon, dinoterbon, dinobuton, dinopenton, dipymetitrone, dipyrithione, diphenylamine, difenoconazole, cyflufenamid, diflumetorim, cyproconazole, cyprodinil, cyprofuram, cypendazole, simeconazole, dimethirimol, dimethomorph, cymoxanil, dimoxystrobin, ziram, silthiofam, streptomycin, spiroxamine, sultropen, sedaxane, zoxamide, dazomet, thiadiazin, tiadinil, thiadifluor, thiabendazole, tioxymid, thiochlorfenphim, thiophanate, thiophanate-methyl, thifluzamide, thicyofen, thioquinox, thiram, decafentin, tecnazene, tecloftalam, tecoram, tetraconazole, debacarb, dehydroacetic acid, tebuconazole, tebufloquin, dodicin, dodine, dodecylbenzenesulfonic acid bis(ethylenediamine) copper(II) complex salt (DBEDC), dodemorph, drazoxolon, triadimenol, triadimefon, triazbutil, triazoxide, triamiphos, triarimol, trichlamide, triclopyricarb, tricyclazole, triticonazole, tridemorph, tributyltin oxide, triflumizole, trifloxystrobin, triforine, tolylfluanid, tolclofos-methyl, tolprocarb, natamycin, nabam, nitrostyrene, nitrothal-isopropyl, nuarimol, copper nonylphenol sulfonate, halacrinate, validamycin, valifenalate, harpin protein, picarbutrazox, bixafen, picoxystrobin, picobenzamide, pydiflumetofen, bithionol, bitertanol, hydroxyisoxazole, hydroisoxazole-potassium, binapacryl, biphenyl, piperalin, hymexazol, pyraoxystrobin, pyracarbolid, pyraclostrobin, pyraziflumid, pyrazophos, pyrapropoyne, pyrametostrobin, pyriofenone, pyridinitril, pydiflumetofen, pyrisoxazole, pyridachlometyl, pyrifenox, pyribencarb, pyriminostrobin, pyrimethanil, pyroxychlor, pyroxyfur, pyroquilon, vinclozolin, ferbam, famoxadone, fenapanil, fenamidone, fenaminosulf, fenaminstrobin, fenarimol, fenitropan, feneptamidoquin, fenoxanil, fenopyramid, ferimzone, ferbam, fentin, fenpiclonil, fenpicoxamid, fenpyrazamine, fenbuconazole, fenfuram, fenpropidin, fenpropimorph, fenhexamid, phthalide, buthiobate, butylamine, bupirimate, fuberidazole, blasticidin-S, furametpyr, furalaxyl, fluacrypyrim, fluazinam, fluindapyr, fluoxastrobin, fluoxapiprolin, fluoxytioconazole, fluotrimazole, fluopicolide, Fluopimomide, fluopyram, fluoroimide, furcarbanil, fluxapyroxad, fluquinconazole, furconazole, furconazole-cis, fludioxonil, flusilazole, flusulfamide, flutianil, flutolanil, flutriafol, flufenoxadiazam, flufenoxystrobin, furfural, flubeneteram, furmecyclox, flumetylsulforim, flumetover, flumorph, proquinazid, prochloraz, procymidone, prothiocarb, prothioconazole, pronitridine, propamocarb, propiconazole, propineb, furophanate, probenazole, bromuconazole, florylpicoxamid, hexachlorobutadiene, hexaconazole, hexylthiofos, bethoxazin, benalaxyl, benalaxyl-M, benodanil, benomyl, pefurazoate, benquinox, penconazole, benzamorf, pencycuron, benzohydroxamic acid, benzovindiflupyr, bentaluron, benthiazole, benthiavalicarb, benthiavalicarb-isopropyl, penthiopyrad, penflufen, boscalid, phosdiphen, fosetyl, fosetyl-Al, polyoxins, polyoxorim, polycarbamate, folpet, formaldehyde, machine oil, maneb, mancozeb, mandipropamid, mandestrobin, myclozolin, myclobutanil, mildiomycin, milneb, mecarbinzid, methasulfocarb, metazoxolon, metam, metam-sodium, metalaxyl, metalaxyl-M, metarylpicoxamid, metiram, methyl isothiocyanate, mepthyldinocap, Metyltetraprole, metconazole, metsulfovax, methfuroxam, metominostrobin, metrafenone, mepanipyrim, mefenoxam, mefentrifluconazole, meptyldinocap, mepronil, mebenil, iodomethane, rabenzazole, methyl bromide, benzalkonium chloride, basic copper chloride, basic copper sulfate, silver, and the like, sodium hypochlorote, cupric hydroxide, wettable sulfur, calcium polysulfide, potassium hydrogen carbonate, sodium hydrogencarbonate, sulfur, copper sulfate anhydride, nickel dimethyldithiocarbamate, copper 8-hydroxyquinoline (oxine copper) and the like, zinc sulfate, copper sulfate pentahydrate, and the like.

Similarly, examples of the herbicide include 1-naphthylacetamide, 2,4-PA, 2,3,6-TBA, 2,4,5-T, 2,4,5-TB, 2,4-D, 2,4-DB, 2,4-DEB, 2,4-DEP, 3,4-DA, 3,4-DB, 3,4-DP, 4-CPA, 4-CPB, 4-CPP, MCP, MCPA, MCPA thioethyl, MCPB, ioxynil, icafolin, icafolin-methyl, aclonifen, azafenidin, acifluorfen, aziprotryne, azimsulfuron, asulam, acetochlor, atrazine, atraton, anisuron, anilofos, aviglycine, abscisic acid, amicarbazone, amidosulfuron, amitrole, aminocyclopyrachlor, aminopyralid, amibuzin, amiprophos-methyl, ametridione, ametryn, alachlor, allidochlor, alloxydim, alorac, iofensulfuron, isouron, isocarbamid, isoxachlortole, isoxapyrifop, isoxaflutole, isoxaben, isocil, isonoruron, isoproturon, isopropalin, isopolinate, isomethiozin, inabenfide, ipazine, iptriazopyrid, ipfencarbazone, iprymidam, imazaquin, imazapic, imazapyr, imazamethapyr, imazamethabenz, imazamethabenz-methyl, imazamox, imazethapyr, imazosulfuron, indaziflam, indanofan, indolebutyric acid, indolauxipyr, indolauxipyr-cyanomethyl, uniconazole-P, eglinazine, esprocarb, ethametsulfuron, ethametsulfuron-methyl, ethalfluralin, ethiolate, ethychlozate-ethyl, ethidimuron, etinofen, ethephon, ethoxysulfuron, ethoxyfen, etnipromid, ethofumesate, etobenzanid, epyrifenacil, epronaz, erbon, endothal, oxadiazon, oxadiargyl, oxaziclomefone, oxasulfuron, oxapyrazon, oxyfluorfen, oryzalin, orthosulfamuron, orbencarb, cafenstrole, cambendichlor, carbasulam, carfentrazone, carfentrazone-ethyl, karbutilate, carbetamide, carboxazole, quizalofop, quizalofop-P, quizalofop-ethyl, xylachlor, quinoclamine, quinonamid, quinclorac, quinmerac, cumyluron, clacyfos, cliodinate, glyphosate, glufosinate, glufosinate-P, credazine, clethodim, cloxyfonac, clodinafop, clodinafop-propargyl, chlorotoluron, clopyralid, cloproxydim, cloprop, chlorbromuron, clofop, clomazone, chlomethoxynil, chlomethoxyfen, clomeprop, chlorazifop, chlorazine, chloranocryl, chloramben, cloransulam, cloransulam-methyl, chloridazon, chlorimuron, chlorimuron-ethyl, chlorsulfuron, chlorthal, chlorthiamid, chlortoluron, chlornitrofen, chlorfenac, chlorfenprop, chlorbufam, chlorphthalim, chlorflurazole, chlorflurenol, chlorprocarb, chlorpropham, chlormequat, chloreturon, chloroxynil, chloroxuron, chlorotoluron, chloropon, saflufenacil, cyanazine, cyanatryn, di-allate, diuron, diethamquat, dioxopyritrione, dicamba, cycluron, cycloate, cycloxydim, diclosulam, cyclosulfamuron, cyclopyranil, cyclopyrimorate, dichlorprop, dichlorprop-P, dichlobenil, diclofop, diclofop-methyl, dichlormate, dichloralurea, diquat, cisanilide, disul, siduron, dithiopyr, dinitramine, cinidon-ethyl, dinosam, cinosulfuron, dinoseb, dinoterb, dinofenate, dinoprop, cyhalofop-butyl, cypyrafluone, diphenamid, difenoxuron, difenopenten, difenzoquat, cybutryne, cyprazine, cyprazole, diflufenican, diflufenzopyr, dipropetryn, cypromid, cyperquat, gibberellin, simazine, dimexano, dimesulfazet, dimethachlor, dimidazon, dimethametryn, dimethenamid, simetryn, simeton, dimepiperate, dimefuron, cinflubrolin, cinmethylin, swep, sulglycapin, sulcotrione, sulfallate, sulfentrazone, sulfosulfuron, sulfometuron, sulfometuron-methyl, secbumeton, sethoxydim, sebuthylazine, terbacil, daimuron, dazomet, dalapon, thiazafluron, thiazopyr, tiafenacil, thiencarbazone, thiencarbazone-methyl, tiocarbazil, tioclorim, thiobencarb, thidiazimin, thidiazuron, thifensulfuron, thifensulfuron-methyl, desmedipham, desmetryn, tetflupyrolimet, tetrafluron, thenylchlor, tebutam, tebuthiuron, terbumeton, tepraloxydim, tefuryltrione, tembotrione, delachlor, terbacil, terbucarb, terbuchlor, terbuthylazine, terbutryn, topramezone, tralkoxydim, triaziflam, triasulfuron, triafamone, triallate, trietazine, tricamba, triclopyr, tridiphane, tritac, tritosulfuron, tripyrasulfone, trifludimoxazin, triflusulfuron, triflusulfuron-methyl, trifluralin, trifloxysulfuron, tripropindan, tribenuron, tribenuron-methyl, trifop, trifopsime, trimeturon, tolpyralate, naptalam, naproanilide, napropamide, nicosulfuron, nitralin, nitrofen, nitrofluorfen, nipyraclofen, neburon, norflurazon, noruron, barban, paclobutrazol, paraquat, parafluron, haloxydine, halauxifen, haloxyfop, haloxyfop-P, haloxyfop-methyl, halosafen, halosulfuron, halosulfuron-methyl, bilanafos, bixlozone, picloram, picolinafen, bicyclopyrone, bispyribac, bispyribac-sodium, pydanon, pinoxaden, bipyrazone, bifenox, piperophos, hymexazol, pyraquinate, pyraclonil, pyrasulfotole, pyrazoxyfen, pyrazosulfuron, pyrazosulfuron-ethyl, pyrazolate, bilanafos, pyraflufen, pyraflufen-ethyl, pyriclor, pyridafol, pyrithiobac, pyrithiobac-sodium, pyridate, pyriftalid, pyributicarb, pyriflubenzoxim, pyribenzoxim, pyrimisulfan, primisulfuron, pyriminobac-methyl, pyroxasulfone, pyroxsulam, fenasulam, phenisopham, fenuron, fenoxasulfone, fenoxaprop, fenoxaprop-P, fenoxaprop-ethyl, phenothiol, fenoprop, phenobenzuron, feproxydim, fenquinotrione, fenthiaprop, fenteracol, fentrazamide, fenpyrazone, phenmedipham, phenmedipham-ethyl, butachlor, butafenacil, butamifos, buthiuron, buthidazole, butylate, buturon, butenachlor, butroxydim, butralin, butroxydim, flazasulfuron, flamprop, furyloxyfen, prynachlor, primisulfuron-methyl, fluazifop, fluazifop-P, fluazifop-butyl, fluazolate, fluroxypyr, fluothiuron, fluometuron, fluoroglycofen, flurochloridone, fluorodifen, fluoronitrofen, fluoromidine, flucarbazone, flucarbazone-sodium, fluchloralin, flusulfinam, flucetosulfuron, fluthiacet, fluthiacet-methyl, flupyrsulfuron, flufenacet, flufenican, flufenoximacil, flufenpyr, flupropacil, flupropanate, flupoxam, flumioxazin, flumiclorac, flumiclorac-pentyl, flumipropyn, flumezin, fluometuron, fluchloraminopyr, fluchloraminopyr-tefuryl, flumetsulam, fluridone, flurtamone, fluroxypyr, pretilachlor, proxan, proglinazine, broclozone, procyazine, prodiamine, prosulfalin, prosulfuron, prosulfocarb, propaquizafop, propachlor, propazine, propanil, propyzamide, propisochlor, prohydrojasmon, propyrisulfuron, propham, profluazol, profluralin, prohexadione-calcium, propoxycarbazone, propoxycarbazone-sodium, profoxydim, bromacil, brompyrazon, prometryn, prometon, bromoxynil, bromofenoxim, bromobutide, bromobonil, florasulam, florpyrauxifen, hexachloroacetone, hexazinone, pethoxamid, benazolin, penoxsulam, pebulate, beflubutamid, beflubutamid-M, vernolate, perfluidone, bencarbazone, benquitrione, benzadox, benzipram, benzylaminopurine, benzthiazuron, benzfendizone, bensulide, bensulfuron-methyl, benzoylprop, benzobicyclon, benzofenap, benzofluor, bentazone, pentanochlor, benthiocarb, pendimethalin, pentoxazone, benfluralin, benfuresate, fosamine, fomesafen, foramsulfuron, forchlorfenuron, maleic hydrazide, mecoprop, mecoprop-P, medinoterb, mesosulfuron, mesosulfuron-methyl, mesotrione, mesoprazine, methoprotryne, metazachlor, methazole, metazosulfuron, methabenzthiazuron, metamitron, metamifop, metam, methalpropalin, methiuron, methiozolin, methiobencarb, methyldymron, metoxuron, metosulam, metsulfuron, metsulfuron-methyl, metflurazon, metproxybicyclone, metobromuron, metobenzuron, methometon, metolachlor, metribuzin, mepiquat-chloride, mefenacet, mefluidide, monalide, monisouron, monuron, monochloroacetic acid, monolinuron, molinate, morfamquat, iodosulfuron, iodosulfuron-methyl-sodium, iodobonil, iodomethane, lactofen, lancotrione, linuron, rimisoxafen, rimsulfuron, lenacil, rhodethanil, calcium peroxide, methyl bromide, and the like.

The same effect as above can be expected by the use in admixture with biological control agents, for example, viral formulations obtained from Nuclear polyhedrosis virus (NPV), Granulosis virus (GV), Cytoplasmic polyhedrosis virus (CPV), Entomopoxi virus (EPV), and the like; microbial pesticides utilized as insecticides or nematicides, such as Monacrosporium phymatophagum, Steinernema carpocapsae, Steinernema kushidai, Pasteuria penetrans, and the like; microbial pesticides utilized as fungicides, such as Trichoderma lignorum, Agrobacterium radiobactor, nonpathogenic Erwinia carotovora, Bacillus subtilis, and the like; biological control agents utilized as herbicides, such as Xanthomonas campestris and the like, and the like.

Furthermore, it is also possible to use biological control agents in combination, for example, natural enemies such as Encarsia formosa, Aphidius colemani, Aphidoletes aphidimyza, Diglyphus isaea, Dacnusa sibirica, Phytoseiulus persimilis, Amblyseius cucumeris, Orius sauteri, and the like; microbial pesticides such as Beauveria brongniartii and the like; pheromones such as (Z)-10-tetradecenyl acetate, (E,Z)-4,10-tetradecadieniel acetate, (Z)-8-dodecenyl acetate, (Z)-11-tetradecenyl acetate, (Z)-13-icosen-10-one, 14-methyl-1-octadecene, and the like, and the like.

Furthermore, the compound represented by the formula (1) or a salt thereof of the present invention is also suitable for controlling parasites in animals. Animal parasites live in the body or on the body surface of host animals and cause disadvantages to the host animals. They are broadly divided into ectoparasites that live on the body surface of the host animal and endoparasites that live inside the body of the host animal. The compound represented by the formula (1) or a salt thereof of the present invention can also be applied as an ectoparasite control agent for animals or an endoparasite control agent for animals.

When the compound represented by the formula (1) or a salt thereof of the present invention is applied as an ectoparasite control agent for animals or endoparasite control agent for animals, examples of the animal to which it is applicable include, but are not limited to, farm animals such as pig, horse, cows sheep, goat, rabbit, camel, buffalo, deer, mink, chinchillas, and the like; pet animals such as dog, cat, small bird, monkey, and the like; laboratory animals such as rat, mouse, golden hamster, guinea pig, and the like; poultry such as chicken, wildduck, aigamo duck, quail, duck, goose, turkey, and the like; human, and the like. In addition, it is also applicable to farmed fish such as sea bream, flounder, tuna, yellowtail, eel, and the like; crustaceans and shellfish such as shrimp, crab, scallop, oyster, Japanese littleneck, hard clam, and the like.

Examples of the ectoparasite that can be controlled by the ectoparasite control agent for animals of the present invention include, but are not limited to, Rhipicephalus (Rhipicaphalus (Boophilus) microplus, Rhipicephalus sanguineus, etc.), Amblyomma, Dermacentor, Haemaphysalis, Hyalomma, Ixodes, Rhipicentor, Margaropus, Argas, Otobius, Ornithodoros, Chorioptes (Chorioptes bovis etc.), Psoroptes (Psoroptes ovis, etc.), Cheyletiella, Dermanyssus (Dermanyssus gallinae etc.), Ortnithonyssus, Demodex (Demodex canis etc.), Sarcoptes (Sarcoptes scabiei etc.), Psorergates, Siphonaptera (Ctenocephalides felis, Ctenocephalides canis etc.), Diptera (Musca spp, Gasterophilus intestinalis, Oestrus ovis etc.), Phthiraptera (Bovicola Ovis, Bovicola Bovis etc.), Lepidoptera, Coleoptera, Homoptera, Haematopota, Tabunus, Haematobia (Haematobia irritans etc.), Stomoxys, Lucilia, Lepeophtheirus, and the like.

Further specifically, examples of the Acari include Ixodoidea such as Boophilus microplus, Rhipicephalus sanguineus, Haemaphysalis longicornis, Haemaphysalis flava, Haemaphysaliscampanulata, Haemaphysalis concinna, Haemaphysalisjaponica, Haemaphysalis kitaokai, Haemaphysalis ias, Ixodes ovatus, Ixodes nipponensis, Ixodespersulcatus, Amblyommatestudinarium, Haemaphysalis megaspinosa, Dermacentor reticulatus, Dermacentor taiwanesis, and the like; Ornithonyssus such as Dermanyssus gallinae, Ornithonyssus sylviarum, Ornithonyssus bursa, and the like; Trombicula such as Eutrombicula wichmanni, Leptotrombidium akamushi, Leptotrombidium pallidum, Leptotrombidium fuji, Leptotrombidium tosa, Neotrombicula autumnalis, Eutrombicula alfreddugesi, Helenicula miyagawai, and the like; Cheyletidae such as Cheyletiella yasguri, Cheyletiella parasitivorax, Cheyletiella blakei, and the like; Sarcoptes such as Psoroptes cuniculi, Chorioptes bovis, Otodectes cynotis, Sarcoptes scabiei, Notoedres cati, and the like; Demodicidae such as Demodex canis, and the like, and the like.

Examples of the Siphonaptera include Pulicidae, Ceratephyllus, and the like. Examples of the flea belonging to Pulicidae include Ctenocephalidescanis, Ctenocephalides felis, Pulex irritans, Echidnophaga gallinacea, Xenopsylla cheopis, Leptopsylla segnis, Nosopsyllus fasciatus, Monopsyllus anisus, and the like.

In addition, ectoparasites that can be controlled by the ectoparasite control agent for animals of the present invention include Anoplura such as Haematopinus eurysternus, Haematopinus asini, Dalmalinia ovis, Linognathus vituli, Haematopinus suis, Phthirus pubis, Pediculus capitis, and the like; biting lices such as Trichodectes canis, and the like; and blood-sucking dipterous insect pests such as Tabanus trigonus, Culicoides schultzei, Simulium ornatum, and the like.

The ectoparasite control agent for animals of the present invention can be used by blending, according to the preparation formulation generally used, with a suitable solid carrier or liquid carrier, as well as adjuvant and the like as necessary, at suitable proportions, and preparing same into a suitable dosage form such as liquid, emulsifier, cream, ointment, suspension, aerosol, or the like according to the purpose of use by dissolving, suspending, mixing, impregnating, adsorbing or attaching. The amount of the active ingredient in these preparations may be in the range of from 0.01 parts by weight to 80.0 parts by weight per 100 parts by weight of the preparation. As the solid carrier or liquid carrier to be used, carriers commonly used for veterinary drugs may be used, and liquid carriers are preferably used in view of the easiness of treatment on target animals. Examples of the liquid carrier include alcohols such as methyl alcohol, ethyl alcohol, isopropyl alcohol, tertiary-butyl alcohol, benzyl alcohol, and the like; propylene carbonate; N-methyl-2-pyrrolidone; water, and the like. Where necessary, an adjuvant can be used, and as the adjuvant, surfactant, antioxidant, emulsifier, and the like can be used. For example, surfactants such as polyoxyethylene alkylaryl ether, polyoxyethylene sorbitan monolaurate, alkylallylsorbitan monolaurate, alkylbenzenesulfonate, alkylnaphthalenesulfonic acid, lignin sulfonate higher alcohol sulfate ester salt, glycol monoalkyl ether, glycols, and the like; emulsifiers such as sorbitan monoeulenate, sorbitan monolaurate, monoglyceride caprylate, monoglyceride caprate, monoglyceride isostearate,

propylene glycol monocaprylate, and the like; and antioxidants such as BHA, BHT, and the like can be mentioned.

The ectoparasite control agent for animals of the present invention may be administered to animals by a route that affords the desired effect, such as topical, oral, parenteral, subcutaneous route or the like, and is not particularly limited, but topical administration is preferred. Topical administration includes administration by a spot-on treatment in which a liquid drug is dropped onto the skin or the like of the dorsal scapula of the target animal; a pour-on treatment in which a liquid drug is applied along the mid-dorsal line of the target animal; spray-on and spray lace in which a liquid aerosol drug is used for treatment by spray or the like; medicinal bath (dip); and a treatment method using an ear tag or a collar or the like carrying a drug. The application dose for the treatment can be appropriately selected from the range of generally about 0.1 mg to 500 mg, in terms of the compound as an active ingredient, and generally about 0.01 ml to 20 ml, in terms of the control agent of the present invention, per 1 kg of body weight of the target animal.

Where necessary, other active ingredients can be used in combination. Examples thereof include afoxolaner, umifoxolaner, tigolaner, fluralaner, mivorilaner, modoflaner, lotilaner, surolaner, albendazole, cambendazole, fenbendazole, flubendazole, mebendazole, oxifendazole, parabendazole, thiabendazole, triclabendazole, amitraz, demiditraz, clorsulon, closantel, oxyclonazide, lafoxanide, cyphenothrin, flumethrin, permethrin, cyromazine, derquantel, diamphenetide, dicyclanil, dinotefuran, imidacloprid, nitenpyram, thiamethoxam, abamectin, doramectin, emamectin, eprinomectin, ivermectin, moxidectin, selamectin, milbemycin oxime, emodepside, epsiplantel, fipronil, fluazuron, fluhexafon, indoxacarb, levamisole, lufenuron, metaflumizone, methoprene, monepantel, morantel, niclosamide, nitroscanate, nitroxinil, novaluron, oxantel, praziquantel, pyrantel, pyriprole, pyriproxyfen, cisapronil, spinosad, spinetoram, triflumezopyrim, and the like. When used in combination, a preparation of two or more kinds of active ingredients mixed together before administration may be used, or two or more different preparations may be administered separately.

The endoparasites that can be controlled by the endoparasite control agent for animals of the present invention are largely classified into Protozoa and Helminth. Examples of Protozoa that parasitize humans include, but are not limited to, amebas (Rhizopoda) such as Entamoeba and the like; Mastigophora such as Leishmania, Trypanosoma, Trichomonas, and the like; Sporozoea such as Plasmodium, Toxoplasmosis, and the like; and Ciliophora such as Balantidium coli and the like, and examples of Helminth that parasitizes humans include Nematoda such as roundworm, anisakis, dog roundworm, hairworm, pinworm, hookworm (Zubini hookworm, American hookworm, Brazilian hookworm, etc.), Schistosoma, Gnathostom, Brugia (filaria, Bancroft filaria, Brugia malayi, etc.), onchocerca, medina worm, Trichinella, Strongyloides, and the like; Acannthocephala such as Macracanthorhynchus hirudinaceus; Gordiacea such as hairworm and the like; Hirudinea such as Hirudo nipponia and the like; Trematoda such as Schistosoma japonicu, Schistosoma mansoni, Schistosoma haematobium, Clonorchis sinensis, Heterophyes, Fasciolidae, Paragonimus, and the like; and Cestoda such as Diphyllobothrium latum, Spirometra mansoni, Sparganum proliferum, Diplogonoporus, Taenia, (Taeniarhynchus, Taenia solium, hydatid (Echinococcus) etc.), Hymenolepis, Dipylidium caninum, Mesocestoides, monkey Anoplocephala, Nybelinia surmenicola, and the like.

Protozoa that parasitize animals belonging to mammals other than human, or birds are exemplified by, but not limited to, Apicomplexa including Cocidia such as Eimeria, Isospora, Toxoplasmosis, Neospora, Sarcocystis, Besnoitia, Hammondia, Cryptosporidium, Caryospora, and the like; Haemosporina such as Leucocytozoon, Plasmodium, and the like; Piroplasma such as Theileria, Anaplasma, Eperythrozoon, Heamobartonella, Rhrlichia, and the like, and the like; other Apicomplexa including Hepatozoon, Haemogregarina, and the like; Microspora including Encephalitozoon, Nosema, and the like; Mastigophora including Trypanosomatid such as Trypanosoma, Leishmania, and the like; Trichomonadida such as Chilomastix, Trichomonas, Monocercomonas, Histomonas, and the like; Diplomonadida such as Hexamita, Giardia, and the like; Sarcodina including amebas such as Entamoeba and the like, and the like; Ciliophora including Balantidium, Buxtonella, Entodinium, and the like,

Helminth that parasitizes animals belonging to mammals other than human, or birds is exemplified by Nematoda including Ascarida such as Ascaris, Toxocara, Toxascaris, Parascaris, Ascaridia, Heterakis, Anisakis, and the like; Oxyurida such as Oxyuris, Passalurus, and the like; Strongylida such as Strongylus, Haemonchus, Ostertagia, Trichostrongylus, Cooperia, Nematodirus, Hystrongylus, Oesophagostomum, Chabertia, Ancylostoma, Uncinaria, Necator, Bunostomum, Dictyocaulus, Metastrongylus, Filaroides, Aelurostrongulus, Angiostrongylus, Syngamus, Stephanurus, and the like; Rhabditida such as Strongyloides, Micronema, and the like; Spirurida such as Thelazia, Oxyspirura, Spirocerca, Gongylonema, Draschia, Habronema, Ascarops, Physaloptera, Gnathostoma, and the like; Filariida such as Dirofilaria, Setaria, Dipetalonema, Parafilaria, Onchocerca, and the like; Enoplida such as Parafilaria, Stephanofilaria, Trichuris, Capillaria, Trichosomoides, Trichinella, Dioctophyma, and the like,

Trematoda including Fasciolata such as Fasciola, Fasciolopsis, and the like; Paramphistomatidae such as Homalogaster and the like; Dicrocoelata such as Eurytrema, Dicrocoelium, and the like; Diplostomata such as Pharyngostomum, Alaria, and the like; Echinostomata such as Echinostoma, Echinochasmus, and the like; Troglotrematoidea such as Paragonimus, Nanophyetus, and the like; Opisthorchiida such as Clonorchis and the like; Heterophyida such as Heterophyes, Metagonimus, and the like; Plagiorchiida such as Prosthogonimus and the like; Schistosoma such as Schistoma and the like; and the like, Cestoda including Pseudophylidea such as Diphyllobothrium, Spirometra, and the like; Cyclophyllidea such as Anoplocephara, Paranoplocephala, Moniezia, Dipylidium, Mesocestoides, Taenia pisiformis,

Tenia, Hydatigera, Multiceps, Echinococcus, Echinococcus, Taenia, Taeniarhynchus, Hymenolepis, Vampirolepis, Raillietina, Amoebotaenia, and the like; and the like, Acannthocephala including Macracanthorhynchus, Moniliformis, and the like; Linguatulida including Linguatula and the like; and various other parasites.

Helminth is exemplified by, but not limited to, other names of Enoplida of Nematoda including Trichuris spp., Capillaria spp., Trichomosoides spp., Trichinella spp., and the like, Rhabditia including Micronema spp., Strongyloides spp., and the like, Strongylida including Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp. and the like,

Oxyurida is exemplified by Oxyuris spp., Enterobius spp., Passalurus spp., Syphacia spp., Aspiculuris spp., Heterakis spp., and the like, Ascaridia is exemplified by Ascaris spp., Toxascaris spp., Toxocara spp., Parascaris spp., Anisakis spp., Ascaridia spp., and the like, Spiruride is exemplified by Gnathosma spp., Physaloptera spp., Thelazia spp., Gongylonema spp., Habronema spp., Parabronema spp., Draschia spp., Dracunculus spp., and the like, Filariida is exemplified by Stephanofilaria spp., Parafilaria spp., Setaria spp., Loa spp., Dirofilaria spp., Litomosoides spp., Brugia spp., Wuchereria spp., Onchocerca spp., and the like,

Acanthocephala is exemplified by Filicollis spp., Moniliformis spp., Macracanthorhynchus spp., Prosthenorchis spp., and the like, Monogenea subclass of Trematoda is exemplified by Gyrodactylus spp., Dactylogyrus spp., Polystoma spp., and the like, Digenea subclass is exemplified by Diplostomum spp., Posthodiplostomum spp., Schistosoma spp., Trichobilharzia spp., Ornithbilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantcotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp., and the like,

Pseudophyllidea is exemplified by Diphyllobothrium spp., Spirometra spp., Schistocephalus spp., Ligula spp., Bothridium spp., and Diplogonoporus spp., and Cyclophyllidea is exemplified by parasites belonging to species such as Mesocestoides spp., Anoplocephala spp., Paranoplocehala spp., Moniezia spp., Thysanosomsa spp., Thysaniezia spp., Avitellina spp., Stilesia spp., Cittotaenia spp., Andyra spp., Bertiella spp., taenia spp., Echinococcus spp., Hydatigera spp., Davainea spp., Raillietina spp., Hymenolepis spp. (Hymenolepis spp.), Echinolepis spp., Echinocotyle spp., Diorchis spp., Dipylidium spp., Joyeuxiella spp., Diplopylidium spp., and the like, and various other parasites belonging to Acannthocephala or Linguatulida are included, but are not limited thereto.

The endoparasite control agent for animals of the present invention is effective in controlling not only parasites living in the bodies of intermediate hosts and final hosts, but also parasites in the living bodies of reservoir hosts. Furthermore, the compound represented by the formula (1) of the present invention exhibits a control effect on parasites at all developmental stages. For example, Protozoa include cyst, precystic form, vegetative form, or asexually reproductive stage meristem, ameboid form, sexual reproductive stage gametocyte, gamete, fusant, sporophyte, and the like. Nematoda include egg, larva, and adult. Furthermore, the compound of the present invention not only exterminates parasites within a living body, but also prevents parasite infection when applied to the environment, which is a route of infection. For example, soil-borne infection from soils in fields and parks; percutaneous infection from water systems such as river, lake, swamp, rice paddy, and the like : oral infection from the feces of animals such as dog, cat, and the like; oral infection from raw meat of saltwater fish, freshwater fish, crustaceans, shellfish, livestock, and the like; infection from mosquito, horsefly, fly, cockroach, tick, flea, louse, assassin bug, chigger, and the like, and the like can be prevented.

The endoparasite control agent for animals of the present invention can be administered to humans, mammals other than human, or birds as a pharmaceutical product for the purpose of treating or preventing parasitic diseases. The administration method can be either oral administration or parenteral administration. In the case of oral administration, it can be administered as capsule, tablet, pill, dust, granule, fine granule, powder, syrup, enteric tablet, suspension, or paste by incorporating into veterinary liquid drink or feed. In the case of parenteral administration, it is administered in a dosage form that maintains mucosal or transdermal absorption, such as injection, drip transfusion, suppository, emulsion, suspension, drop, ointment, cream, liquid, lotion, spray, aerosol, cataplasm, or tape.

When the endoparasite control agent for animals of the present invention is used as a pharmaceutical product for animals belonging to human, mammals other than human, or birds, the most appropriate amount (effective amount) of the active ingredient varies depending on whether it is for treatment or prevention, type of infected parasite, type and degree of infection, dosage form, and the like. Generally, in the case of oral administration, it is within the range of about 0.0001 to

10000 mg/kg body weight per day. In the case of parenteral administration, it is within the range of about 0.0001 mg/kg body weight to 10000 mg/kg body weight per day, which is administered in a single dose or in divided doses.

The concentration of the active ingredient in the endoparasite control agent for animals of the present invention is generally about 0.001% by mass to 100% by mass, preferably about 0.001% by mass to 99% by mass, further preferably about 0.005% by mass to 20% by mass. The endoparasite control agent for animals of the present invention may be a composition to be administered as it is, or may be a highly concentrated composition that is diluted to an appropriate concentration before when in use.

In addition, other active ingredients described above can also be used in combination as necessary for the purpose of reinforcing or supplementing the effects of the endoparasite control agent for animals of the present invention. When used in combination, a preparation obtained by mixing two or more kinds of active ingredients before administration may be used, or two or more kinds of different preparations may be administered separately.

[Example]

Representative examples of the present invention are shown below, which are not to be construed as limitative.

Production Example 1

Production of 2-(bis((4-fluorophenyl)amino)methylene)-5-(4-(trifluoromethyl)phenyl)cyclohexane-1,3-dione (compound No. 1-9)

[Chem. 22]

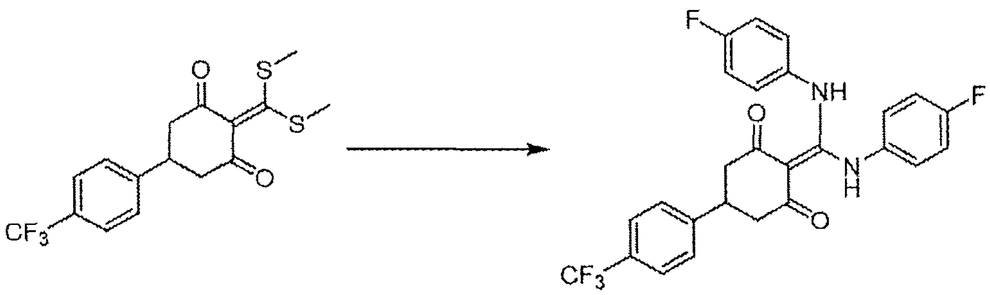

2-(Bis(methylthio)methylene)-5-(4-(trifluoromethyl)phenyl)cyclohexane-1,3-dione (0.24 g, 0.67 mmol) and 4-fluoroaniline (0.22 g, 2.0 mmol) were dissolved in xylene (5 mL), and reacted for 2 hr with heating under reflux. The reaction mixture was concentrated under reduced pressure and then recrystallized from ethyl acetate and normal hexane. The crystals were collected by filtration and washed with normal hexane to give 2-(bis((4-fluorophenyl)amino)methylene)-5-(4-(trifluoromethyl)phenyl)cyclohexane-1,3-dione (0.24 g, 0.50 mmol).

yield: 76%
property: melting point 215-218°C

Production Example 2

Production of 3-(bis((2-fluoropyridin-4-yl)amino)methylene)-6-(5-bromopyridin-2-yl)dihydro-2H-pyran-2,4(3H)-dione (compound No. 2-6)

[Chem. 23]

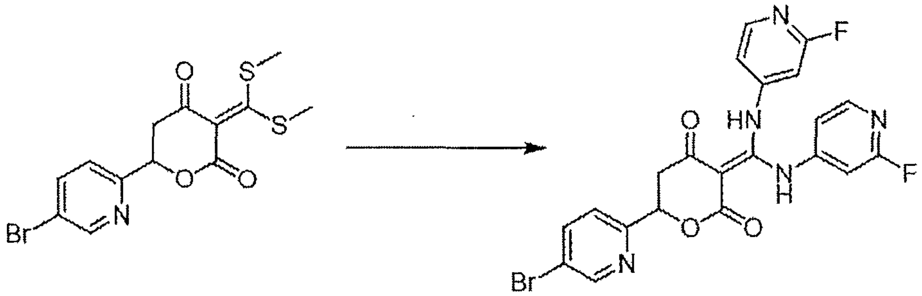

3-(Bis(methylthio)methylene)-6-(5-bromopyridin-2-yl)dihydro-2H-pyran-2,4(3H)-dione (0.15 g, 0.40 mmol) and 4-amino-2-fluoropyridine (0.14 mg, 1.2 mmol) were dissolved in acetonitrile (3 mL), and reacted for 6 hr with heating under reflux. After completion of the reaction, 1N hydrochloric acid was added to the reaction mixture, and the mixture was extracted with ethyl acetate. The obtained organic layer was dried over magnesium sulfate and concentrated under reduced pressure. The obtained residue was purified by silica gel column chromatography to give 3-(bis((2-fluoropyridin-4-yl)amino)methylene)-6-(5-bromopyridin-2-yl)dihydro-2H-pyran-2,4(3H)-dione (0.011 g, 0.022 mmol).

yield: 6%
property: melting point 164-165°C

Production Example 3

Production of 3-(((4-fluorophenyl)amino) (methylamino)methylene)-1-methyl-6-(4-(trifluoromethyl)phenyl)piperidine-2,4-dione (compound No. 3-1)

[Chem. 24]

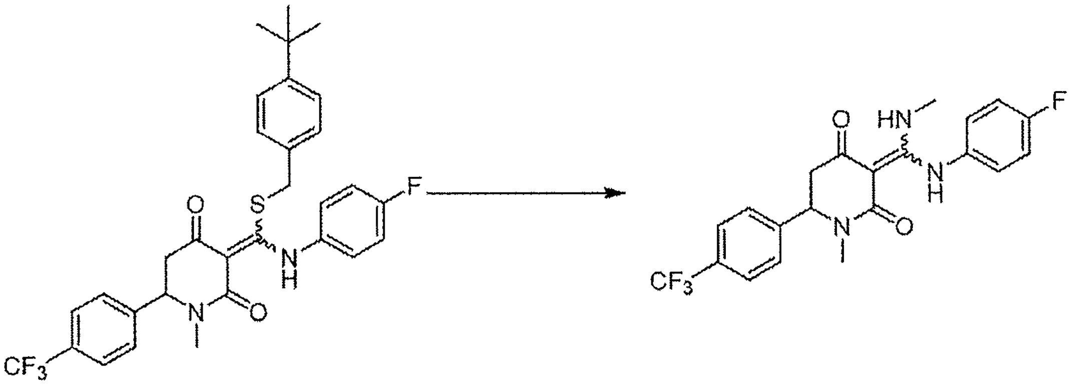

To a solution of 3-(((4-(tertiary butyl)benzyl)thio) ((4-fluorophenyl)amino)methylene)-1-methyl-6-(4-(trifluoromethyl)phenyl)piperidine-2,4-dione (70 mg, 0.12 mmol) in tetrahydrofuran (5 mL) was added methylamine (7% tetrahydrofuran solution) (54 mg, 0.12 mmol) and the mixture was stirred at room temperature for 3 hr. The reaction mixture was concentrated under reduced pressure, the obtained residue was purified by silica gel column chromatography to give 3-(((4-fluorophenyl)amino)(methylamino)methylene)-1-methyl-6-(4-(trifluoromethyl)phenyl)piperidine-2,4-dione (45 mg, 0.11 mmol).

yield: 87%
property: $^1$H-NMR($CDCl_3$):δ 12.9(d,1H), 11.4(d,1H), 7.61(d,2H), 7.31(d,2H), 7.10-7.13(m,2H), 7.02-7.06(m,2H), 4.60-4.62(m,1H), 3.25-3.33(m,1H), 3.00-3.02(m,3H), 2.61-2.66(m,1H), 2.51-2.54(m,3H)

Production Example 4

Production of 5-ethyl-3-((4-fluorophenyl)amino) (methylamino)methylene)-1-methyl-5(6-(trifluoromethyl)pyridin-3-yl)piperidine-2,4-dione (compound No. 3-2)

[Chem. 25]

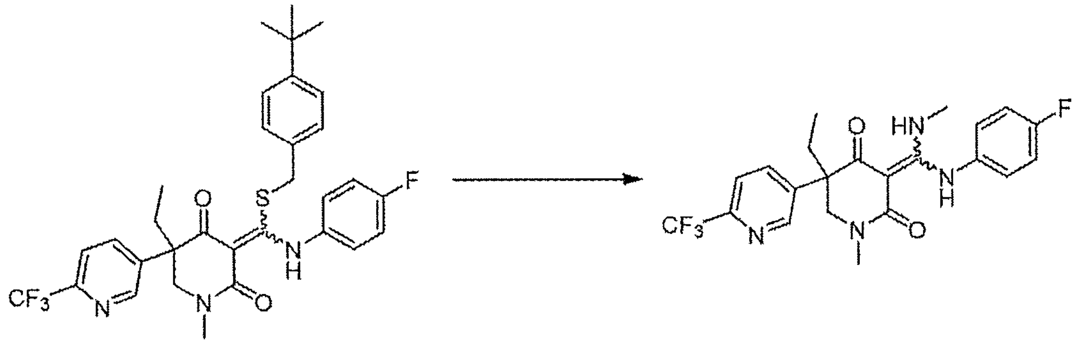

To a solution of 3-(((4-(tertiary butyl)benzyl)thio) ((4-fluorophenyl)amino)methylene)-5-ethyl-1-methyl-5-(6-trifluoromethyl)pyridin-3-yl)piperidine-2,4-dione (70 mg, 0.12 mmol) in tetrahydrofuran (5 mL) was added methylamine (7% tetrahydrofuran solution) (52 mg, 0.12 mmol) and the mixture was stirred at room temperature for 3 hr. The reaction mixture was concentrated under reduced pressure, and the obtained residue was purified by silica gel column chromatography to give 5-ethyl-3-(((4-fluorophenyl)amino) (methylamino)methylene)-1-methyl-5(6-(trifluoromethyl)pyridin-3-yl)piperidine-2,4-dione (43 mg, 0.081 mmol).

yield: 69%
property: $^1$H-NMR($CDCl_3$):δ 12.5-13.4(m,1H), 11.2-12.0(m,1H), 8.68(s,1H), 7.85(d,1H), 7.63(d,1H), 7.00-7.10(m,4H), 3.71(d,1H), 3.60(d,1H), 3.09(s,3H), 2.51(d,3H), 2.14(quint.,1H), 1.95(quint.,1H), 0.78(t,3H)

Production Example 5

Production of tertiary butyl 4-(bis((5-fluoropyridin-2-yl)amino)methylene)-3,5-dioxopiperidine-1-carboxylate (compound No. 4-10)

[Chem. 26]

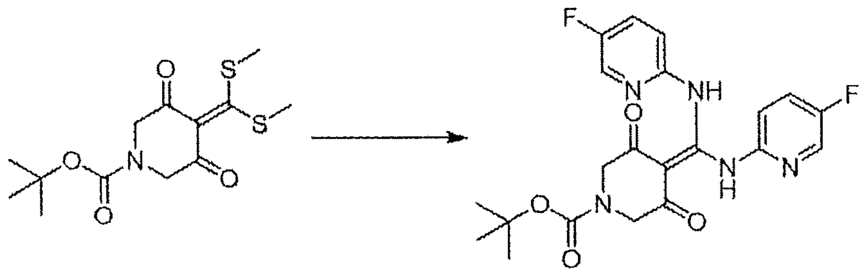

Tertiary butyl 4-(bis(methylthio)methylene)-3,5-dioxopiperidine-1-carboxylate (0.14 g, 0.45 mmol) and 5-fluoropyridin-2-amine (0.11 g, 0.98 mmol) were dissolved in toluene (10 mL), and reacted for 1 hr with heating under reflux. The reaction mixture was concentrated under reduced pressure, and the obtained residue was purified by silica gel column chromatography to give tertiary butyl 4-(bis((5-fluoropyridin-2-yl)amino)methylene)-3,5-dioxopiperidine-1-carboxylate (0.14 g, 0.30 mmol).

yield: 67%
property: melting point 196-198°C

Production Example 6

Production of 3-(amino((4-fluorophenyl)amino)methylene)-1-(4-(trifluoromethyl)phenyl)azepane-2,4-dione (compound No. 5-1)

[Chem. 27]

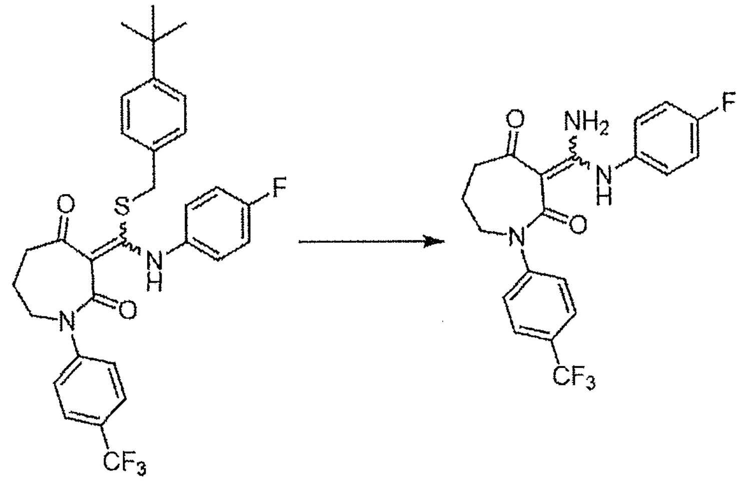

To a solution of 3-(((4-(tertiary butyl)benzyl)thio) ((4-fluorophenyl)amino)methylene)-1-(4-(trifluoromethyl)phenyl)azepane-2,4-dione (80 mg, 0.14 mmol) in tetrahydrofuran (1.0 mL) was added a solution of ammonia in methanol (0.11 mL, 0.21 mmol) at room temperature. The reaction mixture was stirred at room temperature for 1 hr, water was added to the reaction mixture, and the mixture was extracted 3 times with ethyl acetate. The organic layer was concentrated under reduced pressure, and the obtained residue was purified by silica gel column chromatography to give 3-(amino((4-fluorophenyl)amino)methylene)-1-(4-(trifluoromethyl)phenyl)azepane-2,4-dione (44 mg, 0.11 mmol).

yield: 77%
property: melting point 220-221°C

Production Example 7

Production of methyl 2-(6-cyanopyridin-3-yl)-5-(((2,2-difluoroethyl)amino) ((4-fluorophenyl)amino)methylene)-4,6-dioxotetrahydropyridazine-1(2H)-carboxylate (compound No. 6-15)

[Chem. 28]

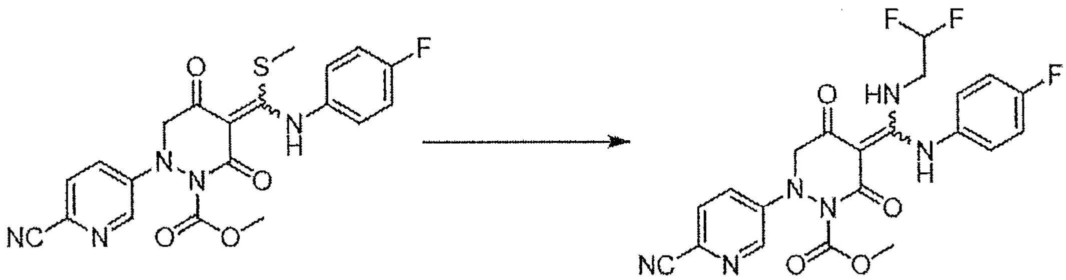

Methyl 2-(6-cyanopyridin-3-yl)-5-(((4-fluorophenyl)amino) (methylthio)methylene)-4,6-dioxotetrahydropyridazine-1(2H)-carboxylate (0.15 g, 0.34 mmol) was dissolved in tetrahydrofuran (2 mL) under an argon atmosphere, triethylamine (41 mg, 0.41 mmol) and difluoroethylamine (33 mg, 0.41 mmol) were added, and the mixture was stirred at room temperature for 40 min. To the reaction mixture was added 1N hydrochloric acid (6 mL), and the mixture was extracted twice with ethyl acetate. The organic layer was washed with saturated brine, dried over anhydrous sodium

sulfate, and concentrated under reduced pressure. The precipitated crystals were washed with tertiary butyl methyl ether to give methyl 2-(6-cyanopyridin-3-yl)-5-(((2,2-difluoroethyl) amino) ((4-fluorophenyl)amino)methylene)-4,6-dioxotetrahydropyridazine-1(2H)-carboxylate (0.12 mg, 0.24 mmol).

yield: 72%
property: melting point 115-120°C

Reference Example 1

Production of 2-(bis(methylthio)methylene)-5-(4-(trifluoromethyl)phenyl)cyclohexane-1,3-dione (compound No. 8-13)

[Chem. 29]

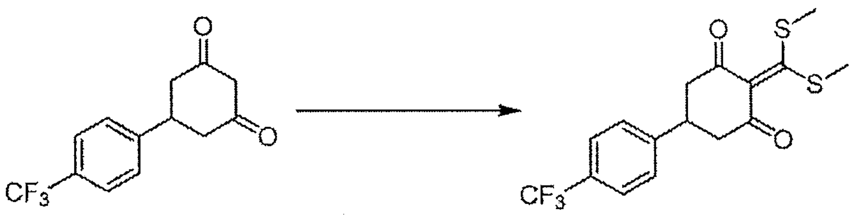

5-(4-(Trifluoromethyl)phenyl)cyclohexane-1,3-dione (2.6 g, 10 mmol), cesium carbonate (9.8 g, 30 mmol), and tetrabutylammonium bromide (32 mg, 0.10 mmol) were added to N,N-dimethylacetamide (10 mL), and the mixture was stirred under an argon atmosphere at room temperature for 1 hr. To the reaction mixture was added dropwise a solution of carbon disulfide (2.3 g, 30 mmol) in N,N-dimethylacetamide (2 mL) at room temperature, and the mixture was stirred at room temperature for 30 min and then allowed to stand overnight at room temperature. To the reaction mixture was added dropwise a solution of methyl iodide (3.20 g, 22.5 mmol) in N,N-dimethylacetamide (2 mL) at room temperature, and the mixture was stirred at room temperature for 2 hr. To the reaction mixture was added saturated aqueous ammonium chloride solution, and the mixture was extracted with ethyl acetate. The organic layer was washed successively with 5% aqueous sodium thiosulfate solution and saturated aqueous ammonium chloride solution, dried over anhydrous sodium sulfate, filtered, and concentrated under reduced pressure. The obtained residue was purified by silica gel column chromatography to give 2-(bis(methylthio)methylene)-5-(4-(trifluoromethyl)phenyl)cyclohexane-1,3-dione (2.9 g, 8.1 mmol).

yield: 81%
property: melting point 110-114°C

Reference Example 2

Production of 3-(bis(methylthio)methylene)-6-(5-bromopyridin-2-yl)dihydro-2H-pyran-2,4(3H)-dione

[Chem. 30]

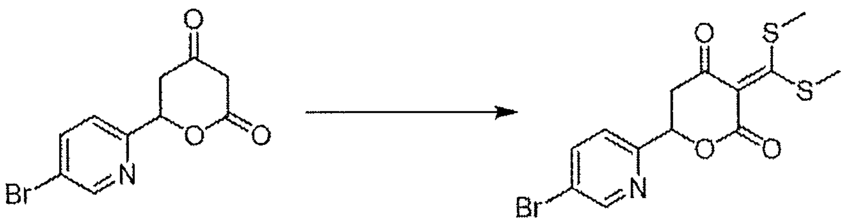

6-(5-Bromopyridin-2-yl)dihydro-2H-pyran-2,4(3H)-dione (1.0 g, 3.7 mmol), cesium carbonate (3.6 g, 11 mmol), and tetrabutylammonium bromide (13 mg, 0.039 mmol) were dissolved in N,N-dimethylacetamide (40 mL), and the mixture was stirred under an argon atmosphere at room temperature for 1 hr. A mixture of carbon disulfide (0.89 g, 11 mmol) and N,N-dimethylacetamide (4 mL) was added dropwise to the reaction mixture at room temperature, and the

mixture was stirred at room temperature for 30 min and then allowed to stand overnight at room temperature. A solution of methyl iodide (1.3 g, 8.8 mmol) in N,N-dimethylacetamide (2 mL) was added dropwise at room temperature to the reaction mixture, and the mixture was stirred at room temperature for 2 hr. To the reaction mixture was added saturated aqueous ammonium chloride solution, and the mixture was extracted with ethyl acetate. The organic layer was washed successively with 5%aqueous sodium thiosulfate solution and saturated aqueous ammonium chloride solution, dried over anhydrous sodium sulfate, filtered, and concentrated under reduced pressure. The obtained residue was purified by silica gel column chromatography to give 3-(bis(methylthio)methylene)-6-(5-bromopyridin-2-yl)dihydro-2H-pyran-2,4(3H)-dione (1.6 g, 4.2 mmol) as a crude product.

Reference Example 3

Production of 3-(((4-tertiary butyl)benzyl)thio) ((4-fluorophenyl)amino)methylene)-1-methyl-6-(4-(trifluoromethyl)phenyl)piperidine-2,4-dione (compound No. 9-7)

Reference Example 3-1

Production of 1-methyl-6-(4-(trifluoromethyl)phenyl)piperidine-2,4-dione

[Chem. 31]

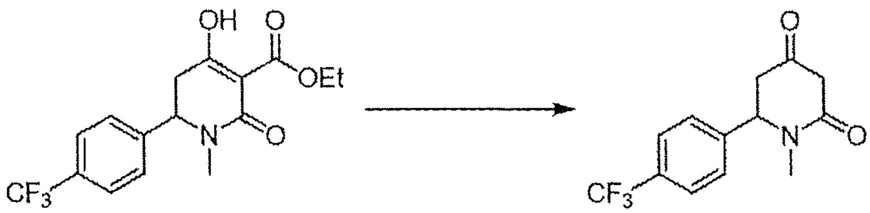

Ethyl 4-hydroxy-1-methyl-2-oxo-6-(4-(trifluoromethyl)phenyl)-1,2,5,6-tetrahydropyridine-3-carboxylate (0.50 g, 1.5 mmol) was dissolved in acetonitrile (14 mL), water (1.5 mL) was added, and the mixture was stirred with heating under reflux for 2 hr. The reaction mixture was concentrated under reduced pressure to give 1-methyl-6-(4-(trifluoromethyl) phenyl)piperidine-2,4-dione (0.20 g, 0.74 mmol) as a crude product. This compound was used in the next step without further purification.

Reference Example 3-2

Production of N-(4-fluorophenyl)-4-hydroxy-1-methyl-6-oxo-2-(4-(trifluoromethyl)phenyl)-2,3-dihydropyridine-5-carbothioamide

[Chem. 32]

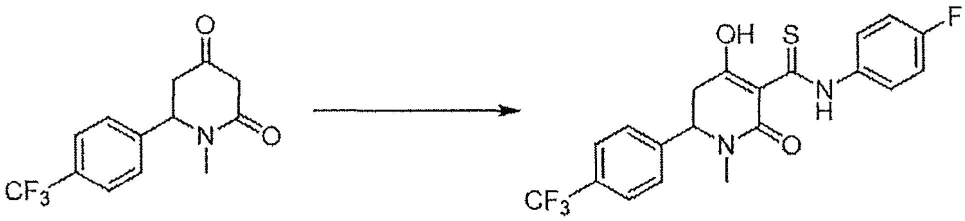

To a solution of 1-methyl-6-(4-(trifluoromethyl)phenyl)piperidine-2,4-dione (0.36 g, 1.2 mmol) and 4-fluorophenylisothiocyanate (0.28 g, 1.8 mmol) in N,N-dimethylacetamide (3 mL) was added 60%wt sodium hydride (72 mg, 1.8 mmol), and the mixture was stirred at room temperature for 3 hr. After completion of the reaction, the reaction mixture was poured into 1N hydrochloric acid, extracted with ethyl acetate, and washed with water. The obtained organic layer was dried over magnesium sulfate and concentrated under reduced pressure. The obtained residue was purified by silica gel

column chromatography to give N-(4-fluorophenyl)-4-hydroxy-1-methyl-2-oxo-6-(4-(trifluoromethyl)phenyl)-1,2,5,6-tetrahydropyridine-3-carbothioamide (0.34 g, 0.63 mmol).

yield: 63%
property: melting point 155-156°C

Reference Example 3-3

Production of 3-(((4-(tertiary butyl)benzyl)thio) ((4-fluorophenyl)amino)methylene)-1-methyl-6-(4-(trifluoromethyl)phenyl)piperidine-2,4-dione

[Chem. 33]

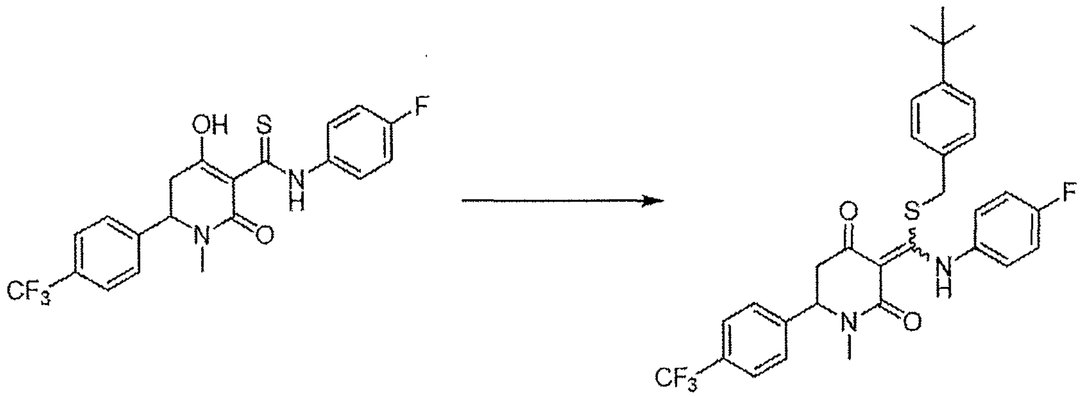

To a solution of N-(4-fluorophenyl)-4-hydroxy-1-methyl-2-oxo-6-(4-(trifluoromethyl)phenyl)-1,2,5,6-tetrahydropyridine-3-carbothioamide (0.34 g, 0.81 mmol) and 4-tertiary butylbenzyl bromide (0.28 g, 1.2 mmol) in tetrahydrofuran (8.1 mL) was added triethylamine (0.25 mg, 2.4 mmol) and the mixture was stirred at room temperature for 5 hr. After completion of the reaction, to the reaction mixture was added saturated aqueous ammonium chloride solution, and the mixture was extracted with ethyl acetate and washed with water. The obtained organic layer was dried over magnesium sulfate and concentrated under reduced pressure to give 3-(((4-(tertiary butyl)benzyl)thio) ((4-fluorophenyl)amino)methylene)-1-methyl-6-(4-(trifluoromethyl)phenyl)piperidine-2,4-dione (0.41 mg, 0.72 mmol).

yield: 90%
property: $^1$H-NMR($CDCl_3$):$\delta$ 14.1(s,1H), 7.61(m,2H), 7.21-7.35(m,6H), 7.08(m,2H), 6.95(m,2H), 4.69(m,1H), 3.55(m,2H), 3.31(m,1H), 3.11(m,3H), 2.77(m,1H), 1.27(m,9H)

Reference Example 4

Production of 3-(((4-tertiary butyl)benzyl)thio) ((4-fluorophenyl)amino)methylene)-5-ethyl-1-methyl-5-(6-(trifluoromethyl)pyridin-3-yl)piperidine-2,4-dione (compound No. 9-8)

Reference Example 4-1

Production of methyl 2-cyano-2-(6-(trifluoromethyl)pyridin-3-yl)butanoate

[Chem. 34]

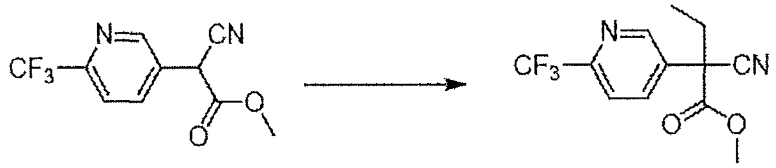

Methyl 2-cyano-2-(6-(trifluoromethyl)pyridin-3-yl)acetate (40 g, 0.16 mol) was dissolved in acetonitrile (0.55 L), cesium carbonate (11 g, 0.33 mol) and iodoethane (38 g, 0.25 mol) were added, and the mixture was stirred at 50°C for 20 hr. The reaction mixture was neutralized with 1 M hydrochloric acid, and organic layer was washed with saturated brine. The obtained organic layer was dried over sodium sulfate and concentrated under reduced pressure. The obtained residue was purified by silica gel chromatography to give methyl 2-cyano-2-(6-(trifluoromethyl)pyridin-3-yl)butanoate (35 g, 0.13 mmol).

yield: 75%
property: $^1$H-NMR($CDCl_3$): δ 8.94(d,1H), 8.10(dd,1H), 7.75(d,1H), 3.85(s,3H), 2.46-2.57(m,1H), 2.16-2.27(m,1H), 1.11(t,3H)

Reference Example 4-2

Production of methyl 2-(((tertiary butoxycarbonyl)amino)methyl)-2-(6-(trifluoromethyl)pyridin-3-yl)butanoate

[Chem. 35]

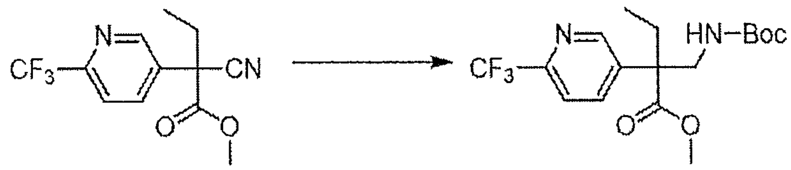

Methyl 2-cyano-2-(6-(trifluoromethyl)pyridin-3-yl)butanoate (35 g, 0.13 mol) was dissolved in methanol (0.40 L), and di-tertiary-butyl dicarbonate (56 g, 0.26 mol) and palladium carbon (8.0 g, 10%wt%) were added. Under a hydrogen atmosphere, the reaction mixture was stirred at 40°C for 96 hr. The reaction mixture was cooled to room temperature, filtered through celite to remove palladium carbon, and extracted with ethyl acetate. The organic layer was washed with saturated brine, and the obtained organic layer was dried over sodium sulfate, dried by filtration, and recrystallized in petroleum ether to give methyl 2-(((tertiary butoxycarbonyl)amino)methyl)-2-(6-(trifluoromethyl)pyridin-3-yl)butanoate (38 g, 0.10 mol). property: $^1$H-NMR ($CDCl_3$): δ 8.53(s,1H), 7.78(d,1H), 7.65(d,1H), 4.89(brs,1H), 3.78(s,3H), 3.64-3.73(m,2H), 2.12(q,2H), 1.29(s,9H), 0.93(t,3H)

Reference Example 4-3

Production of methyl 2-(((tertiary butoxycarbonyl) (methyl)amino)methyl)-2-(6-(trifluoromethyl)pyridin-3-yl)butanoate

[Chem. 36]

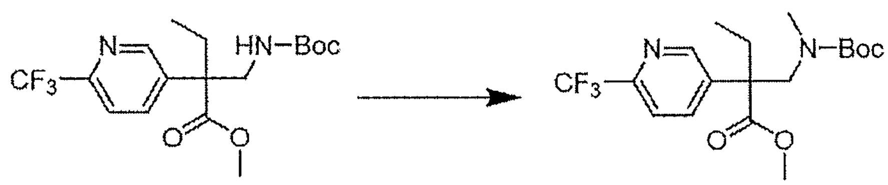

Methyl 2-(((tertiary butoxycarbonyl)amino)methyl)-2-(6-(trifluoromethyl)pyridin-3-yl)butanoate (38 g, 0.10 mol) was dissolved in tetrahydrofuran (0.40 L), sodium hydride (10 g, 0.25 mol, 60%wt%) was added thereto at 0°C, and the

mixture was stirred for 30 min. Thereafter, to the reaction mixture was added methyl iodide (72 g, 0.51 mol) at 25°C, and the mixture was stirred at 55°C for 12 hr. To the reaction mixture was added saturated aqueous ammonium chloride solution, and the mixture was extracted with ethyl acetate. The organic layer was washed with saturated brine, and the obtained organic layer was dried over sodium sulfate and concentrated under reduced pressure. The obtained residue was purified by silica gel column chromatography to give methyl 2-(((tertiary butoxycarbonyl) (methyl)amino)methyl)-2-(6-(trifluoromethyl)pyridin-3-yl)butanoate (37 g, 0.094 mol).
yield: 92%

Reference Example 4-4

Production of methyl 2-((methylamino)methyl)-2-(6-(trifluoromethyl)pyridin-3-yl)butanoate hydrochloride

[Chem. 37]

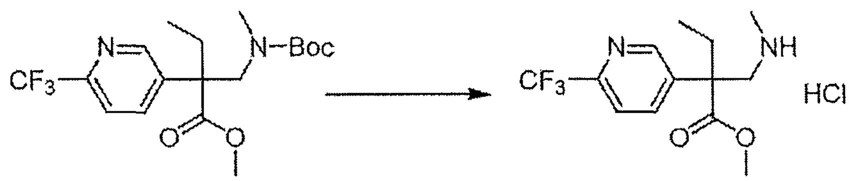

Methyl 2-(((tertiary butoxycarbonyl) (methyl)amino)methyl)-2-(6-(trifluoromethyl)pyridin-3-yl)butanoate (41 g, 0.11 mol) was dissolved in acetic acid (50 mL), 4N hydrogen chloride/ethyl acetate solution (0.20 L) was added, and the mixture was stirred at room temperature for 2 hr. The reaction mixture was concentrated under reduced pressure, and diethyl ether was added to the obtained residue. The precipitated solid was collected by filtration and dried to give methyl 2-((methylamino)methyl)-2-(6-(trifluoromethyl)pyridin-3-yl)butanoate hydrochloride (41 g, 0.10 mol).
yield: 98%

Reference Example 4-5

Production of methyl 2-((3-ethoxy-N-methyl-3-oxopropanamido)methyl)-2-(6-(trifluoromethyl)pyridin-3-yl)butanoate

[Chem. 38]

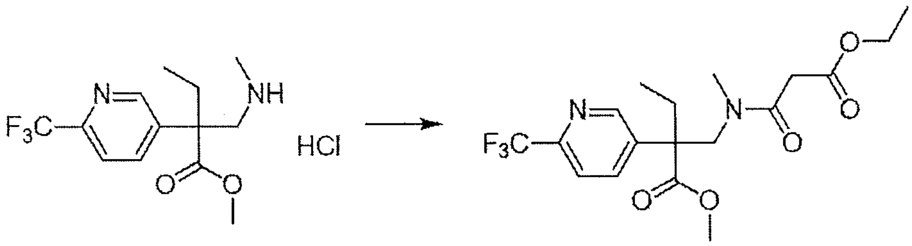

Methyl 2-((methylamino)methyl)-2(6-(trifluoromethyl)pyridin-3-yl)butanoate hydrochloride (32 g, 99 mmol) was dissolved in methylene chloride (0.30 L), diisopropylethylamine (26 g, 0.20 mol) and ethyl 3-chloro-3-oxopropionate (15 g, 99 mmol) were added, and the mixture was stirred at 25°C for 1 hr. The reaction mixture was washed with saturated brine, and the obtained organic layer was dried over sodium sulfate and concentrated under reduced pressure to give methyl 2-((3-ethoxy-N-methyl-3-oxopropanamido)methyl)-2-(6-(trifluoromethyl)pyridin-3-yl)butanoate.

yield: 91%
property: $^1$H-NMR($CDCl_3$): δ 8.56(d,1H), 7.82(dd,1H), 7.65(d,1H), 4.26(d,1H), 4.16(q,2H), 3.82(d,1H), 3.75(s,3H), 3.30(s,2H), 2.65(s,3H), 2.21-2.33(m,1H), 2.05-2.16(m,1H), 1.25(t,3H), 0.91(t,3H)

Reference Example 4-6

Production of ethyl 3-ethyl-4-hydroxy-1-methyl-6-oxo-3-(6-(trifluoromethyl)pyridin-3-yl)-2H-pyridine-5-carboxylate

[Chem. 39]

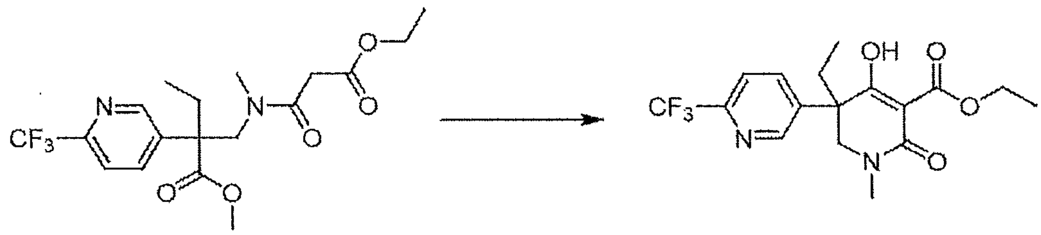

To a solution of methyl 2-((3-ethoxy-N-methyl-3-oxopropanamido)methyl)-2-(6-(trifluoromethyl)pyridin-3-yl)butanoate (30 g, 74 mmol) in ethanol (0.30 L) was added a solution (0.20 L) of sodium ethoxide (51 g, 0.15 mol) in ethanol. The reaction mixture was stirred at 60°C for 1 hr, poured into saturated aqueous ammonium chloride solution, extracted with methylene chloride, and washed with saturated brine. The obtained organic layer was dried over sodium sulfate, concentrated under reduced pressure, and recrystallized from ethanol. The obtained solid was collected by filtration to give ethyl 3-ethyl-4-hydroxy-1-methyl-6-oxo-3-(6-(trifluoromethyl)pyridin-3-yl)-2H-pyridine-5-carboxylate (19 g, 38 mmol).

property: $^1$H-NMR($CDCl_3$): $\delta$ 16.3(s,0.3H), 14.9(s,0.4H), 8.50-8.81(m,1H), 7.85(d,J=6.8Hz,1H), 7.65(d,J=8.0Hz,1H), 4.24-4.44(m,2H), 3.63-3.89(m,2H), 2.99-3.25(m,3H), 2.11-2.23(m,1H), 1.80-2.09(m,1H), 1.39(t,J=7.2Hz,3H), 0.70-0.91(m,3H)

Reference Example 4-7

Production of 5-ethyl-1-methyl-5-(6-(trifluoromethyl)pyridin-3-yl)piperidine-2,4-dione

[Chem. 40]

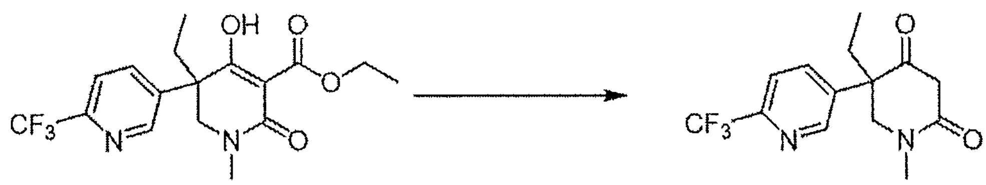

Ethyl 3-ethyl-4-hydroxy-1-methyl-6-oxo-3-(6-(trifluoromethyl)pyridin-3-yl)-2H-pyridine-5-carboxylate (0.50 g, 1.3 mmol) was dissolved in acetonitrile (20 mL), water (2 mL) was added, and the mixture was stirred with heating under reflux for 4 hr. The reaction mixture was concentrated under reduced pressure to give 5-ethyl-1-methyl-5-(6-(trifluoromethyl)pyridin-3-yl)piperidine-2,4-dione (0.40 g, 1.3 mmol) as a crude product. This compound was used in the next step without further purification.

Reference Example 4-8

Production of 5-ethyl-N-(4-fluorophenyl)-4-hydroxy-1-methyl-2-oxo-5-(6-(trifluoromethyl)pyridin-3-yl)-1,2,5,6-tetrahydropyridine-3-carbothioamide

[Chem. 41]

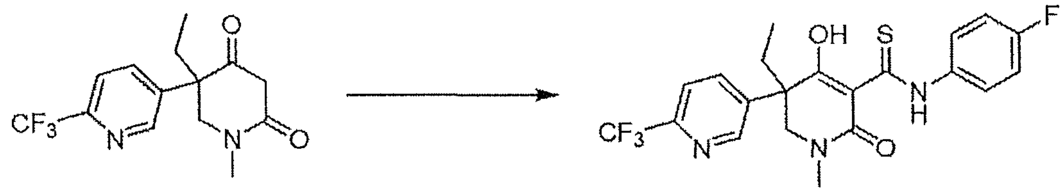

To a solution of 5-ethyl-1-methyl-5-(6-(trifluoromethyl)pyridin-3-yl)piperidine-2,4-dione (0.40 g, 1.3 mmol) and 4-fluorophenylisothiocyanate (0.33 g, 2.0 mmol) in N,N-dimethylacetamide (3 mL) was added 60%wt sodium hydride (80 mg, 2.0 mmol) and the mixture was stirred at room temperature for 2 hr. After completion of the reaction, the reaction mixture was poured into 1N hydrochloric acid, and this was extracted with ethyl acetate and washed with water. The obtained organic layer was dried over magnesium sulfate and concentrated under reduced pressure. The obtained residue was purified by silica gel column chromatography to give 5-ethyl-N-(4-fluorophenyl)-4-hydroxy-1-methyl-2-oxo-5-(6-(trifluoromethyl)pyridin-3-yl)-1,2,5,6-tetrahydropyridine-3-carbothioamide (0.28 g, 0.62 mmol).

yield: 58%
property: melting point 146-147°C

Reference Example 4-9

Production of 3-(((4-(tertiary butyl)benzyl)thio) ((4-fluorophenyl)amino)methylene)-5-ethyl-1-methyl-5-(6-trifluoromethyl)pyridin-3-yl)piperidine-2,4-dione

[Chem. 42]

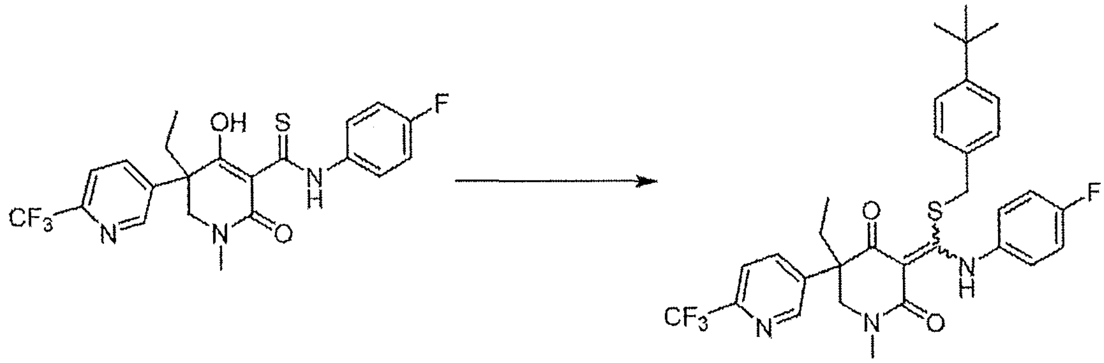

To a solution of 5-ethyl-N-(4-fluorophenyl)-4-hydroxy-1-methyl-2-oxo-5-(6-(trifluoromethyl)pyridin-3-yl)-1,2,5,6-tetrahydropyridine-3-carbothioamide (0.25 g, 0.55 mmol) and 4-tertiary butylbenzyl bromide (0.19 mg, 0.83 mmol) in tetrahydrofuran (5.5 mL) was added triethylamine (0.23 mg, 1.7 mmol), and the mixture was stirred at room temperature for 5 hr. After completion of the reaction, to the reaction mixture was added saturated aqueous ammonium chloride solution, and this was extracted with ethyl acetate and washed with water. The obtained organic layer was dried over magnesium sulfate, filtered, and concentrated under reduced pressure to give 3-(((4-(tertiary butyl)benzyl)thio) ((4-fluorophenyl) amino)methylene)-5-ethyl-1-methyl-5-(6-trifluoromethyl)pyridin-3-yl)piperidine-2,4-dione (0.29 g, 0.49 mmol).

yield: 88%
property: melting point 162-163°C

Reference Example 5

Production of tertiary butyl 4-(bis(methylthio)methylene)-3,5-dioxopiperidine-1-carboxylate (compound No. 8-22)

[Chem. 43]

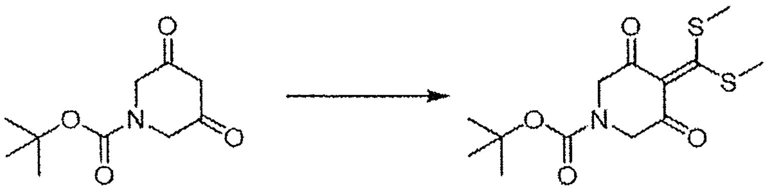

To tertiary butyl 3,5-dioxopiperidine-1-carboxylate (5.0 g, 23 mmo1), cesium carbonate (31 g, 94 mmo1), and tetrabutylammonium bromide (8.0 mg, 0.020 mmo1) was added N,N-dimethylacetamide (100 mL), and the mixture was stirred at room temperature for 3 hr. Carbon disulfide (3.6 g, 47 mmo1) was added dropwise to the reaction mixture and the mixture was allowed to stand at room temperature for 18 hr. Methyl iodide (7.3 g, 52 mmo1) was added dropwise to the reaction mixture and the mixture was stirred at room temperature for 5 hr. To the reaction mixture was added 2 M hydrochloric acid, and the mixture was extracted with ethyl acetate. The organic layer was washed twice with 2 M hydrochloric acid, washed with saturated aqueous ammonium chloride solution, dried over anhydrous sodium sulfate, and concentrated under reduced pressure to give tertiary butyl 4-(bis(methylthio)methylene)-3,5-dioxopiperidine-1-carboxylate (5.47 g, 17.2 mmo1).

yield: 77%
property: melting point 95-97°C

Reference Example 6

Production of (3-(((4-(tertiary butyl)benzyl)thio) ((4-fluorophenyl)amino)methylene)-1-(4-(trifluoromethyl)phenyl)azepane-2,4-dione (compound No. 9-13)

Reference Example 6-1

Production of N-(4-fluorophenyl)-4-hydroxy-2-oxo-1-(4-(trifluoromethyl)phenyl)-2,5,6,7-tetrahydro-1H-azepine-3-carbothioamide

[Chem. 44]

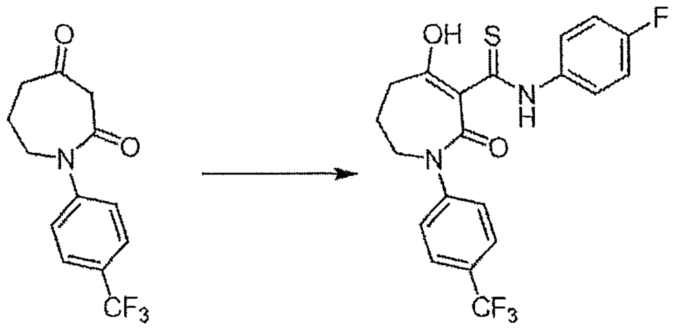

1-(4-Trifluorophenyl)azepane-2,4-dione (0.35 g, 1.3 mmol) was dissolved in N,N-dimethylacetamide (5.0 mL), and sodium hydride (58 mg, 1.4 mmol) and 4-fluorophenylthioisocyanate (0.26 g, 1.7 mmol) were added at room temperature. The reaction mixture was stirred at room temperature for 3 hr, saturated aqueous ammonium chloride solution was added, and the mixture was extracted 3 times with ethyl acetate. The organic layer was concentrated under reduced pressure, and the obtained residue was purified by silica gel column chromatography to give N-(4-fluorophenyl)-4-hydroxy-2-oxo-1-(4-(trifluoromethyl)phenyl)-2,5,6,7-tetrahydro-1H-azepine-3-carbothioamide (0.48 g, 1.1 mmol). yield: 88%

Reference Example 6-2

Production of (3-(((4-(tertiary butyl)benzyl)thio) ((4-fluorophenyl)amino)methylene)-1-(4-(trifluoromethyl)phenyl)azepane-2,4-dione

[Chem. 45]

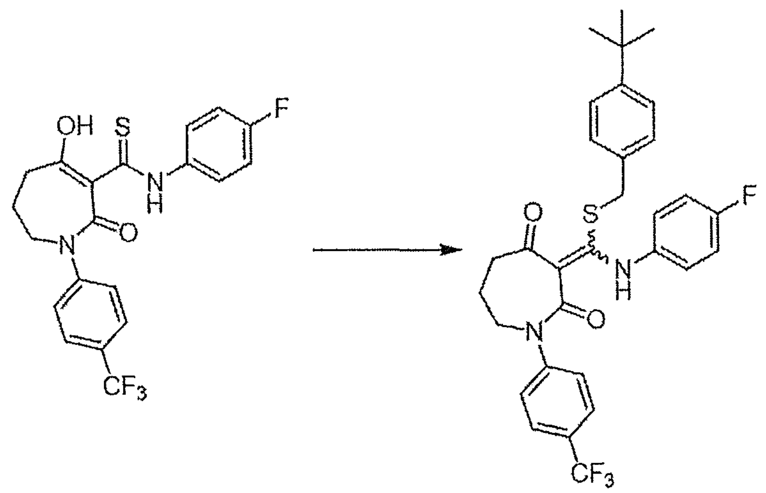

N-(4-fluorophenyl)-4-hydroxy-2-oxo-1-(4-(trifluoromethyl)phenyl)-2,5,6,7-tetrahydro-1H-azepine-3-carbothioamide (0.39 g, 0.92 mmol) was dissolved in tetrahydrofuran (5.0 mL), and 4-tertiary butylbenzyl bromide (0.27 g, 1.2 mmol) and triethylamine (0.39 mL, 2.8 mmol) were added at room temperature. The reaction mixture was stirred at room temperature for 3 hr, saturated aqueous ammonium chloride solution was added, and the mixture was extracted 3 times with ethyl acetate. The organic layer was concentrated under reduced pressure, and the obtained residue was purified by silica gel column chromatography to give (3-(((4-(tertiary butyl)benzyl)thio) ((4-fluorophenyl)amino)methylene)-1-(4-(trifluoromethyl)phenyl)azepane-2,4-dione (0.52 g, 0.91 mmol).

yield: 76%
property: melting point 129-130°C

Reference Example 7

Production of methyl 2-(6-cyanopyridin-3-yl)-5-(((4-fluorophenyl)amino) (methylthio)methylene)-4,6-dioxotetrahydropyridazine-1(2H)-carboxylate

Reference Example 7-1

Production of methyl 2-(6-cyanopyridin-3-yl)-4,6-dioxotetrahydropyridazine-1(2H)-carboxylate

[Chem. 46]

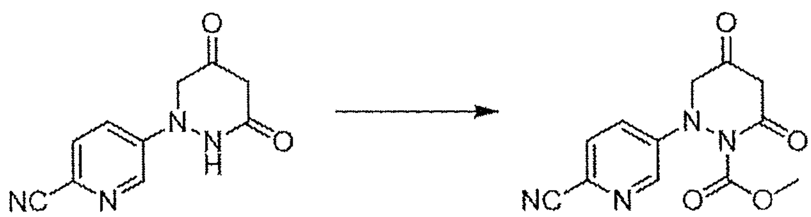

5-(3,5-Dioxotetrahydropyridazin-1(2H)-yl)picolinonitrile (1.0 g, 4.6 mmol) and potassium carbonate (1.5 g, 11 mmol) were dissolved in acetonitrile (10 mL), methyl chloroformate (0.96 g, 10 mmol) was added, and the mixture was stirred at room temperature for 30 min. Thereafter, methanol (2 mL) and 4-dimethylaminopyridine (0.57 g, 4.6 mmol) were

added, and the mixture was stirred at room temperature for 2 min. To the reaction mixture were added 2 M aqueous potassium hydrogen sulfate solution (13 mL) and water (10 mL), and the mixture was extracted twice with ethyl acetate. The organic layer was washed with saturated brine, dried over sodium sulfate, and concentrated under reduced pressure to give methyl-2-(6-cyanopyridin-3-yl)-4,6-dioxotetrahydropyridazine-1(2H)-carboxylate (0.82 g, 3.0 mmol).

yield: 65%
property: $^1$H-NMR($CDCl_3$/TMS, ppm): δ 8.34(d,1H), 7.67(d,1H), 7.24(d,1H), 4.59-4.00(brs,2H), 3.42(s,2H), 1.55(s,9H)

Reference Example 7-2

Production of methyl 2-(6-cyanopyridin-3-yl)-5-((4-fluorophenyl)carbamothioyl)-4-hydroxy-6-oxo-2,3-dihydropyridazine-1(6H)-carboxylate

[Chem. 47]

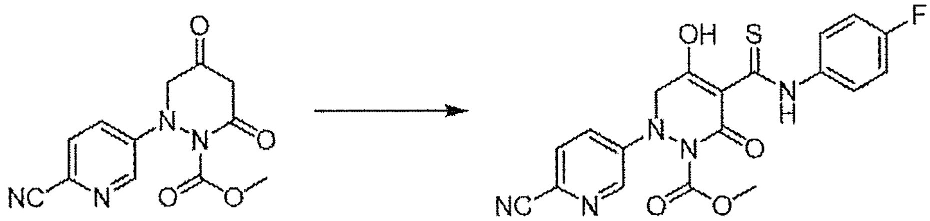

Methyl 2-(6-cyanopyridin-3-yl)-4,6-dioxotetrahydropyridazine-1(2H)-carboxylate (0.30 g, 1.1 mmol) was dissolved in N,N-dimethylacetamide (5 mL), diazabicycloundecene (0.20 g, 1.3 mmol) and 4-fluorophenylisothiocyanate (0.18 g, 1.2 mmol) were added, and the mixture was stirred at room temperature for 3.4 hr. To the reaction mixture was added saturated aqueous ammonium chloride solution, and the mixture was extracted 4 times with ethyl acetate. The organic layer was washed with saturated brine, dried over sodium sulfate, and concentrated under reduced pressure. The obtained residue was purified by silica gel column chromatography to give methyl 2-(6-cyanopyridin-3-yl)-5-((4-fluorophenyl)carbamothioyl)-4-hydroxy-6-oxo-2,3-dihydropyridazine-1(6H)-carboxylate (0.14 g, 0.34 mmol).

yield: 31%
property: melting point 122-124°C

Reference Example 7-3

Production of methyl 2-(6-cyanopyridin-3-yl)-5-(((4-fluorophenyl)amino) (methylthio)methylene)-4,6-dioxotetrahydropyridazine-1(2H)-carboxylate

[Chem. 48]

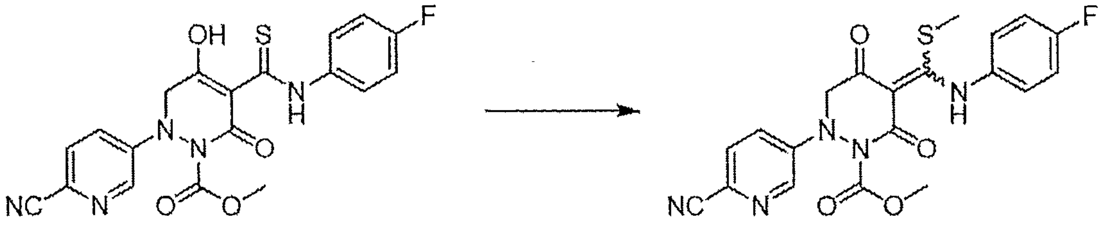

Methyl 2-(6-cyanopyridin-3-yl)-5-((4-fluorophenyl)carbamothioyl)-4-hydroxy-6-oxo-2,3-dihydropyridazine-1(6H)-carboxylate (0.12 g, 0.28 mmol) was dissolved in acetonitrile (4 mL), potassium carbonate (53 mg, 0.39 mmol) and methyl iodide (47 mg, 0.33 mmol) were added, and the mixture was stirred at room temperature for 2 hr. To the reaction mixture was added 1N hydrochloric acid, and the mixture was extracted 4 times with ethyl acetate. The organic layer was washed with saturated brine, dried over sodium sulfate, and concentrated under reduced pressure. The obtained residue

was washed with methyl tertiary butyl ether to give methyl 2-(6-cyanopyridin-3-yl)-5-(((4-fluorophenyl)amino) (methylthio) methylene)-4,6-dioxotetrahydropyridazine-1(2H)-carboxylate (90 mg, 0.20 mmol).

yield: 74%
property: melting point 185-187°C

Formulation Examples are shown below, but are not limitative. In the Formulation Examples, parts indicate parts by weight.

Formulation Example 1.

| | |
|---|---|
| compound of the present invention | 10 parts |
| xylene | 70 parts |
| N-methylpyrrolidone | 10 parts |
| mixture of polyoxyethylene nonyl phenyl ether and calcium alkylbenzenesulfonate | 10 parts |

The above are uniformly mixed and dissolved to give an emulsion.

Formulation Example 2.

| | |
|---|---|
| compound of the present invention | 3 parts |
| clay powder | 82 parts |
| diatomaceous earth powder | 15 parts |

The above are uniformly mixed and pulverized to give a dust formulation.

Formulation Example 3.

| | |
|---|---|
| compound of the present invention | 5 parts |
| mixed powder of bentonite and clay | 90 parts |
| calcium lignin sulfonate | 5 parts |

The above are uniformly mixed, an appropriate amount of water is added, and the mixture is kneaded, granulated, and dried to give granules.

Formulation Example 4.

| | |
|---|---|
| compound of the present invention | 20 parts |
| kaolin and synthetic highly dispersed silica | 75 parts |
| mixture of polyoxyethylene nonyl phenyl etherand calcium alkylbenzenesulfonate | 5 parts |

The above are uniformly mixed and pulverized to give a wettable powder.
Examples of biological tests are shown below; however, the present invention is not limited thereto

Experimental Example 1. Insecticidal test against Plutella xylostella

Adults of Plutella xylostella were released on Chinese cabbage seedlings to spawn, and two days after release, the Chinese cabbage seedlings with eggs were immersed in a drug solution containing a compound represented by the formula (1) of the present invention or (3) or a salt thereof as an active ingredient and diluted to 500 ppm for about 30 sec, air-dried, and left standing in a thermostatic chamber at 25°C. Six days after the immersion in the drug solution, the number of surviving insects was examined. The corrected mortality was calculated by the following equation and the assessment was performed according to the criteria described below. In triplicate using 10 per plot.

[Math. 1]

$$\text{corrected mortality (\%)} = \frac{\text{(survival rate in untreated plot - survival rate in treated plot)}}{\text{(survival rate in untreated plot)}} \times 100$$

| | | |
|---|---|---|
| assessment criteria. | A | mortality 100% |
| | B | mortality 99% to 90% |
| | C | mortality 89% to 80% |
| | D | mortality 79% to 50% |
| | E | mortality 49% to 0% |

As a result, the compound represented by the formula (1) or (3) of the present invention showed a significant insecticidal activity, and particularly the compounds of compound Nos. 1-1, 1-10, 1-23, 1-28, 1-38, 1-43, 1-44, 1-45, 1-74, 1-75, 1-115, 1-116, 3-1, 3-4, 3-5, 3-6, 3-7, 3-11, 3-12, 3-13, 3-14, 3-15, 3-16, 3-21, 3-22, 3-23, 3-24, 3-25, 3-39, 3-42, 3-47, 4-1, 4-3, 4-4, 5-1, 6-6, 6-8, 6-13, 6-16, 6-17, 6-19, 6-33, 9-1, 9-2, 9-4, 9-7, 9-9, 9-10, 9-11, 9-13, 9-16, 9-17, 9-21, 9-22, 9-28, 9-29, 9-30, 9-31, 9-32, 9-35, 9-36, and 9-57 showed superior activity against Plutella xylostella with a rating of assessment A.

Experimental Example 2. Insecticidal test against Laodelphax striatellus

A compound represented by the formula (1) or (3) of the present invention or a salt thereof was dispersed in water and diluted to give a 500 ppm drug solution. Rice seedlings (variety: Nihonbare) were immersed in the drug solution for 30 seconds and air-dried, after which the seedlings were placed in a glass test tube and inoculated with 10 three-instar Laodelphax striatellus, and the test tube was plugged with a cotton plug. Eight days after the inoculation, the numbers of surviving insects and dead insects were examined. The corrected mortality was calculated by the following equation and the assessment was performed according to the criteria of Experimental Example 1.

[Math. 2]

$$\text{corrected mortality (\%)} = \frac{\text{(survival rate in untreated plot - survival rate in treated plot)}}{\text{(survival rate in untreated plot)}} \times 100$$

As a result, the compound represented by the formula (1) or (3) of the present invention showed a significant insecticidal activity, and particularly the compounds of compound Nos. 1-43, 3-4, 3-11, 3-15, 3-16, 3-21, 3-22, 3-23, 3-42, 3-47, 4-10, 5-1, 6-1, 6-2, 6-4, 6-5, 6-6, 6-7, 6-8, 6-10, 6-11, 6-12, 6-13, 6-14, 6-15, 6-16, 6-19, 6-20, 6-22, 6-23, 6-33, 6-37, 6-43, 6-46, 6-47, 9-11, 9-13, 9-21, 9-29, 9-31, 9-32, 9-35, and 9-36 showed superior activity against Laodelphax striatellus with a rating of assessment A.

Experimental Example 3. Insecticidal test against Frankliniella occidentalis

Female adults of Frankliniella occidentalis were inoculated onto Phaseolus vulgaris leaf pieces, and after allowing them to lay eggs for one day, the female adults were removed. Three days later, hatched larvae on the leaf pieces

were counted, and a drug solution containing the compound represented by the formula (1) or (3) of the present invention or a salt thereof as an active ingredient and diluted to 500 ppm was sprayed. Four days after the treatment, the number of surviving larvae of this species larvae was examined. The corrected mortality was calculated by the following equation and the assessment was performed according to the criteria of Experimental Example 1.

[Math. 3]

$$\text{corrected mortality (\%)} = \frac{\text{survival rate in untreated plot} - \text{survival rate in treated plot}}{\text{survival rate in untreated plot}} \times 100$$

As a result, the compound represented by the formula (1) or (3) of the present invention showed a significant insecticidal activity, and particularly the compounds of compound Nos. 1-43, 3-47, 5-1, 6-1, 6-2, 6-4, 6-5, 6-6, 6-10, 6-11, 6-13, 6-15, 6-16, 6-19, 6-20, 6-23, 6-25, 6-28, 6-30, 6-33, 6-34, 6-37, 6-38, 6-43, 6-47, 9-11, 9-13, 9-35, and 9-36 showed superior activity against Frankliniella occidentalis with a rating of assessment A.

Experimental Example 4. Effect test on Dirofilaria immitis

A given prepared solution, 100 L-1 stage larvae of Dirofilaria immitis, and a DMSO-diluted solution of the compound represented by the formula (1) or (3) of the present invention or a salt thereof were added to each well of a 96-well plate to a final concentration of 100 μM. Seven days later, the number of inactive larvae was counted and the motility inhibition rate was calculated.

As a result, among the compounds represented by the formula (1) or (3) of the present invention, the compounds of compound Nos. 1-1, 1-2, 1-3, 1-4, 1-5, 1-6, 1-7, 1-8, 1-9, 1-10, 1-11, 1-14, 1-15, 1-19, 1-23, 1-25, 1-27, 1-28, 1-29, 1-34, 1-36, 1-38, 1-42, 1-44, 1-45, 1-46, 1-47, 1-49, 1-52, 1-54, 1-56, 1-57, 1-64, 1-69, 1-70, 1-72, 1-73, 1-79, 1-80, 1-82, 1-115, 1-116, 1-117, 1-118, 2-1, 2-2, 2-3, 3-1, 3-2, 3-8, 3-9, 3-10, 3-11, 3-20, 3-22, 3-23, 3-24, 3-25, 3-39, 3-42, 3-43, 4-1, 4-3, 5-2, 6-4, 6-6, 6-7, 6-15, 6-20, 6-21, 6-22, 6-27, 6-29, 6-30, 6-31, 6-35, 6-38, 6-44, 6-47, 9-1, 9-32, 9-33, 9-34, 9-35, 9-36, 9-37, and 9-38 showed a motility inhibition rate of not less than 50%.

[Industrial Applicability]

The compound of the present invention and a salt thereof have superior effects as pest control agents.

This application is based on a patent application No. 2023-104156 filed in Japan (filing date: June 26, 2023), the contents of which are incorporated in full herein.

## Claims

1. A compound represented by the formula (1)

[Chem. 1]

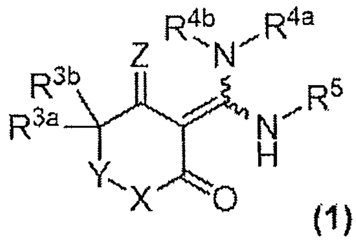

(1)

wherein

X is $CR^{1a}R^{1b}$, $NR^{1c}$, or an oxygen atom,
$R^{1a}$ and $R^{1b}$ are each

(a1) a hydrogen atom;
(a2) a halogen atom;
(a3) a $(C_1-C_6)$alkyl group;
(a4) a halo$(C_1-C_6)$alkyl group;
(a5) a phenyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a $(C_1-C_6)$alkyl group;
(b3) a $(C_2-C_6)$alkenyl group;
(b4) a $(C_2-C_6)$alkynyl group;
(b5) a $(C_3-C_6)$cycloalkyl group;
(b6) a halo$(C_1-C_6)$alkyl group;
(b7) a halo$(C_2-C_6)$alkenyl group;
(b8) a halo$(C_2-C_6)$alkynyl group;
(b9) a halo$(C_3-C_6)$cycloalkyl group;
(b10) a $(C_1-C_6)$alkyl group having 1 to 3 substituents each independently selected from substituent group A;
(b11) a $(C_1-C_6)$alkylcarbonyl group;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b18) a pyridazinyl group;
(b19) a pyridazinyl group having 1 to 3 substituents each independently selected from substituent group B;
(b20) a pyrimidinyl group;
(b21) a pyrimidinyl group having 1 to 3 substituents each independently selected from substituent group B;
(b22) a pyrazinyl group;
(b23) a pyrazinyl group having 1 to 3 substituents each independently selected from substituent group B;
(b24) a phenyl $(C_1-C_6)$ alkyl group;
(b25) a phenyl$(C_1-C_6)$alkyl group having 1 to 5 substituents each independently selected from substituent group B;
(b26) a pyridyl $(C_1-C_6)$ alkyl group;
(b27) a pyridyl$(C_1-C_6)$ alkyl group having 1 to 4 substituents each independently selected from substituent group B;
(b28) a thiazolyl$(C_1-C_6)$alkyl group; or
(b29) a thiazolyl$(C_1-C_6)$alkyl group having 1 to 2 substituents each independently selected from substituent group B,

Y is $CR^{2a}R^{2b}$, $NR^{2c}$, or $CH_2CH_2$,
$R^{2a}$ is

(c1) a hydrogen atom;
(c2) a phenyl group;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;

(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c28) a triazinyl group;
(c29) a triazinyl group having 1 to 2 substituents each independently selected from substituent group B;
(c30) a thiadiazolyl group;
(c31) a thiadiazolyl group having one substituent selected from substituent group B;
(c32) a pyrrolyl group;
(c33) a pyrrolyl group having 1 to 4 substituents each independently selected from substituent group B;
(c34) an imidazolyl group;
(c35) an imidazolyl group having 1 to 3 substituents each independently selected from substituent group B;
(c36) a triazolyl group;
(c37) a triazolyl group having 1 to 2 substituents each independently selected from substituent group B;
(c38) a tetrazolyl group;
(c39) a tetrazolyl group having one substituent selected from substituent group B;
(c40) a naphthyl group;
(c41) a naphthyl group having 1 to 7 substituents each independently selected from substituent group B;
(c42) a quinolyl group;
(c43) a quinolyl group having 1 to 6 substituents each independently selected from substituent group B;
(c44) a carboxyl group; or
(c45) a $(C_1-C_6)$alkoxycarbonyl group,

$R^{2b}$ is

(d1) a hydrogen atom; or
(d2) a $(C_1-C_6)$alkyl group,

$R^{2c}$ is

(e1) a $(C_1-C_6)$alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e10) a pyrazinyl group;
(e11) a pyrazinyl group having 1 to 3 substituents each independently selected from substituent group B;
(e12) a furyl group;
(e13) a furyl group having 1 to 3 substituents each independently selected from substituent group B;
(e14) a thienyl group;
(e15) a thienyl group having 1 to 3 substituents each independently selected from substituent group B;

(e16) an oxazolyl group;
(e17) an oxazolyl group having 1 to 2 substituents each independently selected from substituent group B;
(e18) an isoxazolyl group;
(e19) an isoxazolyl group having 1 to 2 substituents each independently selected from substituent group B;
(e20) a thiazolyl group;
(e21) a thiazolyl group having 1 to 2 substituents each independently selected from substituent group B;
(e22) an isothiazolyl group;
(e23) an isothiazolyl group having 1 to 2 substituents each independently selected from substituent group B;
(e24) a triazinyl group;
(e25) a triazinyl group having 1 to 2 substituents each independently selected from substituent group B;
(e26) an oxadiazolyl group;
(e27) an oxadiazolyl group having one substituent selected from substituent group B;
(e28) a thiadiazolyl group;
(e29) a thiadiazolyl group having one substituent selected from substituent group B;
(e30) a pyrrolyl group;
(e31) a pyrrolyl group having 1 to 4 substituents each independently selected from substituent group B;
(e32) a pyrazolyl group;
(e33) a pyrazolyl group having 1 to 3 substituents each independently selected from substituent group B;
(e34) an imidazolyl group;
(e35) an imidazolyl group having 1 to 3 substituents each independently selected from substituent group B;
(e36) a triazolyl group;
(e37) a triazolyl group having 1 to 2 substituents each independently selected from substituent group B;
(e38) a tetrazolyl group;
(e39) a tetrazolyl group having one substituent selected from substituent group B;
(e40) a naphthyl group;
(e41) a naphthyl group having 1 to 7 substituents each independently selected from substituent group B;
(e42) a quinolyl group;
(e43) a quinolyl group having 1 to 6 substituents each independently selected from substituent group B;
(e44) a pyridylcarbonyl group;
(e45) a pyridylcarbonyl group having 1 to 4 substituents each independently selected from substituent group B; or
(e46) a hydrogen atom,

$R^{3a}$ is

(f1) a hydrogen atom;
(f2) a $(C_1-C_6)$alkyl group;
(f3) a halo$(C_1-C_6)$alkyl group;
(f4) a phenyl group;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom;
(g2) a $(C_1-C_6)$alkyl group; or
(g3) a halo$(C_1-C_6)$alkyl group,

provided that $R^{1a}$, $R^{1b}$, $R^{2a}$, $R^{2b}$, $R^{3a}$, and $R^{3b}$ are not hydrogen atoms at the same time,
$R^{4a}$ and $R^5$ are each

(h1) a hydrogen atom;
(h2) a hydroxyl group;

(h3) a cyano group;
(h4) a $(C_1-C_6)$alkyl group;
(h5) a $(C_2-C_6)$alkenyl group;
(h6) a $(C_2-C_6)$alkynyl group;
(h7) a $(C_3-C_6)$ cycloalkyl group;
(h8) a $(C_1-C_6)$alkoxy group;
(h9) a halo$(C_1-C_6)$alkyl group;
(h10) a halo$(C_2-C_6)$alkenyl group;
(h11) a halo$(C_2-C_6)$alkynyl group;
(h12) a halo $(C_3-C_6)$ cycloalkyl group;
(h13) a halo$(C_1-C_6)$alkoxy group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C
(h29) a phenyl $(C_1-C_6)$alkyl group;
(h30) a phenyl$(C_1-C_6)$alkyl group having 1 to 5 substituents each independently selected from substituent group C;
(h31) a pyridyl$(C_1-C_6)$alkyl group;
(h32) a pyridyl$(C_1-C_6)$ alkyl group having 1 to 4 substituents each independently selected from substituent group C;
(h33) a pyridazinyl$(C_1-C_6)$alkyl group;
(h34) a pyridazinyl$(C_1-C_6)$alkyl group having 1 to 3 substituents each independently selected from substituent group C;
(h35) a pyrimidinyl$(C_1-C_6)$alkyl group;
(h36) a pyrimidinyl$(C_1-C_6)$alkyl group having 1 to 3 substituents each independently selected from substituent group C;
(h37) a pyrazinyl$(C_1-C_6)$alkyl group;
(h38) a pyrazinyl$(C_1-C_6)$alkyl group having 1 to 3 substituents each independently selected from substituent group C;
(h39) a thienyl$(C_1-C_6)$alkyl group;
(h40) a thienyl$(C_1-C_6)$alkyl group having 1 to 3 substituents each independently selected from substituent group C;
(h41) a furyl$(C_1-C_6)$alkyl group;
(h42) a furyl$(C_1-C_6)$alkyl group having 1 to 3 substituents each independently selected from substituent group C;
(h43) an oxazolyl$(C_1-C_6)$alkyl group;
(h44) an oxazolyl$(C_1-C_6)$alkyl group having 1 to 2 substituents each independently selected from substituent group C;
(h45) a thiazolyl$(C_1-C_6)$alkyl group;
(h46) a thiazolyl$(C_1-C_6)$alkyl group having 1 to 2 substituents each independently selected from substituent group C;
(h47) a benzofuranyl$(C_1-C_6)$alkyl group;
(h48) a benzofuranyl$(C_1-C_6)$alkyl group having 1 to 5 substituents each independently selected from substituent group C;
(h49) a tetrahydropyranyl$(C_1-C_6)$alkyl group;
(h50) a tetrahydrofuryl$(C_1-C_6)$alkyl group; or

(h51) a dihydrobenzopyranyl group,

$R^{4a}$ and $R^5$ may be the same or different,
$R^{4b}$ is

(i1) a hydrogen atom;
(i2) a $(C_1-C_6)$alkyl group; or
(i3) a halo$(C_1-C_6)$alkyl group,

when $R^{4a}$ is (h4) a $(C_1-C_6)$alkyl group, or (h14) a $(C_1-C_6)$alkyl group having 1 to 3 substituents each independently selected from substituent group A, and $R^{4b}$ is (i2) a $(C_1-C_6)$alkyl group, then $R^{4a}$ and $R^{4b}$ may form a 3- to 6-membered ring together with the adjacent nitrogen atom, and
Z is an oxygen atom, a sulfur atom, or $NR^7$ wherein $R^7$ is a hydroxyl group, a $(C_1-C_6)$alkoxy group, a halo$(C_1-C_6)$alkoxy group,
a $(C_1-C_6)$alkyl group, a halo$(C_1-C_6)$alkyl group, a $(C_3-C_6)$cycloalkyl group, an amino group, a $(C_1-C_6)$alkylamino group,
or a di$(C_1-C_6)$alkylamino group (the $(C_1-C_6)$alkyl groups may be the same or different),
substituent group A consists of

(j1) a cyano group;
(j2) a $(C_3-C_6)$cycloalkyl group;
(j3) a $(C_1-C_6)$alkoxy group;
(j4) a $(C_1-C_6)$alkylsulfanyl group;
(j5) a $(C_1-C_6)$alkylsulfinyl group;
(j6) a $(C_1-C_6)$alkylsulfonyl group;
(j7) a $(C_1-C_6)$alkylamino group;
(j8) a di$(C_1-C_6)$alkylamino group (the $(C_1-C_6)$alkyl may be the same or different);
(j9) a halo $(C_3-C_6)$ cycloalkyl group;
(j10) a halo$(C_1-C_6)$alkoxy group;
(j11) a halo$(C_1-C_6)$alkylsulfanyl group;
(j12) a halo$(C_1-C_6)$alkylsulfinyl group; and
(j13) a halo$(C_1-C_6)$alkylsulfonyl group,

substituent group B consists of

(k1) a halogen atom;
(k2) a cyano group;
(k3) a nitro group;
(k4) an amino group;
(k5) a hydroxyl group;
(k6) a carboxyl group;
(k7) a $(C_1-C_6)$alkyl group;
(k8) a $(C_2-C_6)$alkenyl group;
(k9) a $(C_2-C_6)$alkynyl group;
(k10) a $(C_1-C_6)$alkoxy group;
(k11) a $(C_3-C_6)$cycloalkyl group;
(k12) a $(C_1-C_6)$alkylsulfanyl group;
(k13) a $(C_1-C_6)$alkylsulfinyl group;
(k14) a $(C_1-C_6)$alkylsulfonyl group;
(k15) a $(C_1-C_6)$alkylamino group;
(k16) a di$(C_1-C_6)$alkylamino group (the $(C_1-C_6)$alkyl may be the same or different);
(k17) a halo$(C_1-C_6)$ alkyl group;
(k18) a halo$(C_2-C_6)$alkenyl group;
(k19) a halo $(C_2-C_6)$ alkynyl group;
(k20) a halo $(C_1-C_6)$alkoxy group;
(k21) a halo$(C_3-C_6)$cycloalkyl group;
(k22) a halo$(C_1-C_6)$alkylsulfanyl group;
(k23) a halo$(C_1-C_6)$alkylsulfinyl group;

(k24) a halo($C_1$-$C_6$)alkylsulfonyl group;
(k25) a ($C_1$-$C_6$)alkoxy($C_1$-$C_6$)alkyl group;
(k26) a phenyl group;
(k27) a phenyl group having 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a halo($C_1$-$C_6$)alkyl group, and a halo ($C_1$-$C_6$)alkoxy group;
(k28) a pyridyl group; and
(k29) a pyridyl group having 1 to 4 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a halo($C_1$-$C_6$)alkyl group, and a halo($C_1$-$C_6$)alkoxy group,

substituent group C consists of

(l1) a halogen atom;
(l2) a cyano group;
(l3) a nitro group;
(l4) an amino group;
(l5) a hydroxyl group;
(l6) a carboxyl group;
(l7) a ($C_1$-$C_6$)alkyl group;
(l8) a ($C_2$-$C_6$)alkenyl group;
(l9) a ($C_2$-$C_6$)alkynyl group;
(l10) a ($C_1$-$C_6$)alkoxy group;
(l11) a ($C_3$-$C_6$) cycloalkyl group;
(l12) a ($C_1$-$C_6$)alkylsulfanyl group;
(l13) a ($C_1$-$C_6$)alkylsulfinyl group;
(l14) a ($C_1$-$C_6$)alkylsulfonyl group;
(l15) a ($C_1$-$C_6$)alkylamino group;
(l16) a di($C_1$-$C_6$)alkylamino group (the ($C_1$-$C_6$)alkyl may be the same or different);
(l17) a halo($C_1$-$C_6$)alkyl group;
(l18) a halo($C_2$-$C_6$)alkenyl group;
(l19) a halo($C_2$-$C_6$)alkynyl group;
(l20) a halo ($C_1$-$C_6$) alkoxy group;
(l21) a halo($C_3$-$C_6$)cycloalkyl group;
(l22) a halo($C_1$-$C_6$)alkylsulfanyl group;
(l23) a halo($C_1$-$C_6$)alkylsulfinyl group;
(l24) a halo($C_1$-$C_6$)alkylsulfonyl group; and
(l25) a ($C_1$-$C_6$)alkoxy($C_1$-$C_6$)alkyl group,

or a salt thereof.

**2.** The compound according to claim 1, wherein

X is $CR^{1a}R^{1b}$, $NR^{1c}$, or an oxygen atom,
$R^{1a}$ and $R^{1b}$ are each

(a1) a hydrogen atom;
(a2) a halogen atom;
(a3) a ($C_1$-$C_6$)alkyl group;
(a4) a halo($C_1$-$C_6$)alkyl group;
(a5) a phenyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a $(C_1-C_6)$alkyl group;
(b5) a $(C_3-C_6)$cycloalkyl group;
(b6) a halo$(C_1-C_6)$alkyl group;
(b9) a halo$(C_3-C_6)$cycloalkyl group;
(b10) a $(C_1-C_6)$alkyl group having 1 to 3 substituents each independently selected from substituent group A;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b18) a pyridazinyl group;
(b19) a pyridazinyl group having 1 to 3 substituents each independently selected from substituent group B;
(b20) a pyrimidinyl group;
(b21) a pyrimidinyl group having 1 to 3 substituents each independently selected from substituent group B;
(b22) a pyrazinyl group;
(b23) a pyrazinyl group having 1 to 3 substituents each independently selected from substituent group B;
(b24) a phenyl $(C_1-C_6)$ alkyl group;
(b25) a phenyl $(C_1-C_6)$ alkyl group having 1 to 5 substituents each independently selected from substituent group B;
(b26) a pyridyl$(C_1-C_6)$alkyl group;
(b27) a pyridyl $(C_1-C_6)$ alkyl group having 1 to 4 substituents each independently selected from substituent group B;
(b28) a thiazolyl $(C_1-C_6)$ alkyl group; or
(b29) a thiazolyl$(C_1-C_6)$alkyl group having 1 to 2 substituents each independently selected from substituent group B,

Y is $CR^{2a}R^{2b}$, $NR^{2c}$ or $CH_2CH_2$,
$R^{2a}$ is

(c1) a hydrogen atom;
(c2) a phenyl group;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;

(c44) a carboxyl group; or
(c45) a ($C_1$-$C_6$)alkoxycarbonyl group,

$R^{2b}$ is

(d1) a hydrogen atom; or
(d2) a ($C_1$-$C_6$)alkyl group,

$R^{2c}$ is

(e1) a ($C_1$-$C_6$)alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e10) a pyrazinyl group;
(e11) a pyrazinyl group having 1 to 3 substituents each independently selected from substituent group B;
(e12) a furyl group;
(e13) a furyl group having 1 to 3 substituents each independently selected from substituent group B;
(e14) a thienyl group;
(e15) a thienyl group having 1 to 3 substituents each independently selected from substituent group B;
(e16) an oxazolyl group;
(e17) an oxazolyl group having 1 to 2 substituents each independently selected from substituent group B;
(e20) a thiazolyl group;
(e21) a thiazolyl group having 1 to 2 substituents each independently selected from substituent group B;
(e44) a pyridylcarbonyl group;
(e45) a pyridylcarbonyl group having 1 to 4 substituents each independently selected from substituent group B; or
(e46) a hydrogen atom,

$R^{3a}$ is

(f1) a hydrogen atom;
(f2) a ($C_1$-$C_6$)alkyl group;
(f4) a phenyl group;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom;
(g2) a ($C_1$-$C_6$)alkyl group; or
(g3) a halo($C_1$-$C_6$)alkyl group,

provided that $R^{1a}$, $R^{1b}$, $R^{2a}$, $R^{2b}$, $R^{3a}$, and $R^{3b}$ are not hydrogen atoms at the same time,
$R^{4a}$ and $R^5$ are each

(h1) a hydrogen atom;
(h2) a hydroxyl group;

(h3) a cyano group;
(h4) a $(C_1-C_6)$alkyl group;
(h5) a $(C_2-C_6)$alkenyl group;
(h6) a $(C_2-C_6)$alkynyl group;
(h7) a $(C_3-C_6)$cycloalkyl group;
(h8) a $(C_1-C_6)$alkoxy group;
(h9) a halo$(C_1-C_6)$alkyl group;
(h12) a halo$(C_3-C_6)$cycloalkyl group;
(h13) a halo$(C_1-C_6)$alkoxy group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C;
(h29) a phenyl$(C_1-C_6)$alkyl group;
(h30) a phenyl$(C_1-C_6)$alkyl group having 1 to 5 substituents each independently selected from substituent group C;
(h31) a pyridyl$(C_1-C_6)$alkyl group;
(h32) a pyridyl $(C_1-C_6)$alkyl group having 1 to 4 substituents each independently selected from substituent group C;
(h37) a pyrazinyl$(C_1-C_6)$alkyl group;
(h38) a pyrazinyl$(C_1-C_6)$alkyl group having 1 to 3 substituents each independently selected from substituent group C;
(h39) a thienyl $(C_1-C_6)$alkyl group;
(h40) a thienyl$(C_1-C_6)$alkyl group having 1 to 3 substituents each independently selected from substituent group C;
(h41) a furyl$(C_1-C_6)$alkyl group;
(h42) a furyl$(C_1-C_6)$alkyl group having 1 to 3 substituents each independently selected from substituent group C;
(h43) an oxazolyl$(C_1-C_6)$alkyl group;
(h44) an oxazolyl$(C_1-C_6)$alkyl group having 1 to 2 substituents each independently selected from substituent group C;
(h45) a thiazolyl $(C_1-C_6)$alkyl group;
(h46) a thiazolyl$(C_1-C_6)$alkyl group having 1 to 2 substituents each independently selected from substituent group C;
(h47) a benzofuranyl$(C_1-C_6)$alkyl group;
(h48) a benzofuranyl$(C_1-C_6)$alkyl group having 1 to 5 substituents each independently selected from substituent group C;
(h49) a tetrahydropyranyl$(C_1-C_6)$alkyl group;
(h50) a tetrahydrofuryl$(C_1-C_6)$alkyl group; or
(h51) a dihydrobenzopyranyl group,

$R^{4a}$ and $R^5$ may be the same or different,
$R^{4b}$ is

(i1) a hydrogen atom;
(i2) a $(C_1-C_6)$alkyl group; or
(i3) a halo$(C_1-C_6)$alkyl group,

when $R^{4a}$ is (h4) a $(C_1\text{-}C_6)$alkyl group, or (h14) a $(C_1\text{-}C_6)$alkyl group having 1 to 3 substituents each independently selected from substituent group A, and $R^{4b}$ is (i2) a $(C_1\text{-}C_6)$alkyl group, then $R^{4a}$ and $R^{4b}$ may form a 3- to 6-membered ring together with the adjacent nitrogen atom, and
Z is an oxygen atom, a sulfur atom, or $NR^7$ wherein $R^7$ is a hydroxyl group or a $(C_1\text{-}C_6)$alkoxy group, or a salt thereof.

**3.** The compound according to claim 1, wherein

X is $CR^{1a}R^{1b}$, $NR^{1c}$, or an oxygen atom,
$R^{1a}$ and $R^{1b}$ are each

(a1) a hydrogen atom;
(a3) a $(C_1\text{-}C_6)$alkyl group;
(a4) a halo $(C_1\text{-}C_6)$alkyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a $(C_1\text{-}C_6)$alkyl group;
(b10) a $(C_1\text{-}C_6)$alkyl group having 1 to 3 substituents each independently selected from substituent group A;
(b12) a $(C_1\text{-}C_6)$alkoxycarbonyl group;
(b13) a $(C_1\text{-}C_6)$alkoxy$(C_1\text{-}C_6)$alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b24) a phenyl$(C_1\text{-}C_6)$alkyl group;
(b25) a phenyl$(C_1\text{-}C_6)$alkyl group having 1 to 5 substituents each independently selected from substituent group B;
(b28) a thiazolyl$(C_1\text{-}C_6)$alkyl group; or
(b29) a thiazolyl$(C_1\text{-}C_6)$alkyl group having 1 to 2 substituents each independently selected from substituent group B,

Y is $CR^{2a}R^{2b}$, $NR^{2c}$, or $CH_2CH_2$,
$R^{2a}$ is

(c1) a hydrogen atom;
(c2) a phenyl group;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;

(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c44) a carboxyl group; or
(c45) a $(C_1-C_6)$alkoxycarbonyl group,

$R^{2b}$ is

(d1) a hydrogen atom; or
(d2) a $(C_1-C_6)$alkyl group,

$R^{2c}$ is

(e1) a $(C_1-C_6)$alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e44) a pyridylcarbonyl group;
(e45) a pyridylcarbonyl group having 1 to 4 substituents each independently selected from substituent group B; or
(e46) a hydrogen atom,

$R^{3a}$ is

(f1) a hydrogen atom;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom; or
(g2) a $(C_1-C_6)$alkyl group,

provided that $R^{1a}$, $R^{1b}$, $R^{2a}$, $R^{2b}$, $R^{3a}$, and $R^{3b}$ are not hydrogen atoms at the same time,
$R^{4a}$ is

(h1) a hydrogen atom;
(h2) a hydroxyl group;
(h3) a cyano group;
(h4) a $(C_1-C_6)$alkyl group;
(h7) a $(C_3-C_6)$ cycloalkyl group;
(h8) a $(C_1-C_6)$alkoxy group;

(h9) a halo $(C_1-C_6)$alkyl group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C;
(h29) a phenyl $(C_1-C_6)$alkyl group;
(h30) a phenyl $(C_1-C_6)$alkyl group having 1 to 5 substituents each independently selected from substituent group C;
(h31) a pyridyl $(C_1-C_6)$alkyl group;
(h32) a pyridyl$(C_1-C_6)$alkyl group having 1 to 4 substituents each independently selected from substituent group C;
(h37) a pyrazinyl$(C_1-C_6)$alkyl group;
(h38) a pyrazinyl$(C_1-C_6)$alkyl group having 1 to 3 substituents each independently selected from substituent group C;
(h39) a thienyl$(C_1-C_6)$alkyl group;
(h40) a thienyl$(C_1-C_6)$alkyl group having 1 to 3 substituents each independently selected from substituent group C;
(h41) a furyl$(C_1-C_6)$alkyl group;
(h42) a furyl$(C_1-C_6)$alkyl group having 1 to 3 substituents each independently selected from substituent group C;
(h45) a thiazolyl$(C_1-C_6)$alkyl group;
(h46) a thiazolyl$(C_1-C_6)$alkyl group having 1 to 2 substituents each independently selected from substituent group C;
(h47) a benzofuranyl$(C_1-C_6)$alkyl group;
(h48) a benzofuranyl$(C_1-C_6)$alkyl group having 1 to 5 substituents each independently selected from substituent group C;
(h49) a tetrahydropyranyl$(C_1-C_6)$alkyl group; or
(h50) a tetrahydrofuryl$(C_1-C_6)$alkyl group,

$R^{4b}$ is

(i1) a hydrogen atom; or
(i2) a $(C_1-C_6)$alkyl group,

when $R^{4a}$ is (h4) a $(C_1-C_6)$alkyl group and $R^{4b}$ is (i2) a $(C_1-C_6)$ alkyl group, then $R^{4a}$ and $R^{4b}$ may form a 3- to 6-membered ring together with the adjacent nitrogen atom,
$R^5$ is

(h1) a hydrogen atom;
(h4) a $(C_1-C_6)$alkyl group;
(h5) a $(C_2-C_6)$alkenyl group;
(h6) a $(C_2-C_6)$alkynyl group;
(h7) a $(C_3-C_6)$cycloalkyl group;
(h9) a halo$(C_1-C_6)$alkyl group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;

(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h27) a pyrazolyl group;
(h28) a pyrazolyl group having 1 to 3 substituents each independently selected from substituent group C;
(h29) a phenyl($C_1$-$C_6$)alkyl group;
(h30) a phenyl($C_1$-$C_6$)alkyl group having 1 to 5 substituents each independently selected from substituent group C;
(h37) a pyrazinyl($C_1$-$C_6$)alkyl group;
(h38) a pyrazinyl($C_1$-$C_6$)alkyl group having 1 to 3 substituents each independently selected from substituent group C; or
(h51) a dihydrobenzopyranyl group, and

Z is an oxygen atom,
or a salt thereof.

**4.** The compound according to claim 1, wherein Y is $CR^{2a}R^{2b}$ or $NR^{2c}$, or a salt thereof.

**5.** The compound according to claim 2, wherein Y is $CR^{2a}R^{2b}$ or $NR^{2c}$, or a salt thereof.

**6.** The compound according to claim 3, wherein Y is $CR^{2a}R^{2b}$ or $NR^{2c}$, or a salt thereof.

**7.** A pest control agent comprising the compound according to any one of claims 1 to 6, or a salt thereof as an active ingredient.

**8.** An agrohorticultural insect pest control agent comprising the compound according to any one of claims 1 to 6, or a salt thereof as an active ingredient.

**9.** An ectoparasite control agent for animals, comprising the compound according to any one of claims 1 to 6, or a salt thereof as an active ingredient.

**10.** An endoparasite control agent for animals, comprising the compound according to any one of claims 1 to 6, or a salt thereof as an active ingredient.

**11.** A method for controlling an agrohorticultural insect pest, comprising treating a plant or soil with an effective amount of the agrohorticultural insect pest control agent according to claim 8.

**12.** A method for controlling an ectoparasite in an animal, comprising orally or parenterally administering an effective amount of the ectoparasite control agent for animals according to claim 9 to a target animal.

**13.** A method for controlling an endoparasite in an animal, comprising orally or parenterally administering an effective amount of the endoparasite control agent for animals according to claim 10 to a target animal.

**14.** Use of the compound according to any one of claims 1 to 6, or a salt thereof as a pest control agent.

**15.** A compound represented by the formula (3)

[Chem. 2]

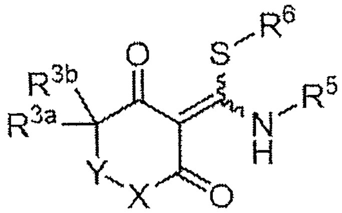

(3)

wherein

X is $CR^{1a}R^{1b}$, $NR^{1c}$, or an oxygen atom,
$R^{1a}$ and $R^{1b}$ are each

(a1) a hydrogen atom;
(a2) a halogen atom;
(a3) a $(C_1-C_6)$alkyl group;
(a4) a halo$(C_1-C_6)$alkyl group;
(a5) a phenyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a $(C_1-C_6)$alkyl group;
(b5) a $(C_3-C_6)$ cycloalkyl group;
(b6) a halo$(C_1-C_6)$alkyl group;
(b9) a halo$(C_3-C_6)$cycloalkyl group;
(b10) a $(C_1-C_6)$alkyl group having 1 to 3 substituents each independently selected from substituent group A;
(b12) a $(C_1-C_6)$alkoxycarbonyl group;
(b13) a $(C_1-C_6)$alkoxy$(C_1-C_6)$alkoxycarbonyl group;
(b14) a phenyl group;
(b15) a phenyl group having 1 to 5 substituents each independently selected from substituent group B;
(b16) a pyridyl group;
(b17) a pyridyl group having 1 to 4 substituents each independently selected from substituent group B;
(b18) a pyridazinyl group;
(b19) a pyridazinyl group having 1 to 3 substituents each independently selected from substituent group B;
(b20) a pyrimidinyl group;
(b21) a pyrimidinyl group having 1 to 3 substituents each independently selected from substituent group B;
(b22) a pyrazinyl group;
(b23) a pyrazinyl group having 1 to 3 substituents each independently selected from substituent group B;
(b24) a phenyl$(C_1-C_6)$alkyl group;
(b25) a phenyl $(C_1-C_6)$alkyl group having 1 to 5 substituents each independently selected from substituent group B;
(b26) a pyridyl$(C_1-C_6)$alkyl group;
(b27) a pyridyl$(C_1-C_6)$alkyl group having 1 to 4 substituents each independently selected from substituent group B;
(b28) a thiazolyl$(C_1-C_6)$alkyl group; or
(b29) a thiazolyl$(C_1-C_6)$alkyl group having 1 to 2 substituents each independently selected from substituent group B,

Y is $CR^{2a}R^{2b}$, $NR^{2c}$, or $CH_2CH_2$,
$R^{2a}$ is

(c1) a hydrogen atom;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c28) a carboxyl group;
(c29) a $(C_1-C_6)$alkoxycarbonyl group
(c44) a carboxyl group; or
(c45) a $(C_1-C_6)$alkoxycarbonyl group,

$R^{2b}$ is

(d1) a hydrogen atom; or
(d2) a $(C_1-C_6)$alkyl group,

$R^{2c}$ is

(e1) a $(C_1-C_6)$alkoxycarbonyl group;
(e2) a phenyl group;
(e3) a phenyl group having 1 to 5 substituents each independently selected from substituent group B;
(e4) a pyridyl group;
(e5) a pyridyl group having 1 to 4 substituents each independently selected from substituent group B;
(e6) a pyridazinyl group;
(e7) a pyridazinyl group having 1 to 3 substituents each independently selected from substituent group B;
(e8) a pyrimidinyl group;
(e9) a pyrimidinyl group having 1 to 3 substituents each independently selected from substituent group B;
(e10) a pyrazinyl group;
(e11) a pyrazinyl group having 1 to 3 substituents each independently selected from substituent group B;
(e12) a furyl group;
(e13) a furyl group having 1 to 3 substituents each independently selected from substituent group B;
(e14) a thienyl group;
(e15) a thienyl group having 1 to 3 substituents each independently selected from substituent group B;
(e16) an oxazolyl group;
(e17) an oxazolyl group having 1 to 2 substituents each independently selected from substituent group B;
(e20) a thiazolyl group;
(e21) a thiazolyl group having 1 to 2 substituents each independently selected from substituent group B;
(e44) a pyridylcarbonyl group; or

(e45) a pyridylcarbonyl group having 1 to 4 substituents each independently selected from substituent group B,

$R^{3a}$ is

(f1) a hydrogen atom;
(f2) a $(C_1-C_6)$alkyl group;
(f4) a phenyl group;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom;
(g2) a $(C_1-C_6)$alkyl group; or
(g3) a halo$(C_1-C_6)$alkyl group,

provided that $R^{1a}$, $R^{1b}$, $R^{2a}$, $R^{2b}$, $R^{3a}$, and $R^{3b}$ are not hydrogen atoms at the same time, and X is not $NR^{1c}$ when Y is$NR^{2c}$,
$R^5$ is

(h1) a hydrogen atom;
(h2) a hydroxyl group;
(h3) a cyano group;
(h4) a $(C_1-C_6)$alkyl group;
(h5) a $(C_2-C_6)$alkenyl group;
(h6) a $(C_2-C_6)$alkynyl group;
(h7) a $(C_3-C_6)$cycloalkyl group;
(h8) a $(C_1-C_6)$alkoxy group;
(h9) a halo$(C_1-C_6)$alkyl group;
(h12) a halo$(C_3-C_6)$cycloalkyl group;
(h13) a halo$(C_1-C_6)$alkoxy group;
(h14) a $(C_1-C_6)$alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h23) a pyrazinyl group;
(h24) a pyrazinyl group having 1 to 3 substituents each independently selected from substituent group C;
(h25) an isoxazolyl group;
(h26) an isoxazolyl group having 1 to 2 substituents each independently selected from substituent group C;
(h27) a pyrazolyl group;
(h28) a pyrazolyl group having 1 to 3 substituents each independently selected from substituent group C;
(h29) a phenyl $(C_1-C_6)$alkyl group;
(h30) a phenyl $(C_1-C_6)$alkyl group having 1 to 5 substituents each independently selected from substituent group C;
(h31) a pyridyl $(C_1-C_6)$alkyl group;
(h32) a pyridyl$(C_1-C_6)$alkyl group having 1 to 4 substituents each independently selected from substituent group C;

(h37) a pyrazinyl ($C_1$-$C_6$)alkyl group;
(h38) a pyrazinyl($C_1$-$C_6$)alkyl group having 1 to 3 substituents each independently selected from substituent group C;
(h39) a thienyl($C_1$-$C_6$)alkyl group;
(h40) a thienyl($C_1$-$C_6$)alkyl group having 1 to 3 substituents each independently selected from substituent group C;
(h41) a furyl ($C_1$-$C_6$)alkyl group;
(h42) a furyl($C_1$-$C_6$)alkyl group having 1 to 3 substituents each independently selected from substituent group C;
(h43) an oxazolyl($C_1$-$C_6$)alkyl group;
(h44) an oxazolyl($C_1$-$C_6$)alkyl group having 1 to 2 substituents each independently selected from substituent group C;
(h45) a thiazolyl($C_1$-$C_6$)alkyl group;
(h46) a thiazolyl($C_1$-$C_6$)alkyl group having 1 to 2 substituents each independently selected from substituent group C;
(h47) a benzofuranyl($C_1$-$C_6$)alkyl group;
(h48) a benzofuranyl($C_1$-$C_6$)alkyl group having 1 to 5 substituents each independently selected from substituent group C;
(h49) a tetrahydropyranyl($C_1$-$C_6$)alkyl group;
(h50) a tetrahydrofuryl ($C_1$-$C_6$)alkyl group; or
(h51) a dihydrobenzopyranyl group, and

$R^6$ is

(m1) a ($C_1$-$C_6$)alkyl group;
(m2) a ($C_2$-$C_6$)alkenyl group;
(m3) a ($C_2$-$C_6$)alkynyl group;
(m4) a halo ($C_1$-$C_6$)alkyl group;
(m5) a phenyl ($C_1$-$C_6$)alkyl group; or
(m6) a phenyl ($C_1$-$C_6$)alkyl group having 1 to 5 substituents each independently selected from substituent group C,

substituent group A consists of

(j1) a cyano group;
(j2) a ($C_3$-$C_6$) cycloalkyl group;
(j3) a ($C_1$-$C_6$)alkoxy group;
(j4) a ($C_1$-$C_6$)alkylsulfanyl group;
(j5) a ($C_1$-$C_6$)alkylsulfinyl group;
(j6) a ($C_1$-$C_6$)alkylsulfonyl group;
(j7) a ($C_1$-$C_6$)alkylamino group;
(j8) a di($C_1$-$C_6$)alkylamino group (the ($C_1$-$C_6$)alkyl may be the same or different);
(j9) a halo ($C_3$-$C_6$) cycloalkyl group;
(j10) a halo ($C_1$-$C_6$)alkoxy group;
(j11) a halo ($C_1$-$C_6$)alkylsulfanyl group;
(j12) a halo($C_1$-$C_6$)alkylsulfinyl group; and
(j13) a halo($C_1$-$C_6$)alkylsulfonyl group,

substituent group B consists of

(k1) a halogen atom;
(k2) a cyano group;
(k3) a nitro group;
(k4) an amino group;
(k5) a hydroxyl group;
(k6) a carboxyl group;
(k7) a ($C_1$-$C_6$)alkyl group;
(k8) a ($C_2$-$C_6$)alkenyl group;

(k9) a ($C_2$-$C_6$)alkynyl group;
(k10) a ($C_1$-$C_6$)alkoxy group;
(k11) a ($C_3$-$C_6$) cycloalkyl group;
(k12) a ($C_1$-$C_6$)alkylsulfanyl group;
(k13) a ($C_1$-$C_6$)alkylsulfinyl group;
(k14) a ($C_1$-$C_6$)alkylsulfonyl group;
(k15) a ($C_1$-$C_6$)alkylamino group;
(k16) a di($C_1$-$C_6$)alkylamino group (the ($C_1$-$C_6$)alkyl may be the same or different);
(k17) a halo($C_1$-$C_6$)alkyl group;
(k18) a halo($C_2$-$C_6$)alkenyl group;
(k19) a halo($C_2$-$C_6$)alkynyl group;
(k20) a halo($C_1$-$C_6$)alkoxy group;
(k21) a halo($C_3$-$C_6$)cycloalkyl group;
(k22) a halo($C_1$-$C_6$)alkylsulfanyl group;
(k23) a halo($C_1$-$C_6$)alkylsulfinyl group;
(k24) a halo($C_1$-$C_6$)alkylsulfonyl group;
(k25) a ($C_1$-$C_6$)alkoxy($C_1$-$C_6$)alkyl group;
(k26) a phenyl group;
(k27) a phenyl group having 1 to 5 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a halo($C_1$-$C_6$)alkyl group, and a halo ($C_1$-$C_6$)alkoxy group;
(k28) a pyridyl group; and
(k29) a pyridyl group having 1 to 4 substituents each independently selected from the group consisting of a halogen atom, a cyano group, a ($C_1$-$C_6$)alkyl group, a ($C_1$-$C_6$)alkoxy group, a ($C_3$-$C_6$)cycloalkyl group, a halo($C_1$-$C_6$)alkyl group, and a halo ($C_1$-$C_6$)alkoxy group,

substituent group C consists of

(11) a halogen atom;
(12) a cyano group;
(13) a nitro group;
(14) an amino group;
(15) a hydroxyl group;
(16) a carboxyl group;
(17) a ($C_1$-$C_6$)alkyl group;
(18) a ($C_2$-$C_6$)alkenyl group;
(19) a ($C_2$-$C_6$)alkynyl group;
(110) a ($C_1$-$C_6$)alkoxy group;
(111) a ($C_3$-$C_6$)cycloalkyl group;
(112) a ($C_1$-$C_6$)alkylsulfanyl group;
(113) a ($C_1$-$C_6$)alkylsulfinyl group;
(114) a ($C_1$-$C_6$)alkylsulfonyl group;
(115) a ($C_1$-$C_6$)alkylamino group;
(116) a di($C_1$-$C_6$)alkylamino group (the ($C_1$-$C_6$)alkyl may be the same or different);
(117) a halo($C_1$-$C_6$)alkyl group;
(118) a halo($C_2$-$C_6$)alkenyl group;
(119) a halo($C_2$-$C_6$)alkynyl group;
(120) a halo($C_1$-$C_6$)alkoxy group;
(121) a halo($C_3$-$C_6$) cycloalkyl group;
(122) a halo($C_1$-$C_6$)alkylsulfanyl group;
(123) a halo($C_1$-$C_6$)alkylsulfinyl group;
(124) a halo($C_1$-$C_6$)alkylsulfonyl group; and
(125) a ($C_1$-$C_6$)alkoxy($C_1$-$C_6$)alkyl group,

or a salt thereof.

**16.** The compound according to claim 15, wherein

X is $CR^{1a}R^{1b}$, $NR^{1c}$, or an oxygen atom,
$R^{1a}$ and $R^{1b}$ are each

(a1) a hydrogen atom;
(a3) a ($C_1$-$C_6$)alkyl group;
(a4) a halo($C_1$-$C_6$)alkyl group;
(a6) a phenyl group having 1 to 5 substituents each independently selected from substituent group B;
(a7) a pyridyl group; or
(a8) a pyridyl group having 1 to 4 substituents each independently selected from substituent group B,

$R^{1a}$ and $R^{1b}$ may be the same or different,
$R^{1c}$ is

(b1) a hydrogen atom;
(b2) a ($C_1$-$C_6$)alkyl group;
(b12) a ($C_1$-$C_6$)alkoxycarbonyl group;
(b14) a phenyl group;
(b15) a phenyl group having 1 to 5 substituents each independently selected from substituent group B;
(b16) a pyridyl group; or
(b17) a pyridyl group having 1 to 4 substituents each independently selected from substituent group B,

Y is $CR^{2a}R^{2b}$, $NR^{2c}$, or $CH_2CH_2$,
$R^{2a}$ is

(c1) a hydrogen atom;
(c3) a phenyl group having 1 to 5 substituents each independently selected from substituent group B;
(c4) a pyridyl group;
(c5) a pyridyl group having 1 to 4 substituents each independently selected from substituent group B;
(c6) a pyridazinyl group;
(c7) a pyridazinyl group having 1 to 3 substituents each independently selected from substituent group B;
(c8) a pyrimidinyl group;
(c9) a pyrimidinyl group having 1 to 3 substituents each independently selected from substituent group B;
(c10) a pyrazinyl group;
(c11) a pyrazinyl group having 1 to 3 substituents each independently selected from substituent group B;
(c12) a furyl group;
(c13) a furyl group having 1 to 3 substituents each independently selected from substituent group B;
(c14) a thienyl group;
(c15) a thienyl group having 1 to 3 substituents each independently selected from substituent group B;
(c16) an oxazolyl group;
(c17) an oxazolyl group having 1 to 2 substituents each independently selected from substituent group B;
(c18) an isoxazolyl group;
(c19) an isoxazolyl group having 1 to 2 substituents each independently selected from substituent group B;
(c20) a thiazolyl group;
(c21) a thiazolyl group having 1 to 2 substituents each independently selected from substituent group B;
(c22) an isothiazolyl group;
(c23) an isothiazolyl group having 1 to 2 substituents each independently selected from substituent group B;
(c24) a pyrazolyl group;
(c25) a pyrazolyl group having 1 to 3 substituents each independently selected from substituent group B;
(c26) an oxadiazolyl group;
(c27) an oxadiazolyl group having one substituent selected from substituent group B;
(c44) a carboxyl group; or
(c45) a ($C_1$-$C_6$)alkoxycarbonyl group,

$R^{2b}$ is

(d1) a hydrogen atom; or
(d2) a ($C_1$-$C_6$)alkyl group,

$R^{2c}$ is

(e1) a ($C_1$-$C_6$)alkoxycarbonyl group;
(e4) a pyridyl group;
(e5) a pyridyl group having 1 to 4 substituents each independently selected from substituent group B;
(e44) a pyridylcarbonyl group; or
(e45) a pyridylcarbonyl group having 1 to 4 substituents each independently selected from substituent group B,

$R^{3a}$ is

(f1) a hydrogen atom;
(f5) a phenyl group having 1 to 5 substituents each independently selected from substituent group B;
(f6) a pyridyl group;
(f7) a pyridyl group having 1 to 4 substituents each independently selected from substituent group B;
(f8) a thiazolyl group;
(f9) a thiazolyl group having 1 to 2 substituents each independently selected from substituent group B;
(f10) a quinolyl group; or
(f11) a quinolyl group having 1 to 6 substituents each independently selected from substituent group B,

$R^{3b}$ is

(g1) a hydrogen atom; or
(g2) a ($C_1$-$C_6$)alkyl group,

provided that $R^{1a}$, $R^{1b}$, $R^{2a}$, $R^{2b}$, $R^{3a}$, and $R^{3b}$ are not hydrogen atoms at the same time, and X is not $NR^{1c}$ when Y is $NR^{2c}$,
$R^5$ is

(h1) a hydrogen atom;
(h4) a ($C_1$-$C_6$)alkyl group;
(h5) a ($C_2$-$C_6$)alkenyl group;
(h6) a ($C_2$-$C_6$)alkynyl group;
(h7) a ($C_3$-$C_6$)cycloalkyl group;
(h9) a halo($C_1$-$C_6$)alkyl group;
(h14) a ($C_1$-$C_6$)alkyl group having 1 to 3 substituents each independently selected from substituent group A;
(h15) a phenyl group;
(h16) a phenyl group having 1 to 5 substituents each independently selected from substituent group C;
(h17) a pyridyl group;
(h18) a pyridyl group having 1 to 4 substituents each independently selected from substituent group C;
(h19) a pyridazinyl group;
(h20) a pyridazinyl group having 1 to 3 substituents each independently selected from substituent group C;
(h21) a pyrimidinyl group;
(h22) a pyrimidinyl group having 1 to 3 substituents each independently selected from substituent group C;
(h27) a pyrazolyl group;
(h28) a pyrazolyl group having 1 to 3 substituents each independently selected from substituent group C;
(h29) a phenyl($C_1$-$C_6$)alkyl group;
(h30) a phenyl($C_1$-$C_6$)alkyl group having 1 to 5 substituents each independently selected from substituent group C;
(h37) a pyrazinyl($C_1$-$C_6$)alkyl group;
(h38) a pyrazinyl($C_1$-$C_6$)alkyl group having 1 to 3 substituents each independently selected from substituent group C; or
(h51) a dihydrobenzopyranyl group, and

$R^6$ is

(m1) a ($C_1$-$C_6$)alkyl group;
(m5) a phenyl($C_1$-$C_6$)alkyl group; or

(m6) a phenyl($C_1$-$C_6$)alkyl group having 1 to 5 substituents each independently selected from substituent group C,

or a salt thereof.

## INTERNATIONAL SEARCH REPORT

International application No.
**PCT/JP2024/022931**

**A. CLASSIFICATION OF SUBJECT MATTER**

***C07D 213/38***(2006.01)i; ***A01N 35/06***(2006.01)i; ***A01N 43/10***(2006.01)i; ***A01N 43/16***(2006.01)i; ***A01N 43/40***(2006.01)i; ***A01N 43/46***(2006.01)i; ***A01N 43/54***(2006.01)i; ***A01N 43/56***(2006.01)i; ***A01N 43/58***(2006.01)i; ***A01N 43/78***(2006.01)i; ***A01N 43/80***(2006.01)i; ***A01N 47/02***(2006.01)i; ***A01N 47/06***(2006.01)i; ***A01N 47/16***(2006.01)i; ***A01P 5/00***(2006.01)i; ***A01P 7/04***(2006.01)i; ***A61K 31/136***(2006.01)i; ***A61K 31/341***(2006.01)i; ***A61K 31/343***(2006.01)i; ***A61K 31/351***(2006.01)i; ***A61K 31/381***(2006.01)i; ***A61K 31/42***(2006.01)i; ***A61K 31/426***(2006.01)i; ***A61K 31/4402***(2006.01)i; ***A61K 31/4439***(2006.01)i; ***A61K 31/444***(2006.01)i; ***A61K 31/4965***(2006.01)i; ***A61K 31/497***(2006.01)i; ***A61K 31/505***(2006.01)i; ***A61K 31/506***(2006.01)i; ***A61P 33/00***(2006.01)i; ***C07C 225/18***(2006.01)i; ***C07D 213/50***(2006.01)i; ***C07D 213/69***(2006.01)i; ***C07D 213/74***(2006.01)i; ***C07D 223/10***(2006.01)i; ***C07D 231/12***(2006.01)i; ***C07D 231/38***(2006.01)i; ***C07D 237/20***(2006.01)i; ***C07D 239/26***(2006.01)i; ***C07D 239/42***(2006.01)i; ***C07D 239/47***(2006.01)i; ***C07D 241/12***(2006.01)i; ***C07D 241/20***(2006.01)i; ***C07D 261/08***(2006.01)i; ***C07D 261/14***(2006.01)i; ***C07D 263/32***(2006.01)i; ***C07D 277/28***(2006.01)i; ***C07D 307/14***(2006.01)i; ***C07D 307/52***(2006.01)i; ***C07D 307/81***(2006.01)i; ***C07D 309/04***(2006.01)i; ***C07D 311/74***(2006.01)i; ***C07D 333/20***(2006.01)i; ***C07D 333/22***(2006.01)i; ***C07D 401/04***(2006.01)i; ***C07D 401/12***(2006.01)i; ***C07D 401/14***(2006.01)i; ***C07D 403/04***(2006.01)i; ***C07D 405/14***(2006.01)i; ***C07D 409/12***(2006.01)i; ***C07D 409/14***(2006.01)i; ***C07D 413/12***(2006.01)i; ***C07D 413/14***(2006.01)i; ***C07D 417/12***(2006.01)i; ***C07D 417/14***(2006.01)i

FI: C07D213/38; A01N35/06; A01N43/10 B; A01N43/16 A; A01N43/40 101A; A01N43/40 101J; A01N43/40 101Q; A01N43/46; A01N43/54 A; A01N43/56 C; A01N43/58 D; A01N43/78 B; A01N43/80 101; A01N47/02; A01N47/06 E; A01N47/16 A; A01P5/00; A01P7/04; A61K31/136; A61K31/341; A61K31/343; A61K31/351; A61K31/381; A61K31/42; A61K31/426; A61K31/4402; A61K31/4439; A61K31/444; A61K31/4965; A61K31/497; A61K31/505; A61K31/506; A61P33/00 171; C07C225/18; C07D213/50; C07D213/69; C07D213/74; C07D223/10; C07D231/12 E; C07D231/38 Z; C07D237/20; C07D239/26; C07D239/42 Z; C07D239/47 Z; C07D241/12; C07D241/20; C07D261/08; C07D261/14; C07D263/32; C07D277/28; C07D307/14; C07D307/52; C07D307/81; C07D309/04; C07D311/74; C07D333/20; C07D333/22; C07D401/04; C07D401/12; C07D401/14; C07D403/04; C07D405/14; C07D409/12; C07D409/14 CSP; C07D413/12; C07D413/14; C07D417/12; C07D417/14

According to International Patent Classification (IPC) or to both national classification and IPC

**B. FIELDS SEARCHED**

Minimum documentation searched (classification system followed by classification symbols)

C07D213/38; A01N35/06; A01N43/10; A01N43/16; A01N43/40; A01N43/46; A01N43/54; A01N43/56; A01N43/58; A01N43/78; A01N43/80; A01N47/02; A01N47/06; A01N47/16; A01P5/00; A01P7/04; A61K31/136; A61K31/341; A61K31/343; A61K31/351; A61K31/381; A61K31/42; A61K31/426; A61K31/4402; A61K31/4439; A61K31/444; A61K31/4965; A61K31/497; A61K31/505; A61K31/506; A61P33/00; C07C225/18; C07D213/50; C07D213/69; C07D213/74; C07D223/10; C07D231/12; C07D231/38; C07D237/20; C07D239/26; C07D239/42; C07D239/47; C07D241/12; C07D241/20; C07D261/08; C07D261/14; C07D263/32; C07D277/28; C07D307/14; C07D307/52; C07D307/81; C07D309/04; C07D311/74; C07D333/20; C07D333/22; C07D401/04; C07D401/12; C07D401/14; C07D403/04; C07D405/14; C07D409/12; C07D409/14; C07D413/12; C07D413/14; C07D417/12; C07D417/14

Documentation searched other than minimum documentation to the extent that such documents are included in the fields searched

Published examined utility model applications of Japan 1922-1996
Published unexamined utility model applications of Japan 1971-2024

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2024** | **10 September 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/022931**

**B. FIELDS SEARCHED**

Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2021/261562 A1 (NIHON NOHYAKU CO., LTD.) 30 December 2021 (2021-12-30) claims, examples | 1-16 |
| A | WO 2021/261563 A1 (NIHON NOHYAKU CO., LTD.) 30 December 2021 (2021-12-30) claims, examples | 1-16 |
| A | US 3976785 A (AMERICAN CYANAMID CO.) 24 August 1976 (1976-08-24) claims, examples | 1-16 |
| A | FADDA, Ahmed A. et al., Synthesis and insecticidal assessment of some innovative heterocycles incorporating a thiadiazole moiety against the cotton leafworm, Spodoptera littoralis, RSC Advances, 2017, 7(63), pp. 39773-39785, DOI: 10.1039/c7ra06087d scheme 2, table 1, compound 4 | 1-16 |
| P, A | WO 2023/190015 A1 (NIHON NOHYAKU CO., LTD.) 05 October 2023 (2023-10-05) entire text | 1-16 |

Form PCT/ISA/210 (second sheet) (July 2022)

**INTERNATIONAL SEARCH REPORT**
**Information on patent family members**

International application No.
**PCT/JP2024/022931**

| Patent document cited in search report | Publication date (day/month/year) | Patent family member(s) | Publication date (day/month/year) |
|---|---|---|---|
| WO 2021/261562 A1 | 30 December 2021 | US 2023/0295108 A1<br>claims, examples<br>EP 4174056 A1<br>BR 112022025816 A<br>CN 115916752 A<br>TW 202216666 A | |
| WO 2021/261563 A1 | 30 December 2021 | US 2023/0303522 A1<br>claims, examples<br>EP 4174057 A1<br>BR 112022026083 A<br>CN 115734965 A<br>CA 3186902 A<br>KR 10-2023-0033708 A<br>AU 2021296100 A<br>MX 2022016133 A<br>TW 202216670 A<br>CL 2022003619 A<br>ZA 202212292 B | |
| US 3976785 A | 24 August 1976 | (Family: none) | |
| WO 2023/190015 A1 | 05 October 2023 | TW 202402740 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021261562 A
- WO 2021261563 A
- US 3976785 A
- WO 2008014311 A
- WO 2017102649 A
- WO 2014021281 A
- JP 61057565 A
- WO 2006003096 A
- WO 2007051062 A
- EP 0374753 A
- WO 9307278 A
- WO 9534656 A
- EP 0427529 A
- EP 451878 A
- WO 03052073 A
- JP 2023104156 A


### Non-patent literature cited in the description


- ORGANIC FUNCTIONAL GROUP PREPARATIONS III. ACADEMIC PRESS, INC.
- Greene's PROTECTIVE GROUPS in ORGANIC SYNTHESIS. WILEY
- *Journal of the Chemical Society*, 1935, 539-540
- *Bioorganic & Medicinal Chemistry*, 2012, vol. 20, 1029-1045
- *Tetrahedron Letters*, 1995, vol. 36, 3659
- *Journal of Heterocyclic Chemistry*, 2017, vol. 54, 1318
- *Proc. Natl. Acad. Sci. USA*, 1990, vol. 87, 7175-7179
- *Weed Science*, 2005, vol. 53, 728-746
- **GURA T.** Repairing the Genome's Spelling Mistakes. *Science*, 1999, vol. 285, 316-318